# FASCICULE DE BREVET EUROPEEN

(11) **EP 2 448 927 B1**
(45) Date de publication et mention de la délivrance du brevet: **12.03.2014**
(21) Numéro de dépôt: 10742014.3
(22) Date de dépôt: 01.07.2010
(51) Int. Cl.: C07D 239/36, C07D 403/12, C07D 409/12, C07D 413/12, A61K 31/513, A61P 35/00, A61K 31/5377, C07D 239/47, C07D 403/06, C07D 403/10, C07D 413/06

(54) **NOUVEAUX DERIVES DE (6-OXO-1,6-DIHYDRO-PYRIMIDIN-2-YL)-AMIDE, LEUR PREPARATION ET LEUR UTILISATION PHARMACEUTIQUE COMME INHIBITEURS DE PHOSPHORYLATION D'AKT**
NEUE (6-OXO-1, 6-DIHYDRO-PYRIMIDIN-2-YL)-AMIDDERIVATE, IHRE ZUBEREITUNG UND PHARMAZEUTISCHE VERWENDUNG ALS AKT-PHOSPHORYLIERUNGSHEMMER
NOVEL (6-OXO-1, 6-DIHYDRO-PYRIMIDIN-2-YL)-AMIDE DERIVATIVES, PREPARATION THEREOF, AND PHARMACEUTICAL USE THEREOF AS AKT PHOSPHORYLATION INHIBITORS

(30) Priorité: 02.07.2009 FR 0903239; 10.09.2009 US 241100 P; 09.10.2009 FR 0957070
(43) Date de publication de la demande: 09.05.2012
(73) Titulaire: SANOFI, 75008 Paris (FR)
(72) Inventeur: CARRY, Jean-Christophe, F-75013 Paris (FR); CERTAL, Victor, F-75013 Paris (FR); HALLEY, Frank, F-75013 Paris (FR); KARLSSON, Karl Andreas, F-75013 Paris (FR); SCHIO, Laurent, F-75013 Paris (FR); THOMPSON, Fabienne, F-75013 Paris (FR)
(74) Mandataire: Nony
(86) Numéro de dépôt international: PCT/FR2010/051375
(87) Numéro de publication internationale: WO 2011/001114

(56) Documents cités:
- WO-A-2009/007749

## Description

La présente invention concerne de nouveaux composés chimiques (6-oxo-1,6-dihydro-pyrimidin-2-yl)-amide), dérivés de pyrimidinones, leur procédé de préparation, les nouveaux intermédiaires obtenus, leur application à titre de médicaments, les compositions pharmaceutiques les renfermant et la nouvelle utilisation de tels dérivés.

La présente invention concerne ainsi également l'utilisation desdits dérivés pour la préparation d'un médicament destiné au traitement de l'homme.

Plus particulièrement, l'invention concerne, de nouveaux dérivés de pyrimidinones et leur utilisation pharmaceutique pour la prévention et le traitement d'affections capables d'être modulées par l'inhibition de la voie PI3K/AKT/mTOR. AKT est un acteur clé dans cette voie de signalisation. Un niveau élevé de phosphorylation d'AKT est le marqueur de l'activation de la voie qui est retrouvée dans de nombreux cancers humains.
Les produits de la présente invention peuvent ainsi notamment être utilisés pour la prévention ou le traitement d'affections capables d'être modulées par l'inhibition de la phosphorylation d'AKT (P-AKT). L'inhibition de P-AKT peut être notamment obtenue par l'inhibition de la voie PI3K/AKT/mTOR, et en particulier par l'inhibition de kinases appartenant à cette voie comme les récepteurs à activité tyrosine kinase tels EGFR, IGFR, ErbB2, la 3'-phosphoinositide-dependent protein kinase-1 (PDK1), la phosphoinositide kinase PI3K, la serine-threonine kinase AKT, la kinase mTOR.
L'inhibition et la régulation de la voie PI3K/AKT/mTOR constitue notamment un nouveau et puissant mécanisme d'action pour le traitement d'un grand nombre de maladies cancéreuses incluant des tumeurs solides et liquides. De telles affections que peuvent traiter les produits de la présente demande sont les tumeurs humaines solides ou liquides.

### Rôle de la voie PI3K/AKT/mTOR

La voie de signalisation PI3K/AKT/mTOR est un réseau complexe qui régule de multiples fonctions cellulaires, comme la croissance, la survie, la prolifération et la motilité cellulaire, qui sont des processus clés de la tumorigénèse.

Cette voie de signalisation est une cible importante dans le traitement du cancer car la plupart de ses effecteurs sont altérés dans les tumeurs humaines. Les principaux effecteurs contribuant à l'activation de la voie sont i) les oncogènes tels que ErbB1 (EGFR), ErbB2 (HER2), PIK3CA et AKT activés par mutation, amplification ou surexpression ; ii) la déficience des gènes suppresseurs de tumeurs tels que PTEN, TSC1/2, LKB et PML qui sont inactivés suite à des mutations ou à des délétions (Jiang L-Z & Liu L-Z, Biochim Biophys Acta, 2008, 1784 :150 ; Vivanco I & Sawyers CL, 2002, Nat Rev Cancer, 2 :489 ; Cully M et al., Nature Rev. Cancer, 2006, 6 :184).

L'activation des oncogènes de cette voie de signalisation est retrouvée dans de nombreuses maladies cancéreuses humaines.
- les mutations activatrices de PIK3CA sont présentes dans 15-30% des cancers du colon, du sein, de l'endomètre, du foie, de l'ovaire et de la prostate (TL Yuan and LC Cantley, Oncogene, 2008, 27:5497; Y. Samuels et al. Science, 2004, 304:554; KE. Bachman et al. Cancer Biol Ther, 2004, 3:772; DA Levine et al. Clin Canc Res. 2005, 11:2875; C. Hartmann et al. Acta Neuropathol. 2005, 109:639).
- les amplifications, mutations activatrices et surexpressions des RTKs tels qu'EGFR et HER2 dans les cancers du cerveau, du sein et du poumon (NSCLC)
- l'amplification et la surexpression activatrice d'AKT dans les cancers du cerveau, du poumon (NSCLC), du sein, du rein, de l'ovaire et du pancréas (Testa JR. and Bellacosa A., Proct. Natl. Acad. Sci. USA 2001, 98:10983 ; Cheng et al., Proct. Natl. Acad. Sci. USA 1992, 89: 9267 ; Bellacosa et al., Int. J. Cancer, 1995, 64:280 ; Cheng et al., Proct. Natl. Acad. Sci. USA 1996, 93 :3636 ; Yuan et al., Oncogene, 2000, 19 :2324).
La déficience des gènes suppresseurs de tumeurs de cette voie de signalisation est également retouvée dans de nombreuses maladies cancéreuses humaines:
o la délétion de *PTEN* dans 50% des cancers du poumon (NSCLC), du foie, du rein, de la prostate, du sein, du cerveau, du pancréas, de l'endomètre et du colon (Maxwell GL et al. Canc. Res. 1998, 58 :2500 ; Zhou X-P et al. Amer. J. Pathol., 2002, 161 :439 ; Endersby R & Baker SJ, Oncogene, 2008, 27 :5416 ; Li et al. Science, 1997, 275:1943; Steack PA et al., Nat. Genet., 1997, 15 :356)
o les mutations de *TSC1*/*2* dans plus de 50% des scléroses tubéreuses
o les mutations ou délétions de *LKB1* (or *STK11*) qui prédisposent aux cancers du tractus gastro-intestinal et au cancer du pancreas et qui sont retouvées en particulier dans 10-38% des adenocarcinomes du poumon (Shah U. et al. Cancer Res. 2008, 68 :3562)
o les modification de *PML* notament par translocation dans les tumeurs humaines (Gurrieri C et al, J. NAtl Cancer Inst. 2004, 96 :269).
De plus cette voie de signalisation est un facteur majeur de résistance à la chimiothérapie, la radiothérapie et à des thérapies ciblées tels que les inhibiteurs d'EGFR et HER2 par exemple (C. Sawyers et al. Nat Rev 2002).

### Role d'AKT

AKT (protéine kinase B ; PKB) est une sérine-thréonine kinase qui occupe une place centrale dans une des voies majeures de signalisation cellulaire, la voie PI3K/AKT. AKT est notamment impliquée dans la croissance, la prolifération et la survie des cellules tumorales. L'activation d'AKT se fait en deux étapes (i) par phosphorylation de la thréonine 308 (P-T308) par PDK1 et (2) par phosphorylation de la sérine 473 (P-S473) par mTORC2 (ou complexe mTOR-Rictor), résultant en une activation totale. AKT à son tour régule un grand nombre de protéines dont mTOR (mammalian target of Rapamycin), BAD, GSK3, p21, p27, FOXO ou FKHRL1 (Manning BD & Cantley LC, Cell, 2007 129 :1261). L'activation d'AKT promeut l'internalisation des nutriments, ce qui déclenche un processus de métabolisation anabolisante soutenant la croissance et la prolifération cellulaire. En particulier, AKT contrôle l'initiation de la synthèse protéique à travers une cascade d'interactions qui procède par l'intermédiaire de TSC1/2 (complexe de sclérose tubéreuse), Rheb, et TOR pour aboutir à deux cibles critiques de la voie de signalisation, p70S6K et 4EBP. AKT induit également une phosphorylation inhibitrice du facteur de transcription Forkhead et l'inactivation de GSK3β qui conduisent à l'inhibition de l'apoptose et à la progression du cycle cellulaire (Franke TF, Oncogene, 2008, 27 :6473). AKT est donc une cible pour la thérapie anti-cancéreuse et l'inhibition de l'activation d'AKT par l'inhibition de sa phosphorylation peut induire l'apoptose des cellules malignes et par la même, présenter un traitement pour le cancer.

### Les récepteurs à activité tyrosyne kinase comme IGF1R

Des niveaux anormalement élevés d'activité protéine kinase ont été impliqués dans de nombreuses maladies résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inappropriée de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.
Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.
Plusieurs rapports cliniques soulignent le rôle important de la voie IGF-I dans le développement des cancers humains : IGF-I-R est souvent trouvé sur-exprimé dans de nombreux types tumoraux (sein, colon, poumon, sarcome, prostate, myelome multiple) et sa présence est souvent associée à un phénotype plus agressif.
De fortes concentrations d'IGF1 circulant sont fortement corrélées à un risque de cancer de la prostate, poumon et sein.
De plus, il a été largement documenté que IGF-I-R est nécessaire à l'établissement et au maintient du phénotype transformé in vitro comme in vivo [Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie, radiation, et l'apoptose induite par des cytokines. De plus, l'inhibition d'IGF-I-R endogène par un dominant négatif, la formation de triple hélice ou l'expression d'un antisens provoque une suppression de l'activité transformante in vitro et la diminution de la croissance de tumeurs dans les modèles animaux.

### PDK1

La 3'-phosphoinositide-dependent protein kinase-1 (PDK1) est une des composantes essentielles de la voie de signalisation PI3K-AKT. C'est une sérine-thréonine (Ser/Thr) kinase dont le rôle est de phosphoryler et d'activer d'autres Ser/Thr kinases de la famille des AGC impliquées dans le contrôle de la croissance, la prolifération, la survie cellulaire et dans la régulation du métabolisme. Ces kinases incluent la protéine kinase B (PKB ou AKT), SGK (ou sérum and glucocorticoïd regulated kinase), RSK (ou p90 ribosomal S6 kinase), p70S6K (ou p70 ribosomal S6 kinase) ainsi que diverses isoformes de la protéine kinase C (PKC) (Vanhaesebroeck B. & Alessi DR., Biochem J, 2000, 346:561). Un des rôles clés de PDK1 est donc l'activation d'AKT : en présence de PIP3, le second messager généré par PI3K, PDK-1 est recruté à la membrane plasmique via son domaine PH (plekstrin homology) et phosphoryle AKT sur la thréonine 308 situé dans la boucle d'activation, une modification essentielle de l'activation d'AKT. PDK1 est exprimée de façon ubiquitaire et est une kinase constitutivement active. PDK1 est un élément clé dans la voie de signalisation PI3K/AKT pour la régulation de processus clés dans la tumorigénèse comme la prolifération et la survie cellulaire. Cette voie étant activée dans plus de 50% des cancers humains, PDK1 représente une cible pour la thérapie anticancéreuse. L'inhibition de PDK1 devrait résulter en une inhibition effective de la proliferation et de la survie des cellules cancéreuses et donc apporter un bénéfice thérapeutique pour les cancers humains (Bayascas JR, Cell cycle, 2008, 7 :2978 ; Peifer C. & Alessi DR, ChemMedChem, 2008, 3 :1810).

### Les phosphoinositides-3 kinases (PI3Ks)

La lipide kinase PI3K est une cible importante dans cette voie de signalisation pour l'oncologie. Les PI3Ks de la classe I sont réparties en classe la (PI3Kα,β,δ) activée par les récepteurs à activité tyrosine kinase (RTKs), les récepteurs couplés aux protéines G (GPCRs), les GTPases de la famille Rho, p21-Ras et en classe Ib (PI3Kγ) activé par les GPCRs et par p21-Ras. Les PI3Ks de la classe Ia sont des hétérodimères qui consistent en une sous unité catalytique p110α, β ou δ et une sous unité régulatrice p85 ou p55. La classe Ib (p110γ) est monomérique. Les PI3Ks de la classe I sont des lipides/protéines kinases qui sont activées par les RTKs, les GPCRs ou Ras après recrutement à la membrane. Ces PI3Ks de la classe I phosphorylent le phosphatidylinositol 4,5 diphosphate (PIP2) sur la position 3 de l'inositol pour donner le phosphatidylinositol 3,4,5 triphosphate (PIP3), messager secondaire clé de cette voie de signalisation. A son tour, PIP3 recrute AKT et PDK1 à la membrane où ils se fixent par leur domaine homologue à la pleckstrine (domaine PH), conduisant à l'activation d'AKT par phosphorylation de PDK1 sur la thréonine 308. AKT phosphoryle de nombreux substrats, jouant ainsi un rôle clé dans de nombreux processus aboutissant à la transformation cellulaire comme la prolifération, la croissance et la survie cellulaire ainsi que l'angiogénèse.

Les PI3Ks de classe I sont impliquées dans les cancers humains : des mutations somatiques du gène PIK3CA qui code pour PI3Kα se retrouvent dans 15-35% des tumeurs humaines avec notamment deux mutations oncogéniques principales H1047R (dans le domaine kinase) et E545K/E542K (dans le domaine hélical) (Y. Samuels et al. Science, 2004, 304:554; TL Yuan and LC Cantley, Oncogene, 2008, 27:5497). Des inhibiteurs de PI3K sont attendus efficaces pour le traitement de nombreux cancers humains présentant des altérations génétiques aboutissant à l'activation de la voie PI3K/AKT/mTOR (Vogt P. et al., Virology, 2006, 344 :131 ; Zhao L & Vogt PK, Oncogene, 2008, 27 :5486).

Des dérivés Morpholino pyrimidinones inhibiteurs de kinases sont connus de l'homme de l'art.

L'application WO2008/148074 décrit des produits qui possèdent une activité inhibitrice de mTOR. Ces produits sont des pyrido[1,2-a]pyrimidin-4-ones qui diffèrent des produits de la présente invention en raison de leur caractère entièrement aromatique et de leurs substitutions.

L'application WO2008/064244 décrit l'application des produits TGX-221 et TGX-155 inhibiteurs de PI3Kβ utiles dans le traitement du cancer et notamment dans le cancer du sein. Ces produits sont des pyrido[1,2-a]pyrimidin-4-ones précédemment décrits dans les applications WO2004/016607 et WO2001/053266 qui diffèrent des produits de la présente invention en raison de leur caractère entièrement aromatique et de leurs substitutions.

Les applications WO2006/109081, WO2006/109084 et WO2006/126010 décrivent des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

L'application WO2003/024949 décrit des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

Des dérivés morpholino pyrimidine inhibiteurs de kinase sont également connus de l'homme de l'art.

Les applications WO2009/007748, WO2009/007749, WO2009/007750 et WO2009/007751 décrivent des produits qui possèdent une activité inhibitrice de mTOR et/ou de PI3K pour le traitement des cancers. Ces produits sont des pyrimidines substituées en 2, 4 et 6 et les produits de la présente invention en différent en raison de la présence du groupement carbonyle sur la pyrimidinone ainsi que par les différent substituants
La présente invention a pour objet les produits de formule (I): 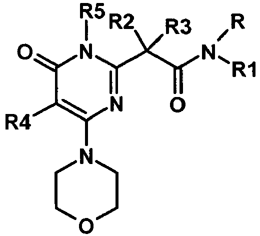 dans laquelle :
R1 représente un radical aryle ou hétéroaryle éventuellement substitués par un ou plusieurs radicaux identiques ou différents choisis parmi les atomes d'halogène et les radicaux hydroxyle, CN, nitro, -COOH, -COOalk, -NRxRy, - CONRxRy, -NRxCORy, -CORy, -NRxCO2Rz, alcoxy, phénoxy, alkylthio, alkyle, alkényle, alkynyle, cycloalkyle, O-cycloalkyle, hétérocycloalkyle ; aryle et hétéroaryle;
ces derniers radicaux alcoxy, phénoxy, alkylthio, alkyle, alkényle, alkynyle, hétérocycloalkyle, aryle et hétéroaryle étant eux-mêmes éventuellement substitués par un ou plusieurs radicaux identiques ou différents choisis parmi les atomes d'halogène et les radicaux hydroxyle, alcoxy, NRvRw ; hetérocycloalkyle ou hétéroaryle ;
les radicaux aryle et hétéroaryle étant de plus éventuellement substitués par un ou plusieurs radicaux alkyle et alcoxy eux-mêmes éventuellement substitués par un ou plusieurs atomes d'halogène ;
les radicaux hétérocycloalkyle et hétéroaryle pouvant de plus renfermer un radical oxo,
R représente un atome d'hydrogène ou bien forme avec R1 un cycle à 5 ou 6 chaînons saturé ou partiellement ou totalement insaturé fusionné à un reste aryle ou hétéroaryle et renfermant éventuellement un ou plusieurs autres hétéroatomes choisi(s) parmi O, S, N, NH et Nalk, ce radical bicyclique étant éventuellement substitué par un ou plusieurs radicaux identiques ou différents choisis parmi les atomes d'halogène et les radicaux CO-NH2, hydroxyle, alkyle et alcoxy; ce dernier radical alkyle étant lui-même éventuellement substitué par un radical hydroxyle, alcoxy, NH2, NHAlk ou N(alk)2 ;
R2, R3, identiques ou différents représentent indépendamment un atome d'hydrogène, un atome d'halogène ou un radical alkyle éventuellement substitué par un ou plusieurs atomes d'halogène ;
R4 représente un atome d'hydrogène;
R5 représente un atome d'hydrogène ou un radical alkyle éventuellement substitué par un ou plusieurs atomes d'halogène;
NRvRw étant tel que Rv représente un atome d'hydrogène ou un radical alkyle et Rw représente un atome d'hydrogène, un radical cycloalkyle, CO2alk ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
Rz représente les valeurs de Ry à l'exception de hydrogéne ;
Rx, Ry et Rz dans les radicaux -NRxCORy, -CORy et NRxCO₂Rz étant choisis parmi les significations indiquées ci-dessus pour Rx, Ry, et Rz ;
tous les radicaux alkyle (alk), alcoxy et alkylthio ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).
Les produits de formule (I) selon la présente invention sont donc tels que :
- soit R représente H et R1 représente un radical aryle ou hétéroaryle éventuellement substitués tels que définis ci-dessus ou ci-après,
- soit R forme avec R1 un cycle à 5 ou 6 chaînons saturé ou partiellement ou totalement insaturé fusionné à un reste aryle ou hétéroaryle et renfermant éventuellement un ou plusieurs autres hétéroatomes choisi(s) parmi O, S, N, NH et Nalk, ce radical bicyclique étant éventuellement substitué tel que défini ci-dessus ou ci-après,
les substituants R2, R3, R4 et R5 desdits produits de formule (I) ayant les définitions indiquées ci-dessus.
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).
La présente invention a ainsi pour objet les produits de formule (I) tels que définis ci-dessus dans laquelle :
R1 représene un radical phényle, pyridine, thiényle, benzoxazolyle, benzofuryle, indazolyle, indolyle, benzothiényle, benzimidazolyle, benzoxazinyle, tetrahydroquinolyle, éventuellement substitués par un ou plusieurs radicaux identiques ou différents choisis parmi les atomes d'halogène et les radicaux CN, nitro, -COOH, -COOalk, -NRxRy, alcoxy, alkyle, alkynyle et cycloalkyle ;
ces derniers radicaux alcoxy, alkyle et alkynyle, étant eux-mêmes éventuellement substitués par un ou plusieurs radicaux identiques ou différents choisis parmi les atomes d'halogène et les radicaux hydroxyle, alcoxy, NRvRw ; pipéridyle, pyrrolidinyle ou hétéroaryle;
les radicaux phényle et hétéroaryle étant de plus éventuellement substitués par un ou plusieurs radicaux alkyle et alcoxy ;
R représente un atome d'hydrogène ou bien forme avec R1
un cycle benzoxazinyle, dihydroindolyle, tetrahydroisoquinolyle, tetrahydroquinolyle, dihydropyrrolopyridyle, ces cycles étant éventuellement substitués par un ou plusieurs radicaux identiques ou différents choisis parmi les atomes d'halogène et les radicaux CO-NH2, hydroxyle, alkyle et alcoxy; ce dernier radical alkyle étant lui-même éventuellement substitué par un radical hydroxyle, alcoxy, NH2, NHAlk ou N(alk)2 ;
R2, R3, identiques ou différents représentent indépendamment un atome d'hydrogène, un atome de fluor ou un radical alkyle;
R4 représente un atome d'hydrogène;
R5 représente un atome d'hydrogène ou un radical alkyle;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène ou un radical alkyle;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).
La présente invention a pour objet les produits de formule (I): 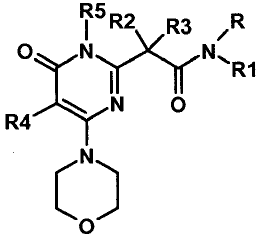 dans laquelle :
R1 représente un radical aryle ou hétéroaryle éventuellement substitués par un ou plusieurs radicaux identiques ou différents choisis parmi les atomes d'halogène et les radicaux hydroxyle, CN, nitro, -COOH, -COOalk, -NRxRy, - CONRxRy, -NRxCORy, -CORy, -NRxCO2Rz, alcoxy, phénoxy, alkylthio, alkyle, alkényle, alkynyle, cycloalkyle, O-cycloalkyle, hétérocycloalkyle ; aryle et hétéroaryle;
ces derniers radicaux alcoxy, phénoxy, alkylthio, alkyle, alkényle, alkynyle, hétérocycloalkyle, aryle et hétéroaryle étant eux-mêmes éventuellement substitués par un ou plusieurs radicaux identiques ou différents choisis parmi les atomes d'halogène et les radicaux hydroxyle, alcoxy et NRvRw ;
les radicaux aryle et hétéroaryle étant de plus éventuellement substitués par un ou plusieurs radicaux alkyle et alcoxy eux-mêmes éventuellement substitués par un ou plusieurs atomes d'halogène ;
les radicaux hétérocycloalkyle et hétéroaryle pouvant de plus renfermer un radical oxo,
R représente un atome d'hydrogène ou bien forme avec R1 un cycle à 5 ou 6 chaînons saturé ou partiellement ou totalement insaturé fusionné à un reste aryle ou hétéroaryle et renfermant éventuellement un ou plusieurs autres hétéroatomes choisi(s) parmi O, S, N, NH et Nalk, ce radical bicyclique étant éventuellement substitué par un ou plusieurs radicaux identiques ou différents choisis parmi les atomes d'halogène et les radicaux hydroxyle, alkyle et alcoxy;
R2, R3, identiques ou différents représentent indépendamment un atome d'hydrogène, un atome d'halogène ou un radical alkyle éventuellement substitué par un ou plusieurs atomes d'halogène ;
R4 représente un atome d'hydrogène;
R5 représente un atome d'hydrogène ou un radical alkyle éventuellement substitué par un ou plusieurs atomes d'halogène;
NRxRy étant tel que Rx représente un atome d'hydrogène ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
Rz représente les valeurs de Ry à l'exception de hydrogène ;
Rx, Ry et Rz dans les radicaux -NRxCORy, -CORy et NRxCO₂Rz étant choisis parmi les significations indiquées ci-dessus pour Rx, Ry, et Rz ;
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).
Dans les produits de formule (I) :
- le terme radical alkyle (ou alk) désigne les radicaux, linéaires et le cas échéant ramifiés, méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alcoxy désigne les radicaux linéaires et le cas échéant ramifiés, méthoxy, éthoxy, propoxy, isopropoxy, butoxy linéaire, secondaire ou tertiaire, pentoxy ou hexoxy ainsi que leurs isomères de position linéaires ou ramifiés : on préfère les radicaux alkoxy renfermant de 1 à 4 atomes de carbone de la liste ci-dessus ;

- le terme radical alkylthio désigne les radicaux linéaires et le cas échéant ramifiés, méthylthio, éthylthio, propylthio , isopropylthio, butylthio linéaire, secondaire ou tertiaire, pentylthio ou hexylthio ainsi que leurs isomères de position linéaires ou ramifiés : on préfère les radicaux alkylthio renfermant de 1 à 4 atomes de carbone de la liste ci-dessus ;
- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropyle, cyclobutyle, cyclopentyle et cyclohexyle et tout particulièrement les radicaux cyclopropyle, cyclopentyle et cyclohexyle ;
- dans le radical -O-cycloalkyle, cycloalkyle est tel que défini ci-dessus ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, homomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, tétrahydropyranne, oxodihydropyridazinyle, ou encore oxétanyle tous ces radicaux étant éventuellement substitués ; on peut citer notamment les radicaux morpholinyle, thiomorpholinyle, homomorpholinyle, pipérazinyle, pipéridyle, homopipérazinyle ou encore pyrrolidinyle,
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
le terme radical aryle désigne ainsi des radicaux monocycliques ou bicycliques renfermant 6 à 12 chaînons tels que par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone ;
le terme radical hétéroaryle désigne ainsi des radicaux monocycliques ou bicycliques renfermant 5 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, thiazolyle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués ; des radicaux hétéroaryles bicycliques tels que par exemple les radicaux benzothiényle (benzothiophène) tel que 3-benzothiényle, benzothiazolyle, quinolyle, isoquinolyle, dihydroquinolyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazinyle, benzoxazolyle, thionaphtyle, indolyle, dihydroindolyle, azaindolyle, indazolyle, purinyle, thiénopyrazolyle, tétrahydroindazolyle, tetrahydroquinolyle, tetrahydroisoquinolyle tétrahydrocyclopentapyrazolyle, dihydrofuropyrazolyle, dihydropyrrolopyridyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;
Comme exemples de radicaux hétéroaryles ou bicycliques, on peut citer plus particulièrement les radicaux pyrimidinyle, pyridyle, pyrrolyle, azaindolyle, indazolyle ou pyrazolyle, benzothiazolyle ou benzimidazolyle éventuellement substitués par un ou plusieurs substituants identiques ou différents comme indiqué ci-dessus.
Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.
La présente invention a pour objet les produits de formule (I) tels que définis ci-dessus dans laquelle :
R1 représente un radical phényle, pyridine, thiényle, benzoxazole-4-yle et indazole-6-yle, éventuellement substitués par un ou plusieurs radicaux identiques ou différents choisis parmi les atomes d'halogène et les radicaux CN, nitro, -COOH, -COOalk, -NRxRy, alcoxy, alkyle, alkynyle et cycloalkyle ;
ces derniers radicaux alcoxy, alkyle et alkynyle, étant eux-mêmes éventuellement substitués par un ou plusieurs radicaux identiques ou différents choisis parmi les atomes d'halogène et les radicaux hydroxyle, alcoxy et NRvRw ;
les radicaux phényle et hétéroaryle étant de plus éventuellement substitués par un ou plusieurs radicaux alkyle et alcoxy ;
R représente un atome d'hydrogène ou bien forme avec R1 un cycle 1,4-benzoxazin-4-yle ou 2,3-dihydro-indol-1-yle, ces cycles étant éventuellement substitués par un ou plusieurs radicaux identiques ou différents choisis parmi les atomes d'halogène et les radicaux hydroxyle, alkyle et alcoxy;
R2, R3, identiques ou différents représentent indépendamment un atome d'hydrogène, un atome de fluor ou un radical alkyle;
R4 représente un atome d'hydrogène;
R5 représente un atome d'hydrogène ou un radical alkyle;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène ou un radical alkyle;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).
Notamment, lorsque NRxRy ou NRvRw forme un cycle comme défini ci-dessus, un tel cycle aminé peut être choisi notamment parmi les radicaux pyrrolidinyle, pyrazolidinyle, pyrazolinyle, pipéridyle, azépinyle, morpholinyle, homomorpholinyle, pipérazinyle ou homopipérazinyle, ces radicaux étant eux-mêmes éventuellement substitués comme indiqué ci-dessus ou ci-après.
Le cycle NRxRy ou NRvRw peut plus particulièrement être choisi parmi les radicaux pyrrolidinyle, morpholinyle éventuellement substitué par un ou deux radicaux alkyle ou pipérazinyle éventuellement substitué sur le second atome d'azote par un radical alkyle, phényle, ou et CH2-phényle, eux-mêmes éventuellement substitués par un ou plusieurs radicaux identiques ou différents choisis parmi les atomes d'halogène et les radicaux alkyle, hydroxyle et alcoxy.
La présente invention a tout particulièrement pour objet les produits de formule (I) tels que définis ci-dessus répondant aux formules suivantes :
- 2-[4-(morpholin-4-yl)-6-oxo-1,6-dihydropyrimidin-2-yl]-N-phénylacétamide
- N-(4-fluorophényl)-2-[4-(morpholin-4-yl)-6-oxo-1,6-dihydropyrimidin-2-yl]acétamide
- N-(3-chlorophényl)-2-[4-(morpholin-4-yl)-6-oxo-1,6-dihydropyrimidin-2-yl]acétamide
- N-[3-(diméthylamino)phényl]-2-[4-(morpholin-4-yl)-6-oxo-1,6-dihydropyrimidin-2-yl]acétamide
- N-(2,4-difluorophényl)-2-[4-(morpholin-4-yl)-6-oxo-1,6-dihydropyrimidin-2-yl]acétamide
- N-(3,4-difluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(thiophén-3-yl)acétamide
- N-(4-fluoro-3-méthoxyphényl)-2-[4-(morpholin-4-yl)-6-oxo-1,6-dihydropyrimidin-2-yl]acétamide
- N-(2-fluorophényl)-2-[4-(morpholin-4-yl)-6-oxo-1,6-dihydropyrimidin-2-yl]acétamide
- N-(2-méthylphényl)-2-[4-(morpholin-4-yl)-6-oxo-1,6-dihydropyrimidin-2-yl]acétam ide
- N-(2-méthoxyphényl)-2-[4-(morpholin-4-yl)-6-oxo-1,6-dihydropyrimidin-2-yl]acétamide
- N-(2,3-difluorophényl)-2-[4-(morpholin-4-yl)-6-oxo-1,6-dihydropyrimidin-2-yl]acétamide
- N-(3,5-difluorophényl)-2-[4-(morpholin-4-yl)-6-oxo-1,6-dihydropyrimidin-2-yl]acétamide
- N-(3-fluorophényl)-2-[4-(morpholin-4-yl)-6-oxo-1,6-dihydropyrimidin-2-yl]acétamide
- N-(4-chlorophényl)-2-[4-(morpholin-4-yl)-6-oxo-1,6-dihydropyrimidin-2-yl]acétamide
- N-(3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhyl)phényl]acétamide
- N-(3-bromophényl)-2-[4-(morpholin-4-yl)-6-oxo-1,6-dihydropyrimidin-2-yl]acétamide
- N-[3-(2-méthylpropan-2-yl)phényl]-2-[4-(morpholin-4-yl)-6-oxo-1,6-dihydropyrimidin-2-yl]acétamide
- 3-({[4-(morpholin-4-yl)-6-oxo-1,6-dihydropyrimidin-2-yl]acétyl}amino)benzoate de méthyle
- acide 3-({[4-(morpholin-4-yl)-6-oxo-1,6-dihydropyrimidin-2-yl]acétyl}amino)benzoïque
- 2-[4-(morpholin-4-yl)-6-oxo-1,6-dihydropyrimidin-2-yl]-N-[3-(propan-2-yl)phényl]acétamide
- N-(3-méthylphényl)-2-[4-(morpholin-4-yl)-6-oxo-1,6-dihydropyrimidin-2-yl]acétamide
- N-(3-cyano-4-fluorophényl)-2-[4-(morpholin-4-yl)-6-oxo-1,6-dihydropyrimidin-2-yl]acétamide
- N-(1H-indazol-6-yl)-2-[4-(morpholin-4-yl)-6-oxo-1,6-dihydropyrimidin-2-yl]acétamide
- N-(3-cyanophényl)-2-[4-(morpholin-4-yl)-6-oxo-1,6-dihydropyrimidin-2-yl]acétamide
- N-(5-fluoropyridin-2-yl)-2-[4-(morpholin-4-yl)-6-oxo-1,6-dihydropyrimidin-2-yl]acétamide
- N-(4-fluoro-3-méthylphényl)-2-[4-(morpholin-4-yl)-6-oxo-1,6-dihydropyrimidin-2-yl]acétamide
- N-(3-chloro-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(pyridin-3-yl)acétamide
- N-(4-fluoro-2-méthylphényl)-2-[4-(morpholin-4-yl)-6-oxo-1,6-dihydropyrimidin-2-yl]acétamide
- N-(3-hydroxyphényl)-2-[4-(morpholin-4-yl)-6-oxo-1,6-dihydropyrimidin-2-yl]acétamide
- N-(3-bromo-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(3,4,5-trifluorophényl)acétamide
- N-[4-fluoro3-(hydroxyméthyl)phényl)-2-[4-(morpholin-4-yl)-6-oxo-1,6-dihydropyrimidin-2-yl]acétamide
- N-(3-cyclopropylephényl)-2-[4-(morpholin-4-yl)-6-oxo-1,6-dihydropyrimidin-2-yl]acétamide
- N-(2-hydroxyphényl)-2-[4-(morpholin-4-yl)-6-oxo-1,6-dihydropyrimidin-2-yl]acétamide
- N-[3-(difluorométhoxy)phényl]-2-[4-(morpholin-4-yl)-6-oxo-1,6-dihydropyrimidin-2-yl]acétamide
- N-(4-fluoro-3-méthoxyphényl)-2-[4-(morpholin-4-yl)-6-oxo-1,6-dihydropyrimidin-2-yl]propanamide
- N-(2,3-diméthylphényl)-2-[4-(morpholin-4-yl)-6-oxo-1,6-dihydropyrimidin-2-yl]acétamide
- N-(2-fluoro-3-méthylphényl)-2-[4-(morpholin-4-yl)-6-oxo-1,6-dihydropyrimidin-2-yl]acétamide
- N-(1,3-benzoxazol-4-yl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhoxy)phényl]acétamide
- 2-[4-(morpholin-4-yl)-6-oxo-1,6-dihydropyrimidin-2-yl]-N-[3-(propan-2-yloxy)phényl]acétamide
- N-(4-fluoro-2-méthoxyphényl)-2-[4-(morpholin-4-yl)-6-oxo-1,6-dihydropyrimidin-2-yl]acétamide
- {2-[3-({[4-(morpholin-4-yl)-6-oxo-1,6-dihydropyrimidin-2-yl]acétyl}amino)phényl]éthyl}carbamate de 2-méthylpropan-2-yle
- N-[4-fluoro3-(trifluorométhyl)phényl]-2-[4-(morpholin-4-yl)-6-oxo-1,6-dihydropyrimidin-2-yl]acétamide
- N-(3-éthynylphényl)-2-[4-(morpholin-4-yl)-6-oxo-1,6-dihydropyrimidin-2-yl]acétamide
- N-[3-(cyclopentyloxy)phényl)-2-[4-(morpholin-4-yl)-6-oxo-1,6-dihydropyrimidin-2-yl]acétamide
- N-(4-fluoro-2-hydroxyphényl)-2-[4-(morpholin-4-yl)-6-oxo-1,6-dihydropyrimidin-2-yl]acétamide
- 2-[2-(2,3-dihydro-1H-indol-1-yl)-2-oxoéthyl]-6-(morpholin-4-yl)pyrimidin-4(3H)-one
- N-(3-cyclopropyl-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,3,4-trifluorophényl)acétamide
- N-[4-fluoro3-(trifluorométhoxy)phényl]-2-[4-(morpholin-4-yl)-6-oxo-1,6-dihydropyrimidin-2-yl]acétamide
- N-[3-(2-hydroxyéthoxy)phényl]-2-[4-(morpholin-4-yl)-6-oxo-1,6-dihydropyrimidin-2-yl]acétamide
- N-(3-iodophényl)-2-[4-(morpholin-4-yl)-6-oxo-1,6-dihydropyrimidin-2-yl]acétamide
- 2-fluoro-5-({[4-(morpholin-4-yl)-6-oxo-1,6-dihydropyrimidin-2-yl]acétyl}amino)benzoate de méthyle
- N-(3-éthoxyphényl)-2-[4-(morpholin-4-yl)-6-oxo-1,6-dihydropyrimidin-2-yl]acétamide
- N-(2,4-difluoro-3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,4,5-trifluorophényl)acétamide
- N-(3,5-dichloro-4-fluorophényl)-2-[4-(morpholin-4-yl)-6-oxo-1,6-dihydropyrimidin-2-yl]acétamide
- 2-[2-(2,3-dihydro-4H-1,4-benzoxazin-4-yl)-2-oxoéthyl]-6-(morpholin-4-yl)pyrimidin-4(3H)-one
- N-(4-fluoro-3-nitrophényl)-2-[4-(morpholin-4-yl)-6-oxo-1,6-dihydropyrimidin-2-yl]acétamide
- acide 2-fluoro-5-({[4-(morpholin-4-yl)-6-oxo-1,6-dihydropyrimidin-2-yl]acétyl}amino)benzoïque
- N-(5-fluoro-2-hydroxyphényl)-2-[4-(morpholin-4-yl)-6-oxo-1,6-dihydropyrimidin-2-yl]acétamide
- N-(2-bromo-4-fluorophényl)-2-[4-(morpholin-4-yl)-6-oxo-1,6-dihydropyrimidin-2-yl]acétamide
- N-(4-fluorophényl)-2-[1-méthyl-4-(morpholin-4-yl)-6-oxo-1,6-dihydropyrimidin-2-yl]acétamide
- N-(3-chloro-4-fluorophényl)-2-[1-méthyl-4-(morpholin-4-yl)-6-oxo-1,6-dihydropyrimidin-2-yl]acétamide
- N-(3-bromophényl)-2-[1-méthyl-4-(morpholin-4-yl)-6-oxo-1,6-dihydropyrimidin-2-yl]acétamide
- 2-[1-éthyl-4-(morpholin-4-yl)-6-oxo-1,6-dihydropyrimidin-2-yl]-N-(4-fluorophényl)acétamide
- N-(1H-indol-4-yl)-2-[4-(morpholin-4-yl)-6-oxo-1,6-dihydropyrimidin-2-yl]acétamide
- N-(4-fluorophényl)-3-méthyl-2-[4-(morpholin-4-yl)-6-oxo-1,6-dihydropyrimidin-2-yl]butanamide
- N-[4-fluoro3-(méthoxyméthyl)phényl]-2-[4-(morpholin-4-yl)-6-oxo-1,6-dihydropyrimidin-2-yl]acétamide
- N-(4-fluoro-3-iodophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(1,1,2,2-tétrafluoroéthoxy)phényl]acétamide
- N-[3-(difluorométhyl)-4-fluorophényl]-2-[4-(morpholin-4-yl)-6-oxo-1,6-dihydropyrimidin-2-yl]acétamide
- 2,2-difluoro-N-(4-fluorophényl)-2-[4-(morpholin-4-yl)-6-oxo-1,6-dihydropyrimidin-2-yl]acétamide
- N-(3,4-difluorophényl)-2-[1-méthyl-4-(morpholin-4-yl)-6-oxo-1,6-dihydropyrimidin-2-yl]acétamide
- 2-[2-(2,3-dihydro-1H-indol-1-yl)-2-oxoéthyl]-3-méthyl-6-(morpholin-4-yl)pyrimidin-4(3H)-one
- N-(3-bromo-4-fluorophényl)-2-[1-méthyl-4-(morpholin-4-yl)-6-oxo-1,6-dihydropyrimidin-2-yl]acétamide
- N-[4-fluoro3-(hydroxyméthyl)phényl]-2-[1-méthyl-4-(morpholin-4-yl)-6-oxo-1,6-dihydropyrimidin-2-yl]acétamide
- N-(3-cyclopropylphényl)-2-[1-méthyl-4-(morpholin-4-yl)-6-oxo-1,6-dihydropyrimidin-2-yl]acétamide
- N-(4-fluoro-3-méthoxyphényl)-2-[1-méthyl-4-(morpholin-4-yl)-6-oxo-1,6-dihydropyrimidin-2-yl]acétamide
- N-(1-benzofur-4-yl)-2-[4-(morpholin-4-yl)-6-oxo-1,6-dihydropyrimidin-2-yl]acétamide
- 2-[1-méthyl-4-(morpholin-4-yl)-6-oxo-1,6-dihydropyrimidin-2-yl]-N-phénylacétamide
- N-(3-cyclopropyl-4-fluorophényl)-2-[1-méthyl-4-(morpholin-4-yl)-6-oxo-1,6-dihydropyrimidin-2-yl]acétamide
- N-(3-fluoro-2-hydroxyphényl)-2-[4-(morpholin-4-yl)-6-oxo-1,6-dihydropyrimidin-2-yl]acétamide
- 2-[2-(4-fluoro-2,3-dihydro-1H-indol-1-yl)-2-oxoéthyl]-6-(morpholin-4-yl)pyrimidin-4(3H)-one
- 2-[2-(4-chloro-2,3-dihydro-1H-indol-1-yl)-2-oxoéthyl]-6-(morpholin-4-yl)pyrimidin-4(3H)-one
- N-(3-éthynyl-4-fluorophényl)-2-[4-(morpholin-4-yl)-6-oxo-1,6-dihydropyrimidin-2-yl]acétamide
- 2-[2-(4-hydroxy-2,3-dihydro-1H-indol-1-yl)-2-oxoéthyl]-6-(morpholin-4-yl)pyrimidin-4(3H)-one
- 2-[2-(4,6-difluoro-2,3-dihydro-1H-indol-1-yl)-2-oxoéthyl]-6-(morpholin-4-yl)pyrimidin-4(3H)-one
- N-(4-Fluoro-3-iodo-phenyl)-2-(1-methyl-4-morpholin-4-yl-6-oxo-1,6-dihydro-pyrimidin-2-yl)-acetamide
- 2-[2-(4,5-difluoro-2,3-dihydro-1H-indol-1-yl)-2-oxoéthyl]-6-(morpholin-4-yl)pyrimidin-4(3H)-one
- 2-[2-(6-fluoro-2,3-dihydro-1H-indol-1-yl)-2-oxoéthyl]-6-(morpholin-4-yl)pyrimidin-4(3H)-one
- 2-[2-(2-méthyl-2,3-dihydro-1H-indol-1-yl)-2-oxoéthyl]-6-(morpholin-4-yl)pyrimidin-4(3H)-one
- N-[3-(difluorométhyl)-4-fluorophényl]-2-[1-méthyl-4-(morpholin-4-yl)-6-oxo-1,6-dihydropyrimidin-2-yl]acétamide
- 2-[1-methyl-4-(morpholin-4-yl)-6-oxo-1,6-dihydropyrimidin-2-yl]-N-(3,4,5-trifluorophenyl)acetamide
- N-(1-methyl-1H-indol-4-yl)-2-[4-(morpholin-4-yl)-6-oxo-1,6-dihydropyrimidin-2-yl]acetamide
- 2-[2-(4-methyl-2,3-dihydro-1H-indol-1-yl)-2-oxoethyl]-6-(morpholin-4-yl)pyrimidin-4(3H)-one
- 2-[2-(3-methyl-2,3-dihydro-1H-indol-1-yl)-2-oxoethyl]-6-(morpholin-4-yl)pyrimidin-4(3H)-one
- 2-[2-(4-fluoro-2,3-dihydro-1H-indol-1-yl)-2-oxoethyl]-3-methyl-6-(morpholin-4-yl)pyrimidin-4(3H)-one
- 2-[2-(5-Fluoro-2,3-dihydro-indol-1-yl)-2-oxo-ethyl]-6-morpholin-4-yl-3H-pyrimidin-4-one
- 2-[2-(4-chloro-2,3-dihydro-1H-indol-1-yl)-2-oxoethyl]-3-methyl-6-(morpholin-4-yl)pyrimidin-4(3H)-one
- N-(1-benzothiophen-4-yl)-2-[4-(morpholin-4-yl)-6-oxo-1,6-dihydropyrimidin-2-yl]acetamide
- 2-{2-[2-(hydroxymethyl)-2,3-dihydro-1H-indol-1-yl]-2-oxoethyl}-6-(morpholin-4-yl)pyrimidin-4(3H)-one
- 2-[4-(morpholin-4-yl)-6-oxo-1,6-dihydropyrimidin-2-yl]-N-{2-[2-(piperidin-1-yl)ethoxy]phenyl}acetamide
- N-[2-(2-methoxyethoxy)phenyl]-2-[4-(morpholin-4-yl)-6-oxo-1,6-dihydropyrimidin-2-yl]acetamide
- 2-[2-(4-hydroxy-2,3-dihydro-1H-indol-1-yl)-2-oxoethyl]-3-methyl-6-(morpholin-4-yl)pyrimidin-4(3H)-one
- 2-[2-(4-methoxy-2,3-dihydro-1H-indol-1-yl)-2-oxoethyl]-3-methyl-6-(morpholin-4-yl)pyrimidin-4(3H)-one
- 2-[2-(3,4-dihydroisoquinolin-2(1H)-yl)-2-oxoethyl]-6-(morpholin-4-yl)pyrimidin-4(3H)-one
- 2-[4-(morpholin-4-yl)-6-oxo-1,6-dihydropyrimidin-2-yl]-N-{2-[2-(pyrrolidin-1-yl)ethoxy]phenyl}acetamide
- 2-[4-(morpholin-4-yl)-6-oxo-1,6-dihydropyrimidin-2-yl]-N-[2-(pyridin-3-ylmethoxy)phenyl]acetamide
- 3-methyl-2-[2-(4-methyl-2,3-dihydro-1H-indol-1-yl)-2-oxoethyl]-6-(morpholin-4-yl)pyrimidin-4(3H)-one
- 2-(2-{3-[(dimethylamino)methyl]-2,3-dihydro-1H-indol-1-yl}-2-oxoethyl)-6-(morpholin-4-yl)pyrimidin-4(3H)-one
- 2-[2-(4-bromo-2,3-dihydro-1H-indol-1-yl)-2-oxoethyl]-3-methyl-6-(morpholin-4-yl)pyrimidin-4(3H)-one
- 2-{2-[(2S)-2-methyl-2,3-dihydro-1H-indol-1-yl]-2-oxoethyl}-6-(morpholin-4-yl)pyrimidin-4(3H)-one
- 2-{2-[(2R)-2-methyl-2,3-dihydro-1H-indol-1-yl]-2-oxoethyl}-6-(morpholin-4-yl)pyrimidin-4(3H)-one
- 3-methyl-2-[2-(3-methyl-2,3-dihydro-1H-indol-1-yl)-2-oxoethyl]-6-(morpholin-4-yl)pyrimidin-4(3H)-one
- 2-{2-[2-(methoxymethyl)-2,3-dihydro-1H-indol-1-yl]-2-oxoethyl}-6-(morpholin-4-yl)pyrimidin-4(3H)-one
- 2-[2-(4-ethoxy-2,3-dihydro-1H-indol-1-yl)-2-oxoethyl]-6-(morpholin-4-yl)pyrimidin-4(3H)-one
- 1-{[4-(morpholin-4-yl)-6-oxo-1,6-dihydropyrimidin-2-yl]acetyl}-2,3-dihydro-1H-indole-2-carboxamide
- 3-methyl-2-[2-(2-methyl-2,3-dihydro-1H-indol-1-yl)-2-oxoethyl]-6-(morpholin-4-yl)pyrimidin-4(3H)-one
- 2-[2-(6-fluoro-2,3-dihydro-1H-indol-1-yl)-2-oxoethyl]-3-methyl-6-(morpholin-4-yl)pyrimidin-4(3H)-one
- 2-{2-[(3S)-3-methyl-2,3-dihydro-1H-indol-1-yl]-2-oxoethyl}-6-(morpholin-4-yl)pyrimidin-4(3H)-one
- 2-{2-[(3R)-3-methyl-2,3-dihydro-1H-indol-1-yl]-2-oxoethyl}-6-(morpholin-4-yl)pyrimidin-4(3H)-one
- 2-[2-(5,6-difluoro-2,3-dihydro-1H-indol-1-yl)-2-oxoethyl]-3-methyl-6-(morpholin-4-yl)pyrimidin-4(3H)-one
- 2-[2-(4,5-difluoro-2,3-dihydro-1H-indol-1-yl)-2-oxoethyl]-3-methyl-6-(morpholin-4-yl)pyrimidin-4(3H)-one
- 2-[2-(1,3-dihydro-2H-isoindol-2-yl)-2-oxoethyl]-6-(morpholin-4-yl)pyrimidin-4(3H)-one
- N-(1-benzothiophen-4-yl)-2-[1-methyl-4-(morpholin-4-yl)-6-oxo-1,6-dihydropyrimidin-2-yl]acetamide
- 2-[2-(5-chloro-3,4-dihydroquinolin-1 (2H)-yl)-2-oxoethyl]-6-(morpholin-4-yl)pyrimidin-4(3H)-one
- 2-{2-[4-(hydroxymethyl)-2,3-dihydro-1H-indol-1-yl]-2-oxoethyl)-6-(morpholin-4-yl)pyrimidin-4(3H)-one
- N-[4-fluoro-2-(piperidin-4-ylmethoxy)phenyl]-2-[4-(morpholin-4-yl)-6-oxo-1,6-dihydropyrimidin-2-yl]acetamide
- 2-[2-(5-Chloro-2,3-dihydro-indol-1-yl)-2-oxo-ethyl]-6-morpholin-4-yl-3H-pyrimidin-4-one
- 2-[2-(4-Bromo-2,3-dihydro-indol-1-yl)-2-oxo-ethyl]-6-morpholin-4-yl-3H-pyrimidin-4-one
- 2-(2-{(3S)-3-[(dimethylamino)methyl]-2,3-dihydro-1H-indol-1-yl}-2-oxoethyl)-6-(morpholin-4-yl)pyrimidin-4(3H)-one
- 2-(2-{(3R)-3-[(dimethylamino)methyl]-2,3-dihydro-1H-indol-1-yl}-2-oxoethyl)-6-(morpholin-4-yl)pyrimidin-4(3H)-one
- N-[4-fluoro-2-(2-methoxyethoxy)phenyl]-2-[4-(morpholin-4-yl)-6-oxo-1,6-dihydropyrimidin-2-yl]acetamide
- N-(1H-benzimidazol-4-yl)-2-[4-(morpholin-4-yl)-6-oxo-1,6-dihydropyrimidin-2-yl]acetam ide
- methyl 2-hydroxy-3-({[4-(morpholin-4-yl)-6-oxo-1,6-dihydropyrimidin-2-yl]acetyl}amino)benzoate
- 2-[2-(4-Methoxy-2,3-dihydro-indol-1-yl)-2-oxo-ethyl]-6-morpholin-4-yl-3H-pyrimidin-4-one
- N-(3-bromo-2-hydroxyphenyl)-2-[4-(morpholin-4-yl)-6-oxo-1,6-dihydropyrimidin-2-yl]acetamide
- N-(3,4-dihydro-2H-1,4-benzoxazin-8-yl)-2-[4-(morpholin-4-yl)-6-oxo-1,6-dihydropyrimidin-2-yl]acetamide
- methyl 5-fluoro-2-({[4-(morpholin-4-yl)-6-oxo-1,6-dihydropyrimidin-2-yl]acetyl}amino)benzoate
- 2-(2-{3-[(diethylamino)methyl]-2,3-dihydro-1H-indol-1-yl}-2-oxoethyl)-6-(morpholin-4-yl)pyrimidin-4(3H)-one
- 2-[2-(2,3-dihydro-1H-pyrrolo[3,2-b]pyridin-1-yl)-2-oxoethyl]-6-(morpholin-4-yl)pyrimidin-4(3H)-one
- 2-[2-(5,6-Difluoro-2,3-dihydro-indol-1-yl)-2-oxo-ethyl]-6-morpholin-4-yl-3H-pyrimidin-4-one
- 2-[4-(morpholin-4-yl)-6-oxo-1,6-dihydropyrimidin-2-yl]-N-(1,2,3,4-tetrahydroquinolin-8-yl)acetamide
- 2-[2-(8-chloro-2,3-dihydro-4H-1,4-benzoxazin-4-yl)-2-oxoéthyl]-6-(morpholin-4-yl)pyrimidin-4(3H)-one
- N-(2-hydroxy-3-méthylphényl)-2-[4-(morpholin-4-yl)-6-oxo-1,6-dihydropyrimidin-2-yl]acétamide
- N-(2-hydroxy-3-nitrophényl)-2-[4-(morpholin-4-yl)-6-oxo-1,6-dihydropyrimidin-2-yl]acétamide
- N-(3-cyano-2-hydroxyphényl)-2-[4-(morpholin-4-yl)-6-oxo-1,6-dihydropyrimidin-2-yl]acétamide
- N-[2-hydroxy-3-(trifluorométhyl)phényl]-2-[4-(morpholin-4-yl)-6-oxo-1,6-dihydropyrimidin-2-yl]acétamide
- 2-[2-(3,3-diméthyl-2,3-dihydro-1H-indol-1-yl)-2-oxoéthyl]-6-(morpholin-4-yl)pyrimidin-4(3H)-one
ainsi que les sels d'addition avec les acides minéraux et organiques ou avec les bases minérales et organiques desdits produits de formule (I).
La présente invention a tout particulièrement pour objet les produits de formule (I) tels que définis ci-dessus répondant aux formules suivantes :
- 2-[4-(morpholin-4-yl)-6-oxo-1,6-dihydropyrimidin-2-yl]-N-phénylacétamide
- N-(4-fluorophényl)-2-[4-(morpholin-4-yl)-6-oxo-1,6-dihydropyrimidin-2-yl]acétamide
- N-(3-chlorophényl)-2-[4-(morpholin-4-yl)-6-oxo-1,6-dihydropyrimidin-2-yl]acétamide
- N-[3-(diméthylamino)phényl]-2-[4-(morpholin-4-yl)-6-oxo-1,6-dihydropyrimidin-2-yl]acétamide
- N-(2,4-difluorophényl)-2-[4-(morpholin-4-yl)-6-oxo-1,6-dihydropyrimidin-2-yl]acétamide
- N-(3,4-difluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(thiophén-3-yl)acétamide
- N-(4-fluoro-3-méthoxyphényl)-2-[4-(morpholin-4-yl)-6-oxo-1,6-dihydropyrimidin-2-yl]acétamide
- N-(2-fluorophényl)-2-[4-(morpholin-4-yl)-6-oxo-1,6-dihydropyrimidin-2-yl]acétamide
- N-(2-méthylphényl)-2-[4-(morpholin-4-yl)-6-oxo-1,6-dihydropyrimidin-2-yl]acétamide
- N-(2-méthoxyphényl)-2-[4-(morpholin-4-yl)-6-oxo-1,6-dihydropyrimidin-2-yl]acétamide
- N-(2,3-difluorophényl)-2-[4-(morpholin-4-yl)-6-oxo-1,6-dihydropyrimidin-2-yl]acétamide
- N-(3,5-difluorophényl)-2-[4-(morpholin-4-yl)-6-oxo-1,6-dihydropyrimidin-2-yl]acétamide
- N-(3-fluorophényl)-2-[4-(morpholin-4-yl)-6-oxo-1,6-dihydropyrimidin-2-yl]acétamide
- N-(4-chlorophényl)-2-[4-(morpholin-4-yl)-6-oxo-1,6-dihydropyrimidin-2-yl]acétamide
- N-(3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhyl)phényl]acétamide
- N-(3-bromophényl)-2-[4-(morpholin-4-yl)-6-oxo-1,6-dihydropyrimidin-2-yl]acétamide
- N-[3-(2-méthylpropan-2-yl)phényl]-2-[4-(morpholin-4-yl)-6-oxo-1,6-dihydropyrimidin-2-yl]acétamide
- 3-({[4-(morpholin-4-yl)-6-oxo-1,6-dihydropyrimidin-2-yl]acétyl}amino)benzoate de méthyle
- acide 3-({[4-(morpholin-4-yl)-6-oxo-1,6-dihydropyrimidin-2-yl]acétyl}amino)benzoïque
- 2-[4-(morpholin-4-yl)-6-oxo-1,6-dihydropyrimidin-2-yl]-N-[3-(propan-2-yl)phényl]acétamide
- N-(3-méthylphényl)-2-[4-(morpholin-4-yl)-6-oxo-1,6-dihydropyrimidin-2-yl]acétamide
- N-(3-cyano-4-fluorophényl)-2-[4-(morpholin-4-yl)-6-oxo-1,6-dihydropyrimidin-2-yl]acétamide
- N-(1H-indazol-6-yl)-2-[4-(morpholin-4-yl)-6-oxo-1,6-dihydropyrimidin-2-yl]acétamide
- N-(3-cyanophényl)-2-[4-(morpholin-4-yl)-6-oxo-1,6-dihydropyrimidin-2-yl]acétamide
- N-(5-fluoropyridin-2-yl)-2-[4-(morpholin-4-yl)-6-oxo-1,6-dihydropyrimidin-2-yl]acétamide
- N-(4-fluoro-3-méthylphényl)-2-[4-(morpholin-4-yl)-6-oxo-1,6-dihydropyrimidin-2-yl]acétamide
- N-(3-chloro-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(pyridin-3-yl)acétamide
- N-(4-fluoro-2-méthylphényl)-2-[4-(morpholin-4-yl)-6-oxo-1,6-dihydropyrimidin-2-yl]acétamide
- N-(3-hydroxyphényl)-2-[4-(morpholin-4-yl)-6-oxo-1,6-dihydropyrimidin-2-yl]acétamide
- N-(3-bromo-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(3,4,5-trifluorophényl)acétamide
- N-[4-fluoro3-(hydroxyméthyl)phényl]-2-[4-(morpholin-4-yl)-6-oxo-1,6-dihydropyrimidin-2-yl]acétamide
- N-(3-cyclopropylephényl)-2-[4-(morpholin-4-yl)-6-oxo-1,6-dihydropyrimidin-2-yl]acétamide
- N-(2-hydroxyphényl)-2-[4-(morpholin-4-yl)-6-oxo-1,6-dihydropyrimidin-2-yl]acétamide
- N-[3-(difluorométhoxy)phényl]-2-[4-(morpholin-4-yl)-6-oxo-1,6-dihydropyrimidin-2-yl]acétamide
- N-(4-fluoro-3-méthoxyphényl)-2-[4-(morpholin-4-yl)-6-oxo-1,6-dihydropyrimidin-2-yl]propanamide
- N-(2,3-diméthylphényl)-2-[4-(morpholin-4-yl)-6-oxo-1,6-dihydropyrimidin-2-yl]acétamide
- N-(2-fluoro-3-méthylphényl)-2-[4-(morpholin-4-yl)-6-oxo-1,6-dihydropyrimidin-2-yl]acétamide
- N-(1,3-benzoxazol-4-yl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhoxy)phényl]acétamide
- 2-[4-(morpholin-4-yl)-6-oxo-1,6-dihydropyrimidin-2-yl]-N-[3-(propan-2-yloxy)phényl]acétamide
- N-(4-fluoro-2-méthoxyphényl)-2-[4-(morpholin-4-yl)-6-oxo-1,6-dihydropyrimidin-2-yl]acétamide
- {2-[3-({[4-(morpholin-4-yl)-6-oxo-1,6-dihydropyrimidin-2-yl]acétyl}amino)phényl]éthyl}carbamate de 2-méthylpropan-2-yle
- N-[4-fluoro3-(trifluorométhyl)phényl]-2-[4-(morpholin-4-yl)-6-oxo-1,6-dihydropyrimidin-2-yl]acétamide
- N-(3-éthynylphényl)-2-[4-(morpholin-4-yl)-6-oxo-1,6-dihydropyrimidin-2-yl]acétamide
- N-[3-(cyclopentyloxy)phényl]-2-[4-(morpholin-4-yl)-6-oxo-1,6-dihydropyrimidin-2-yl]acétamide
- N-(4-fluoro-2-hydroxyphényl)-2-[4-(morpholin-4-yl)-6-oxo-1,6-dihydropyrimidin-2-yl]acétamide
- 2-[2-(2,3-dihydro-1H-indol-1-yl)-2-oxoéthyl]-6-(morpholin-4-yl)pyrimidin-4(3H)-one
- N-(3-cyclopropyl-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,3,4-trifluorophényl)acétamide.
- N-[4-fluoro3-(trifluorométhoxy)phényl]-2-[4-(morpholin-4-yl)-6-oxo-1,6-dihydropyrimidin-2-yl]acétamide
- N-[3-(2-hydroxyéthoxy)phényl]-2-[4-(morpholin-4-yl)-6-oxo-1,6-dihydropyrimidin-2-yl]acétamide
- N-(3-iodophényl)-2-[4-(morpholin-4-yl)-6-oxo-1,6-dihydropyrimidin-2-yl]acétamide
- 2-fluoro-5-({[4-(morpholin-4-yl)-6-oxo-1,6-dihydropyrimidin-2-yl]acétyl}amino)benzoate de méthyle
- N-(3-éthoxyphényl)-2-[4-(morpholin-4-yl)-6-oxo-1,6-dihydropyrimidin-2-yl]acétam ide
- N-(2,4-difluoro-3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,4,5-trifluorophényl)acétamide
- N-(3,5-dichloro-4-fluorophényl)-2-[4-(morpholin-4-yl)-6-oxo-1,6-dihydropyrimidin-2-yl]acétamide
- 2-[2-(2,3-dihydro-4H-1,4-benzoxazin-4-yl)-2-oxoéthyl]-6-(morpholin-4-yl)pyrimidin-4(3H)-one
- N-(4-fluoro-3-nitrophényl)-2-[4-(morpholin-4-yl)-6-oxo-1,6-dihydropyrimidin-2-yl]acétam ide
- acide 2-fluoro-5-({[4-(morpholin-4-yl)-6-oxo-1,6-dihydropyrimidin-2-yl]acétyl}amino)benzoïque
- N-(5-fluoro-2-hydroxyphényl)-2-[4-(morpholin-4-yl)-6-oxo-1,6-dihydropyrimidin-2-yl]acétamide
- N-(2-bromo-4-fluorophényl)-2-[4-(morpholin-4-yl)-6-oxo-1,6-dihydropyrimidin-2-yl]acétamide
- N-(4-fluorophényl)-2-[1-méthyl-4-(morpholin-4-yl)-6-oxo-1,6-dihydropyrimidin-2-yl]acétamide
- N-(3-chloro-4-fluorophényl)-2-[1-méthyl-4-(morpholin-4-yl)-6-oxo-1,6-dihydropyrimidin-2-yl]acétamide
- N-(3-bromophényl)-2-[1-méthyl-4-(morpholin-4-yl)-6-oxo-1,6-dihydropyrimidin-2-yl]acétamide
- 2-[1-éthyl-4-(morpholin-4-yl)-6-oxo-1,6-dihydropyrimidin-2-yl]-N-(4-fluorophényl)acétamide
- N-(1H-indol-4-yl)-2-[4-(morpholin-4-yl)-6-oxo-1,6-dihydropyrimidin-2-yl]acétamide
- N-(4-fluorophényl)-3-méthyl-2-[4-(morpholin-4-yl)-6-oxo-1,6-dihydropyrimidin-2-yl]butanamide
- N-[4-fluoro3-(méthoxyméthyl)phényl]-2-[4-(morpholin-4-yl)-6-oxo-1,6-dihydropyrimidin-2-yl]acétamide
- N-(4-fluoro-3-iodophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(1,1,2,2-tétrafluoroéthoxy)phényl]acétamide
- N-[3-(difluorométhyl)-4-fluorophényl]-2-[4-(morpholin-4-yl)-6-oxo-1,6-dihydropyrimidin-2-yl]acétamide
- 2,2-difluoro-N-(4-fluorophényl)-2-[4-(morpholin-4-yl)-6-oxo-1,6-dihydropyrimidin-2-yl]acétamide
- N-(3,4-difluorophényl)-2-[1-méthyl-4-(morpholin-4-yl)-6-oxo-1,6-dihydropyrimidin-2-yl]acétamide
- 2-[2-(2,3-dihydro-1H-indol-1-yl)-2-oxoéthyl]-3-méthyl-6-(morpholin-4-yl)pyrimidin-4(3H)-one
- N-(3-bromo-4-fluorophényl)-2-[1-méthyl-4-(morpholin-4-yl)-6-oxo-1,6-dihydropyrimidin-2-yl]acétamide
- N-[4-fluoro3-(hydroxyméthyl)phényl]-2-[1-méthyl-4-(morpholin-4-yl)-6-oxo-1,6-dihydropyrimidin-2-yl]acétamide
- N-(3-cyclopropylphényl)-2-[1-méthyl-4-(morpholin-4-yl)-6-oxo-1,6-dihydropyrimidin-2-yl]acétamide
- N-(4-fluoro-3-méthoxyphényl)-2-[1-méthyl-4-(morpholin-4-yl)-6-oxo-1,6-dihydropyrimidin-2-yl]acétamide
- N-(1-benzofur-4-yl)-2-[4-(morpholin-4-yl)-6-oxo-1,6-dihydropyrimidin-2-yl]acétamide
- 2-[1-méthyl-4-(morpholin-4-yl)-6-oxo-1,6-dihydropyrimidin-2-yl]-N-phénylacétamide
- N-(3-cyclopropyl-4-fluorophényl)-2-[1-méthyl-4-(morpholin-4-yl)-6-oxo-1,6-dihydropyrimidin-2-yl]acétamide
- N-(3-fluoro-2-hydroxyphényl)-2-[4-(morpholin-4-yl)-6-oxo-1,6-d ihydropyrim id in-2-yl]acétamide
- 2-[2-(4-fluoro-2,3-dihydro-1H-indol-1-yl)-2-oxoéthyl]-6-(morpholin-4-yl)pyrimidin-4(3H)-one
- 2-[2-(4-chloro-2,3-dihydro-1H-indol-1-yl)-2-oxoéthyl]-6-(morpholin-4-yl)pyrimidin-4(3H)-one
- N-(3-éthynyl-4-fluorophényl)-2-[4-(morpholin-4-yl)-6-oxo-1,6-dihydropyrimidin-2-yl]acétamide
- 2-[2-(4-hydroxy-2,3-dihydro-1H-indol-1-yl)-2-oxoéthyl]-6-(morpholin-4-yl)pyrimidin-4(3H)-one
- 2-[2-(4,6-difluoro-2,3-dihydro-1H-indol-1-yl)-2-oxoéthyl]-6-(morpholin-4-yl)pyrimidin-4(3H)-one
ainsi que les sels d'addition avec les acides minéraux et organiques ou avec les bases minérales et organiques desdits produits de formule (I).
La présente invention a encore pour objet tout procédé de préparation des produits de formule (I) tels que définis ci-dessus.

Les produits selon l'invention peuvent être préparés à partir de méthodes conventionnelles de chimie organique.

### Préparation de composés de formule (I)

Les produits de formule générale (I) selon la présente invention peuvent notamment être préparés comme indiqué dans les Schémas Généraux 1A-1C ci-dessous. A ce titre, les méthodes décrites ne sauraient constituer une limitation de la portée de l'invention, en ce qui concerne les méthodes de préparation des composés revendiqués.

Les préparations des exemples de la présente invention donnent des illustrations des schémas ci-dessous.

De tels schémas de synthèse font partie de la présente invention : la présente invention a ainsi également pour objet les procédés de préparation des produits de formule C à (I)-d tels que définis dans les Schémas Généraux 1A-1C ci-dessous.

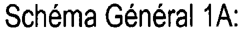

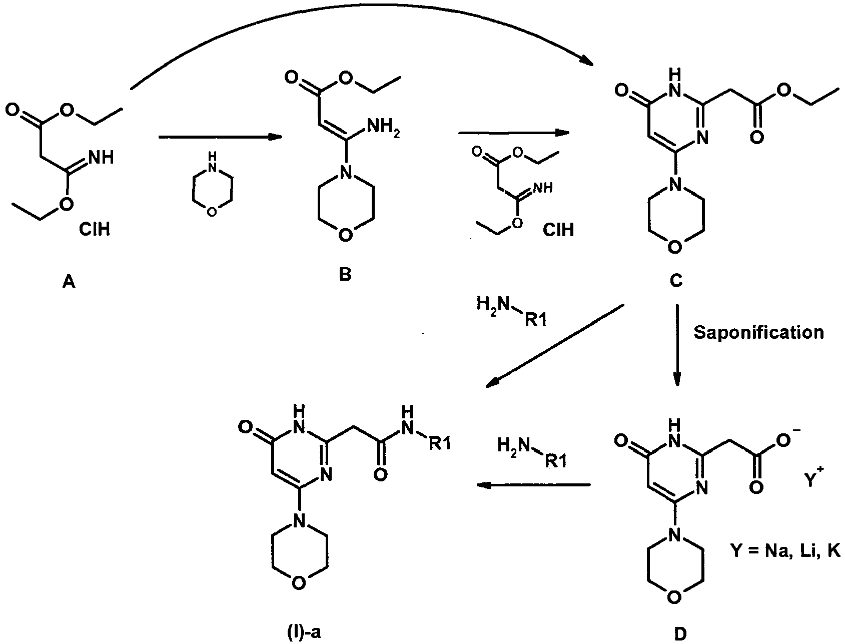

Dans le Schéma Général 1 A :
Le cétène aminal B peut être obtenu à partir de l'imino-éther A ou de son tautomère amino-acrylate commercial, par réaction avec la morpholine dans un solvant tel que l'éthanol, à une température comprise entre 0°C et la température d'ébullition du solvant, selon le procédé décrit par Landwehr J. et coll. dans J. Med. Chem. 2006, 49, 4327-4332.

L'ester C peut être obtenu par réaction du cétène aminal B avec l'imino-éther A, ou son tautomère amino-acrylate, dans un solvant tel que l'éthanol, à une température comprise entre 20°C et le point d'ébullition du solvant.

Alternativement, l'ester C peut être obtenu par réaction « one-pot » entre la morpholine et un excès (par exemple 3 équivalents) d'imino-éther A (ou de son tautomère amino-acrylate), dans un solvant tel que l'éthanol, à une température comprise entre 20°C et le point d'ébullition du solvant.

Le carboxylate D peut être obtenu par hydrolyse de l'ester C en présence d'une base telle que la soude ou la lithine, dans un solvant tel que le tétrahydrofuranne ou le méthanol, à une température comprise entre 0°C et 30°C

Les amides (I)-a peuvent être obtenus à partir du carboxylate D par condensation d'une amine R1-NH2 en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans Tetrahedron 2001, 57, 1551-1558.

Les amides (I)-a peuvent également être obtenus à partir de l'ester C par réaction d'une amine R1-NH2 en présence d'un agent tel que le triméthyl aluminium ou le tertiobutylate de potassium, dans un solvant tel que le toluène, le tétrahydrofuranne ou la N,N-diméthylformamide, à une température comprise entre 20°C et 150°C, comme par exemple dans les conditions décrites par Perreux L. et Coll. Dans Tetrahedron 2003 (59) 2185-2189 et par Auzeloux, P et coll. dans J. Med. Chem. 2000,43 (2), 190-197. 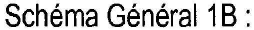 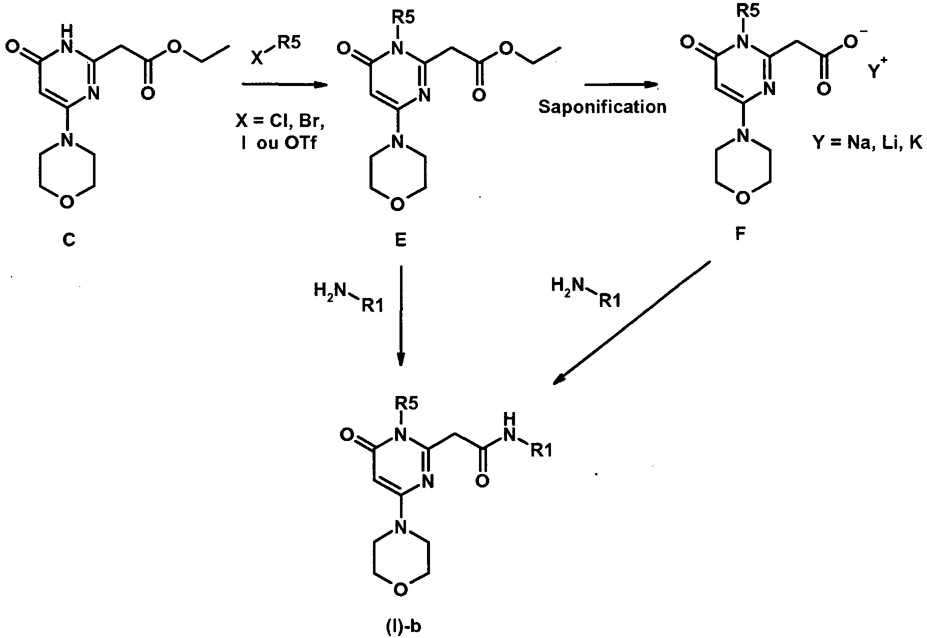 Dans le Schéma Général 1B :
Les esters E peuvent être obtenus à partir de l'ester C par réaction avec un composé R5-X (X= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans J. Med. Chem. 2008, 51, 5766-5779.

Les carboxylates F peuvent être obtenus par hydrolyse des esters E, en présence d'une base telle que la soude ou la lithine, dans un solvant tel que le tétrahydrofuranne ou le méthanol, à une température comprise entre 0°C et 30°C

Les amides (I)-b peuvent être obtenus à partir des carboxylates F par condensation d'une amine R1-NH2 en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCl [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans Tetrahedron 2001, 57, 1551-1558.

Les amides (I)-b peuvent également être obtenus à partir des esters E par réaction d'une amine R1-NH2, en présence d'un agent tel le triméthyl aluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197. 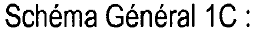 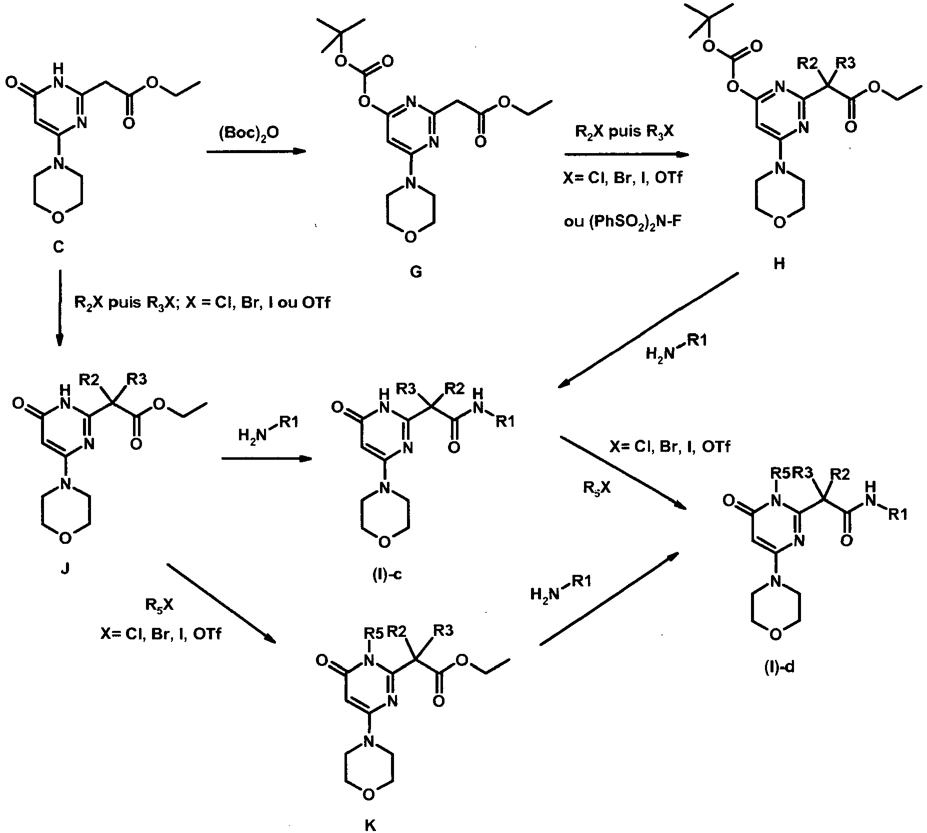 Dans le Schéma Général 1C :
l'ester G peut être obtenu à partir de l'ester C par réaction avec le (Boc)2O (dicarbonate de tertiobutyle), dans un solvant tel que la N,N-diméthylformamide, le dioxanne, l'acétonitrile ou le dichlorométhane, en présence d'une base comme par exemple, l'hydrure de sodium, la triéthylamine, la N,N-diisopropyléthylamine ou la pyridine, à une température comprise entre 0°C et 60°C, selon par exemple le procédé décrit par Hioki K. et Coll. Synthesis 2006, 12, 1931-1933

Les produits H peuvent être obtenus à partir de l'ester G par réaction avec R2-X puis éventuellement avec R3-X (X = Cl, Br, I ou OTf et R2 et R3 sont des groupes alkyles), en présence d'une base telle que la soude, le tertiobutylate de potassium ou le carbonate de césium, dans un solvant tel que le méthanol, l'éthanol ou le dioxanne, à une température comprise entre 0°C et 100°C, selon par exemple le procédé décrit par Noël D. D'Angelo et coll. dans J. Med. Chem. 2008, 51, 5766-5779.

Le produit H où R2 = R3 = F peut être obtenu par réaction du produit G avec la N-fluorobenzènesulfonimide, en présence d'une base telle que le sel de potassium de l'héxaméthyl-disilylazane, dans un solvant tel que le tétrahydrofuranne, à une température comprise entre -78°C et 20°C, selon par exemple le procédé décrit par Christopher S. Burgey et Coll. Dans J. Med. Chem. 2003, 46, 461-473.

Les esters J où les groupes R2 et R3 sont des radicaux alkyles peuvent être obtenus à partir de l'ester C de la même façon que les produits H, en présence d'une base telle que le butyllithium, l'hydrure de sodium, le tertiobutylate de potassium ou le carbonate de césium dans un solvant tel que le méthanol, l'éthanol, le tétrahydrofurane, le N,N-diméthylformamide ou le dioxanne, à une température comprise entre 0°C et 50°C.

Les amides (I)-c peuvent être obtenus à partir des esters H ou J par réaction d'une amine R1-NH2, en présence d'un agent tel le triméthyl aluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les amides (I)-d peuvent être obtenus à partir des amides (I)-c par réaction avec un composé R5-X (X= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noel D. D'Angelo et coll. dans J. Med. Chem. 2008, 51, 5766-5779.

Alternativement, les amides (I)-d peuvent être obtenus à partir des esters K par réaction d'une amine R1-NH2, en présence d'un agent tel le triméthylaluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les esters K peuvent être obtenus à partir des esters J par réaction avec un composé R5-X (X= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans J. Med. Chem. 2008, 51, 5766-5779.

Parmi les produits de départs de formule A ou B certains sont connus et peuvent être obtenus soit commercialement, soit selon les méthodes usuelles connues de l'homme du métier, par exemple à partir de produits commerciaux.
Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires.
La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyles, trityle, benzyle, tert-butoxycarbonyle, BOC, benzyloxycarbonyle, phtalimido ou d'autres radicaux connus dans la chimie des peptides,
Les fonctions acide peuvent être protégées par exemple sous forme d'esters formés avec les esters facilement clivables tels que les esters benzyliques ou ter butyliques ou des esters connus dans la chimie des peptides.
On trouvera une liste de différents groupements protecteurs utilisables dans les manuels connus de l'homme du métier et par exemple dans le brevet BF 2 499 995.

On peut noter que l'on peut soumettre, si désiré et si nécessaire, des produits intermédiaires ou des produits de formule (I) ainsi obtenus par les procédés indiqués ci-dessus, pour obtenir d'autres intermédiaires ou d'autres produits de formule (I), à une ou plusieurs réactions de transformations connues de l'homme du métier telles que par exemple :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I) ainsi obtenus étant sous toutes les formes isomères possibles racémiques, énantiomères et diastéréoisomères.
Les réactions a) à g) peuvent être réalisées dans les conditions usuelles connues de l'homme du métier telles que, par exemple, celles indiquées ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
   La réaction de saponification peut être réalisée selon les méthodes usuelles connues de l'homme du métier, telles que par exemple dans un solvant tel que le méthanol ou l'éthanol, le dioxanne ou le diméthoxyéthane, en présence de soude ou de potasse.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxanne ou l'éther éthylique.
   Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol .
g) Les éventuelles formes optiquement actives des produits décrits ci-dessus peuvent être préparées par dédoublement des racémiques selon les méthodes usuelles connues de l'homme du métier.
Les produits de formule (I) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.
Les produits de la présente invention sont notamment utiles pour la thérapie de tumeurs.

Les produits de l'invention peuvent également ainsi augmenter les effets thérapeutiques d'agents anti-tumoraux couramment utilisés.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a tout particulièrement pour objet, à titre de médicaments, les produits répondant aux formules suivantes :
- 2-[4-(morpholin-4-yl)-6-oxo-1,6-dihydropyrimidin-2-yl]-N-phénylacétamide
- N-(4-fluorophényl)-2-[4-(morpholin-4-yl)-6-oxo-1,6-dihydropyrimidin-2-yl]acétamide
- N-(3-chlorophényl)-2-[4-(morpholin-4-yl)-6-oxo-1,6-dihydropyrimidin-2-yl]acétamide
- N-[3-(diméthylamino)phényl]-2-[4-(morpholin-4-yl)-6-oxo-1,6-dihydropyrimidin-2-yl]acétamide
- N-(2,4-difluorophényl)-2-[4-(morpholin-4-yl)-6-oxo-1,6-dihydropyrimidin-2-yl]acétamide
- N-{3,4-difluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(thiophén-3-yl)acétamide
- N-(4-fluoro-3-méthoxyphényl)-2-[4-(morpholin-4-yl)-6-oxo-1,6-dihydropyrimidin-2-yl]acétamide
- N-(2-fluorophényl)-2-[4-(morpholin-4-yl)-6-oxo-1,6-dihydropyrimidin-2-yl]acétamide
- N-(2-méthylphényl)-2-[4-(morpholin-4-yl)-6-oxo-1,6-dihydropyrimidin-2-yl]acétamide
- N-(2-méthoxyphényl)-2-[4-(morpholin-4-yl)-6-oxo-1,6-dihydropyrimidin-2-yl]acétamide
- N-(2,3-difluorophényl)-2-[4-(morpholin-4-yl)-6-oxo-1,6-dihydropyrimidin-2-yl]acétamide
- N-(3,5-difluorophényl)-2-[4-(morpholin-4-yl)-6-oxo-1,6-dihydropyrimidin-2-yl]acétamide
- N-(3-fluorophényl)-2-[4-(morpholin-4-yl)-6-oxo-1,6-dihydropyrimidin-2-yl]acétamide
- N-(4-chlorophényl)-2-[4-(morpholin-4-yl)-6-oxo-1,6-dihydropyrimidin-2-yl]acétamide
- N-(3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhyl)phényl]acétamide
- N-(3-bromophényl)-2-[4-(morpholin-4-yl)-6-oxo-1,6-dihydropyrimidin-2-yl]acétam ide
- N-[3-(2-méthylpropan-2-yl)phényl]-2-[4-(morpholin-4-yl)-6-oxo-1,6-dihydropyrimidin-2-yl]acétamide
- 3-({[4-(morpholin-4-yl)-6-oxo-1,6-dihydropyrimidin-2-yl]acétyl}amino)benzoate de méthyle
- acide 3-({[4-(morpholin-4-yl)-6-oxo-1,6-dihydropyrimidin-2-yl]acétyl}amino)benzoïque
- 2-[4-(morpholin-4-yl)-6-oxo-1,6-dihydropyrimidin-2-yl]-N-[3-(propan-2-yl)phényl]acétamide
- N-(3-méthylphényl)-2-[4-(morpholin-4-yl)-6-oxo-1,6-dihydropyrimidin-2-yl]acétamide
- N-(3-cyano-4-fluorophényl)-2-[4-(morpholin-4-yl)-6-oxo-1,6-dihydropyrimidin-2-yl]acétamide
- N-(1H-indazol-6-yl)-2-[4-(morpholin-4-yl)-6-oxo-1,6-dihydropyrimidin-2-yl]acétamide
- N-(3-cyanophényl)-2-[4-(morpholin-4-yl)-6-oxo-1,6-dihydropyrimidin-2-yl]acétamide
- N-(5-fluoropyridin-2-yl)-2-[4-(morpholin-4-yl)-6-oxo-1,6-dihydropyrimidin-2-yl]acétamide
- N-(4-fluoro-3-méthylphényl)-2-[4-(morpholin-4-yl)-6-oxo-1,6-dihydropyrimidin-2-yl]acétamide
- N-(3-chloro-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(pyridin-3-yl)acétamide
- N-(4-fluoro-2-méthylphényl)-2-[4-(morpholin-4-yl)-6-oxo-1,6-dihydropyrimidin-2-yl]acétamide
- N-(3-hydroxyphényl)-2-[4-(morpholin-4-yl)-6-oxo-1,6-dihydropyrimidin-2-yl]acétamide
- N-(3-bromo-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(3,4,5-trifluorophényl)acétamide
- N-[4-fluoro3-(hydroxyméthyl)phényl]-2-[4-(morpholin-4-yl)-6-oxo-1,6-dihydropyrimidin-2-yl]acétamide
- N-(3-cyclopropylephényl)-2-[4-(morpholin-4-yl)-6-oxo-1,6-dihydropyrimidin-2-yl]acétamide
- N-(2-hydroxyphényl)-2-[4-(morpholin-4-yl)-6-oxo-1,6-dihydropyrimidin-2-yl]acétamide
- N-[3-(difluorométhoxy)phényl]-2-[4-(morpholin-4-yl)-6-oxo-1,6-dihydropyrimidin-2-yl]acétamide
- N-(4-fluoro-3-méthoxyphényl)-2-[4-(morpholin-4-yl)-6-oxo-1,6-dihydropyrimidin-2-yl]propanamide
- N-(2,3-diméthylphényl)-2-[4-(morpholin-4-yl)-6-oxo-1,6-dihydropyrimidin-2-yl]acétam ide
- N-(2-fluoro-3-méthylphényl)-2-[4-(morpholin-4-yl)-6-oxo-1,6-dihydropyrimidin-2-yl]acétamide
- N-(1,3-benzoxazol-4-yl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhoxy)phényl]acétamide
- 2-[4-(morpholin-4-yl)-6-oxo-1,6-dihydropyrimidin-2-yl]-N-[3-(propan-2-yloxy)phényl]acétamide
- N-(4-fluoro-2-méthoxyphényl)-2-[4-(morpholin-4-yl)-6-oxo-1,6-dihydropyrimidin-2-yl]acétamide
- {2-[3-({[4-(morpholin-4-yl)-6-oxo-1,6-dihydropyrimidin-2-yl]acétyl}amino)phényl]éthyl}carbamate de 2-méthylpropan-2-yle
- N-[4-fluoro3-(trifluorométhyl)phényl]-2-[4-(morpholin-4-yl)-6-oxo-1,6-dihydropyrimidin-2-yl]acétamide
- N-(3-éthynylphényl)-2-[4-(morpholin-4-yl)-6-oxo-1,6-dihydropyrimidin-2-yl]acétamide
- N-[3-(cyclopentyloxy)phényl]-2-[4-(morpholin-4-yl)-6-oxo-1,6-dihydropyrimidin-2-yl]acétamide
- N-(4-fluoro-2-hydroxyphényl)-2-[4-(morpholin-4-yl)-6-oxo-1,6-dihydropyrimidin-2-yl]acétamide
- 2-[2-(2,3-dihydro-1H-indol-1-yl)-2-oxoéthyl]-6-(morpholin-4-yl)pyrimidin-4(3H)-one
- N-(3-cyclopropyl-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,3,4-trifluorophényl)acétam ide
- N-[4-fluoro3-(trifluorométhoxy)phényl]-2-[4-(morpholin-4-yl)-6-oxo-1,6-dihydropyrimidin-2-yl]acétamide
- N-[3-(2-hydroxyéthoxy)phényl]-2-[4-(morpholin-4-yl)-6-oxo-1,6-dihydropyrimidin-2-yl]acétamide
- N-(3-iodophényl)-2-[4-(morpholin-4-yl)-6-oxo-1,6-dihydropyrimidin-2-yl]acétamide
- 2-fluoro-5-({[4-(morpholin-4-yl)-6-oxo-1,6-dihydropyrimidin-2-yl]acétyl}amino)benzoate de méthyle
- N-(3-éthoxyphényl)-2-[4-(morpholin-4-yl)-6-oxo-1,6-dihydropyrimidin-2-yl]acétamide
- N-(2,4-difluoro-3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,4,5-trifluorophényl)acétamide
- N-(3,5-dichloro-4-fluorophényl)-2-[4-(morpholin-4-yl)-6-oxo-1,6-dihydropyrimidin-2-yl]acétamide
- 2-[2-(2,3-dihydro-4H-1,4-benzoxazin-4-yl)-2-oxoéthyl]-6-(morpholin-4-yl)pyrimidin-4(3H)-one
- N-(4-fluoro-3-nitrophényl)-2-[4-(morpholin-4-yl)-6-oxo-1,6-dihydropyrimidin-2-yl]acétamide
- acide 2-fluoro-5-({[4-(morpholin-4-yl)-6-oxo-1,6-dihydropyrimidin-2-yl]acétyl}amino)benzoïque
- N-(5-fluoro-2-hydroxyphényl)-2-[4-(morpholin-4-yl)-6-oxo-1,6-dihydropyrimidin-2-yl]acétamide
- N-(2-bromo-4-fluorophényl)-2-[4-(morpholin-4-yl)-6-oxo-1,6-dihydropyrimidin-2-yl]acétamide
- N-(4-fluorophényl)-2-[1-méthyl-4-(morpholin-4-yl)-6-oxo-1,6-dihydropyrimidin-2-yl]acétamide
- N-(3-chloro-4-fluorophényl)-2-[1-méthyl-4-(morpholin-4-yl)-6-oxo-1,6-dihydropyrimidin-2-yl]acétamide
- N-(3-bromophényl)-2-[1-méthyl-4-(morpholin-4-yl)-6-oxo-1,6-dihydropyrimidin-2-yl]acétamide
- 2-[1-éthyl-4-(morpholin-4-yl)-6-oxo-1,6-dihydropyrimidin-2-yl]-N-(4-fluorophényl)acétamide
- N-(1H-indol-4-yl)-2-[4-(morpholin-4-yl)-6-oxo-1,6-dihydropyrimidin-2-yl]acétamide
- N-(4-fluorophényl)-3-méthyl-2-[4-(morpholin-4-yl)-6-oxo-1,6-dihydropyrimidin-2-yl]butanamide
- N-[4-fluoro3-(méthoxyméthyl)phényl]-2-[4-(morpholin-4-yl)-6-oxo-1,6-dihydropyrimidin-2-yl]acétamide
- N-(4-fluoro-3-iodophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(1,1,2,2-tétrafluoroéthoxy)phényl]acétamide
- N-[3-(difluorométhyl)-4-fluorophényl]-2-[4-(morpholin-4-yl)-6-oxo-1,6-dihydropyrimidin-2-yl]acétamide
- 2,2-difluoro-N-(4-fluorophényl)-2-[4-(morpholin-4-yl)-6-oxo-1,6-dihydropyrimidin-2-yl]acétamide
- N-(3,4-difluorophényl)-2-[1-méthyl-4-(morpholin-4-yl)-6-oxo-1 ,6-dihydropyrimidin-2-yl]acétamide
- 2-[2-(2,3-dihydro-1 H-indol-1-yl)-2-oxoéthyl]-3-méthyl-6-(morpholin-4-yl)pyrimidin-4(3H)-one
- N-(3-bromo-4-fluorophényl)-2-[1-méthyl-4-(morpholin-4-yl)-6-oxo-1,6-dihydropyrimidin-2-yl]acétamide
- N-[4-fluoro3-(hydroxyméthyl)phényl]-2-[1-méthyl-4-(morpholin-4-yl)-6-oxo-1,6-dihydropyrimidin-2-yl]acétamide
- N-(3-cyclopropylphényl)-2-[1-méthyl-4-(morpholin-4-yl)-6-oxo-1,6-dihydropyrimidin-2-yl]acétamide
- N-(4-fluoro-3-méthoxyphényl)-2-[1-méthyl-4-(morpholin-4-yl)-6-oxo-1,6-dihydropyrimidin-2-yl]acétamide
- N-(1-benzofur-4-yl)-2-[4-(morpholin-4-yl)-6-oxo-1,6-dihydropyrimidin-2-yl]acétamide
- 2-[1-méthyl-4-(morpholin-4-yl)-6-oxo-1,6-dihydropyrimidin-2-yl]-N-phénylacétamide
- N-(3-cyclopropyl-4-fluorophényl)-2-[1-méthyl-4-(morpholin-4-yl)-6-oxo-1,6-dihydropyrimidin-2-yl]acétamide
- N-(3-fluoro-2-hydroxyphényl)-2-[4-(morpholin-4-yl)-6-oxo-1,6-dihydropyrimidin-2-yl]acétamide
- 2-[2-(4-fluoro-2,3-dihydro-1 H-indol-1-yl)-2-oxoéthyl]-6-(morpholin-4-yl)pyrimidin-4(3H)-one
- 2-[2-(4-chloro-2,3-dihydro-1H-indol-1-yl)-2-oxoéthyl]-6-(morpholin-4-yl)pyrimidin-4(3H)-one
- N-(3-éthynyl-4-fluorophényl)-2-[4-(morpholin-4-yl)-6-oxo-1,6-dihydropyrimidin-2-yl]acétamide
- 2-[2-(4-hydroxy-2,3-dihydro-1H-indol-1-yl)-2-oxoéthyl]-6-(morpholin-4-yl)pyrimidin-4(3H)-one
- 2-[2-(4,6-difluoro-2,3-dihydro-1H-indol-1-yl)-2-oxoéthyl]-6-(morpholin-4-yl)pyrimidin-4(3H)-one
- N-(4-Fluoro-3-iodo-phenyl)-2-(1-methyl-4-morpholin-4-yl-6-oxo-1,6-dihydro-pyrimid in-2-yl)-acetamide
- 2-[2-(4,5-difluoro-2,3-dihydro-1H-indol-1-yl)-2-oxoéthyl]-6-(morpholin-4-yl)pyrimidin-4(3H)-one
- 2-[2-(6-fluoro-2,3-dihydro-1H-indol-1-yl)-2-oxoéthyl]-6-(morpholin-4-yl)pyrimidin-4(3H)-one
- 2-[2-(2-méthyl-2,3-dihydro-1 H-indol-1-yl)-2-oxoéthyl]-6-(morpholin-4-yl)pyrimidin-4(3H)-one
- N-[3-(difluorométhyl)-4-fluorophényl]-2-[1-méthyl-4-{morpholin-4-yl)-6-oxo-1,6-dihydropyrimidin-2-yl]acétamide
- 2-[1-methyl-4-(morpholin-4-yl)-6-oxo-1,6-dihydropyrimidin-2-yl]-N-(3,4,5-trifluorophenyl)acetamide
- N-(1-methyl-1H-indol-4-yl)-2-[4-(morpholin-4-yl)-6-oxo-1,6-dihydropyrimidin-2-yl]acetamide
- 2-[2-(4-methyl-2,3-dihydro-1H-indol-1-yl)-2-oxoethyl]-6-(morpholin-4-yl)pyrimidin-4(3H)-one
- 2-[2-(3-methyl-2,3-dihydro-1H-indol-1-yl)-2-oxoethyl]-6-(morpholin-4-yl)pyrimidin-4(3H)-one
- 2-[2-(4-fluoro-2,3-dihydro-1H-indol-1-yl)-2-oxoethyl]-3-methyl-6-(morpholin-4-yl)pyrimidin-4(3H)-one
- 2-[2-(5-Fluoro-2,3-dihydro-indol-1-yl)-2-oxo-ethyl]-6-morpholin-4-yl-3H-pyrimidin-4-one
- 2-[2-(4-chloro-2,3-dihydro-1H-indol-1-yl)-2-oxoethyl]-3-methyl-6-(morpholin-4-yl)pyrimidin-4(3H)-one
- N-(1-benzothiophen-4-yl)-2-[4-(morpholin-4-yl)-6-oxo-1,6-dihydropyrimidin-2-yl]acetamide
- 2-{2-[2-(hydroxymethyl)-2,3-dihydro-1H-indol-1-yl]-2-oxoethyl}-6-(morpholin-4-yl)pyrimidin-4(3H)-one
- 2-[4-(morpholin-4-yl)-6-oxo-1,6-dihydropyrimidin-2-yl]-N-{2-[2-(piperidin-1-yl)ethoxy]phenyl}acetamide
- N-[2-(2-methoxyethoxy)phenyl]-2-[4-(morpholin-4-yl)-6-oxo-1,6-dihydropyrimidin-2-yl]acetamide
- 2-[2-(4-hydroxy-2,3-dihydro-1H-indol-1-yl)-2-oxoethyl]-3-methyl-6-(morpholin-4-yl)pyrimidin-4(3H)-one
- 2-[2-(4-methoxy-2,3-dihydro-1H-indol-1-yl)-2-oxoethyl]-3-methyl-6-(morpholin-4-yl)pyrimidin-4(3H)-one
- 2-[2-(3,4-dihydroisoquinolin-2(1H)-yl)-2-oxoethyl]-6-(morpholin-4-yl)pyrimidin-4(3H)-one
- 2-[4-(morpholin-4-yl)-6-oxo-1,6-dihydropyrimidin-2-yl]-N-{2-[2-(pyrrolidin-1-yl)ethoxy]phenyl}acetamide
- 2-[4-(morpholin-4-yl)-6-oxo-1,6-dihydropyrimidin-2-yl]-N-[2-(pyridin-3-ylmethoxy)phenyl]acetamide
- 3-methyl-2-[2-(4-methyl-2,3-dihydro-1H-indol-1-yl)-2-oxoethyl]-6-(morpholin-4-yl)pyrimidin-4(3H)-one
- 2-(2-{3-[(dimethylamino)methyl]-2,3-dihydro-1H-indol-1-yl}-2-oxoethyl)-6-(morpholin-4-yl)pyrimidin-4(3H)-one
- 2-[2-(4-bromo-2,3-dihydro-1H-indol-1-yl)-2-oxoethyl]-3-methyl-6-(morpholin-4-yl)pyrimidin-4(3H)-one
- 2-{2-[(2S)-2-methyl-2,3-dihydro-1H-indol-1-yl]-2-oxoethyl}-6-(morpholin-4-yl)pyrimidin-4(3H)-one
- 2-{2-[(2R)-2-methyl-2,3-dihydro-1H-indol-1-yl]-2-oxoethyl}-6-(morpholin-4-yl)pyrimidin-4(3H)-one
- 3-methyl-2-[2-(3-methyl-2,3-dihydro-1H-indol-1-yl)-2-oxoethyl]-6-(morpholin-4-yl)pyrimidin-4(3H)-one
- 2-{2-[2-(methoxymethyl)-2,3-dihydro-1H-indol-1-yl]-2-oxoethyl}-6-(morpholin-4-yl)pyrimidin-4(3H)-one
- 2-[2-(4-ethoxy-2,3-dihydro-1 H-indol-1-yl)-2-oxoethyl]-6-(morpholin-4-yl)pyrimidin-4(3H)-one
- 1-{[4-(morpholin-4-yl)-6-oxo-1,6-dihydropyrimidin-2-yl]acetyl}-2,3-dihydro-1H-indole-2-carboxamide
- 3-methyl-2-[2-(2-methyl-2,3-dihydro-1H-indol-1-yl)-2-oxoethyl]-6-(morpholin-4-yl)pyrimidin-4(3H)-one
- 2-[2-(6-fluoro-2,3-dihydro-1H-indol-1-yl)-2-oxoethyl]-3-methyl-6-(morpholin-4-yl)pyrimidin-4(3H)-one
- 2-{2-[(3S)-3-methyl-2,3-dihydro-1H-indol-1-yl]-2-oxoethyl}-6-(morpholin-4-yl)pyrimidin-4(3H)-one
- 2-{2-[(3R)-3-methyl-2,3-dihydro-1H-indol-1-yl]-2-oxoethyl}-6-(morpholin-4-yl)pyrimidin-4(3H)-one
- 2-[2-(5,6-difluoro-2,3-dihydro-1H-indol-1-yl)-2-oxoethyl]-3-methyl-6-(morpholin-4-yl)pyrimidin-4(3H)-one
- 2-[2-(4,5-difluoro-2,3-dihydro-1H-indol-1-yl)-2-oxoethyl]-3-methyl-6-(morpholin-4-yl)pyrimidin-4(3H)-one
- 2-[2-(1,3-dihydro-2H-isoindol-2-yl)-2-oxoethyl]-6-(morpholin-4-yl)pyrimidin-4(3H)-one
- N-(1-benzothiophen-4-yl)-2-[1-methyl-4-(morpholin-4-yl)-6-oxo-1,6-dihydropyrimidin-2-yl]acetamide
- 2-[2-(5-chloro-3,4-dihydroquinolin-1 (2H)-yl)-2-oxoethyl]-6-(morpholin-4-yl)pyrimidin-4(3H)-one
- 2-{2-[4-(hydroxymethyl)-2,3-dihydro-1H-indol-1-yl]-2-oxoethyl}-6-(morpholin-4-yl)pyrimidin-4(3H)-one
- N-[4-fluoro-2-(piperidin-4-ylmethoxy)phenyl]-2-[4-(morpholin-4-yl)-6-oxo-1,6-dihydropyrimidin-2-yl]acetamide
- 2-[2-(5-Chloro-2,3-dihydro-indol-1-yl)-2-oxo-ethyl]-6-morpholin-4-yl-3H-pyrimidin-4-one
- 2-[2-(4-Bromo-2,3-dihydro-indol-1-yl)-2-oxo-ethyl]-6-morpholin-4-yl-3H-pyrimidin-4-one
- 2-(2-{(3S)-3-[(dimethylamino)methyl]-2,3-dihydro-1H-indol-1-yl}-2-oxoethyl)-6-(morpholin-4-yl)pyrimidin-4(3H)-one
- 2-(2-{(3R)-3-[(dimethylamino)methyl]-2,3-dihydro-1H-indol-1-yl}-2-oxoethyl)-6-(morpholin-4-yl)pyrimidin-4(3H)-one
- N-[4-fluoro-2-(2-methoxyethoxy)phenyl]-2-[4-(morpholin-4-yl)-6-oxo-1,6-dihydropyrimidin-2-yl]acetamide
- N-(1H-benzimidazol-4-yl)-2-(4-(morpholin-4-yl)-6-oxo-1,6-dihydropyrimidin-2-yl]acetamide
- methyl2-hydroxy-3-({[4-(morpholin-4-yl)-6-oxo-1,6-dihydropyrimidin-2-yl]acetyl}amino)benzoate
- 2-[2-(4-Methoxy-2,3-dihydro-indol-1-yl)-2-oxo-ethyl]-6-morpholin-4-yl-3H-pyrimidin-4-one
- N-(3-bromo-2-hydroxyphenyl)-2-[4-(morpholin-4-yl)-6-oxo-1,6-dihydropyrimidin-2-yl]acetamide
- N-(3,4-dihydro-2H-1,4-benzoxazin-8-yl)-2-[4-(morpholin-4-yl)-6-oxo-1,6-dihydropyrimidin-2-yl]acetamide
- methyl 5-fluoro-2-({[4-(morpholin-4-yl)-6-oxo-1,6-dihydropyrimidin-2-yl]acetyl}amino)benzoate
- 2-(2-{3-[(diethylamino)methyl]-2,3-dihydro-1H-indol-1-yl}-2-oxoethyl)-6-(morpholin-4-yl)pyrimidin-4(3H)-one
- 2-[2-(2,3-dihydro-1H-pyrrolo[3,2-b]pyridin-1-yl)-2-oxoethyl]-6-(morpholin-4-yl)pyrimidin-4(3H)-one
- 2-[2-(5,6-Difluoro-2,3-dihydro-indol-1-yl)-2-oxo-ethyl]-6-morpholin-4-yl-3H-pyrimidin-4-one
- 2-[4-(morpholin-4-yl)-6-oxo-1,6-dihydropyrimidin-2-yl]-N-(1,2,3,4-tetrahydroquinolin-8-yl)acetamide
- 2-[2-(8-chloro-2,3-dihydro-4H-1,4-benzoxazin-4-yl)-2-oxoéthyl]-6-(morpholin-4-yl)pyrimidin-4(3H)-one
- N-(2-hydroxy-3-méthylphényl)-2-[4-(morpholin-4-yl)-6-oxo-1,6-dihydropyrimidin-2-yl]acétamide
- N-(2-hydroxy-3-nitrophényl)-2-[4-(morpholin-4-yl)-6-oxo-1,6-dihydropyrimidin-2-yl]acétamide
- N-(3-cyano-2-hydroxyphényl)-2-[4-(morpholin-4-yl)-6-oxo-1,6-dihydropyrimidin-2-yl]acétamide
- N-[2-hydroxy-3-(trifluorométhyl)phényl]-2-[4-(morpholin-4-yl)-6-oxo-1,6-dihydropyrimidin-2-yl]acétamide
- 2-[2-(3,3-diméthyl-2,3-dihydro-1H-indol-1-yl)-2-oxoéthyl]-6-(morpholin-4-yl)pyrimidin-4(3H)-one
ainsi que les sels d'addition avec les acides minéraux et organiques ou avec les bases minérales et organiques pharmaceutiquement acceptables desdits produits de formule (I).

L'invention concerne aussi des compositions pharmaceutiques contenant à titre de principe actif l'un au moins des produits de formule (I) tels que définis ci-dessus ou un sel pharmaceutiquement acceptable de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

Un tel médicament peut notamment être destiné au traitement ou à la prévention d'une maladie chez un mammifère.

Est également décrit l'utilisation d'un produit de formule (I) tel que défini ci-dessus pour la préparation d'un médicament destiné à la prévention ou an traitement de maladies liées à une prolifération non contrôlée.

La présente invention a ainsi tout particulièrement pour objet l'utilisation d'un produit de formule (I) tel que défini ci-dessus pour la préparation d'un médicament destiné au traitement ou à la prévention de maladies en oncologie et notament destiné au traitement de cancers.

Parmi ces cancers, on s'intéresse au traitement de tumeurs solides ou liquides, au traitement de cancers résistant à des agents cytotoxiques Les produits de la présente invention cités peuvent notamment être utilisés pour le traitement de tumeurs primaires et/ou de métastases en particulier dans les cancers gastriques, hépatiques, rénaux, ovariens, du colon, de la prostate, de l'endométre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

La présente invention a aussi pour objet l'utilisation des produits de formule (I) telle que définie ci-dessus pour la préparation de médicaments destinés à la chimiothérapie de cancers.
La présente invention a ainsi pour objet les produits de formule (I) tels que définis ci-dessus pour leur utilisation pour le traitement de cancers.
La présente invention a pour objet les produit de formule (I) tels que définis ci-dessus pour leur utilisation pour le traitement de tumeurs solides ou liquides.
La présente invention a donc pour objet les produits de formule (I) tels que définis ci-dessus pour leur utilisation pour le traitement de cancers résistant à des agents cytotoxiques.
La présente invention a donc pour objet les produits de formule (I) tels que définis ci-dessus pour leur utilisation pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.
La présente invention a donc pour objet les produits de formule (I) tels que définis ci-dessus pour leur utilisation pour la chimiothérapie de cancers.
De tels médicaments destinés à la chimiothérapie de cancers peuvent être utilisés seuls ou en en association.
La présente invention a donc pour objet les produits de formule (I) tels que définis ci-dessus pour leur utilisation pour la chimiothérapie de cancers, seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

La présente invention a encore pour objet à titre de produits industriels nouveaux, les intermédiaires de synthèse de formules C, D, E et F tels que définis ci-dessus et rappelés ci-après : 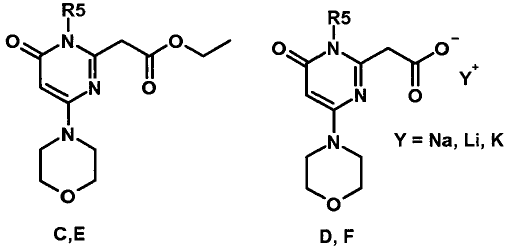 dans lesquels R5 a la définition indiquée à l'une quelconque des revendications 1 à 2.

Les exemples suivants qui sont des produits de formule (I) illustrent l'invention sans toutefois la limiter.

### Partie expérimentale

La nomenclature des composés de cette présente invention a été effectuée avec le logiciel ACDLABS version 10.0.

Le four à microondes utilisé est un appareil Biotage, Initiator^{™} 2.0, 400W max, 2450 MHz.

Les spectres de RMN ¹H à 400 MHz et ¹H à 500 MHz ont été effectués sur spectromètre BRUKER AVANCE DRX-400 ou BRUKER AVANCE DPX-500 avec les déplacements chimiques (δ en ppm) dans le solvant diméthylsulfoxide-d₆ (DMSO-d₆) référencé à 2,5 ppm à la température de 303K.

Les spectres de masse (SM) ont été obtenus soit par la méthode A, soit par la méthode B :

### Méthode A :

Appareil WATERS UPLC-SQD ; Ionisation: électrospray en mode positif et/ou négatif (ES+/-) ; Conditions chromatographiques : Colonne : ACQUITY BEH C18 1,7 µm - 2,1 x 50 mm ; Solvants : A : H₂O (0,1 % acide formique) B : CH₃CN (0,1 % acide formique) ; Température de colonne : 50 °C ; Débit : 1 ml/min ; Gradient (2 min) : de 5 à 50 % de B en 0,8 min ; 1,2 min : 100 % de B ; 1,85 min : 100 % de B ; 1,95 : 5 % de B ; Temps de rétention = Tr (min).

### Méthode B :

Appareil WATERS ZQ ; Ionisation : électrospray en mode positif et/ou négatif (ES+/-) ; Conditions chromatographiques : Colonne : XBridge C18 2,5 µm - 3 x 50 mm ; Solvants : A : H2O (0,1 % acide formique) B : CH3CN (0,1 % acide formique) ; Température de colonne : 70°C ; Débit : 0,9 ml/min ; Gradient (7 min) : de 5 à 100 % de B en 5,3 min ; 5,5 min : 100 % de B ; 6,3 min : 5 % de B ; Temps de rétention = Tr (min).

### Exemple 1 : Synthèse du 2-[4-(morpholin-4-yl)-6-oxo-1,6-dihydropyrimidin-2-yl]-N-phénylacétamide

### Etape 1 :

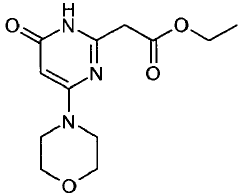

A une solution de 25 g de morpholine dans 400 mL d'éthanol chauffée à 95 °C sont ajoutés 168.5 mL de chlorhydrate de 3-éthoxy-3-iminopropanoate d'éthyle, puis 155 mL de N,N-diisopropyléthylamine dans 200 mL d'éthanol. Le mélange réactionnel est chauffé à 95 °C pendant 30 heures puis laissé revenir à température ambiante. Le précipité formé est filtré sur verre fritté puis lavé avec 100 mL d'éthanol, 2 fois 500 mL d'eau et enfin 500 mL d'éther éthylique. Le solide est séché sous vide pour donner 35 g de [4-(morpholin-4-yl)-6-oxo-1,6-dihydropyrimidin-2-yl]acétate d'éthyle sous forme de solide blanc dont les caractéristiques sont les suivantes :
Spectre RMN 1 H (400MHz) : 1,19 (t, J=7,1 Hz, 3 H); 3,38 à 3,44 (m, 4 H); 3,56 (s, 2H); 3,61 (dd, J=4,0 et 5,7 Hz, 4 H); 4,12 (q, J=7,1 Hz, 2 H); 5,20 (s, 1 H); 11,69 (s large, 1 H)
Spectrométrie de Masse : méthode A
Temps de rétention Tr (min) = 0.48;
[M+H]+ : m/z 268 ; [M-H]- : m/z 266

### Etape 2:

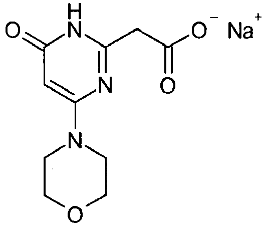

A une solution de 10 g de [4-(morpholin-4-yl)-6-oxo-1,6-dihydropyrimidin-2-yl]acétate d'éthyle préparé à l'étape 1 de l'exemple 1 dans 300 mL de tétrahydrofuranne, est ajouté 18.7 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif pour donner 8.7 g de [4-(morpholin-4-yl)-6-oxo-1,6-dihydropyrimidin-2-yl]acétate de sodium sous forme de solide blanc dont les caractéristiques sont les suivantes:
Spectre RMN 1 H :3,08 (s, 2 H); 3,38 (t, J=4,6 Hz, 4 H); 3,61 (t, J=4,6Hz, 4 H); 5,08 (s, 1 H); 13,16 (s large, 1 H)
Spectrométrie de Masse : méthode A
Temps de rétention Tr (min) = 0.29;
[M+H]+ : m/z 240 ; [M-H]- : m/z 238

### Etape 2':

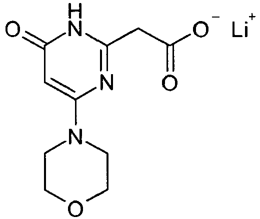

A une solution de 2 g de [4-(morpholin-4-yl)-6-oxo-1,6-dihydropyrimidin-2-yl]acétate d'éthyle préparé à l'étape 1 de l'exemple 1 dans 75 mL de méthanol, est ajouté 7.5 mL d'eau et 197 mg d'hydroxyde de lithium. Après 48 heures d'agitation à température ambiante le mélange réactionnel est concentré sous pression réduite. On ajoute 50 mL d'eau. La phase aqueuse est alors lavée à l'acétate d'éthyle puis lyophilisée. On obtient 1.73 g de [4-(morpholin-4-yl)-6-oxo-1,6-dihydropyrimidin-2-yl]acétate de lithium sous forme de solide blanc, dont les caractéristiques sont similaires au produit de l'étape 2.

### Etape 3 :

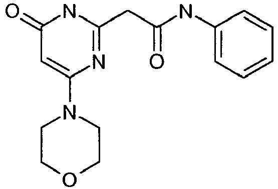

A une solution de 200 mg de [4-(morpholin-4-yl)-6-oxo-1,6-dihydropyrimidin-2-yl]acétate de sodium préparé à l'étape 2 de l'exemple 1 dans 3 mL de N,N-diméthylformamide sont ajoutés 370 mg de benzotriazol-1-yloxytris(dimethylamino)phosphonium hexafluorophosphate, 113 mg de 1-hydroxybenzotriazole et 0.140 mL d'aniline. Le mélange réactionnel est agité à température ambiante pendant 3 heures, puis concentré sous pression réduite. On ajoute de l'eau et de l'acétate d'éthyle et agite ainsi pendant 30 minutes. Le précipité formé est filtré et séché à l'évaporateur rotatif. Après purification par chromatographie sur colonne de silice, éluant : dichlorométhane/méthanol 90/10, on obtient 161 mg du 2-[4-(morpholin-4-yl)-6-oxo-1,6-dihydropyrimidin-2-yl]-N-phénylacétamide sous forme de solide blanc dont les caractéristiques sont les suivantes:
Spectre RMN 1 H :3,38 à 3,44 (m, 4 H); 3,56 à 3,63 (m, 6 H); 5,20 (s, 1 H); 7,06 (t, J=7,8 Hz, 1 H); 7,31 (t, J=8,6 Hz, 2 H); 7,56 (d, J=8,6 Hz, 2 H); 10,14 (s, 1 H); 11,64 (s large, 1 H)
Spectrométrie de Masse : méthode A
Temps de rétention Tr (min) = 0.55;
[M+H]+ : m/z 315 ; [M-H]- : m/z 313

### Exemple 2 : Synthèse du N-(4-fluorophényl)-2-[4-(morpholin-4-yl)-6-oxo-1,6-dihydropyrimidin-2-yl]acétamide

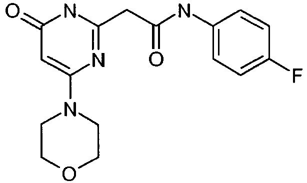

Le produit est préparé en suivant le mode opératoire décrit à l'étape 3 de l'exemple 1 à partir de 300 mg de [4-(morpholin-4-yl)-6-oxo-1,6-dihydropyrimidin-2-yl]acétate de sodium préparé à l'étape 2 de l'exemple 1, 595 mg de benzotriazol-1-yloxytris(dimethylamino)phosphonium hexafluorophosphate, 182 mg de 1-hydroxybenzotriazole et 0.235 mL de 4-fluoroaniline au lieu de l'aniline. On obtient 110 mg du N-(4-fluorophényl)-2-[4-(morpholin-4-yl)-6-oxo-1,6-dihydropyrimidin-2-yl]acétamide sous forme de solide blanc cassé dont les caractéristiques sont les suivantes:
Spectre RMN 1 H :3,41 (t, J=4,9 Hz, 4 H); 3,55 à 3,64 (m, 6 H); 5,20 (s, 1 H); 7,15 (t, J=8,9 Hz, 2 H); 7,58 (dd, J=5,6 et 9,0 Hz, 2 H); 10,20 (s, 1 H); 11,65 (slarge, 1 H)
Spectrométrie de Masse : méthode B
Temps de rétention Tr (min) = 2.86;
[M+H]+ : m/z 333 ; [M-H]- : m/z 331

### Exemple 3 : Synthèse du N-(3-chlorophényl)-2-[4-(morpholin-4-yl)-6-oxo-1,6-dihydropyrimidin-2-yl]acétamide

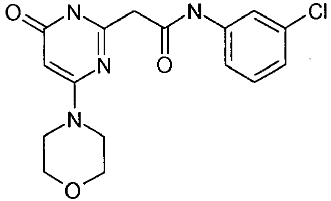

Le produit est préparé en suivant le mode opératoire décrit à l'étape 3 de l'exemple 1 à partir de 300 mg de [4-(morpholin-4-yl)-6-oxo-1,6-dihydropyrimidin-2-yl]acétate de lithium obtenu à étape 2' de l'exemple 1 et 0.235 mL de 3-chlororoaniline au lieu de l'aniline. Après purification par chromatographie sur colonne de silice, éluant : dichlorométhane/méthanol/acétonitrile 90/5/5, on obtient 71 mg du N-(3-chlorophényl)-2-[4-(morpholin-4-yl)-6-oxo-1,6-dihydropyrimidin-2-yl]acétamide dont les caractéristiques sont les suivantes:
Spectre RMN 1 H :3,42 (d, J=5,1 Hz, 4 H); 3,53 à 3,69 (m, 6 H); 5,20 (s,1 H); 7,12 (d, J=9,0 Hz, 1 H); 7,34 (t, J=8,1 Hz, 1 H); 7,42 (dt, J=1,3 et 7,8 Hz, 1 H);7,77 (t, J=2,1 Hz, 1 H); 10,34 (s large, 1 H); 11,66 (s large, 1 H)
Spectrométrie de Masse : méthode B
Temps de rétention Tr (min) = 0.68;
[M+H]+ : m/z 349 ; [M-H]- : m/z 347

### Exemple 4: Synthèse du N-[3-(diméthylamino)phényl]-2-[4-(morpholin-4-yl)-6-oxo-1,6-dihydropyrimidin-2-yl]acétamide

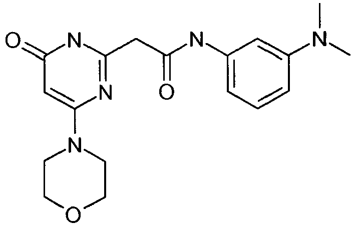

A une solution de 250 mg de [4-(morpholin-4-yl)-6-oxo-1,6-dihydropyrimidin-2-yl]acétate de sodium préparé à l'étape 2 de l'exemple 1 dans 4 mL de N,N-diméthylformamide sont ajoutés 0.160 mL de pyridine, 240 mg de N-[3-(diméthylamino)propyl]-N'-éthylcarbodiimide chlorhydrate et 400 mg de N,N-diméthyl-m-phénylènediamine. On agite à température ambiante pendant 1 nuit, puis le mélange réactionnel est concentré sous pression réduite. On ajoute de l'eau et de l'acétate d'éthyle et agite ainsi pendant 30 minutes. Le précipité formé est filtré, rincé à l'éther éthylique et séché à l'évaporateur rotatif. Après purification par chromatographie sur colonne de silice en dépôt solide, éluant : dichlorométhane/méthanol 95/05, on obtient un solide que l'on reprend dans un mélange de dichlorométhane, de méthanol et d'éther éthylique. Le solide est filtré et séché. On obtient 30 mg de N-[3-(diméthylamino)phényl]-2-[4-(morpholin-4-yl)-6-oxo-1 ,6-dihydropyrimidin-2-yl]acétamide sous forme de solide jaune dont les caractéristiques sont les suivantes:
Spectre RMN 1 H :2,86 (s, 6 H); 3,41 (t, J=5,0 Hz, 4 H); 3,57 (s, 2 H);3,60 (t, J=4,9 Hz, 4 H); 5,19 (s, 1 H); 6,44 (d, J=9,3 Hz, 1 H); 6,86 (d, J=7,8 Hz, 1 H);7,01 (s, 1 H); 7,09(t, J=8,3 Hz, 1 H); 9,98 (s large, 1 H); 11,61 (s large, 1 H)
Spectrométrie de Masse : méthode B
Temps de rétention Tr (min) =0.40;
[M+H]+ : m/z 358 ; [M-H]- : m/z 356

### Exemple 5 : Synthèse du N-(2,4-difluorophényl)-2-[4-(morpholin-4-yl)-6-oxo-1,6-dihydropyrimidin-2-yl]acétamide

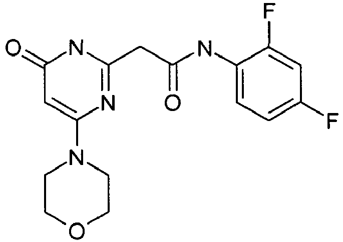

A une solution de 260 mg de [4-(morpholin-4-yl)-6-oxo-1,6-dihydropyrimidin-2-yl]acétate de sodium préparé à l'étape 2 de l'exemple 1 dans 2 mL de N,N-diméthylformamide sont ajoutés 2.5 mL de pyridine, 233 mg de N-[3-(diméthylamino)propyl]-N'-éthylcarbodiimide chlorhydrate et 400 mg de 2,4-difluoroaniline. Le mélange réactionnel est agité à température ambiante pendant 16 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 205 mg de N-(2,4-difluorophényl)-2-[4-(morpholin-4-yl)-6-oxo-1,6-dihydropyrimidin-2-yl]acétamide sous forme de solide rosé dont les caractéristiques sont les suivantes:
Spectre RMN 1 H :3,43 (t, J=5,0 Hz, 4 H); 3,61 (t, J=5,0 Hz, 4 H); 3,66(s, 2 H); 5,20 (s, 1 H); 7,01 à 7,13 (m, 1 H); 7,25 à 7,40 (m, 1 H); 7,83 (q, J=7,1 Hz, 1 H); 9,97 (s large, 1 H); 11,67 (s large, 1 H)
Spectrométrie de Masse : méthode B
Temps de rétention Tr (min) = 0.59;
[M+H]+ : m/z 351 ; [M-H]- : m/z 349

### Exemple 6 : Synthèse du N-(3,4-difluorophényl)-2-[4-(morpholin-4-yl)-6-oxo-1,6-dihydropyrimidin-2-yl]acétamide

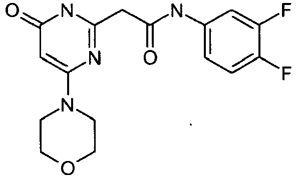

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 400 mg de 3,4-difluoroaniline au lieu de la 2,4-difluoroaniline. On obtient 210 mg de N-(3,4-difluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz) : 3,41 (t, J=4,9 Hz, 4 H); 3,54 à 3,64 (m, 6 H); 5,20 (s, 1 H); 7,27 (d, J=8,8 Hz,1 H); 7,39 (dt, J=9,0 et 10,6 Hz, 1 H); 7,64 à 7,79 (m, 1 H);10,38 (s, 1 H); 11,66 (s large, 1 H)
Spectrométrie de Masse : méthode A
Temps de rétention Tr (min) = 0.64;
[M+H]+ : m/z 351 ; [M-H]- : m/z 349

### Exemple 7: Synthèse du 2-[4-(morpholin-4-yl)-6-oxo-1,6-dihydropyrimidin-2-yl]-N-(thiophèn-3-yl)acétamide

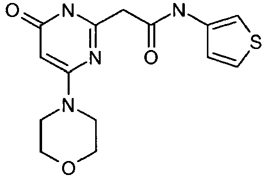

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 400 mg de 3-aminothiophène chlorhydrate au lieu de la 2,4-difluoroaniline. On obtient 252 mg de 2-[4-(morpholin-4-yl)-6-oxo-1,6-dihydropyrimidin-2-yl]-N-(thiophén-3-yl)acétamide sous forme de solide beige dont les caractéristiques sont les suivantes:
Spectre RMN 1 H :3,41 (t, J=5,0 Hz, 4 H); 3,51 à 3,64 (m, 6 H); 5,20 (s, 1 H); 7,08 (d, J=5,6 Hz, 1 H); 7,35 à 7,56 (m, 2 H); 10,55 (s large, 1 H); 11,64 (s large,1 H)
Spectrométrie de Masse : méthode A
Temps de rétention Tr (min) = 0.52;
[M+H]+ : m/z 321 ; [M-H]- : m/z 319

**Exemple 8: Synthèse du N-(4-fluoro-3-méthoxyphényl)-2-[4-(morpholin-4-yl)-6-oxo-1,6-dihydropyrimidin-2-yl]acétamide**

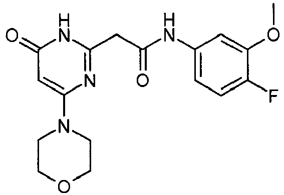

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 520 mg de 4-fluoro-3-méthoxyaniline au lieu de la 2,4-difluoroaniline. On obtient 262 mg de N-(4-fluoro-3-méthoxyphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz) : 3,41 (t, J=4,9 Hz, 4 H); 3,56 à 3,62 (m, 6 H); 3,79 (s, 3H); 5,20 (s, 1 H); 7,02 à 7,09 (m, 1 H); 7,10 à 7,17 (m, 1 H); 7,47 (dd, J=2,4 et 8,1 Hz, 1 H); 10,20 (s, 1 H);11,64 (s large, 1 H)
Spectrométrie de Masse : méthode A
Temps de rétention Tr (min) = 0.60;
[M+H]+ : m/z 363 ; [M-H]- : m/z 361

### Exemple 9 : Synthèse du N-(2-fluorophényl)-2-[4-(morpholin-4-yl)-6-oxo-1,6-dihydropyrimidin-2-yl]acétamide

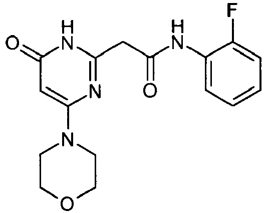

Dans un tube micro-ondes, on introduit 300 mg de [4-(morpholin-4-yl)-6-oxo-1,6-dihydropyrimidin-2-yl]acétate d'éthyle préparé à l'étape 1 de l'exemple 1 dans 1 mL de N,N-diméthylformamide, 623 mg de 2-fluoroaniline et 378 mg de tert-butylate de potassium. On ajoute ensuite 2 mL de N,N-diméthylformamide. Le tube est alors chauffé sous micro-ondes à 150°C pendant 20 minutes. Le mélange réactionnel est concentré sous pression réduite. On ajoute 30 mL d'eau et 10 mL d'acétate d'éthyle et agite ainsi pendant 1 H30. Le précipité formé est filtré et rincé avec de l'acétate d'éthyle, de l'éther éthylique puis de l'éther de pétrole. Le solide obtenu est séché à l'évaporateur rotatif et on obtient ainsi 93 mg de N-(2-fluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,42 (t, J=4,9 Hz, 4 H); 3,61 (t, J=4,6 Hz, 4 H); 3,67(s, 2 H); 5,19 (s, 1 H); 7,12 à 7,20 (m, 2 H); 7,21 à 7,32 (m, 1 H); 7,81 à 7,98 (m,J=8,3 et 8,3 Hz, 1 H);10,00 (s large, 1 H); 11,66 (s large, 1 H)
Spectrométrie de Masse : méthode A
Temps de rétention Tr (min) = 0.56;
[M+H]+ : m/z 333 ; [M-H]- : m/z 331
Pureté : 94 %
Point de fusion (Kofler) : 279°C

### Exemple 10 : Synthèse du N-(2-méthylphényl)-2-[4-(morpholin-4-yl)-6-oxo-1,6-dihydropyrimidin-2-yl]acétamide

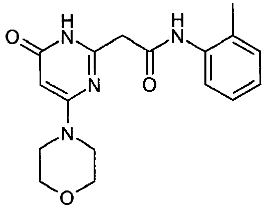

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 et 582 mg de 2-méthylaniline au lieu de la 2-fluoroaniline. On obtient 76 mg de N-(2-méthylphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :2,21 (s, 3 H); 3,43 (t, J=5,0 Hz, 4 H); 3,55 à 3,67 (m, 6H); 5,18 (s, 1 H); 7,08 (t, J=7,8 Hz, 1 H); 7,16 (t, J=7,8 Hz, 1 H); 7,21 (d, J=7,8 Hz, 1 H); 7,42 (d, J=7,8Hz, 1 H); 9,65 (s large, 1 H); 11,70 (s, 1 H)
Spectrométrie de Masse : méthode B
Temps de rétention Tr (min) = 2.81;
[M+H]+ : m/z 329 ; [M-H]- : m/z 327
Point de fusion (Kofler) : 194°C

### Exemple 11 : Synthèse du N-(2-méthoxyphényl)-2-[4-(morpholin-4-yl)-6-oxo-1,6-dihydropyrimidin-2-yl]acétamide

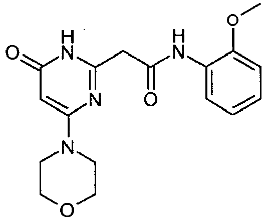

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 dans 2 mL de N,N-diméthylformamide, 637 mg de 2-méthoxyaniline au lieu de la 2-fluoroaniline et 300 mg de tert-butylate de potassium. On ajoute 3 mL de N,N-diméthylformamide et le tube est chauffé sous micro-ondes à 150°C pendant 20 minutes. Le mélange réactionnel est concentré sous pression réduite. On ajoute 15 mL d'eau et 5 mL d'acétate d'éthyle et agite ainsi pendant 2 heures. La phase aqueuse est extraite avec 5 mL d'acétate d'éthyle. Les phases organiques combinées sont séchées sur sulfate de magnésium, filtrées et concentrées sous pression réduite. Après purification du solide obtenu sur plaque préparative (épaisseur : 2 mm), en éluant par un mélange de dichlorométhane et de méthanol (90/10 en volumes), on obtient ainsi 7 mg de N-(2-méthoxyphényl)-2-[4-(morpholin-4-yl)-6-oxo-1,6-dihydropyrimidin-2-yl]acétamide sous forme de solide rosé dont les caractéristiques sont les suivantes:
Spectre RMN 1 H :3,43 (t, J=4,9 Hz, 4 H); 3,59 à 3,65 (m, 4 H); 3,69 (s, 2H); 3,83 (s, 3 H); 5,20 (s, 1 H); 6,90 (ddd, J=2,2 et 6,5 et 8,2 Hz, 1 H); 6,99 à 7,13 (m,2 H); 7,97 (d, J=8,6Hz, 1 H); 9,44 (s large, 1 H); 11,66 (s large, 1 H)
Spectrométrie de Masse : méthode A
Temps de rétention Tr (min) = 0.59;
[M+H]+ : m/z 345 ; [M-H]- : m/z 343

### Exemple 12 : Synthèse du N-(2,3-difluorophényl)-2-[4-(morpholin-4-yl)-6-oxo-1,6-dihydropyrimidin-2-yl]acétamide

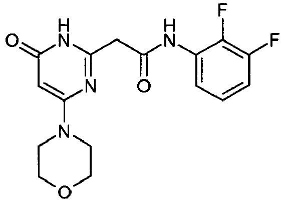

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 et 840 mg de 2,3-difluoroaniline au lieu de la 2-fluoroaniline. On obtient 83 mg de N-(2,3-difluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,42 (t, J=5,1 Hz, 4H); 3,61 (t, J=5.1 Hz, 4 H); 3,69 (s,2 H); 5,20 (s, 1 H); 7,18 (t, J=7,0 Hz, 2 H); 7,69 (s large, 1 H); 10,24 (s large, 1 H);11,63 (s large, 1 H)
Spectrométrie de Masse : méthode A
Temps de rétention Tr (min) = 0.61;
[M+H]+ : m/z 351 ; [M-H]- : m/z 349
Point de fusion (Kofler) : 248°C

### Exemple 13 : Synthèse du N-(3,5-difluorophényl)-2-[4-(morpholin-4-yl)-6-oxo-1,6-dihydropyrimidin-2-yl]acétamide

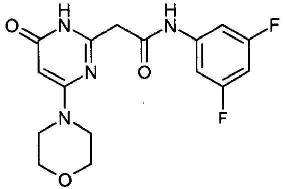

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 et 780 mg de 3,5-difluoroaniline au lieu de la 2-fluoroaniline. On obtient 133 mg de N-(3,5-difluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,41 (t, J=4,9 Hz, 4 H); 3,56 à 3,65 (m, 6 H); 5,20 (s, 1 H); 6,92 (t, J=9,3 Hz, 1 H); 7,28 (dd, J=2,2 et 9,5 Hz, 2 H); 10,55 (s large, 1 H); 11,66(s large, 1 H)
Spectrométrie de Masse : méthode A
Temps de rétention Tr (min) = 0.66;
[M+H]+ : m/z 351 ; [M-H]- : m/z 349
Point de fusion (Kofler) > 260°C

### Exemple 14: Synthèse du N-(3-fluorophényl)-2-[4-(morpholin-4-yl)-6-oxo-1,6-dihydropyrimidin-2-yl]acétamide

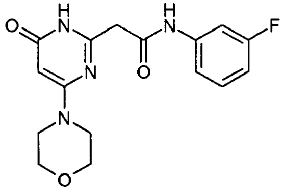

Le produit est préparé en suivant le mode opératoire décrit à l'exemple 9 à partir de 250 mg de [4-(morpholin-4-yl)-6-oxo-1,6-dihydropyrimidin-2-yl]acétate d'éthyle préparé à l'étape 1 de l'exemple 1 et 0.90 mL de 3-fluoroaniline au lieu de la 2-fluoroaniline. On obtient 160 mg de N-(3-fluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz) : 3,42 (d, J=5,0 Hz, 4 H); 3,57 à 3,62 (m, 6 H); 5,20 (s,1 H); 6,89 (t, J=9,0 Hz, 1 H); 7,27 (d, J=7,5 Hz, 1 H); 7,31 à 7,40 (m, 1 H); 7,55 (d, J=12,1 Hz, 1 H); 10,38(s large, 1 H); 11,66 (s large, 1 H)
Spectrométrie de Masse : méthode A
Temps de rétention Tr (min) = 2.93;
[M+H]+ : m/z 333 ; [M-H]- : m/z 331

### Exemple 15: Synthèse du N-(4-chlorophényl)-2-[4-(morpholin-4-yl)-6-oxo-1,6-dihydropyrimidin-2-yl]acétamide

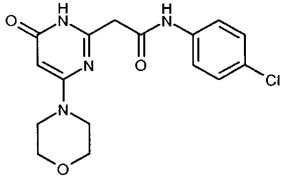

Le produit est préparé en suivant le mode opératoire décrit à l'exemple 9 à partir de 250 mg de [4-(morpholin-4-yl)-6-oxo-1,6-dihydropyrimidin-2-yl]acétate d'éthyle préparé à l'étape 1 de l'exemple 1 et 1.193 g de 4-chloroaniline au lieu de la 2-fluoroaniline. On obtient 140 mg de N-(4-chlorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,37 à 3,46 (m, J=4,9 Hz, 4 H); 3,51 à 3,66 (m, 6 H); 5,21 (s, 1 H); 7,38 (d, J=8,8 Hz, 2 H); 7,60 (d, J=8,8 Hz, 2 H); 10,34 (s large, 1 H); 11,71 (s large, 1 H)
Spectrométrie de Masse : méthode A
Temps de rétention Tr (min) = 0.68;
[M+H]+ : m/z 349 ; [M-H]- : m/z 347

### Exemple 16 : Synthèse du N-(3-méthoxyphényl)-2-[4-(morpholin-4-yl)-6-oxo-1,6-dihydropyrimidin-2-yl]acétamide

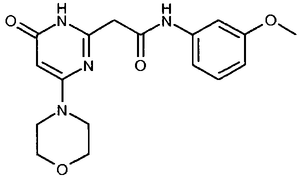

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 et 1.254 mL de 3-méthoxyaniline au lieu de la 2-fluoroaniline. On obtient 56 mg de N-(3-méthoxyphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,41 (t, J=5,0 Hz, 4 H); 3,55 à 3,63 (m, 6 H); 3,72 (s, 3H); 5,20 (s, 1 H); 6,64 (dd, J=2,4 et 8,1 Hz, 1 H); 7,08 (ddd, J=0,9 et 1,0 et 8.1 Hz, 1 H); 7,21 (t, J=8,3 Hz,1 H); 7,27 (s, 1 H); 10,14 (s large, 1 H); 11,63 (s large, 1 H)
Spectrométrie de Masse : méthode A
Temps de rétention Tr (min) = 0.58;
[M+H]+ : m/z 345 ; [M-H]- : m/z 343

### Exemple 17 : Synthèse du 2-[4-(morpholin-4-yl)-6-oxo-1,6-dihydropyrimidin-2-yl]-N-[3-(trifluorométhyl)phényl]acétamide

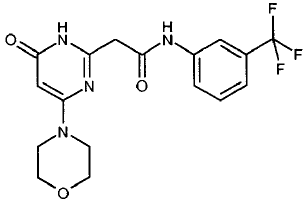

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 et 0.705 mL de 3-(trifluorométhyl)aniline au lieu de la 2-fluoroaniline. On obtient 228 mg de 2-[4-(morpholin-4-yl)-6-oxo-1,6-dihydropyrimidin-2-yl]-N-[3-(trifluorométhyl)phényl]acétamide sous forme de solide blanc dont les caractéristiques sont les suivantes:
Spectre RMN 1 H :3,37 à 3,46 (m, 4 H); 3,54 à 3,66 (m, 6 H); 5,21 (slarge, 1 H); 7,42 (d, J=8.1 Hz, 1 H); 7,57 (t, J=8,1 Hz, 1 H); 7,75 (d, J=8,3 Hz, 1 H); 8,05 (s large, 1 H); 10,50 (s large, 1 H); 11,67 (s large, 1 H)
Spectrométrie de Masse : méthode A
Temps de rétention Tr (min) = 3.39;
[M+H]+ : m/z 383 ; [M-H]- : m/z 381

### Exemple 18 : Synthèse du N-(3-bromophényl)-2-[4-(morpholin-4-yl)-6-oxo-1,6-dihydropyrimidin-2-yl]acétamide

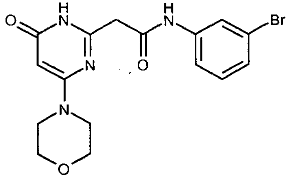

Le produit est préparé en suivant le mode opératoire décrit à l'exemple 9 à partir de 300 mg de [4-(morpholin-4-yl)-6-oxo-1,6-dihydropyrimidin-2-yl]acétate d'éthyle préparé à l'étape 1 de l'exemple 1 dans et 0.61 mL de 3-bromoaniline au lieu de la 2-fluoroaniline. On obtient 105 mg de N-(3-bromophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz) : 3,37 à 3,46 (m, 4 H); 3,54 à 3,66 (m, 6 H); 5,20 (slarge, 1 H); 7,15 à 7,34 (m, 2 H); 7,47 (d, J=8,1 Hz, 1 H); 7,91 (s large, 1 H); 10,33 (s large, 1 H); 11,66 (slarge, 1 H)
Spectrométrie de Masse : méthode A
Temps de rétention Tr (min) = 0.70;
[M+H]+ : m/z 395 ; [M-H]- : m/z 393

### Exemple 19 : Synthèse du N-[3-(2-méthylpropan-2-yl)phényl]-2-[4-(morpholin-4-yl)-6-oxo-1,6-dihydropyrimidin-2-yl]acétamide

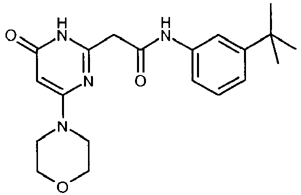

A une solution de 261 mg de [4-(morpholin-4-yl)-6-oxo-1,6-dihydropyrimidin-2-yl]acétate de sodium préparé à l'étape 2 de l'exemple 1 dans 10 mL de méthanol sont ajoutés 224 mg de 3-(tert-butyl)aniline. Le mélange réactionnel est agité à température ambiante pendant 5 minutes puis on ajoute 442 mg chlorure de 4-(4,6-diméthoxy-1,3,5-triazin-2-yl)-4-méthylmorpholin-4-ium hydrate. On agite ainsi pendant 30 minutes à température ambiante. Le mélange réactionnel est concentré sous pression réduite. Le résidu d'évaporation est repris par 10 mL d'eau et 10 mL d'acétate d'éthyle. On agite ensuite pendant 30 minutes. Le précipité formé est filtré. On obtient ainsi 235 mg de N-[3-(2-méthylpropan-2-yl)phényl]-2-[4-(morpholin-4-yl)-6-oxo-1 ,6-dihydropyrimidin-2-yl]acétamide sous forme de solide blanc dont les caractéristiques sont les suivantes:
Spectre RMN 1 H :1,26 (s, 9 H); 3,42 (t, J=4,8 Hz, 4 H); 3,53 à 3,64 (m, 6H); 5,20 (s, 1 H); 7,10 (d, J=8,1 Hz, 1 H); 7,23 (t, J=8,1 Hz, 1 H); 7,41 (d, J=8,1 Hz, 1 H); 7,58 (s large, 1 H); 10,09 (s large, 1 H); 11,65 (s large, 1 H)
Spectrométrie de Masse : méthode A
Temps de rétention Tr (min) = 0.83;
[M+H]+ : m/z 371 ; [M-H]- : m/z 369

### Exemple 20 : Synthèse du 3-({[4-(morpholin-4-yl)-6-oxo-1,6-dihydropyrimidin-2-yl]acétyl}amino)benzoate de méthyle

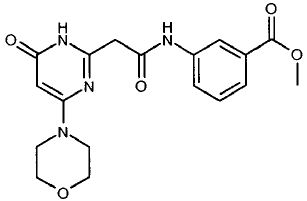

Le produit est préparé en suivant le mode opératoire décrit à l'exemple 19 à partir de 653 mg de [4-(morpholin-4-yl)-6-oxo-1,6-dihydropyrimidin-2-yl]acétate de sodium préparé à l'étape 2 de l'exemple 1 et 567 mg de 3-aminobenzoate de méthyle au lieu de la 3-(tert-butyl)aniline. On obtient 400 mg de 3-({[4-(morpholin-4-yl)-6-oxo-1,6-dihydropyrimidin-2-yl]acétyl}amino)benzoate de méthyle sous forme de solide blanc dont les caractéristiques sont les suivantes:
Spectre RMN 1 H :3,41 (dd, J=4,8 et 5,3 Hz, 4 H); 3,53 à 3,68 (m, 6 H);3,85 (s large, 3 H); 5,21 (s large, 1 H); 7,47 (t, J=8,1 Hz, 1 H); 7,66 (d, J=8,1 Hz, 1 H); 7,80 (d, J=8,3 Hz, 1 H); 8,24 (s, 1 H); 10,00 à 10,64 (m, 1 H); 11,67 (s large, 1 H)
Spectrométrie de Masse : méthode A
Temps de rétention Tr (min) = 0.59;
[M+H]+ : m/z 373 ; [M-H]- : m/z 371
Pureté : 95 %

### Exemple 21 : Synthèse du acide 3-({[4-(morpholin-4-yl)-6-oxo-1,6-dihydropyrimidin-2-yl]acétyl}amino)benzoïque

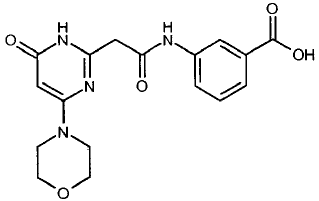

A une solution de 335 mg de 3-({[4-(morpholin-4-yl)-6-oxo-1,6-dihydropyrimidin-2-yl]acétyllamino)benzoate de méthyle dans 25 mL de méthanol , on ajoute 1,8 mL de soude 2 M. Le mélange réactionnel est chauffé à 60°C pendant 2h30, puis ilest concentré sous pression réduite. Le résidu d'évaporation est repris dans 50 mL d'eau. La phase aqueuse est extraite à l'acétate d'éthyle puis acidifiée (pH=6) par ajout d'une solution d'acide chlorhydrique 1 N. Le précipité formé est filtré puis lavé avec 20 mL d'eau, 5 mL d'acétate d'éthyle et enfin 20 mL d'éther éthylique. Le solide est séché sous vide et on obtient ainsi 175 mg d'acide 3-({[4-(morpholin-4-yl)-6-oxo-1,6-dihydropyrimidin-2-yl]acétyl}amino)benzoïque sous forme de solide blanc dont les caractéristiques sont les suivantes:
Spectre RMN 1 H (400MHz) : 3,42 (t, J=4,9 Hz, 4 H); 3,52 à 3,72 (m, 6 H); 5,21 (s, 1H); 7,43 (t, J=7,9 Hz, 1 H); 7,64 (d, J=7,8 Hz, 1H); 7,79 (ddd, J=1,5 et 1,6 et 8,2 Hz, 1 H); 8,19 (s, 1 H);10,36 (s, 1 H); 11,77 (s large, 1 H); 12,94 (s large, 1 H)
Spectrométrie de Masse : méthode A
Temps de rétention Tr (min) = 0.46;
[M+H]+ : m/z 359 ; [M-H]- : m/z 357

### Exemple 22 : Synthèse du 2-[4-(morpholin-4-yl)-6-oxo-1,6-dihydropyrimidin-2-yl]-N-[3-(propan-2-yl)phényl]acétamide

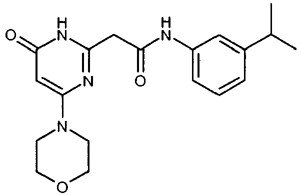

Le produit est préparé en suivant le mode opératoire décrit à l'exemple 19 à partir de 653 mg de [4-(morpholin-4-yl)-6-oxo-1,6-dihydropyrimidin-2-yl]acétate de sodium préparé à l'étape 2 de l'exemple 1 en utilisant un mélange de 10 mL d'eau et 2 mL de tétrahydrofurane au lieu du méthanol et 270 mg d'isopropylaniline au lieu de la 3-(tert-butyl)aniline. On obtient 235 mg de 2-[4-(morpholin-4-yl)-6-oxo-1,6-dihydropyrimidin-2-yl]-N-[3-(propan-2-yl)phényl]acétamide sous forme de solide blanc dont les caractéristiques sont les suivantes:
Spectre RMN 1 H :1,18 (d, J=6,8 Hz, 6 H); 2,84 (quin, J=6,9 Hz, 1 H);3,42 (t, J=4,9 Hz, 4 H); 3,54 à 3,65 (m, 6 H); 5,20 (s, 1 H); 6,94 (d, J=7,6 Hz, 1 H); 7,22 (t, J=7,8 Hz, 1 H); 7,38 (d, J=8,8 Hz, 1 H); 7,45 (s, 1 H); 10,09 (s, 1 H); 11,64 (s large, 1 H)
Spectrométrie de Masse : méthode A
Temps de rétention Tr (min) =0.78;
[M+H]+ : m/z 357 ; [M-H]- : m/z 355

### Exemple 23 : Synthèse du N-(3-méthylphényl)-2-[4-(morpholin-4-yl)-6-oxo-1,6-dihydropyrimidin-2-yl]acétamide

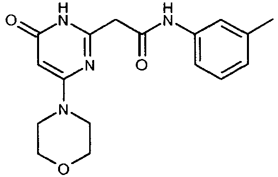

Le produit est préparé en suivant le mode opératoire décrit à l'exemple 19 à partir de 261 mg de [4-(morpholin-4-yl)-6-oxo-1,6-dihydropyrimidin-2-yl]acétate de sodium préparé à l'étape 2 de l'exemple 1 en utilisant un mélange de 1 mL d'eau et 9 mL d'éthanol au lieu du méthanol et, 214 mg de 3-méthyl-aniline au lieu de la 3-(tert-butyl)aniline. On obtient 196 mg de N-(3-méthylphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :2,27 (s, 3 H); 3,38 à 3,45 (m, 4 H); 3,54 à 3,64 (m, 6H); 5,20 (s, 1 H); 6,88 (d, J=7,8 Hz, 1 H); 7,19 (t, J=7,8 Hz, 1 H); 7,35 (d, J=8,8 Hz, 1 H); 7,39 (s, 1 H);10,07 (s, 1 H); 11,65 (s large, 1 H)
Spectrométrie de Masse : méthode A
Temps de rétention Tr (min) = 0.63;
[M+H]+ : m/z 329 ; [M-H]- : m/z 327

### Exemple 24 : Synthèse du N-(3-cyano-4-fluorophényl)-2-[4-(morpholin-4-yl)-6-oxo-1,6-dihydropyrimidin-2-yl]acétamide

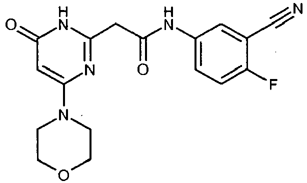

Le produit est préparé en suivant le mode opératoire décrit à l'exemple 19 à partir de 261 mg de [4-(morpholin-4-yl)-6-oxo-1,6-dihydropyrimidin-2-yl]acétate de sodium préparé à l'étape 2 de l'exemple 1 et 163 mg de 5-amino-2-fluorobenzonitrile au lieu de la 3-(tert-butyl)aniline. Le mélange réactionnel est concentré à sec sous pression réduite puis le résidu est purifié par chromatographie sur colonne de silice en éluant avec un gradient de l'éluant CH2Cl2/MeOH : 90/10 dans le dichlorométhane de 0% à 100%. On obtient 81 mg de N-(3-cyano-4-fluorophényl)-2-[4-(morpholin-4-yl)-6-oxo-1,6-dihydropyrimidin-2-yl]acétamide sous forme de solide ambré dont les caractéristiques sont les suivantes:
Spectre RMN 1 H :3,34 à 3,47 (m, 4 H); 3,53 à 3,67 (m, 6 H); 5,21 (s, 1 H); 7,51 (t, J=9,2 Hz, 1 H); 7,73 à 7,89 (m, 1 H); 8,07(dd, J=2,8 et 5,7 Hz, 1 H); 10,53 (s, 1 H); 11,68 (s large, 1 H)
Spectrométrie de Masse : méthode A
Temps de rétention Tr (min) = 0.60;
[M+H]+ : m/z 358 ; [M-H]- : m/z 356
Pureté : 95 %

### Exemple 25 : Synthèse du N-(1H-indazol-6-yl)-2-[4-(morpholin-4-yl)-6-oxo-1,6-dihydropyrimidin-2-yl]acétamide

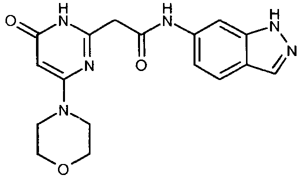

Le produit est préparé en suivant le mode opératoire décrit à l'exemple 19 à partir de 261 mg de [4-(morpholin-4-yl)-6-oxo-1,6-dihydropyrimidin-2-yl]acétate de sodium préparé à l'étape 2 de l'exemple 1 et 280 mg de 1-Boc-6-amino-indazole au lieu de la 3-(tert-butyl)aniline. Le mélange réactionnel est concentré sous pression réduite et repris dans 13 mL de 1,4-dioxanne et 3 mL d'acide chlorhydrique 1 N, puis il est chauffé sous micro-ondes pendant 10 minutes à 100°C. Après refroidissement à température ambiante, le mélange réactionnel est concentré sous pression réduite et repris dans 30 mL d'eau puis on ajoute une solution aqueuse saturée en bicarbonate de sodium afin d'obtenir un pH proche de 8. Le précipité formé est filtré et lavé avec de l'eau, de l'acétate d'éthyle et de l'éther éthylique. Après purification par chromatographie sur colonne de silice en éluant avec un gradient de l'éluant CH2Cl2/MeOH : 70/30 dans le dichlorométhane de 0% à 100%, on obtient 20 mg de N-(1H-indazol-6-yl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,42 (t, J=4,9 Hz, 4 H); 3,60 (dd, J=4,0 et 5,7 Hz, 4H); 3,64 (s, 2 H); 5,20 (s, 1 H); 7,08 (dd, J=1,8 et 8,6 Hz,1 H); 7,67 (d, J=8,6 Hz, 1 H); 7,96 (s, 1 H); 8,09 (s, 1 H); 10,32 (s large, 1 H); 11,67 (s large, 1 H); 12,88 (s large, 1 H)
Spectrométrie de Masse : méthode A
Temps de rétention Tr (min) = 0.45;
[M+H]+ : m/z 355 ; [M-H]- : m/z 353

### Exemple 26 : Synthèse du N-(3-cyanophényl)-2-[4-(morpholin-4-yl)-6-oxo-1,6-dihydropyrimidin-2-yl]acétamide

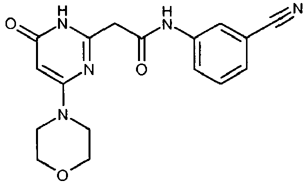

Le produit est préparé en suivant le mode opératoire décrit à l'exemple 19 à partir de 261 mg de [4-(morpholin-4-yl)-6-oxo-1,6-dihydropyrimidin-2-yl]acétate de sodium préparé à l'étape 2 de l'exemple 1 en utilisant un mélange de 6 mL d'eau et 1 mL d'acétate d'éthyle au lieu du méthanol et, 118 mg de 3-aminobenzonitrile au lieu de la 3-(tert-butyl)aniline. On obtient 120 mg de N-(3-cyanophényl)-2-[4-(morpholin-4-yl)-6-oxo-1,6-dihydropyrimidin-2-yl]acétamide sous forme de solide blanc cassé dont les caractéristiques sont les suivantes:
Spectre RMN 1 H :3,41 (t, J=4,8 Hz, 4 H); 3,60 (dd, J=4,8 et 5,3 Hz, 4H); 3,63 (s, 2 H); 5,21 (s, 1 H); 7,49 à 7,59 (m, 2 H); 7,78 (dt, J=2,3 et 6,8 Hz, 1 H); 8,04 (s, 1 H); 10,51 (s large, 1 H); 11,69 (s large, 1 H)
Spectrométrie de Masse : méthode A
Temps de rétention Tr (min) = 0.55;
[M+H]+ : m/z 340 ; [M-H]- : m/z 338

### Exemple 27 : Synthèse du N-(5-fluoropyridin-2-yl)-2-[4-(morpholin-4-yl)-6-oxo-1,6-dihydropyrimidin-2-yl]acétamide

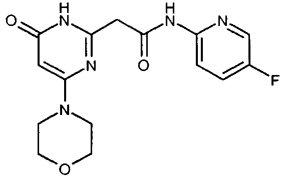

Le produit est préparé en suivant le mode opératoire décrit à l'exemple 19 à partir de 261 mg de [4-(morpholin-4-yl)-6-oxo-1,6-dihydropyrimidin-2-yl]acétate de sodium préparé à l'étape 2 de l'exemple 1 en utilisant un mélange de 7 mL d'eau et 1.5 mL de tétrahydrofurane au lieu du méthanol et 134 mg de 2-amino-5-fluoropyridine au lieu de la 3-(tert-butyl)aniline. Après 1 H30 d'agitation à température ambiante, un précipité se forme. Le mélange réactionnel est filtré sur verre fritté. Le solide obtenu est lavé avec de l'eau, de l'acétate d'éthyle et de l'éther éthylique. On obtient 130 mg de N-(5-fluoropyridin-2-yl)-2-[4-(morpholin-4-yl)-6-oxo-1,6-dihydropyrimidin-2-yl]acétamide sous forme de solide blanc cassé dont les caractéristiques sont les suivantes:
Spectre RMN 1 H :3,36 à 3,46 (m, 4 H); 3,60 (t, J=5,0 Hz, 4 H); 3,67 (s, 2H); 5,20 (s, 1 H); 7,75 (td, J=2.7 et 6,1 Hz, 1 H); 8,07 (dd, J=9.4 et 4 Hz, 1 H); 8,33 (d, J= 2,7 Hz, 1 H);10,80 (s, 1 H); 11,66 (s large, 1 H)
Spectrométrie de Masse : méthode A
Temps de rétention Tr (min) = 2.67;
[M+H]+ : m/z 334 ; [M-H]- : m/z 332

### Exemple 28: Synthèse du N-(4-fluoro-3-méthylphényl)-2-[4-(morpholin-4-yl)-6-oxo-1,6-dihydropyrimidin-2-yl]acétamide

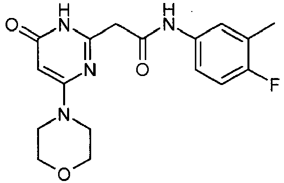

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 240 mg de 4-fluoro-3-méthylaniline au lieu de la 2,4-difluoroaniline. On obtient 204 mg de N-(4-fluoro-3-méthylphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :2,20 (d, J=1,2 Hz, 3 H); 3,41 (t, J=4,9 Hz, 4 H); 3,55 à3,63 (m, 6 H); 5,20 (s, 1 H); 7,07 (t, J=9,2 Hz, 1 H); 7,33 à 7,41 (m, 1 H); 7,46 (dd, J=2,7 et 6,8 Hz, 1 H);10,13 (s, 1 H); 11,63 (s large, 1 H)
Spectrométrie de Masse : méthode A
Temps de rétention Tr (min) = 0.66;
[M+H]+ : m/z 347 ; [M-H]- : m/z 345

### Exemple 29: Synthèse du N -(3-chloro-4-fluorophényl)-2-[4-(morpholin-4-yl)-6-oxo-1,6-dihydropyrimidin-2-yl]acétamide

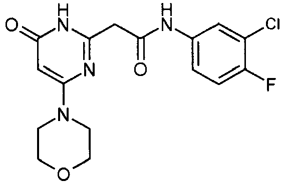

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 278 mg de 3-chloro-4-fluoroaniline au lieu de la 2,4-difluoroaniline. On obtient 218 mg de N-(3-chloro-4-fluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,41 (t, J=5,0 Hz, 4 H); 3,56 à 3,63 (m, 6 H); 5,20 (s, 1 H); 7,38 (t, J=8,8 Hz, 1 H); 7,41 à 7,50 (m, 1 H); 7,88 (dd, J=2,7 et 6,8 Hz, 1 H);10,38 (s, 1 H); 11,66 (s large, 1 H)
Spectrométrie de Masse : méthode A
Temps de rétention Tr (min) = 0.70;
[M+H]+ : m/z 367 ; [M-H]- : m/z 365

### Exemple 30: Synthèse du 2-[4-(morpholin-4-yl)-6-oxo-1,6-dihydropyrimidin-2-yl]-N-(pyridin-3-yl)acétamide

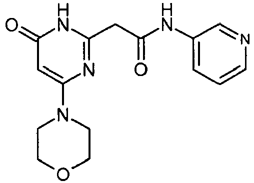

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 427 mg de 3-aminopyridine au lieu de la 2,4-difluoroaniline. On obtient 168 mg de 2-[4-(morpholin-4-yl)-6-oxo-1,6-dihydropyrimidin-2-yl]-N-(pyridin-3-yl)acétamide sous forme de solide blanc dont les caractéristiques sont les suivantes:
Spectre RMN 1 H :3,39 à 3,46 (m, 4 H); 3,56 à 3,66 (m, 6 H); 5,20 (slarge, 1 H); 7,35 (s large, 1 H); 8,00 (d, J=8,6 Hz, 1 H); 8,28 (s large, 1 H); 8,71 (s large, 1 H); 10,37 (slarge, 1 H); 11,67 (s large, 1 H)
Spectrométrie de Masse : méthode A
Temps de rétention Tr (min) =0.25;
[M+H]+ : m/z 316 ; [M-H]- : m/z 314

### Exemple 31 : Synthèse du N-(4-fluoro-2-méthylphényl)-2-[4-(morpholin-4-yl)-6-oxo-1,6-dihydropyrimidin-2-yl]acétamide

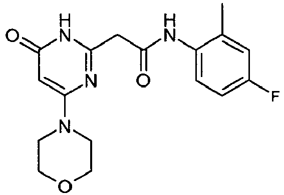

Le produit est préparé en suivant le mode opératoire décrit à l'exemple 5 à partir de 200 mg de [4-(morpholin-4-yl)-6-oxo-1,6-dihydropyrimidin-2-yl]acétate de sodium préparé à l'étape 2 de l'exemple 1 et 278 mg de 4-fluoro-2-méthylaniline au lieu de la 2,4-difluoroaniline. On obtient 92 mg de N-(4-fluoro-2-méthylphényl)-2-[4-(morpholin-4-yl)-6-oxo-1,6-dihydropyrimidin-2-yl]acétamide sous forme de solide rosé dont les caractéristiques sont les suivantes:
Spectre RMN 1 H :2,21 (s, 3 H); 3,44 (t, J=4,9 Hz, 4 H); 3,57 à 3,66 (m, 6H); 5,19 (s, 1 H); 6,99 (td, J=3,2 et 8,6 Hz, 1 H); 7,08 (dd, J=3,1 et 9,7 Hz, 1 H); 7,36 (dd, J=5,6 et 8,6 Hz,1 H); 9,56 (s large, 1 H); 11,67 (s large, 1 H)
Spectrométrie de Masse : méthode A
Temps de rétention Tr (min) = 0.59;
[M+H]+ : m/z 347 ; [M-H]- : m/z 345

### Exemple 32 : Synthèse du N-(3-hydroxyphényl)-2-[4-(morpholin-4-yl)-6-oxo-1,6-dihydropyrimidin-2-yl]acétamide

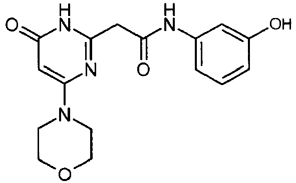

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 418 mg de 3-aminophenol au lieu de la 2,4-difluoroaniline. On obtient 210 mg de N-(3-hydroxyphényl)-2-[4-(morpholin-4-yl)-6-oxo-1,6-dihydropyrimidin-2-yl]acétamide sous forme de solide rosé dont les caractéristiques sont les suivantes:
Spectre RMN 1 H :3,37 à 3,47 (m, 4 H); 3,51 à 3,63 (m, 6 H); 5,20 (s, 1 H); 6,45 (d, J=8,6 Hz, 1 H); 6,93 (d, J=8,6 Hz, 1 H); 7,07 (t, J=8,6 Hz, 1 H); 7,12 (s large, 1 H); 9,36 (s large, 1 H); 10,00 (s large, 1 H); 11,63 (s large, 1 H)
Spectrométrie de Masse : méthode A
Temps de rétention Tr (min) = 0.43;
[M+H]+ : m/z 331 ; [M-H]- : m/z 329

### Exemple 33: Synthèse de N-(3-bromo-4-fluorophényl)-2-[4-(morpholin-4-yl)-6-oxo-1,6-dihydropyrimidin-2-yl]acétamide

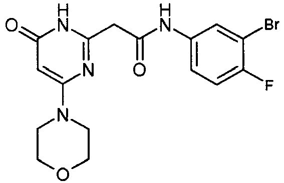

Le produit est préparé en suivant le mode opératoire décrit à l'exemple 19 à partir de 261 mg de [4-(morpholin-4-yl)-6-oxo-1,6-dihydropyrimidin-2-yl]acétate de sodium préparé à l'étape 2 de l'exemple 1 en utilisant un mélange de 7 mL d'eau et 1.5 mL de tétrahydrofurane au lieu du méthanol et 190 mg de 3-bromo-4-fluoroaniline au lieu de la 3-(tert-butyl)aniline. On obtient 266 mg de N-(3-bromo-4-fluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,41 (t, J=4,9 Hz, 4 H); 3,55 à 3,65 (m, 6 H); 5,20 (s, 1H); 7,34 (t, J=8,8 Hz, 1 H); 7,44 à 7,53 (m, 1 H); 8,00 (dd, J=2,2 et 6,1 Hz, 1 H); 10,35 (s large, 1 H); 11,67 (s large, 1 H)
Spectrométrie de Masse : méthode A
Temps de rétention Tr (min) = 3.30
[M+H]+ : m/z 411 ; [M-H]- : m/z 409

### Exemple 34: Synthèse du 2-[4-(morpholin-4-yl)-6-oxo-1,6-dihydropyrimidin-2-yl]-N-(3,4,5-trifluorophényl)acétamide

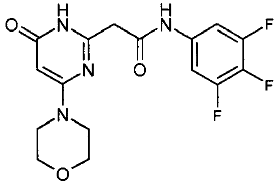

Le produit est préparé en suivant le mode opératoire décrit à l'exemple 5 à partir de 200 mg de [4-(morpholin-4-yl)-6-oxo-1,6-dihydropyrimidin-2-yl]acétate de sodium et 113 mg de 3,4,5-trifluoroaniline au lieu de la 2,4-difluoroaniline. On obtient 33 mg de 2-[4-(morpholin-4-yl)-6-oxo-1,6-dihydropyrimidin-2-yl]-N-(3,4,5-trifluorophényl)acétamide dont les caractéristiques sont les suivantes:
Spectre RMN 1 H :3,41 (dd, J=4,8 et 5,3 Hz, 4 H); 3,54 à 3,66 (m, 6 H); 5,20 (s, 1 H); 7,46 (dd, J=6,5 et 10,1 Hz, 2 H); 10,55 (s, 1 H); 11,64 (s, 1 H)

### Exemple 35 : Synthèse du N-[4-fluoro-3-(hydroxyméthyl)phényl]-2-[4-(morpholin-4-yl)-6-oxo-1,6-dihydropyrimidin-2-yl]acétamide

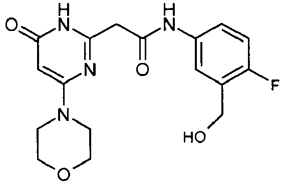

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 525 mg de (5-amino-2-fluorophényl)méthanol au lieu de la 2,4-difluoroaniline. On obtient 218 mg de N-[4-fluoro-3-(hydroxyméthyl)phényl]-2-[4-(morpholin-4-yl)-6-oxo-1,6-dihydropyrimidin-2-yl]acétamide sous forme de solide beige dont les caractéristiques sont les suivantes:
Spectre RMN 1 H :3,42 (d, J=4,9 Hz, 4 H); 3,55 à 3,64 (m, 6 H); 4,51 (d,J=6,1 Hz, 2 H); 5,20 (s large, 1 H); 5,27 (t, J=6,0 Hz, 1 H); 7,08 (t, J=9,4 Hz, 1 H); 7,46 à 7,54 (m, 1 H);7,63 (d, J=7,3 Hz, 1 H); 10,18 (s large, 1 H); 11,64 (s large, 1H)
Spectrométrie de Masse : méthode A
Temps de rétention Tr (min) = 0.46
[M+H]+ : m/z 363 ; [M-H]- : m/z 361
Point de fusion (Kofler) > 260°C

### Exemple 36 : Synthèse du N-(3-cyclopropylephényl)-2-[4-(morpholin-4-yl)-6-oxo-1,6-dihydropyrimidin-2-yl]acétamide

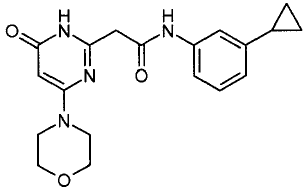

Le produit est préparé en suivant le mode opératoire 250 mg de [4-(morpholin-4-yl)-6-oxo-1 ,6-dihydropyrimidin-2-yl]acétate de sodium et 395 mg de 3-cyclopropyleaniline (préparée selon Wallace et al. dans Tetrahedron Lett. 2002, 43, 6987) au lieu de la 2,4-difluoroaniline. On obtient 225 mg de N-(3-cyclopropylephényl)-2-[4-(morpholin-4-yl)-6-oxo-1 ,6-dihydropyrimidin-2-yl]acétamide sous forme de solide blanc dont les caractéristiques sont les suivantes:
Spectre RMN 1 H :0,50 à 0,68 (m, J=2,0 et 4,9 Hz, 2 H); 0,88 à 1,02 (m, 2H); 1,79 à 1,94 (m, J=4,4 et 4,4 Hz, 1 H); 3,42 (t, J=5,1 Hz, 4 H); 3,51 à 3,68 (m, 6 H); 5,20 (s, 1 H); 6,80 (d, J=8,3 Hz, 1 H); 7,17 (t, J=7,8 Hz, 1 H); 7,26 à 7,36 (m, 2 H); 10,06 (s large, 1 H); 11,63 (s large, 1 H)
Spectrométrie de Masse : méthode A
Temps de rétention Tr (min) =0.72
[M+H]+ : m/z 353 ; [M-H]- : m/z 355
Point de fusion (Kofler) = 246°C

### Exemple 37 : Synthèse du N-(2-hydroxyphényl)-2-[4-(morpholin-4-yl)-6-oxo-1,6-dihydropyrimidin-2-yl]acétamide

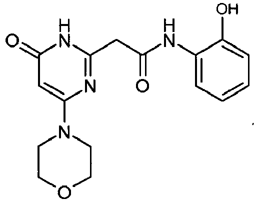

Le produit est préparé en suivant le mode opératoire décrit à l'exemple 5 à partir de 500 mg de [4-(morpholin-4-yl)-6-oxo-1,6-dihydropyrimidin-2-yl]acétate de sodium et 278 mg de 2-aminophénol au lieu de la 2,4-difluoroaniline. On obtient 370 mg de N-(2-hydroxyphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,45 (t, J=5,0 Hz, 4 H); 3,59 à 3,65 (m, 4 H); 3,69 (s, 2H); 5,21 (s, 1 H); 6,76 (t, J=7,8 Hz, 1 H); 6,87 (d, J=7,8 Hz, 1 H); 6,92 (t, J=8,5 Hz, 1 H); 7,86 (d, J=7,3Hz, 1 H); 9,46 (s large, 1 H); 9,82 (s large, 1 H); 11,63 (s large, 1 H)
Spectrométrie de Masse : méthode A
Temps de rétention Tr (min) = 2.58;
[M+H]+ : m/z 331 ; [M-H]- : m/z 329
Point de fusion (Kofler) > 260°C

### Exemple 38 : Synthèse du N-[3-(difluorométhoxy)phényl]-2-[4-(morpholin-4-yl)-6-oxo-1,6-dihydropyrimidin-2-yl]acétamide

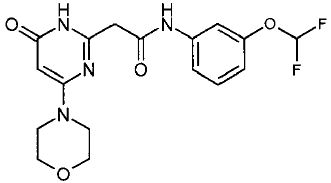

Le produit est préparé en suivant le mode opératoire décrit à l'exemple 5 à partir de 200 mg de [4-(morpholin-4-yl)-6-oxo-1,6-dihydropyrimidin-2-yl]acétate de sodium et 520 mg de 3-(difluorométhoxy)aniline au lieu de la 2,4-difluoroaniline. On obtient 135 mg de N-[3-(difluorométhoxy)phényl]-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz) : 3,36 à 3,48 (m, 4 H); 3,54 à 3,65 (m, 6 H); 5,20 (slarge, 1 H); 6,88 (s large, 2 H); 7,17 (t, J=69,0 Hz, 1 H); 7,36 (s, 1 H); 7,48 à 7,57 (m, 1 H); 10,34 (s large,1 H); 11,66 (s large, 1 H)
Spectrométrie de Masse : méthode A
Temps de rétention Tr (min) = 0.67;
[M+H]+ : m/z 381 ; [M-H]- : m/z 379

### Exemple 39: Synthèse du N-(4-fluoro-3-méthoxyphényl)-2-[4-(morpholin-4-yl)-6-oxo-1,6-dihydropyrimidin-2-yl]propanamide

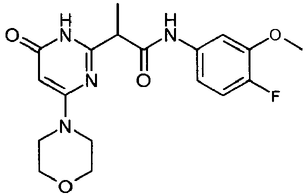

### Etape 1:

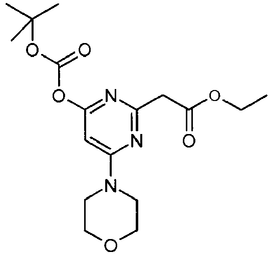

A une solution de 535 mg de [4-(morpholin-4-yl)-6-oxo-1,6-dihydropyrimidin-2-yl]acétate d'éthyle préparé à l'étape 1 de l'exemple 1dans 9 mL de N,N-diméthylformamide sous argon sont ajoutés 88 mg d'hydrure de sodium. Le mélange réactionnel est alors agité pendant 5 minutes à température ambiante, puis une solution de 655 mg de di-tert-butyl-dicarbonate dans 2 mL de N,N-diméthylformamide est ajoutée. Après 1 nuit d'agitation à température ambiante, le mélange réactionnel est concentré sous pression réduite. On ajoute 10 mL d'eau puis une solution d'acide chlorhydrique 1 N jusqu'à obtention d'un pH proche de 6. On extrait à l'acétate d'éthyle et la phase organique est séchée sur sulfate de magnésium puis concentrée à sec sous pression réduite. On obtient 735 mg de [4-({[(2-méthylpropan-2-yl)oxy]carbonyl}oxy)-6-(morpholin-4-yl)pyrimidin-2-yl]acétate d'éthyle sous forme d'une huile jaune.

### Etape 2 :

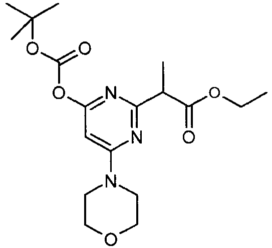

A une solution de 700 mg de [4-({[(2-méthylpropan-2-yl)oxy]carbonyl}oxy)-6-(morpholin-4-yl)pyrimidin-2-yl]acétate d'éthyle dans 8 mL de N,N-diméthylformamide sous argon sont ajoutés 84 mg d'hydrure de sodium. Le mélange réactionnel est alors agité pendant 15 minutes à 0°C. On ajoute alors 0.130 mL de d'iodure de méthane et agite à température ambiante pendant 1 nuit. On ajoute 0.5 mL d'eau et le mélange réactionnel est concentré à sec sous pression réduite. Après purification par chromatographie sur colonne de silice en éluant avec un gradient d'éluant heptane/acétate d'éthyle de 0% à 50% puis avec l'acétate d'éthyle à 100%. On obtient 150 mg de 2-[4-({[(2-méthylpropan-2-yl)oxy]carbonyl}oxy)-6-(morpholin-4-yl)pyrimidin-2-yl]propanoate d'éthyle sous forme d'une huile incolore.

### Etape 3 :

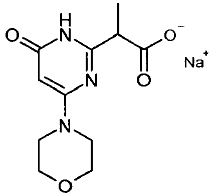

A une solution de 145 mg de 2-[4-({[(2-méthylpropan-2-yl)oxy]carbonyl}oxy)-6-(morpholin-4-yl)pyrimidin-2-yl]propanoate d'éthyle dans 5 mL de tétrahydrofurane, est ajouté 0.190 mL de soude 1 M. Après 1 nuit d'agitation à température ambiante, le mélange réactionnel est concentré à sec sous pression réduite. On obtient 100 mg de 2-[4-(morpholin-4-yl)-6-oxo-1,6-dihydropyrimidin-2-yl]propanoate de sodium sous forme de solide utilisé tel quel à l'étape d'après.

### Etape 4 :

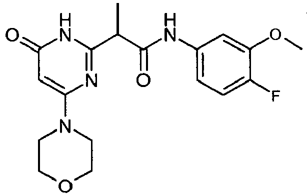

Le produit est préparé en suivant le mode opératoire décrit à l'exemple 5 à partir de 100 mg de 2-[4-(morpholin-4-yl)-6-oxo-1,6-dihydropyrimidin-2-yl]propanoate de sodium et 195 mg de 4-fluoro-3-méthoxyaniline au lieu de la 2,4-difluoroaniline. On obtient 30 mg de N-(4-fluoro-3-méthoxyphényl)-2-[4-(morphol in-4-yl)-6-oxo-1,6-dihydropyrimidin-2-yl]propanamide sous forme de solide blanc dont les caractéristiques sont les suivantes:
Spectre RMN 1H (400MHz) : 1,44 (d, J=7,1 Hz, 3 H); 3,36 à 3,47 (m, 4 H); 3,50 à3,65 (m, 4 H); 3,71 (q, J=6,8 Hz, 1 H); 3,79 (s, 3 H); 5,19 (s large, 1 H); 7,01 à 7,18(m, 2 H); 7,47 (dd,J=2,0 et 7,8 Hz, 1 H); 10,00 (s, 1 H); 11,55 (s large, 1 H)
Spectrométrie de Masse : méthode A
Temps de rétention Tr (min) = 0.65
[M+H]+ : m/z 377 ; [M-H]- : m/z 375

### Exemple 40 : Synthèse du N-(2,3-diméthylphényl)-2-[4-(morpholin-4-yl)-6-oxo-1,6-dihydropyrimidin-2-yl]acétamide

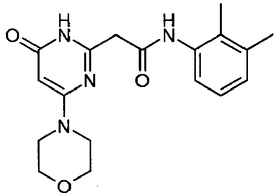

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 242 mg de 2,3-diméthylaniline au lieu de la 2,4-difluoroaniline. On obtient 190 mg de N-(2,3-d iméthylphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :2,08 (s, 3 H); 2,24 (s, 3 H); 3,41 à 3,47 (m, 4 H); 3,58à 3,65 (m, 6 H); 5,20 (s, 1 H); 6,99 à 7,08 (m, 2 H); 7,13 (d, J=7,6 Hz, 1 H); 9,57 (s, 1 H); 11,67 (s large, 1 H)
Spectrométrie de Masse : méthode A
Temps de rétention Tr (min) = 0.62
[M+H]+ : m/z 343 ; [M-H]- : m/z 341

### Exemple 41 : Synthèse du N-(2-fluoro-3-méthylphényl)-2-[4-(morpholin-4-yl)-6-oxo-1,6-dihydropyrimidin-2-yl]acétamide

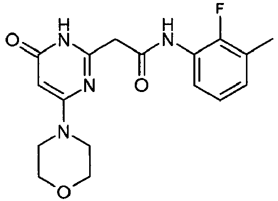

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 250 mg de 2-fluoro-3-méthylaniline au lieu de la 2,4-difluoroaniline. On obtient 208 mg de N-(2-fluoro-3-méthylphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :2,24 (s large, 3 H); 3,37 à 3,48 (m, 4 H); 3,56 à 3,72(m, 6 H); 5,20 (s large, 1 H); 6,97 à 7,08 (m, 2 H); 7,71 (s large, 1 H); 9,89 (s large, 1 H); 11,66 (s large, 1 H)
Spectrométrie de Masse : méthode A
Temps de rétention Tr (min) = 0.65
[M+H]+ : m/z 347 ; [M-H]- : m/z 345

### Exemple 42 : Synthèse du N-(1,3-benzoxazol-4-yl)-2-[4-(morpholin-4-yl)-6-oxo-1,6-dihydropyrimidin-2-yl]acétamide

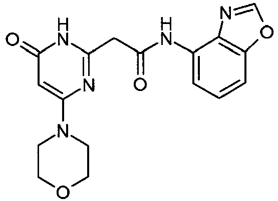

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 268 mg de 1,3-benzoxazol-4-amine au lieu de la 2,4-difluoroaniline. On obtient 193 mg de N-(1,3-benzoxazol-4-yl)-2-[4-(morpholin-4-yl)-6-oxo-1,6-dihydropyrimidin-2-yl]acétamide sous forme de solide écru dont les caractéristiques sont les suivantes:
Spectre RMN 1 H (400MHz) : 3,43 à 3,48 (m, 4 H); 3,62 (t, J=4,9 Hz, 4 H); 3,80 (s, 2H); 5,21 (s, 1 H); 7,36 à 7,42 (m, 1 H); 7,49 (d, J=8,3 Hz, 1 H); 8,08 (d, J=7,8 Hz, 1 H); 8,78 (s, 1 H); 10,44(s large, 1 H); 11,73 (s large, 1 H)
Spectrométrie de Masse : méthode A
Temps de rétention Tr (min) = 0.55
[M+H]+ : m/z 356 ; [M-H]- : m/z 354

### Exemple 43: Synthèse du 2-[4-(morpholin-4-yl)-6-oxo-1,6-dihydropyrimidin-2-yl]-N-[3-(trifluorométhoxy)phényl]acétamide

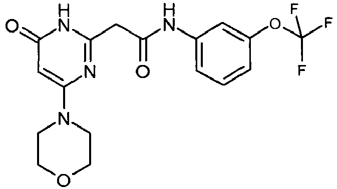

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 270 mg de 3-(trifluorométhoxy)aniline au lieu de la 2,4-difluoroaniline. On obtient 230 mg de 2-[4-(morpholin-4-yl)-6-oxo-1,6-dihydropyrimidin-2-yl]-N-[3-(trifluorométhoxy)phényl]acétamide sous forme de solide blanc dont les caractéristiques sont les suivantes:
Spectre RMN 1 H :3,37 à 3,44 (m, 4 H); 3,54 à 3,64 (m, 6 H); 5,20 (s, 1 H); 7,05 (d, J=6,6 Hz, 1 H); 7,41 à 7,48 (m, 2 H); 7,74 (s, 1 H); 10,45 (s large, 1 H); 11,66 (s large, 1 H)
Spectrométrie de Masse : méthode A
Temps de rétention Tr (min) = 0.78;
[M+H]+ : m/z 399 ; [M-H]- : m/z 397

### Exemple 44: Synthèse du 2-[4-(morpholin-4-yl)-6-oxo-1,6-dihydropyrimidin-2-yl]-N-[3-(propan-2-yloxy)phényl]acétamide

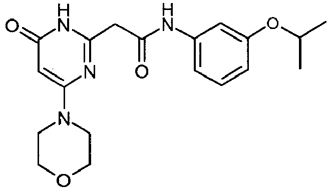

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 572 mg de 3-isopropoxyaniline au lieu de la 2,4-difluoroaniline. On obtient 228 mg de 2-[4-(morpholin-4-yl)-6-oxo-1,6-dihydropyrimidin-2-yl]-N-[3-(propan-2-yloxy)phényl]acétamide sous forme de solide blanc dont les caractéristiques sont les suivantes:
Spectre RMN 1 H :3,35 (t, J=5,0 Hz, 4 H); 3,56 (t, J=4,9 Hz, 4 H); 4,25(s, 2 H); 5,25 (s large, 1 H); 7,31 à 7,40 (m, 2 H); 7,62 à 7,74 (m, 2 H); 11,92 (s large, 1 H)
Spectrométrie de Masse : méthode A
Temps de rétention Tr (min) = 0.60;
[M+H]+ : m/z 313 ; [M-H]- : m/z 311

### Exemple 45: Synthèse du N-(4-fluoro-2-méthoxyphényl)-2-[4-(morpholin-4-yl)-6-oxo-1,6-dihydropyrimidin-2-yl]acétamide

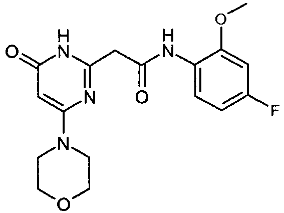

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 460 mg de 4-fluoro-2-méthoxyaniline au lieu de la 2,4-difluoroaniline. On obtient 245 mg de N-(4-fluoro-2-méthoxyphényl)-2-[4-(morpholin-4-yl)-6-oxo-1,6-dihydropyrimidin-2-yl]acétamide sous forme de solide rosé dont les caractéristiques sont les suivantes:
Spectre RMN 1 H :3,43 (m, 4 H) ; 3,62 (m, 4 H) ; 3,67 (s, 2 H) ; 3,85 (s,3 H) ; 5,20 (s, 1 H) ; 6,74 (dt, J=2,8 et 8,6 Hz, 1 H) ; 6,98 (dd, J=2,8 et 10,9 Hz, 1 H) ;7,88 (dd, J=6,7 et8,6 Hz, 1 H); 9,44 (s, 1 H) ; 11,67 (s large, 1 H)
Spectrométrie de Masse : méthode A
Temps de rétention Tr (min) = 0,62 ;
[M+H]+ : m/z 363 ; [M-H]- : m/z 361

### Exemple 46: Synthèse du {2-[3-({[4-(morpholin-4-yl)-6-oxo-1,6-dihydropyrimidin-2-yl]acétyl}amino)phényl]éthyl}carbamate de 2-méthylpropan-2-yle

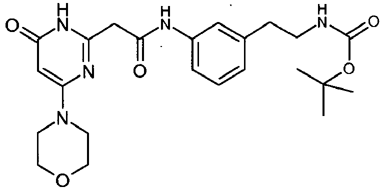

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 930 mg de [2-(3-aminophényl)éthyl]carbamate de 2-méthylpropan-2-yle au lieu de la 2,4-difluoroaniline. On obtient 285 mg de {2-[3-({[4-(morpholin-4-yl)-6-oxo-1,6-dihydropyrimidin-2-yl]acétyl}amino)phényl]éthyl}carbamate de 2-méthylpropan-2-yle sous forme de solide rosé dont les caractéristiques sont les suivantes:
Spectre RMN 1 H :1,36 (s, 9 H) ; 2,61 à 2,68 (m, 2 H) ; 3,04 à 3,14 (m,2 H) ; 3,41 m, 4 H) ; 3,56 à 3,63 (m, 6 H) ; 5,20 (s, 1 H) ; 6,84 (t large, J=6,7 Hz, 1 H) ; 6,89 (d, J=8,1 Hz, 1 H) ; 7,22 (t, J=8,1 Hz, 1 H) ; 7,36 à 7,44 (m, 2 H) ; 10,09 (s large, 1 H) ; 11,63 (s large,1 H)
Spectrométrie de Masse : méthode A
Temps de rétention Tr (min) = 0,75 ;
[M+H]+ : m/z 458 ; [M-H]- : m/z 456
Point de fusion = 194 °C

### Exemple 47 : Synthèse du N-[4-fluoro-3-(trifluorométhyl)phényl]-2-[4-(morpholin-4-yl)-6-oxo-1,6-dihydropyrimidin-2-yl]acétamide

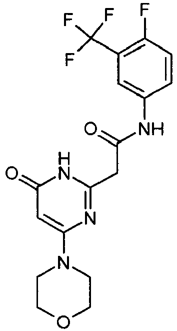

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 358 mg de 4-fluoro-3-(trifluorométhylaniline au lieu de la 2,4-difluoroaniline. On obtient 222 mg de N-[4-fluoro-3-(trifluorométhyl)phényl]-2-[4-(morpholin-4-yl)-6-oxo-1,6-dihydropyrimidin-2-yl]acétamide sous forme de solide blanc dont les caractéristiques sont les suivantes:
Spectre RMN 1 H :3,39 à 3,42 (m, 4 H) ; 3,57 à 3,61 (m, 4 H) ; 3,62 (s,2 H) ; 5,21 (s, 1 H) ; 7,49 (t, J=9,8 Hz, 1 H) ; 7,75 à 7,83 (m, 1 H) ; 8,06 (dd, J=2,4 et6,4 Hz, 1 H) ; 10,53 (s large, 1 H) ; 11,68 (s large, 1 H)
Spectrométrie de Masse : méthode A
Temps de rétention Tr (min) = 0,77 ;
[M+H]+ : m/z 401 ; [M-H]- : m/z 399

### Exemple 48 : Synthèse du N-(3-éthynylphényl)-2-[4-(morpholin-4-yl)-6-oxo-1,6-dihydropyrimidin-2-yl]acétamide

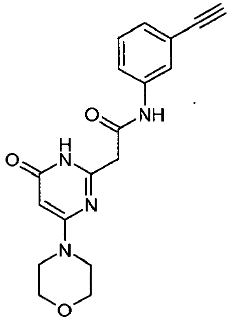

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 201 mg de 3-aminophénylacétylène au lieu de la 2,4-difluoroaniline. On obtient 190 mg de N-(3-éthynylphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H :3,39 à 3,44 (m, 4 H) ; 3,58 à 3,64 (m, 6 H) ; 4,16 (s,1 H) ; 5,20 (s, 1 H) ; 7,17 (d, J=7,8 Hz, 1 H) ; 7,33 (t, J=7,8 Hz, 1 H) ; 7,54 (d, J=7,8Hz, 1 H) ; 7,75 (s, 1H) ; 10,27 (s, 1 H) ; 11,66 (s large, 1 H)
Spectrométrie de Masse : méthode A
Temps de rétention Tr (min) = 0,64 ;
[M+H]+ : m/z 339 ; [M-H]- : m/z 337

### Exemple 49: Synthèse du N-[3-(cyclopentyloxy)phényl]-2-[4-(morpholin-4-yl)-6-oxo-1,6-dihydropyrimidin-2-yl]acétamide

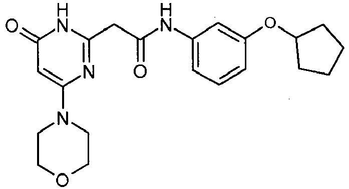

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 354 mg de 3-(cyclopentyloxy)aniline au lieu de la 2,4-difluoroaniline. On obtient 269 mg de N-[3-(cyclopentyloxy)phényl]-2-[4-(morpholin-4-yl)-6-oxo-1,6-dihydropyrimidin-2-yl]acétamide sous forme de solide blanc dont les caractéristiques sont les suivantes:
Spectre RMN 1 H :1,50 à 1,75 (m, 6 H) ; 1,83 à 1,96 (m, 2 H) ; 3,39 à3,44 (m, 4 H) ; 3,54 à 3,63 (m, 6 H) ; 4,73 (m, 1 H) ; 5,20 (s, 1 H) ; 6,60 (dd, J=2,0 et8,3 Hz, 1 H) ; 7,03 (dlarge, J=8,3 Hz, 1 H) ; 7,18 (t, J=8,3 Hz, 1 H) ; 7,26 (t, J=2,2 Hz, 1 H) ; 10,10 (s, 1 H) ; 11,64 (s large, 1 H)
Spectrométrie de Masse : méthode A
Temps de rétention Tr (min) = 0,84 ;
[M+H]+ : m/z 399 ; [M-H]- : m/z 397

### Exemple 50: Synthèse du N-(4-fluoro-2-hydroxyphényl)-2-[4-(morpholin-4-yl)-6-oxo-1,6-dihydropyrimidin-2-yl]acétamide (VAC. SON4.056.1)

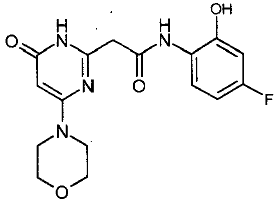

Le produit est préparé en suivant le mode opératoire décrit à l'exemple 5 à partir de 500 mg de [4-(morpholin-4-yl)-6-oxo-1,6-dihydropyrimidin-2-yl]acétate de sodium et 815 mg de 2-amino-5-fluorophénol au lieu de la 2,4-difluoroaniline. Après purification par chromatographie sur colonne de silice, éluant : CH2Cl2/MeOH 95/5, on obtient 149 mg de N-(4-fluoro-2-hydroxyphényl)-2-[4-(morpholin-4-yl)-6-oxo-1,6-dihydropyrimidin-2-yl]acétamide sous forme de solide gris dont les caractéristiques sont les suivantes:
Spectre RMN 1 H :3,41 à 3,47 (m, 4 H) ; 3,59 à 3,64 (m, 4 H) ; 3,67 (s, 2 H) ; 5,20 (s, 1 H) ; 6,58 (dt, J=2,8 et 8,8 Hz, 1 H) ; 6,66 (dd, J=2,8 et 10,4 Hz, 1 H) ; 7,80 (dd, J=6,4 et 8,8 Hz, 1 H) ; 8,66 à 12,13 (m étalé, 3 H)
Spectrométrie de Masse : méthode B
Temps de rétention Tr (min) = 2,74
[M+H]+ : m/z 349 ; [M-H]- : m/z 347

### Exemple 51 : Synthèse du 2-[2-(2,3-dihydro-1H-indol-1-yl)-2-oxoéthyl]-6-(morpholin-4-yl)pyrimidin-4(3H)-one (VAC. PSB2.078.6)

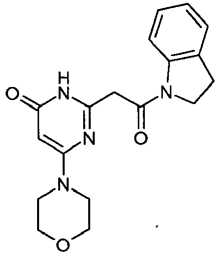

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 238 mg de indoline au lieu de la 2,4-difluoroaniline. On obtient 230 mg de 2-[2-(2,3-dihydro-1H-indol-1-yl)-2-oxoéthyl]-6-(morpholin-4-yl)pyrimidin-4(3H)-one sous forme de solide rose pâle dont les caractéristiques sont les suivantes:
Spectre RMN 1 H :3,17 (t, J=8,3 Hz, 2 H) ; 3,41 (m, 4 H) ; 3,60 (m, 4 H) ; 3,75 (s, 2 H) ; 4,14 (t, J=8,3 Hz, 2 H) ; 5,21 (s, 1 H) ; 7,01 (t, J=7,6 Hz, 1 H) ; 7,16 (t, J=7,6 Hz, 1 H) ; 7,25 (d, J=7,6 Hz, 1 H) ; 8,02 (d, J=7,6 Hz, 1 H) ; 11,61 (s large, 1 H)
Spectrométrie de Masse : méthode A
Temps de rétention Tr (min) = 0,64
[M+H]+ : m/z 341 ; [M-H]- : m/z 339

### Exemple 52: Synthèse du N-(3-cyclopropyle-4-fluorophényl)-2-[4-(morpholin-4-yl)-6-oxo-1,6-dihydropyrimidin-2-yl]acétamide (VAC. PSB2.078.11)

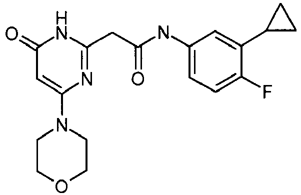

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 302 mg de 3-cyclopropyle-4-fluoroaniline (préparé selon la demande de brevet US 2007/0185058.) au lieu de la 2,4-difluoroaniline. On obtient 225 mg de N-(3-cyclopropyle-4-fluorophényl)-2-[4-(morpholin-4-yl)-6-oxo-1,6-dihydropyrimidin-2-yl]acétamide sous forme de solide jaune pâle dont les caractéristiques sont les suivantes:
Spectre RMN 1 H :0,62 (m, 2 H) ; 0,99 (m, 2 H) ; 1,97 à 2,09 (m, 1 H) ; 3,41 (m, 4 H) ; 3,56 (s, 2 H) ; 3,58 à 3,63 (m, 4 H) ; 5,20 (s, 1 H) ; 7,07 (t, J=9,5 Hz, 1 H) ; 7,18 (dd, J=2,3 et 7,0 Hz, 1 H) ; 7,30 à 7,37 (m, 1 H) ; 10,11 (s, 1 H) ; 11,63 (s large, 1 H)
Spectrométrie de Masse : méthode A
Temps de rétention Tr (min) = 0,75
[M+H]+ : m/z 373 ; [M-H]- : m/z 371

### Exemple 53 : Synthèse du 2-[4-(morpholin-4-yl)-6-oxo-1,6-dihydropyrimidin-2-yl]-N-(2,3,4-trifluorophényl)acétamide (VAC. PSB2.078.12)

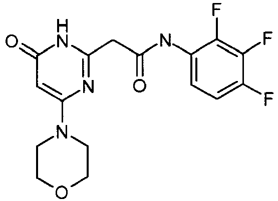

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 294 mg de 2,3,4-trifluoroaniline au lieu de la 2,4-difluoroaniline. On obtient 195 mg de 2-[4-(morpholin-4-yl)-6-oxo-1,6-dihydropyrimidin-2-yl]-N-(2,3,4-trifluorophényl)acétamide sous forme de solide blanc dont les caractéristiques sont les suivantes:
Spectre RMN 1 H :3,43 (m, 4 H) ; 3,61 (m, 4 H) ; 3,67 (s, 2 H) ; 5,20(s, 1 H) ; 7,24 à 7,35 (m, 1 H) ; 7,56 à 7,67 (m, 1 H) ; 10,18 (s large, 1 H) ; 11,67 (s large, 1 H)
Spectrométrie de Masse : méthode A
Temps de rétention Tr (min) = 0,65
[M+H]+ : m/z 369 ; [M-H]- : m/z 367

**Exemple 54 : Synthèse du N-[4-fluoro-3-(trifluorométhoxy)phényl]-2-[4-(morpholin-4-yl)-6-oxo-1,6-dihydropyrimidin-2-yl]acétamide**

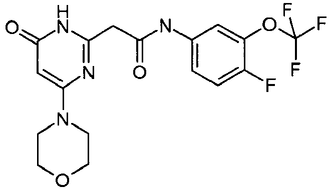

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 270 mg de 4-fluoro-3-(trifluorométhoxy)aniline au lieu de la 2,4-difluoroaniline. On obtient 270 mg de N-[4-fluoro-3-(trifluorométhoxy)phényl]-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,37 à 3,44 (m, 4 H) ; 3,55 à 3,65 (m, 6 H) ; 5,20 (s, 1 H) ; 7,40 à 7,59 (m, 2 H) ; 7,90 (d large, *J*=6,8 Hz, 1 H) ; 10,48 (s large, 1H) ; 11,66 (s large, 1 H)
Spectrométrie de Masse : méthode A
Temps de rétention Tr (min) = 0,80
[M+H]+ : m/z 417 ; [M-H]- : m/z 415

### Exemple 55: Synthèse du N-[3-(2-hydroxyéthoxy)phényl]-2-[4-(morpholin-4-yl)-6-oxo-1,6-dihydropyrimidin-2-yl]acétamide

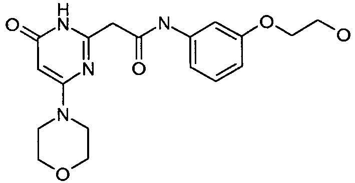

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 294 mg de 2-(3-aminophénoxy)-éthanol au lieu de la 2,4-difluoroaniline. On obtient 180 mg de N-[3-(2-hydroxyéthoxy)phényl]-2-[4-(morpholin-4-yl)-6-oxo-1,6-dihydropyrimidin-2-yl]acétamide sous forme de solide rosé dont les caractéristiques sont les suivantes:
Spectre RMN 1 H (400MHz):
   Pour ce lot, nous observons des signaux larges avec : 3,42 (m, 4 H) ; 3,59 (m, 6 H) ; 3,70 (m, 2 H) ; 3,93 (m, 2 H) ; 4,82 (m, 1 H) ; 5,20 (s, 1 H) ; 6,64 (d, *J*=8,1 Hz, 1 H) ; 7,07 (d, *J*=8,1 Hz, 1 H) ; 7,15 à 7,23 (t, *J*=8,1 Hz, 1H) ; 7,28 (s, 1H) ; 10,12 (s, 1H) ; 11,65 (s, 1H)
Spectrométrie de Masse: méthode A
Temps de rétention Tr (min) = 0,46
[M+H]+ : m/z 375 ; [M-H]- : m/z 373

### Exemple 56 : Synthèse du N-(3-iodophényl)-2-[4-(morpholin-4-yl)-6-oxo-1,6-dihydropyrimidin-2-yl]acétamide

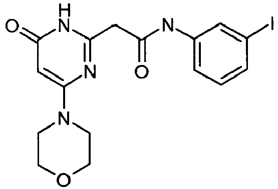

Le produit est préparé en suivant le mode opératoire décrit à l'exemple 5 à partir de 268 mg de [4-(morpholin-4-yl)-6-oxo-1,6-dihydropyrimidin-2-yl]acétate de sodium et 324 mg de 3-iodoaniline au lieu de la 2,4-difluoroaniline. On obtient 345 mg de N-(3-iodophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,41 (m, 4 H) ; 3,58 à 3,64 (m, 6 H) ; 5,20 (s, 1 H) ; 7,12 (t, *J*=8,1 Hz, 1 H) ; 7,42 (d, *J*=8,1 Hz, 1 H) ; 7,50 (d, *J*=8,1 Hz, 1 H) ; 8,06 (s, 1 H) ; 10,26 (s large, 1 H) ; 11,66 (s large, 1 H)
Spectrométrie de Masse : méthode B
Temps de rétention Tr (min) = 3,41
[M+H]+ : m/z 441 ; [M-H]- : m/z 439

### Exemple 57: Synthèse du 2-fluoro-5-({[4-(morpholin-4-yl)-6-oxo-1,6-dihydropyrimidin-2-yl]acétyl}amino)benzoate de méthyle

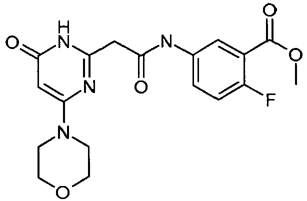

Le produit est préparé en suivant le mode opératoire décrit à l'exemple 5 à partir de 500 mg de [4-(morpholin-4-yl)-6-oxo-1,6-dihydropyrimidin-2-yl]acétate de sodium et 466 mg de 5-amino-2-fluorobenzoate de méthyle au lieu de la 2,4-difluoroaniline. On obtient 625 mg de 2-fluoro-5-({[4-(morpholin-4-yl)-6-oxo-1,6-dihydropyrimidin-2-yl]acétyl}amino)benzoate de méthyle sous forme de solide rosé dont les caractéristiques sont les suivantes :
Spectre RMN 1 H (400MHz) :
   3,41 (m, 4 H) ; 3,57 à 3,62 (m, 6 H) ; 3,85 (s, 3 H) ; 5,20 (s, 1 H) ; 7,32 (dd, *J*=9,0 et 10,6 Hz, 1 H) ; 7,79 (ddd, *J*=2,9 et 4,1 et 9,0 Hz, 1 H) ; 8,15 (dd, *J*=2,9 et 6,4 Hz, 1 H) ; 10,40 (s, 1 H) ; 11,67 (s large, 1 H)
Spectrométrie de Masse : méthode A
Temps de rétention Tr (min) = 0,59
[M+H]+ : m/z 391 ; [M-H]- : m/z 389

### Exemple 58 : Synthèse du N-(3-éthoxyphényl)-2-[4-(morpholin-4-yl)-6-oxo-1,6-dihydropyrimidin-2-yl]acétamide

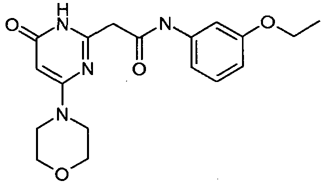

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 540 mg de 3-éthoxyaniline au lieu de la 2,4-difluoroaniline. On obtient 235 mg de N-(3-éthoxyphényl)-2-[4-(morpholin-4-yl)-6-oxo-1,6-dihydropyrimidin-2-yl]acétamide sous forme de solide blanc dont les caractéristiques sont les suivantes :
Spectre RMN 1 H (400MHz):
   1,31 (t, *J*=7,0 Hz, 3 H) ; 3,41 (m, 4 H) ; 3,55 à 3,64 (m, 6 H) ; 3,98 (q, J=7,0 Hz, 2 H) ; 5,20 (s, 1 H) ; 6,62 (d, *J*=8,0 Hz, 1 H) ; 7,06 (d, *J*=8,0 Hz, 1 H) ; 7,19 (t, *J*=8,0 Hz, 1 H) ; 7,26 (s large, 1 H) ; 10,12 (s large, 1 H) ; 11,64 (s large, 1 H)
Spectrométrie de Masse : méthode A
Temps de rétention Tr (min) = 0,66
[M+H]+ : m/z 359 ; [M-H]- : m/z 357

### Exemple 59 : Synthèse du N-(2,4-difluoro-3-méthoxyphényl)-2-[4-(morpholin-4-yl)-6-oxo-1,6-dihydropyrimidin-2-yl]acétamide

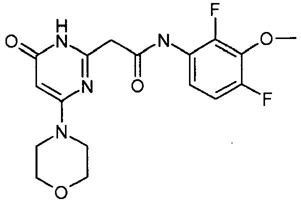

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 318 mg de 2,4-difluoro-3-méthoxyaniline au lieu de la 2,4-difluoroaniline. On obtient 255 mg de N-(2,4-difluoro-3-méthoxyphényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,43 (m, 4 H) ; 3,61 (m, 4 H) ; 3,66 (s, 2 H) ; 3,93 (s, 3 H) ; 5,20 (s, 1 H) ; 7,11 (ddd, *J*=2,1 et 9,0 et 10,9 Hz, 1 H) ; 7,50 (dt, *J*=5,5 et 9,0 Hz, 1H) ; 10,00 (s, 1 H) ; 11,65 (s large, 1 H)
Spectrométrie de Masse : méthode B
Temps de rétention Tr (min) = 3,01
[M+H]+ : m/z 381 ; [M-H]- : m/z 379

### Exemple 60 : Synthèse du 2-[4-(morpholin-4-yl)-6-oxo-1,6-dihydropyrimidin-2-yl]-N-(2,4,5-trifluorophényl)acétamide

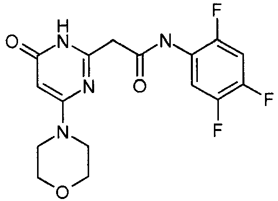

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 294 mg de 2,4,5-trifluoroaniline au lieu de la 2,4-difluoroaniline. On obtient 230 mg de 2-[4-(morpholin-4-yl)-6-oxo-1,6-dihydropyrimidin-2-yl]-N-(2,4,5-trifluorophényl)acétamide sous forme de solide blanc dont les caractéristiques sont les suivantes:
Spectre RMN 1 H (400MHz):
   3,42 (m, 4 H) ; 3,61 (m, 4 H) ; 3,69 (s, 2 H) ; 5,20 (s, 1 H) ; 7,65 (dt, *J*=7,6 et 10,8 Hz, 1 H); 8,01 (td, *J*=8,0 et 12,3 Hz, 1 H) ; 10,18 (s large, 1 H) ; 11,67 (s large, 1 H)
Spectrométrie de Masse : méthode B
Temps de rétention Tr (min) = 3,10
[M+H]+ : m/z 369 ; [M-H]- : m/z 367

### Exemple 61 : Synthèse du N-(3,5-dichloro-4-fluorophényl)-2-[4-(morpholin-4-yl)-6-oxo-1,6-dihydropyrimidin-2-yl]acétamide

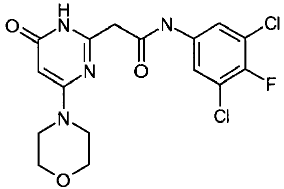

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 360 mg de 3,5-dichloro-4-fluoroaniline au lieu de la 2,4-difluoroaniline. On obtient 259 mg de N-(3,5-dichloro-4-fluorophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,41 (m, 4 H) ; 3,56 à 3,64 (m, 6 H) ; 5,21 (s, 1 H) ; 7,74 (d, *J*=6,1 Hz, 2 H) ; 10,50 (s large, 1 H) ; 11,66 (s large, 1 H)
Spectrométrie de Masse : méthode A
Temps de rétention Tr (min) = 0,82
[M+H]+ : m/z 401 ; [M-H]- : m/z 399

### Exemple 62 : Synthèse du 2-[2-(2,3-dihydro-4H-1,4-benzoxazin-4-yl)-2-oxoéthyl]-6-(morpholin-4-yl)pyrimidin-4(3H)-one

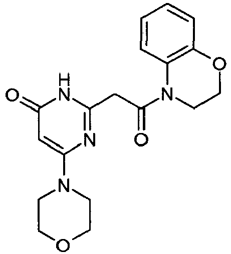

Le produit est préparé en suivant le mode opératoire décrit à l'exemple 5 à partir de 260 mg de [4-(morpholin-4-yl)-6-oxo-1,6-dihydropyrimidin-2-yl]acétate de sodium et 270 mg de 3,4-dihydro-2H-1,4-benzoxazine au lieu de la 2,4-difluoroaniline. Après purification par chromatographie sur colonne de silice, éluant CH2Cl2/MeOH 95/05, on obtient 150 mg de 2-[2-(2,3-dihydro-4H-1,4-benzoxazin-4-yl)-2-oxoéthyl]-6-(morpholin-4-yl)pyrimidin-4(3H)-one sous forme de solide blanc dont les caractéristiques sont les suivantes:
Spectre RMN 1 H (400MHz):
   3,36 à 3,45 (m, 4 H) ; 3,52 à 3,70 (m, 4 H) ; 3,81 à 3,95 (m, 4 H) ; 4,30 (m, 2 H) ; 5,18 (s, 1 H) ; 6,81 à 6,95 (m, 2 H) ; 6,99 à 7,16 (m, 1H) ; 7,22 à 8,18 (m, 1H) ; 11,59 (s large, 1H)
Spectrométrie de Masse : méthode A
Temps de rétention Tr (min) = 0,62
[M+H]+ : m/z 357 ; [M-H]- : m/z 355

### Exemple 63 : Synthèse du N-(4-fluoro-3-nitrophényl)-2-[4-(morpholin-4-yl)-6-oxo-1,6-dihydropyrimidin-2-yl]acétamide

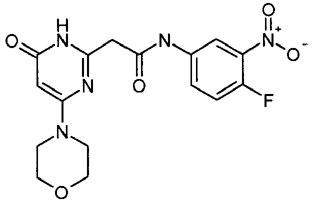

Le produit est préparé en suivant le mode opératoire décrit à l'exemple 5 à partir de 500 mg de [4-(morpholin-4-yl)-6-oxo-1,6-dihydropyrimidin-2-yl]acétate de sodium et 510 mg de 4-fluoro-3-nitroaniline au lieu de la 2,4-difluoroaniline. On obtient 339 mg de N-(4-fluoro-3-nitrophényl)-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,41 (m, 4 H) ; 3,58 à 3,62 (m, 4 H) ; 3,64 (s, 2 H) ; 5,21 (s, 1 H) ; 7,56 (dd, *J*=9,0 et 11,2 Hz, 1 H) ; 7,83 à 7,88 (ddd, *J*=2,9 et 4,0 et 9,0 Hz, 1 H) ; 8,47 (dd, *J*=2,9 et 6,8 Hz, 1 H) ; 10,63 (s large, 1 H) ; 11,69 (s large, 1 H)
Spectrométrie de Masse : méthode A
Temps de rétention Tr (min) = 0,63
[M+H]+ : m/z 378 ; [M-H]- : m/z 376

### Exemple 64 : Synthèse de l'acide 2-fluoro-5-({[4-(morpholin-4-yl)-6-oxo-1,6-dihydropyrimidin-2-yl]acétyl}amino)benzoïque

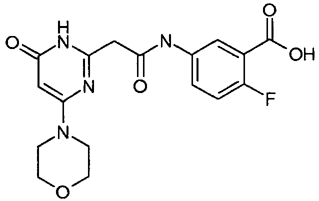

A une solution de 310 mg de 2-fluoro-5-({[4-(morpholin-4-yl)-6-oxo-1,6-dihydropyrimidin-2-yl]acétyl}amino)benzoate de méthyle préparé à l'exemple 57 dans 7 mL de méthanol sont ajoutés 0.4 mL de soude 2M. Après une nuit d'agitation à température ambiante, on ajoute de nouveau 0.4 mL de soude 2M et porte au reflux pendant 3 heures. Après refroidissement, le mélange réactionnel est concentré sous pression réduite. Le résidu d'évaporation est repris par de l'eau. La phase aqueuse est extraite avec de l'acétate d'éthyle puis, acidifiée avec une solution aqueuse d'acide chlorhydrique 1 N (pH = 5). On filtre l'insoluble. Le filtrat est concentré sous pression réduite puis, repris par de l'eau et quelques gouttes d'une solution aqueuse d'acide chlorhydrique 1 N. Le précipité formé est filtré, rincé à l'éther de pétrole et concentré à sec sous pression réduite. On obtient 52 mg de l'acide 2-fluoro-5-({[4-(morpholin-4-yl)-6-oxo-1,6-dihydropyrimidin-2-yl]acétyl}amino)benzoïque sous forme de solide rose pâle dont les caractéristiques sont les suivantes:
Spectre RMN 1 H (400MHz) :
   3,41 (m, 4 H) ; 3,60 (t, *J*=4,9 Hz, 6 H) ; 5,20 (s, 1 H) ; 7,24 (t, *J*=9,8 Hz, 1 H) ; 7,75 (m, 1 H) ; 8,06 (m, 1H) ; 10,34 (s large, 1H) ; 11,67 (s large, 1 H)
Spectrométrie de Masse : méthode A
Temps de rétention Tr (min) = 0,46
[M+H]+ : m/z 377 ; [M-H]- : m/z 375

### Exemple 65 : Synthèse du N-(5-fluoro-2-hydroxyphényl)-2-[4-(morpholin-4-yl)-6-oxo-1,6-dihydropyrimidin-2-yl]acétamide

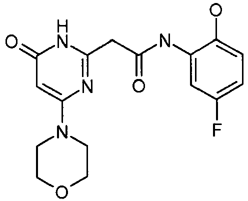

Le produit est préparé en suivant le mode opératoire décrit à l'exemple 5 à partir de 1 g de [4-(morpholin-4-yl)-6-oxo-1,6-dihydropyrimidin-2-yl]acétate de sodium et 466 mg de 2-amino-4-fluorophénol au lieu de la 2,4-difluoroaniline. On obtient 795 mg de 2 N-(5-fluoro-2-hydroxyphényl)-2-[4-(morpholin-4-yl)-6-oxo-1,6-dihydropyrimidin-2-yl]acétamide sous forme de solide marron dont les caractéristiques sont les suivantes :
Spectre RMN 1 H (400MHz):
   3,44 (m, 4 H) ; 3,59 à 3,65 (m, 4 H) ; 3,72 (s, 2 H) ; 5,21 (s, 1 H) ; 6,70 à 6,78 (m, 1 H) ; 6,81 à 6,87 (m, 1 H) ; 7,85 (dd, *J*=3,0 et 10,8 Hz, 1 H) ; 9,10 à 10,09 (m étalé, 1 H) ; 10,64 à 11,96 (m étalé, 1 H)
Spectrométrie de Masse : méthode A
Temps de rétention Tr (min) = 0,55
[M+H]+ : m/z 349 ; [M-H]- : m/z 347

### Exemple 66: Synthèse du N-(2-bromo-4-fluorophényl)-2-[4-(morpholin-4-yl)-6-oxo-1,6-dihydropyrimidin-2-yl]acétamide

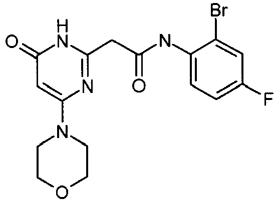

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 260 mg de 2-bromo-4-fluoroaniline au lieu de la 2,4-difluoroaniline. On obtient 310 mg de N-(2-bromo-4-fluorophényl)-2-[4-(morpholin-4-yl)-6-oxo-1,6-dihydropyrimidin-2-yl]acétamide sous forme de solide marron dont les caractéristiques sont les suivantes :
Spectre RMN 1 H (400MHz):
   Pour ce lot, tous les signaux sont larges avec : 3,44 (m, 4 H) ; 3,62 (m, 4 H) ; 3,65 (s, 2 H) ; 5,20 (s, 1 H) ; 7,22 à 7,32 (m, 1 H) ; 7,58 à 7,67 (m, 2 H) ; 9,73 (s, 1 H) ; 11,68 (s, 1 H)
Spectrométrie de Masse: méthode A
Temps de rétention Tr (min) = 0,65
[M+H]+ : m/z 411 ; [M-H]- : m/z 409

### Exemple 67 : Synthèse du N-(4-fluorophényl)-2-[1-méthyl-4-(morpholin-4-yl)-6-oxo-1,6-dihydropyrimidin-2-yl]acétamide

### Etape 1:

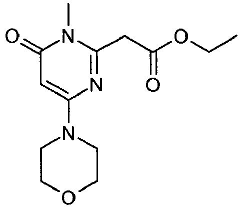

A une solution de 500 mg de [4-(morpholin-4-yl)-6-oxo-1,6-dihydropyrimidin-2-yl]acétate d'éthyle préparé à l'étape 1 de l'exemple 1 préparé à l'étape 1 de l'exemple 1 dans 1.5 mL de dioxanne, 330 mg de carbonate de potassium et 150 mL d'iodure de méthyle. Le mélange réactionnel est chauffé à 40 °C pendant 16 puis refroidi à température ambiante. La suspension est filtrée sur verre fritté puis rincée avec du dioxanne et le filtrat est concentré sous pression réduite. Le résidu est purifié par chromatographie sur colonne de silice, en éluant avec un mélange de dichlorométhane, d'acétonitrile et de méthanol (98/01/01, 96/02/02 puis, 90/05/05 V/V/V). On obtient 200 mg de [1-méthyl-4-(morpholin-4-yl)-6-oxo-1,6-dihydropyrimidin-2-yl]acétate d'éthyle sous forme de solide blanc dont les caractéristiques sont les suivantes :
Spectrométrie de Masse : méthode A
Temps de rétention Tr (min) = 0,53
[M+H]+ : m/z 282 ; [M-H]- : m/z 280

### Etape 2:

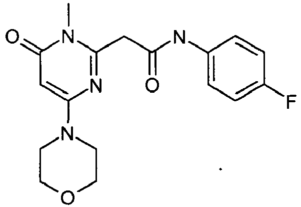

A une soluiton de 190 mg de [1-méthyl-4-(morpholin-4-yl)-6-oxo-1,6-dihydropyrimidin-2-yl]acétate d'éthyle préparé à l'étape 1 de l'exemple 67 dans 10 mL de toluène sont ajoutés 0.135 mL de 4-fluoroaniline et 0.670 mL d'une solution de triméthylaluminium 2M goutte à goutte. Après agitation pendant 30 minutes à température ambiante, on ajoute 10 mL de toluène. Après 4 heures d'agitation à température ambiante, le mélange réactionnel est coulé sur de l'eau et on ajoute une solution de phosphate de potassium 1 M. Le précipité est filtré sur verre fritté puis rincé avec de l'acétate d'éthyle. Le filtrat est alors lavé avec de l'eau puis avec une solution aqueuse saturée en chlorure de sodium. La phase organique est extraite puis séchée sur sulfate de magnésium, filtrée sur verre fritté et concentrée sous pression reduite. Après purification du résidu par chromatographie sur colonne de silice en éluant avec un mélange de dichlorométhane et de méthanol (95/05, V/V) on obtient 20 mg de N-(4-fluorophényl)-2-[1-méthyl-4-(morpholin-4-yl)-6-oxo-1,6-dihydropyrimidin-2-yl]acétamide sous forme de solide blanc dont les caractéristiques sont les suivantes :
Spectre RMN 1 H (400MHz):
   3,34 (s, 3 H) ; 3,40 (m, 4 H) ; 3,58 (m, 4 H) ; 3,90 (s, 2 H) ; 5,35 (s, 1 H) ; 7,15 (t, *J*=9,0 Hz, 2 H) ; 7,57 (dd, *J*=5,1 et 9,0 Hz, 2 H) ; 10,25 (s, 1 H)
Spectrométrie de Masse : méthode A
Temps de rétention Tr (min) = 0,62
[M+H]+ : m/z 347 ; [M-H]- : m/z 345

### Exemple 68 : Synthèse du N-(3-chloro-4-fluorophényl)-2-[1-méthyl-4-(morpholin-4-yl)-6-oxo-1,6-dihydropyrimidin-2-yl]acétamide

### Etape 1 :

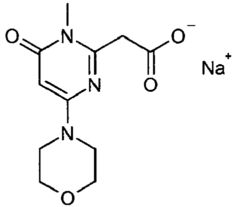

A une solution de 1.62 g de [1-méthyl-4-(morpholin-4-yl)-6-oxo-1,6-dihydropyrimidin-2-yl]acétate d'éthyle préparé à l'étape 1 de l'exemple 67, dans 20 mL de tétrahydrofurane, sont ajoutés 2.88 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif. On obtient 730 mg de [1-méthyl-4-(morpholin-4-yl)-6-oxo-1,6-dihydropyrimidin-2-yl]acétate de sodium sous forme de solide blanc dont les caractéristiques sont les suivantes:
Spectrométrie de Masse : méthode B
Temps de rétention Tr (min) = 1,67
[M+H]+ : m/z 254 ; [M-H]- : m/z 252

### Etape 2 :

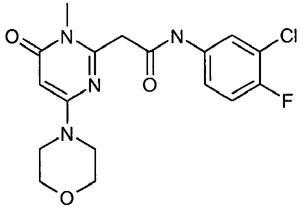

A une solution de 200 mg de [1-méthyl-4-(morpholin-4-yl)-6-oxo-1,6-dihydropyrimidin-2-yl]acétate de sodium dans 2.5 mL de N,N-diméthylformamide sont ajoutés 120 mL de pyridine, 182 mg de N-[3-(diméthylamino)propyl]-N'-éthylcarbodiimide chlorhydrate et 210 mg de 3-chloro-4-fluoroaniline. On agite à température ambiante pendant 1 nuit, puis le mélange réactionnel est concentré sous pression réduite. On ajoute de l'eau, puis on extrait à l'acétate d'éthyle, lave de nouveau avec de l'eau et concentre sous pression réduite. Le résidu obtenu est lavé avec de l'acétate d'éthyle, puis rincé avec de l'éther éthylique. On obtient 76 mg de N-(3-chloro-4-fluorophényl)-2-[1-méthyl-4-(morpholin-4-yl)-6-oxo-1,6-dihydropyrimidin-2-yl]acétamide sous forme de solide parme dont les caractéristiques sont les suivantes:
Spectre RMN 1 H (400MHz):
   3,34 (s, 3 H) ; 3,37 à 3,42 (m, 4 H) ; 3,55 à 3,60 (m, 4 H); 3,91 (s, 2 H) ; 5,36 (s, 1 H) ; 7,38 (t, *J*=9,0 Hz, 1 H) ; 7,41 à 7,47 (m, 1 H) ; 7,87 (dd, *J*=2,4 et 6,8 Hz, 1 H) ; 10,42 (s, 1 H)
Spectrométrie de Masse : méthode A
Temps de rétention Tr (min) = 0,75
[M+H]+ : m/z 381 ; [M-H]- : m/z 379
Point de fusion (Kofler) : 248°C

### Exemple 69: Synthèse du N-(3-bromophényl)-2-[1-méthyl-4-(morpholin-4-yl)-6-oxo-1,6-dihydropyrimidin-2-yl]acétamide

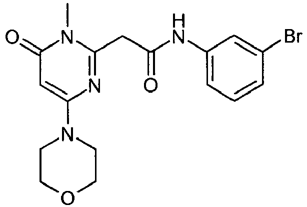

Le produit est préparé en suivant le mode opératoire décrit à l'exemple 68 à partir de 200 mg de [1-méthyl-4-(morpholin-4-yl)-6-oxo-1,6-dihydropyrimidin-2-yl]acétate de sodium et de 257 mg de 3-bromoaniline. On obtient 51 mg de N-(3-bromophényl)-2-[1-méthyl-4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,34 (s, 3 H) ; 3,37 à 3,42 (m, 4 H) ; 3,58 ( m, 4 H) ; 3,92 (s, 2 H) ; 5,36 (s, 1 H) ; 7,19 à 7,33 (m, 2 H) ; 7,45 (m, 1 H) ; 7,91 (s large, 1 H) ; 10,37 (s large, 1 H)
Spectrométrie de Masse : méthode A
Temps de rétention Tr (min) = 0,75
[M+H]+ : m/z 407 ; [M-H]- : m/z 405
Point de fusion (Kofler) : 266°C

### Exemple 70 : Synthèse de 2-[1-éthyl-4-(morpholin-4-yl)-6-oxo-1,6-dihydropyrimidin-2-yl]-N-(4-fluorophényl)acétamide

### Etape 1 :

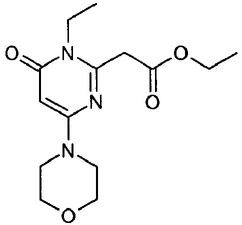

Le produit est préparé en suivant le mode opératoire décrit à l'exemple 67 à partir de 600 mg de [4-(morpholin-4-yl)-6-oxo-1,6-dihydropyrimidin-2-yl]acétate d'éthyle préparé à l'étape 1 de l'exemple 1 et 0.23 mL iodure d'éthyle au lieu du iodure de méthyle ainsi qu'en remplaçant le carbonate de potassium par le carbonate de césium. Après purification par chromatographie sur colonne de silice, éluant: CH₂Cl₂/ CH₃CN/ MeOH, 96/02/02, on obtient 190 mg de [1-éthyl-4-(morpholin-4-yl)-6-oxo-1,6-dihydropyrimidin-2-yl]acétate d'éthyle sous forme de solide blanc dont les caractéristiques sont les suivantes :
Spectrométrie de Masse: méthode A
Temps de rétention Tr (min) = 0,59 ;
[M+H]+ : m/z 296 ; [M-H]- : m/z 294
Pureté : 86 %

### Etape 2:

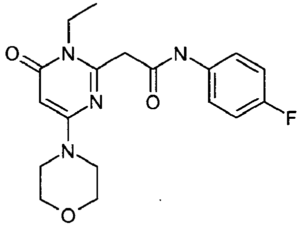

A une solution de 0.13 mL de 4-fluoroaniline dans 4 mL de toluène sont ajoutés successivement goutte à goutte 0.640 mL d'une solution de triméthylaluminium 2M dans le toluène, puis après 40 minutes d'agitation à température ambiante, une solution de 190 mg de [1-éthyl-4-(morpholin-4-yl)-6-oxo-1,6-dihydropyrimidin-2-yl]acétate d'éthyle dans 6 mL de toluène. Le mélange réactionnel est chauffé à 80°C pendant 4 heures, refroidit à l'aide d'un bain de glace et coulé sur de l'eau. Une solution 1 M de phosphate de potassium et de l'acétate d'éthyle sont ensuite ajoutés, la phase organique est extraite, lavée de nouveau avec une solution 1 M de phosphate de potassium puis séchée sur sulfate de magnésium, filtrée et enfin concentrée sous pression réduite. Après purification par chromatographie sur colonne de silice, éluant: CH₂Cl₂/ CH₃CN/ MeOH, 98/01/01 puis, 96/02/02, on obtient 60 mg de 2-[1-éthyl-4-(morpholin-4-yl)-6-oxo-1,6-dihydropyrimidin-2-yl]-N-(4-fluorophényl)acétamide sous forme de solide blanc dont les caractéristiques sont les suivantes :
Spectre RMN 1 H (400MHz):
   1,16 (t, J=7,1 Hz, 3 H) ; 3,39 (m, 4 H) ; 3,57 (m, 4 H) ; 3,88 (s, 2 H) ; 3,91 (q, J=7,1 Hz, 2 H) ; 5,33 (s, 1 H) ; 7,15 (t, J=8,9 Hz, 2 H) ; 7,57 (dd, J=5,0 et 8,9 Hz, 2 H) ; 10,26 (s, 1 H)
Spectrométrie de Masse: méthode A
Temps de rétention Tr (min) = 0,66
[M+H]+ : m/z 361 ; [M-H]- : m/z 359
Point de fusion (Kofler) : 238°C

### Exemple 71: Synthèse de N-(1H-indol-4-yl)-2-[4-(morpholin-4-yl)-6-oxo-1,6-dihydropyrimidin-2-yl]acétamide

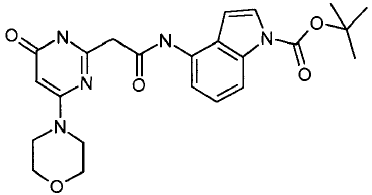

### Etape 1 :

Le produit est préparé en suivant le mode opératoire décrit à l'exemple 5 à partir de 500 mg de [4-(morpholin-4-yl)-6-oxo-1,6-dihydropyrimidin-2-yl]acétate de sodium et 445 mg de 4-amino-1H-indole-1-carboxylate de 2-méthylpropan-2-yle au lieu du 2,4-difluoroaniline. On obtient 500 mg de 4-({[4-(morpholin-4-yl)-6-oxo-1,6-dihydropyrimidin-2-yl]acétyl}amino)-1H-indole-1-carboxylate de 2-méthylpropan-2-yle sous forme de solide marron dont les caractéristiques sont les suivantes :
Spectrométrie de Masse: méthode A
Temps de rétention Tr (min) = 0,88
[M+H]+ : m/z 454 ; [M-H]- : m/z 452

**Etape 2 :**

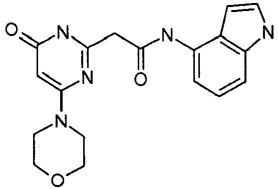

A une solution de 200 mg de 4-({[4-(morpholin-4-yl)-6-oxo-1,6-dihydropyrimidin-2-yl]acétyl}amino)-1H-indole-1-carboxylate de 2-méthylpropan-2-yle dans 8 mL de dichlorométhane sont ajoutés goutte à goutte 0.940 mL d'acide trifluoroacétique. Après 3 jours d'agitation à température ambiante, le milieu réactionnel est concentré sous pression réduite, repris par du toluène et concentré sous pression réduite. Après purification par chromatographie sur colonne de silice, éluant: CH₂Cl₂/MeOH, 90/10, on obtient 48 mg de N-(1 H-indol-4-yl)-2-[4-(morpholin-4-yl)-6-oxo-1,6-dihydropyrimidin-2-yl]acétamide dont les caractéristiques sont les suivantes :
Spectre RMN 1 H (400MHz):
   3,43 (m, 4 H) ; 3,60 (m, 4 H) ; 3,73 (s, 2 H) ; 5,21 (s, 1 H) ; 6,68 (s large, 1 H) ; 7,01 (t, J=7,9 Hz, 1 H) ; 7,16 (d, J=7,9 Hz, 1 H) ; 7,30 (t, J=2,4 Hz, 1 H) ; 7,55 (d, J=7,9 Hz, 1 H) ; 9,80 (s large, 1 H) ; 11,11 (s large, 1 H) ; 11,67 (m étalé, 1 H)
Spectrométrie de Masse: méthode A
Temps de rétention Tr (min) = 0,48
[M+H]+ : m/z 354 ; [M-H]- : m/z 352

### Exemple 72 : Synthèse de N-(4-fluorophényl)-3-méthyl-2-[4-(morpholin-4-yl)-6-oxo-1,6-dihydropyrimidin-2-yl]butanamide

### Etape 1:

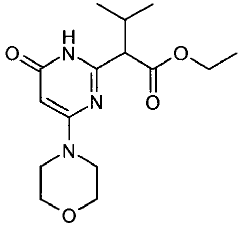

Le produit est préparé en suivant le mode opératoire décrit à l'exemple 67 à partir de 600 mg de [4-(morpholin-4-yl)-6-oxo-1,6-dihydropyrimidin-2-yl]acétate d'éthyle préparé à l'étape 1 de l'exemple 1 préparé à l'étape 1 de l'exemple 1 et 0.29 mL 2-iodopropane au lieu du iodure de méthyle ainsi qu'en remplaçant le carbonate de potassium par le carbonate de césium. Après purification par chromatographie sur colonne de silice, éluant: CH₂Cl₂/ CH₃CN/ MeOH, 90/05/05, on obtient 20 mg de 3-méthyl-2-[4-(morpholin-4-yl)-6-oxo-1,6-dihydropyrimidin-2-yl]butanoate d'éthyle sous forme de solide blanc dont les caractéristiques sont les suivantes:
Spectrométrie de Masse: méthode A
Temps de rétention Tr (min) = 0,70
[M+H]+ : m/z 310 ; [M-H]- : m/z 308

### Etape 2:

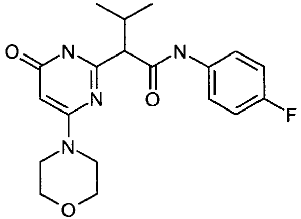

Le produit est préparé en suivant le mode opératoire décrit à l'exemple 70 à partir de 0.023 mL de 4-fluoroaniline et 36 mg de 3-méthyl-2-[4-(morpholin-4-yl)-6-oxo-1,6-dihydropyrimidin-2-yl]butanoate d'éthyle au lieu du [1-éthyl-4-(morpholin-4-yl)-6-oxo-1,6-dihydropyrimidin-2-yl]acétate d'éthyle. Après purification par chromatographie sur colonne de silice, éluant: CH₂Cl₂/ CH₃CN/ MeOH, 94/03/03, on obtient 15 mg de N-(4-fluorophényl)-3-méthyl-2-[4-(morpholin-4-yl)-6-oxo-1,6-dihydropyrimidin-2-yl]butanamide sous forme de solide blanc dont les caractéristiques sont les suivantes:
Spectre RMN 1 H (400MHz):
   0,87 (d, J=6,6 Hz, 3 H) ; 0,97 (d, J=6,6 Hz, 3 H) ;2,53 à 2,57 (m, 1 H) ; 3,24 (d, J=10,3 Hz, 1 H) ; 3,42 à 3,47 (m, 4 H) ; 3,59 à 3,65 (m, 4 H) ; 5,21 (s, 1 H) ;7,15 (t, J=8,8 Hz, 2 H) ; 7,59 (dd, J=5,1 et 8,8 Hz, 2 H) ; 10,09 (s large, 1 H) ; 11,30 (s large, 1 H)
Spectrométrie de Masse: méthode A
Temps de rétention Tr (min) = 0,76
[M+H]+ : m/z 375 ; [M-H]- : m/z 373
Point de fusion (Kofler) : 266°C

### Exemple 73 : Synthèse de N-[4-fluoro-3-(méthoxyméthyl)phényl]-2-[4-(morpholin-4-yl)-6-oxo-1,6-dihydropyrimidin-2-yl]acétamide

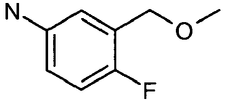

### Etape 1:

Dans un ballon, on introduit 374 mg d'hydrure de sodium à 60% dans 10 mL de tétrahydrofurane, 1 g de (5-amino-2-fluorophényl)méthanol et 0.441 mL de iodure de méthyle. Après une heure d'agitation à température ambiante, on ajoute une solution de chlorure de sodium et de l'éther diéthylique. La phase organique est extraite puis lavée avec de l'eau jusqu'à pH = 7. Elle est ensuite séchée sur sulfate de magnésium, filtrée et concentrée à pression réduite. On obtient 900 mg de 4-fluoro-3-(méthoxyméthyl)aniline sous forme d'huile noire utilisée tel quel dans l'étape suivante.

### Etape 2:

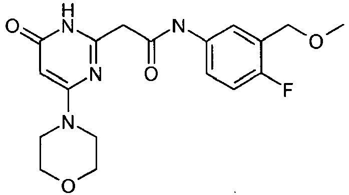

Le produit est préparé en suivant le mode opératoire décrit à l'exemple 5 à partir de 250 mg de [4-(morpholin-4-yl)-6-oxo-1,6-dihydropyrimidin-2-yl]acétate de sodium et 900 mg de 4-fluoro-3-(méthoxyméthyl)aniline préparé précédemment au lieu du 2,4-difluoroaniline. On obtient 168 mg de N-[4-fluoro-3-(méthoxyméthyl)phényl]-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,31 (s partiellement masqué, 3 H) ; 3,41 (m, 4 H) ; 3,59 (m, 6 H) ; 4,43 (s, 2 H) ; 5,20 (s, 1 H) ; 7,14 (t, J=9,3 Hz, 1 H) ; 7,51 (m, 1 H) ; 7,63 (m, 1 H) ; 10,21 (s large, 1 H) ; 11,63 (s large, 1 H)
Spectrométrie de Masse: méthode A
Temps de rétention Tr (min) = 0,60
[M+H]+ : m/z 377 ; [M-H]- : m/z 375
Point de fusion (Kofler) : 220°C

### Exemple 74: Synthèse de N-(4-fluoro-3-iodophenyl)-2-[4-(morpholin-4-yl)-6-oxo-1,6-dihydropyrimidin-2-yl]acétamide

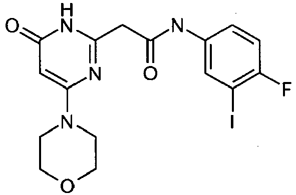

### Etape 1 :

A une suspension de 1 g de limaille de fer dans 10 mL d'éthanol, 5 mL d'eau et 250.4 mg de chlorure d'ammonium, porté au reflux est ajouté 1 g de 4-fluoro-3-nitrobenzène solubilisé dans 5 mL d'éthanol. Après 1 heure de chauffage au reflux, le mélange réactionnel est filtré et les extraits organiques sont évaporés. Le résidu d'évaporation est repris dans un mélange d'eau et de dichlorométhane. La phase aqueuse est extraite avec 3 fois du dichlorométhane. Les extraits organiques sont regroupés, sechés sur sulfate de magnésium et concentrés sous pression réduite. On obtient 840 mg de 4-fluoro-3-iodoaniline dont les caractéristiques sont les suivantes :
Spectrométrie de Masse: méthode A
Temps de rétention Tr (min) = 0,60
[M+H]+ : m/z 238

### Etape 2 :

Le produit est préparé en suivant le mode opératoire décrit à l'exemple 5 à partir de 300 mg de [4-(morpholin-4-yl)-6-oxo-1,6-dihydropyrimidin-2-yl]acétate de sodium et 272 mg de 4-fluoro-3-iodoaniline. On obtient 309 mg de N-(4-fluoro-3-iodophenyl)-2-[4-(morpholin-4-yl)-6-oxo-1,6-dihydropyrimidin-2-yl]acétamide dont les caractéristiques sont les suivantes :
Spectre RMN 1 H (400MHz):
   3,42 (m, 4 H) ; 3,57 à 3,63 (m, 6 H) ; 5,21 (s, 1 H) ; 7,23 (t, J=8,6 Hz, 1 H) ; 7,52 (ddd, J=2,5 et 4,4 et 8,6 Hz, 1 H) ; 8,12 (dd, J=2,5 et 5,4 Hz, 1 H) ; 10,29 (s large, 1 H) ; 11,65 (s large, 1 H)
Spectrométrie de Masse: méthode A
Temps de rétention Tr (min) = 0,74
[M+H]+ : m/z 459 ; [M-H]- : m/z 457

### Exemple 75 : Synthèse de 2-[4-(morpholin-4-yl)-6-oxo-1,6-dihydropyrimidin-2-yl]-N-[3-(1,1,2,2-tétrafluoroéthoxy)phényl]acétamide

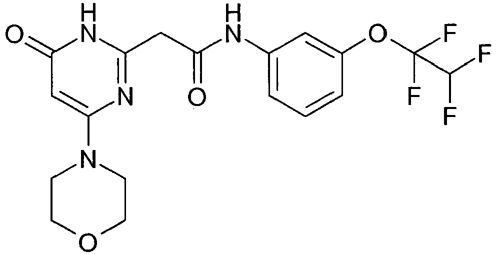

Le produit est préparé en suivant le mode opératoire décrit à l'exemple 5 à partir de 250 mg de [4-(morpholin-4-yl)-6-oxo-1,6-dihydropyrimidin-2-yl]acétate de sodium, de 300 mg de 3-(1,1,2,2-tétrafluoroéthoxy)aniline, de 300 mg de N-[3-(diméthylamino)propyl]-N'-éthylcarbodiimide chlorhydrate dans un mélange de 2 mL de pyridine et de 2 mL de N,N-diméthylformamide . On obtient 300 mg de 2-[4-(morpholin-4-yl)-6-oxo-1,6-dihydropyrimidin-2-yl]-N-[3-(1,1,2,2-tétrafluoroéthoxy)phényl]acétamide sous forme d'un solide blanc dont les caractéristiques sont les suivantes:
Spectre RMN 1 H (400MHz):
   tous les signaux sont larges avec : 3,41 (m, 4 H) ; 3,60 (m, 6 H) ; 5,20 (s, 1 H) ; 6,79 (t, J=52,5 Hz, 1 H) ; 6,94 à 7,01 (m, 1 H) ; 7,35 à 7,50 (m, 2 H) ; 7,69 (s, 1 H) ; 10,40 (s, 1 H) ; 11,66 (s, 1 H)
Spectrométrie de Masse: méthode A
Temps de rétention Tr (min) = 0,77
[M+H]+ : m/z 431 ; [M-H]- : m/z 429
Point de fusion (Kofler) : 229°C

### Exemple 76 : Synthèse de N-[3-(difluorométhyl)-4-fluorophényl]-2-[4-(morpholin-4-yl)-6-oxo-1,6-dihydropyrimidin-2-yl]acétamide

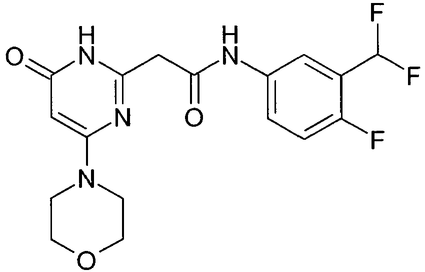

### Etape 1 :

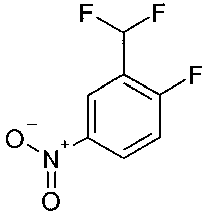

A une solution de 3.36 g de 2-fluoro-5-nitrobenzaldéhyde dans 100 mL de dichlorométhane sont ajoutés, à une température voisine de 20°C et sous argon, 4.48 g (3.5 mL) de N-éthyl-N-(trifluoro-lambda~4~sulfanyl)éthanamine. Après 3h30 d'agitation à une température voisine de 20°C sont ajoutés lentement 300 mL d'une solution aqueuse saturée en hydrogénocarbonate de sodium. Après une heure d'agitation, la phase organique est lavée par 40 mL d'eau, puis séchée sur sulfate de magnésium anhydre, filtrée et concentrée à sec sous pression réduite. On obtient 3.6 g de 2-(difluorométhyl)-1-fluoro-4-nitrobenzène sous forme d'un liquide jaune dont les caractéristiques sont les suivantes :
Spectrométrie de Masse : Les spectres ont été réalisés par introduction directe sur un appareil WATERS GCTof (introduction directe sans LC) : El : [M]+.m/z = 191

### Etape 2 :

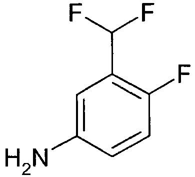

A une suspension de 7 g de fer, dans 30 mL d'acide acétique au reflux, sous argon, est ajoutée lentement une solution de 2 g de 2-(difluorométhyl)-1-fluoro-4-nitrobenzène dans 10 mL d'acide acétique. Le mélange réactionnel est agité pendant 1 heure au reflux, puis refroidi à température ambiante. 20 mL d'acétate d'éthyle sont ensuite ajoutés et le milieu réactionnel est filtré sur Clarcel, rincé avec de l'actétate d'éthyle puis concentré à sec sous pression réduite. Le résidu est repris par 100 mL d'acétate d'éthyle et filtré à nouveau sur Clarcel et concentré à sec sous pression réduite. Après purification sur colonne de silice du résidu huileux, éluant: CH₂Cl₂/ MeOH, 95/05, on obient 1.1 g d'un mélange contenant la 3-(difluorométhyl)-4-fluoroaniline sous la forme d'un liquide marron utilisé tel que dans l'étape suivante.

### Etape 3 :

Le produit est préparé en suivant le mode opératoire décrit à l'exemple 5 à partir de 250 mg de [4-(morpholin-4-yl)-6-oxo-1,6-dihydropyrimidin-2-yl]acétate de sodium, de 1.1 g de 3-(difluorométhyl)-4-fluoroaniline (étape 2), et de 300 mg de N-[3-(diméthylamino)propyl]-N'-éthylcarbodiinide chlorhydrate dans un mélange de 2 mL de pyridine et de 2 mL de N,N-diméthylformamide . On obtient 260 mg de N-[3-(difluorométhyl)-4-fluorophényl]-2-[4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,41 (m, 4 H) ; 3,52 à 3,67 (m, 6 H) ; 5,21 (s, 1 H) ; 7,20 (t, J=54,3 Hz, 1 H) ; 7,34 (t, J=8,9 Hz, 1 H) ; 7,68 (m, 1 H) ; 7,90 (m, 1 H) ; 10,41 (s, 1 H) ; 11,67 (s large, 1 H)
Spectrométrie de Masse : méthode A
Temps de rétention Tr (min) = 0,67
[M+H]+ : m/z 383 ; [M-H]- : m/z 381
Point de fusion (Kofler) : supérieur à 260°C

### Exemple 77 : Synthèse de 2,2-difluoro-N-(4-fluorophényl)-2-[4-(morpholin-4-yl)-6-oxo-1,6-dihydropyrimidin-2-yl]acétamide

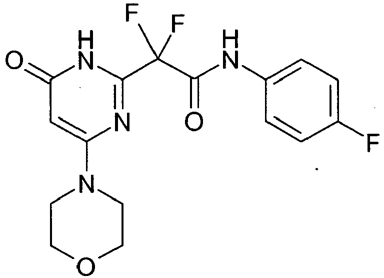

### Etape 1 :

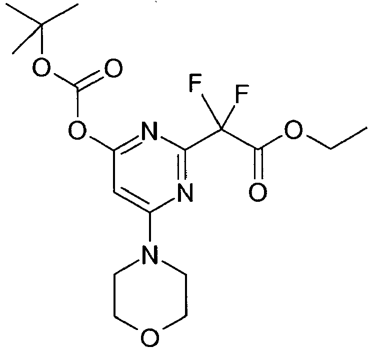

A une solution de 570 mg de 1,1,1,3,3,3-hexaméthyldisilazan-2-ide de potassium dans 9 mL de tétrahydrofurane placée à une température de - 78°C et sous argon, sont ajoutés 350 mg de [4-({[(2-méthylpropan-2-yl)oxy]carbonyl}oxy)-6-(morpholin-4-yl)pyrimidin-2-yl]acétate d'éthyle préalablement dissous dans 6 mL de tétrahydrofurane. Après 45 mn d'agitation à cette température, sont ajoutés 409 mg de dibromure de manganèse(2+). Après trente minutes d'agitation de la suspension obtenue, toujours à une température de - 78°C, sont ajoutés 847 mg de N-fluoro-N-(phénylsulfonyl)benzènesulfonamide. Après retour à une température voisine de 20°C, la suspention est agitée une nuit. Le milieu est versé sur une solution aqueuse saturée en bicarbonate de sodium, extrait par de l'acétate d'éthyle, la phase organique lavée par une solution aqueuse saturée en chlorure de sodium, séchée sur sulfate de magnésium anhydre, filtrée et concentrée à sec sous pression réduite. Le résidu est repris par 10 mL de chlorure de méthylène puis l'insoluble est filtré et lavé à par trois fois 5 mL de chlorure de méthylène. Le filtrat est concentré à sec sous pression réduite et après purification sur colonne de silice du résidu huileux, éluant: CH₂Cl₂/ AcOEt, 95/05, on obtient 278 mg de difluoro[4-({[(2-méthylpropan-2-yl)oxy]carbonyl}oxy)-6-(morpholin-4-yl)pyrimidin-2-yl]acétate d'éthyle sous forme d'une huile incolore dont les caractéristiques sont les suivantes :
Spectrométrie de masse : méthode A
Temps de rétention Tr (min) = 1,06
[M+Na]+ : m/z 426 ; pic de base : m/z 304

### Etape 2 :

A une solution de 0.136 mL de 4-fluoroaniline dans 5 mL de toluène à une température comprise entre 0°C et 10°C on introduit lentement 0.756 mL d'une solution de trichlorure d'aluminium (2N) dans le toluène. Après 40 mn d'agitation à une température voisine de 20°C on ajoute lentement 271 mg de difluoro[4-({[(2-méthylpropan-2-yl)oxy]carbonyl}oxy)-6-(morpholin-4-yl)pyrimidin-2-yl]acétate d'éthyle solubilisés dans 6 mL de toluène. Après 2h30 de reflux, on revient à une température voisine de 20°C puis à 0°C ajoute 26 mL d'eau et 26 mL d'une solution molaire de dihydrogénophosphate de potassium et extrait par de l'acétate d'éthyle. La phase organique est lavée par une solution aqueuse saturée en chlorure de sodium, séchée sur sulfate de magnésium anhydre, filtrée et concentrée à sec sous pression réduite. Après purification sur colonne de silice du résidu, éluant: CH₂Cl₂/MeOH, 95/05, le solide est repris par 5 mL de dichlorométhane, le mélange est chauffé à 40°C pendant 5mn puis on revient à une température voisine de 20°C. On essore l'insoluble, celui-ci est lavé par trois empâtages dans 1 mL de dichlorométhane, puis séché sous cloche à vide. On obtient 28 mg de 2,2-difluoro-N-(4-fluorophényl)-2-[4-(morpholin-4-yl)-6-oxo-1,6-dihydropyrimidin-2-yl]acétamide sous forme d'une poudre blanche dont les caractéristiques sont les suivantes :
Spectre RMN 1 H (400MHz):
   3,51 (m, 4 H) ; 3,58 à 3,64 (m, 4 H) ; 5,92 (s large, 1 H) ; 7,20 (t, J=9,0 Hz, 2 H) ; 7,70 (dd, J=5,2 et 9,0 Hz, 2 H) ; 10,70 (s large, 1H) ; 11,82 (m étalé, 1 H)
Spectrométrie de Masse : méthode A
Temps de rétention Tr (min) = 0,69
[M+H]+ : m/z 369 ; [M-H]- : m/z 367

### Exemple 78: Synthèse de N-(3,4-difluorophényl)-2-[1-méthyl-4-(morpholin-4-yl)-6-oxo-1,6-dihydropyrimidin-2-yl]acétamide

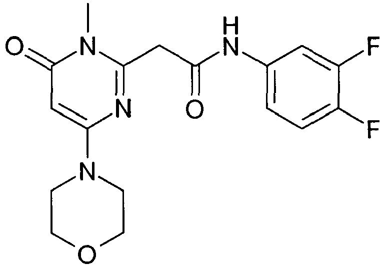

Le produit est préparé en suivant le mode opératoire décrit à l'exemple 5 à partir de 250 mg de [1-méthyl-4-(morpholin-4-yl)-6-oxo-1,6-dihydropyrimidin-2-yl]acétate de sodium préparé à l'étape 1 de l'exemple 68, de 169 mg 3,4-difluoroaniline, et de 285 mg de N-[3-(diméthylamino)propyl]-N'-éthylcarbodiimide chlorhydrate dans un mélange de 2 mL de pyridine et de 2 mL de N,N-diméthylformamide. On obtient 180 mg de N-(3,4-difluorophényl)-2-[1-méthyl-4-(morpholin-4-yl)-6-oxo-1,6-dihydropyrimidin-2-yl]acétamide sous forme de solide blanc dont les caractéristiques sont les suivantes :
Spectre RMN 1 H (400MHz):
   3,33 (s, 3H) ; 3,39 (m, 4 H) ; 3,58 (m, 4H) ; 3,91 (s, 2 H) ; 5,36 (s, 1 H) ; 7,26 (m, 1 H) ; 7,39 (q, J=9,5 Hz, 1H) ; 7,73 (ddd, J=2,2 et 7,5 et 13,0 Hz, 1H) ; 10,43 (s, 1 H)
Spectrométrie de Masse : méthode A
Temps de rétention Tr (min) = 0,69
[M+H]+ : m/z 365 ; [M-H]- : m/z 363
Point de fusion (Kofler) : supérieur à 260°C

### Exemple 79: Synthèse de 2-[2-(2,3-dihydro-1H-indol-1-yl)-2-oxoéthyl]-3-méthyl-6-(morpholin-4-yl)pyrimidin-4(3H)-one

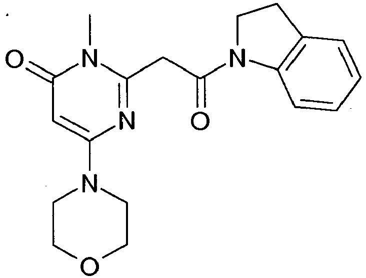

Le produit est préparé en suivant le mode opératoire décrit à l'exemple 5 à partir de 200 mg de [1-méthyl-4-(morpholin-4-yl)-6-oxo-1,6-dihydropyrimidin-2-yl]acétate de sodium préparé à l'étape 1 de l'exemple 68, de 174 mg de 2,3-dihydro-1H-indole, et de 185 mg de N-[3-(diméthylamino)propyl]-N'-éthylcarbodiimide chlorhydrate dans un mélange de 0.12 mL de pyridine et de 3 mL de N,N-diméthylformamide. Après purification sur colonne de silice, éluant: CH₂Cl₂/MeOH, 93/07, le solide est repris par 4 mL de méthanol et quelques gouttes d'acétone. La suspension est chauffée à une température de 80°C, filtrée à chaud puis recristallisée à une température de 20°C puis de 0°C. Le solide est filtré, rincé par de l'oxyde de diéthyle, puis séché sous cloche à vide. On obtient 52 mg de 2-[2-(2,3-dihydro-1H-indol-1-yl)-2-oxoéthyl]-3-méthyl-6-(morpholin-4-yl)pyrimidin-4(3H)-one sous forme de cristaux blancs dont les caractéristiques sont les suivantes:
Spectre RMN 1 H (400MHz):
   3,17 (t, J=8,4 Hz, 2 H) ; 3,32 (s, 3 H) ; 3,39 (m, 4H) ; 3,58 (m, 4 H) ; 4,11 (s, 2 H) ; 4,17 (t, J=8,4 Hz, 2 H) ; 5,36 (s, 1 H) ; 7,02 (t, J=7,5 Hz, 1 H) ; 7,16 (t, J=7,5 Hz, 1 H) ; 7,26 (d, J=7,5 Hz, 1 H) ; 8,01 (d, J=7,5 Hz, 1 H)
Spectrométrie de Masse : méthode A
Temps de rétention Tr (min) = 0,68
[M+H]+ : m/z 355 ; [M-H]- : m/z 353
Point de fusion (Kofler) : supérieur à 250°C

### Exemple 80: Synthèse de N-(3-bromo-4-fluorophényl)-2-[1-méthyl-4-(morpholin-4-yl)-6-oxo-1,6-dihydropyrimidin-2-yl]acétamide

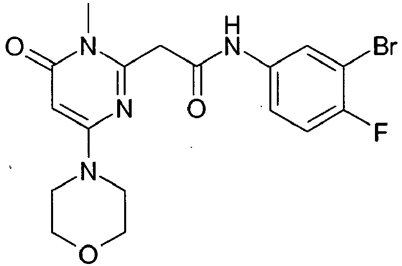

Le produit est préparé en suivant le mode opératoire décrit à l'exemple 5 à partir de 200 mg de [1-méthyl-4-(morpholin-4-yl)-6-oxo-1,6-dihydropyrimidin-2-yl]acétate de sodium préparé à l'étape 1 de l'exemple 68, de 199 mg de 3-bromo-4-fluoroaniline, et de 228 mg de N-[3-(diméthylamino)propyl]-N'-éthylcarbodiimide chlorhydrate dans un mélange de 1.5 mL de pyridine et de 1.5 mL de N,N-diméthylformamide. On obtient 185 mg de N-(3-bromo-4-fluorophényl)-2-[1-méthyl-4-(morpholin-4-yl)-6-oxo-1,6-dihydropyrimidin-2-yl]acétamide sous forme de solide blanc dont les caractéristiques sont les suivantes:
Spectre RMN 1 H (400MHz):
   3,34 (s, 3 H) ; 3,39 (m, 4 H) ; 3,58 (m, 4 H) ; 3,91 (s, 2 H) ; 5,36 (s, 1 H) ; 7,30 à 7,38 (t, J=8,9 Hz, 1 H) ; 7,48 (ddd, J=2,7 et 4,4 et 8,9 Hz, 1 H) ; 7,99 (dd, J=2,7 et 6,4 Hz, 1 H) ; 10,40 (s, 1 H)
Spectrométrie de Masse : méthode A
Temps de rétention Tr (min) = 0,76
[M+H]+ : m/z 425 ; [M-H]- : m/z 423
Point de fusion (Kofler) : supérieur à 262°C

### Exemple 81 : Synthèse de N-[4-fluoro-3-(hydroxyméthyl)phényl]-2-[1-méthyl-4-(morpholin-4-yl)-6-oxo-1,6-dihydropyrimidin-2-yl]acétamide

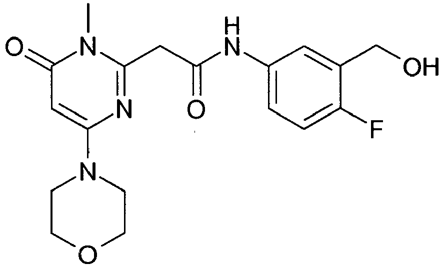

Le produit est préparé en suivant le mode opératoire décrit à l'exemple 5 à partir de 250 mg de [1-méthyl-4-(morpholin-4-yl)-6-oxo-1,6-dihydropyrimidin-2-yl]acétate de sodium préparé à l'étape 1 de l'exemple 68, de 184 mg de (5-amino-2-fluorophényl)méthanol, et de 285 mg de N-[3-(diméthylamino)propyl]-N'-éthylcarbodiimide chlorhydrate dans un mélange de 2 mL de pyridine et de 2 mL de N,N-diméthylformamide. On obtient 157 mg de N-[4-fluoro-3-(hydroxyméthyl)phényl]-2-[1-méthyl-4-(morpholin-4-yl)-6-oxo-1,6-dihydropyrimidin-2-yl]acétamide sous forme de solide gris dont les caractéristiques sont les suivantes:
Spectre RMN 1 H (400MHz):
   3,34 (s, 3 H) ; 3,40 (m, 4 H) ; 3,59 (m, 4 H) ; 3,90 (s, 2 H) ; 4,52 (d, J=5,6 Hz, 2 H) ; 5,27 (t, J=5,6 Hz, 1 H) ; 5,35 (s, 1 H) ; 7,08 (t, J=9,3 Hz, 1 H) ; 7,43 à 7,53 (m, 1 H) ; 7,64 (dd, J=2,7 et 6,6 Hz, 1H) ; 10,23 (s, 1 H)
Spectrométrie de Masse : méthode A
Temps de rétention Tr (min) = 0,49
[M+H]+ : m/z 377 ; [M-H]- : m/z 375
Point de fusion (Kofler) : supérieure à 224°C

### Exemple 82 : Synthèse de N-(3-cyclopropylphényl)-2-[1-méthyl-4-(morpholin-4-yl)-6-oxo-1,6-dihydropyrimidin-2-yl]acétamide

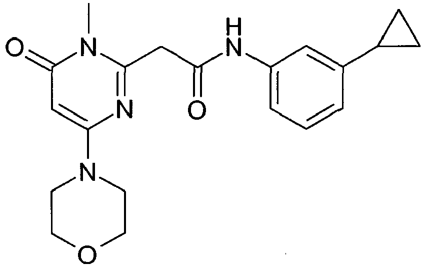

Le produit est préparé en suivant le mode opératoire décrit à l'exemple 5 à partir de 200 mg de [1-méthyl-4-(morpholin-4-yl)-6-oxo-1,6-dihydropyrimidin-2-yl]acétate de sodium préparé à l'étape 1 de l'exemple 68, de 194 mg de 3-cyclopropyleaniline (préparée selon Wallace et al. dans Tetrahedron Lett. 2002, 43, 6987), et de 185 mg de N-[3-(diméthylamino)propyl]-N'-éthylcarbodiimide chlorhydrate dans un mélange de 0.12 mL de pyridine et de 4 mL de N,N-diméthylformamide . Après extractions à l'acétate d'éthyle et purification sur colonne de silice du résidu obtenu, éluant: CH2Cl2/MeOH, 95/05, on obtient 129 mg de N-(3-cyclopropylphényl)-2-[1-méthyl-4-(morpholin-4-yl)-6-oxo-1,6-dihydropyrimidin-2-yl]acétamide sous forme d'un solide blanc dont les caractéristiques sont les suivantes:
Spectre RMN 1 H (400MHz):
   0,57 à 0,64 (m, 2 H) ; 0,90 à 0,98 (m, 2 H) ; 1,82 à 1,91 (m, 1 H) ; 3,33 (s, 3 H) ; 3,38 à 3,43 (m, 4 H) ; 3,57 à 3,62 (m, 4 H) ; 3,89 (s, 2 H) ; 5,35 (s, 1 H) ; 6,79 (d large, J=7,8 Hz, 1 H) ; 7,17 (t, J=7,8 Hz, 1 H) ; 7,28 (t, J=1,5 Hz, 1 H) ; 7,31 (d large, J=7,8 Hz, 1 H) ; 10,11 (s, 1 H)
Spectrométrie de Masse : méthode A
Temps de rétention Tr (min) = 0,76
[M+H]+ : m/z 369 ; [M-H]- : m/z 367
Point de fusion (Kofler) : 216°C

### Exemple 83 : Synthèse de N-(4-fluoro-3-méthoxyphényl)-2-[1-méthyl-4-(morpholin-4-yl)-6-oxo-1,6-dihydropyrimidin-2-yl]acétamide

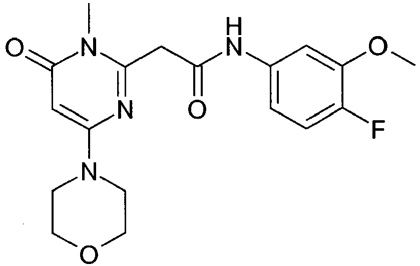

Le produit est préparé en suivant le mode opératoire décrit à l'exemple 5 à partir de 200 mg de [1-méthyl-4-(morpholin-4-yl)-6-oxo-1,6-dihydropyrimidin-2-yl]acétate de sodium préparé à l'étape 1 de l'exemple 68, de 205 mg de 4-fluoro-3-méthoxyaniline, et de 185 mg de N-[3-(diméthylamino)propyl]-N'-éthylcarbodiimide chlorhydrate dans un mélange de 0.12 mL de pyridine et de 3 mL de N,N-diméthylformamide . Après extractions à l'acétate d'éthyle et purification sur colonne de silice du résidu obtenu, éluant: CH2Cl2/MeOH, 95/05 puis 90/10, on obtient 42 mg de N-(4-fluoro-3-méthoxyphényl)-2-[1-méthyl-4-(morpholin-4-yl)-6-oxo-1,6-dihydropyrimidin-2-yl]acétamide sous forme d'un solide blanc dont les caractéristiques sont les suivantes:
Spectre RMN 1 H (400MHz):
   3,34 (s, 3 H) ; 3,38 à 3,42 (m, 4 H) ; 3,55 à 3,62 (m, 4 H) ; 3,79 (s, 3 H) ; 3,90 (s, 2 H) ; 5,35 (s, 1 H) ; 7,05 (ddd, J=2,4 et 4,0 et 8,8 Hz, 1 H) ; 7,14 (dd, J=8,8 et 11,2 Hz, 1 H) ; 7,47 (dd, J=2,4 et 8,1 Hz, 1 H) ; 10,26 (s, 1H)
Spectrométrie de Masse : méthode A
Temps de rétention Tr (min) = 0,64
[M+H]+ : m/z 377 ; [M-H]- : m/z 375
Point de fusion (Kofler) : supérieur à 260°C

### Exemple 84 : Synthèse de N-(1-benzofur-4-yl)-2-[4-(morpholin-4-yl)-6-oxo-1,6-dihydropyrimidin-2-yl]acétamide

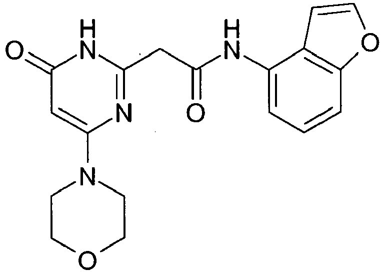

Le produit est préparé en suivant le mode opératoire décrit à l'exemple 5 à partir de 250 mg de [de [4-(morpholin-4-yl)-6-oxo-1,6-dihydropyrimidin-2-yl]acétate de sodium, de 178 mg de 1-benzofuran-4-amine, de 300 mg de N-[3-(diméthylamino)propyl]-N'-éthylcarbodiimide chlorhydrate dans un mélange de 2 mL de pyridine et de 3 mL de N,N-diméthylformamide . On obtient 275 mg de 2-[1-méthyl-4-(morpholin-4-yl)-6-oxo-1,6-dihydropyrimidin-2-yl]-N-phénylacétamide sous forme d'un solide blanc cassé dont les caractéristiques sont les suivantes :
Spectre RMN 1 H (400MHz):
   3,43 (m, 4 H) ; 3,60 (m, 4 H) ; 3,73 (s, 2 H) ; 5,21 (s, 1 H) ; 7,12 (d large, J=2,2 Hz, 1 H) ; 7,25 (t, J=8,1 Hz, 1 H) ; 7,35 (d large, J=8,1 Hz, 1 H) ; 7,71 (d, J=8,1 Hz, 1 H) ; 7,94 (d, J=2,2 Hz, 1 H) ; 10,12 (s, 1 H) ; 11,70 (m étalé, 1 H)
Spectrométrie de Masse : méthode B
Temps de rétention Tr (min) = 2,96
[M+H]+ : m/z 355 ; [M-H]- : m/z 353
Point de fusion (Kofler) : supérieur à 260°C

### Exemple 85 : Synthèse de 2-[1-méthyl-4-(morpholin-4-yl)-6-oxo-1,6-dihydropyrimidin-2-yl]-N-phénylacétamide

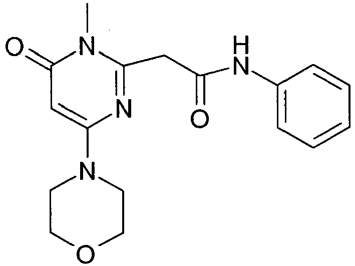

Le produit est préparé en suivant le mode opératoire décrit à l'exemple 5 à partir de 200 mg de [1-méthyl-4-(morpholin-4-yl)-6-oxo-1,6-dihydropyrimidin-2-yl]acétate de sodium préparé à l'étape 1 de l'exemple 68, de 134mg d' aniline, et de 180 mg de N-[3-(diméthylamino)propyl]-N'-éthylcarbodiimide chlorhydrate dans un mélange de 0.12 mL de pyridine et de 2.5 mL de N,N-diméthylformamide . On obtient 95mg de 2-[1-méthyl-4-(morpholin-4-yl)-6-oxo-1,6-dihydropyrimidin-2-yl]-N-phénylacétamide sous forme d'un solide violet dont les caractéristiques sont les suivantes:
Spectre RMN 1 H (400MHz):
   3,35 (s, 3 H) ; 3,41 (m, 4 H) ; 3,58 (m, 4 H) ; 3,91 (s, 2 H) ; 5,35 (s, 1 H) ; 7,06 (t, J=7,8 Hz, 1 H) ; 7,31 (t, J=7,8 Hz, 2 H) ; 7,55 (d, J=7,8 Hz, 2 H) ; 10,19 (s, 1 H)
Spectrométrie de Masse : méthode B
Temps de rétention Tr (min) = 2,87
[M+H]+ : m/z 329 ; [M-H]- : m/z 327
Point de fusion (Kofler) : 212°C

### Exemple 86 : Synthèse de N-(3-cyclopropyl-4-fluorophényl)-2-[1-méthyl-4-(morpholin-4-yl)-6-oxo-1,6-dihydropyrimidin-2-yl]acétamide

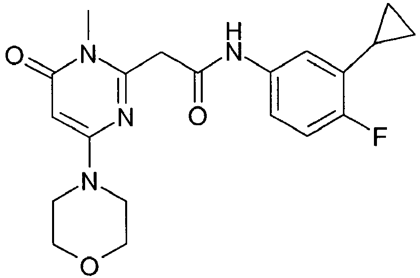

### Etape 1

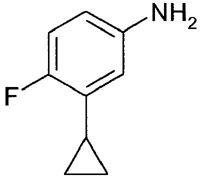

A une solution de 950 mg de 3-bromo-4-fluoroaniline dans 20 mL de tolène sous agitation et sous argon sont ajoutés 558 mg d'acide cyclopropylboronique, *3.7g de phosphonate de potassium tribasic, 70* mg de tricyclohexylphosphane et de 56 mg de diacétate de palladium(2+) dans 1.5 mL d'eau. Le mélange est dégazé puis chauffé à une température de 100°C. Après 15 heures, le milieu réactionnel est refroidit à une température voisine de 20°C, versé dans 100 mL d'eau, puis extrait par quatre fois 60 mL d'oxyde de diéthyle. Les phases organiques réunies sont séchées sur sulfate de magnésium anhydre, filtrées et concentrées sous pression réduite. Le résidu est purifié sur colonne de silice, éluant: cyclohexane/Acétate d'éthyle 70/30, on obtient 494 mg de 3-cyclopropyl-4-fluoroaniline sous forme d'une huile marron dont les caractéristiques sont les suivantes :
Spectrométrie de Masse : méthode A
Temps de rétention Tr (min) = 0,40
[M+H]+ : m/z 152 ;

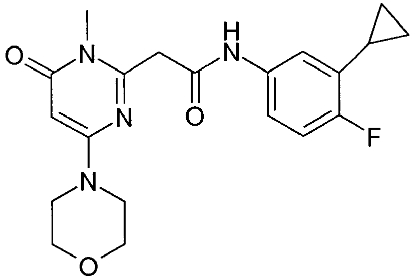

### Etape 2

Le produit est préparé en suivant le mode opératoire décrit à l'exemple 5 à partir de 200 mg de [1-méthyl-4-(morpholin-4-yl)-6-oxo-1,6-dihydropyrimidin-2-yl]acétate de sodium préparé à l'étape 1 de l'exemple 68, de 220 mg de 3-cyclopropyl-4-fluoroaniline, et de 180 mg de N-[3-(diméthylamino)propyl]-N'-éthylcarbodiimide chlorhydrate dans un mélange de 0.12 mL de pyridine et de 2.5 mL de N,N-diméthylformamide . Après extractions à l'acétate d'éthyle et purification sur colonne de silice éluant: CH2Cl2/MeOH, 90/10, le résidu est repris par 10 mL d'oxyde de diisopropyl, le précipité est essoré et séché sous vide pour obtenir 69 mg de N-(3-cyclopropyl-4-fluorophényl)-2-[1-méthyl-4-(morpholin-4-yl)-6-oxo-1,6-dihydropyrimidin-2-yl]acétamide sous forme d'un solide jaune dont les caractéristiques sont les suivantes:
Spectre RMN 1 H (400MHz):
   0,57 à 0,66 (m, 2 H) ; 0,94 à 1,03 (m, 2 H) ; 1,93 à 2,08 (m, 1 H) ; 3,33 (s, 3 H) ; 3,37 à 3,42 (m, 4 H) ; 3,55 à 3,62 (m, 4 H) ; 3,87 (s, 2 H) ; 5,35 (s, 1 H) ; 7,07 (dd, J=8,8 et 10,3 Hz, 1 H) ; 7,16 (dd, J=2,6 et 7,0 Hz, 1 H) ; 7,29 à 7,38 (m, 1 H) ; 10,16 (s, 1 H)
Spectrométrie de Masse : méthode B
Temps de rétention Tr (min) = 3,53
[M+H]+ : m/z 387 ; [M-H]- : m/z 385
Point de fusion (Kofler) : 245°C

### Exemple 87 : Synthèse de N-(3-fluoro-2-hydroxyphényl)-2-[4-(morpholin-4-yl)-6-oxo-1,6-dihydropyrimidin-2-yl]acétamide

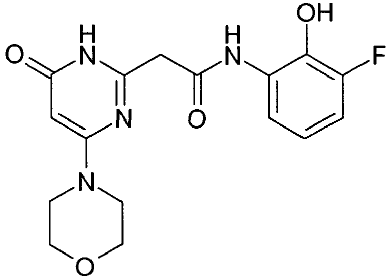

Le produit est préparé en suivant le mode opératoire décrit à l'exemple 5 à partir de 500 mg de [4-(morpholin-4-yl)-6-oxo-1,6-dihydropyrimidin-2-yl]acétate de sodium, de 357 mg de 2-amino-6-fluorophénol, de 600 mg de N-[3-(diméthylamino)propyl]-N'-éthylcarbodiimide chlorhydrate dans un mélange de 4 mL de pyridine et de 4 mL de N,N-diméthylformamide. On obtient 335 mg de N-(3-fluoro-2-hydroxyphényl)-2-[4-(morpholin-4-yl)-6-oxo-1,6-dihydropyrimidin-2-yl]acétamide sous forme d'un solide beige dont les caractéristiques sont les suivantes:
Spectre RMN 1 H (400MHz):
   3,44 (m, 4 H) ; 3,62 (m, 4 H) ; 3,71 (s, 2 H) ; 5,21 (s, 1 H) ; 6,78 (dt, J=6,0 et 8,1 Hz, 1 H) ; 6,94 (t large, J=8,1 Hz, 1 H) ; 7,64 (d large, J=8,1 Hz, 1 H) ; 9,55 à 10,10 (m étalé, 2 H) ; 11,69 (m étalé, 1 H)
Spectrométrie de Masse : méthode B
Temps de rétention Tr (min) = 2,66
[M+H]+ : m/z 349 ; [M-H]- : m/z 347
Point de fusion (Kofler) : supérieur à 260°C

### Exemple 88 : Synthèse de 2-[2-(4-fluoro-2,3-dihydro-1H-indol-1-yl)-2-oxoéthyl]-6-(morpholin-4-yl)pyrimidin-4(3H)-one

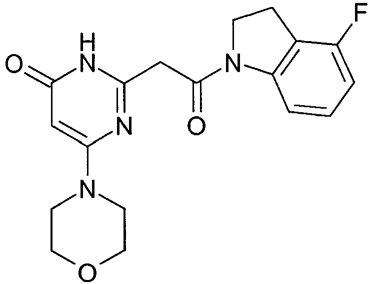

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 274 mg de 4-fluoro-2,3-dihydro-1H-indole, de 254 mg de N-[3-(diméthylamino)propyl]-N'-éthylcarbodiimide chlorhydrate dans un mélange de 0.16 mL de pyridine et de 4 mL de N,N-diméthylformamide. On obtient 249 mg de 2-[2-(4-fluoro-2,3-dihydro-1H-indol-1-yl)-2-oxoéthyl]-6-(morpholin-4-yl)pyrimidin-4(3H)-one sous forme d'un solide blanc dont les caractéristiques sont les suivantes :
Spectre RMN 1 H (400MHz):
   3,20 (t, J=8,4 Hz, 2 H) ; 3,41 (m, 4 H) ; 3,61 (m, 4 H) ; 3,76 (s, 2 H) ; 4,21 (t, J=8,4 Hz, 2 H) ; 5,21 (s, 1 H) ; 6,86 (t, J=8,6 Hz, 1 H) ; 7,12 à 7,31 (m, 1 H) ; 7,84 (d, J=8,1 Hz, 1 H) ; 11,61 (s large, 1 H)
Spectrométrie de Masse : méthode B
Temps de rétention Tr (min) = 3,20
[M+H]+ : m/z 359 ; [M-H]- : m/z 357
Point de fusion (Kofler) : supérieur à 260°C

### Exemple 89 : Synthèse de 2-[2-(4-chloro-2,3-dihydro-1H-indol-1-yl)-2-oxoéthyl]-6-(morpholin-4-yl)pyrimidin-4(3H)-one

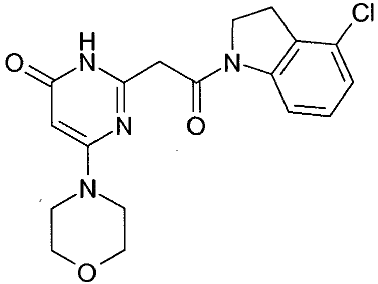

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 307 mg de 4-chloro-2,3-dihydro-1H-indole, de 254 mg de N-[3-(diméthylamino)propyl]-N'-éthylcarbodiimide chlorhydrate dans un mélange de 0.16 mL de pyridine et de 4 mL de N,N-diméthylformamide . On obtient 247 mg de 2-[2-(4-chloro-2,3-dihydro-1H-indol-1-yl)-2-oxoéthyl]-6-(morpholin-4-yl)pyrimidin-4(3H)-one sous forme d'un solide blanc dont les caractéristiques sont les suivantes :
Spectre RMN 1 H (400MHz):
   3,18 (t, J=8,4 Hz, 2 H) ; 3,41 (m, 4 H) ; 3,60 (m, 4 H) ; 3,76 (s, 2 H) ; 4,20 (t, J=8,4 Hz, 2 H) ; 5,21 (s, 1 H) ; 7,09 (d, J=8,1 Hz, 1 H) ; 7,22 (t, J=8,1 Hz, 1 H) ; 7,97 (d, J=8,1 Hz, 1 H) ; 11,62 (s large, 1 H)
Spectrométrie de Masse : méthode B
Temps de rétention Tr (min) = 3,46 ;
[M+H]+ : m/z 375 ; [M-H]- : m/z 373
Point de fusion (Kofler) : supérieur à 260°C

### Exemple 90 : Synthèse de N-(3-éthynyl-4-fluorophényl)-2-[4-(morpholin-4-yl)-6-oxo-1,6-dihydropyrimidin-2-yl]acétamide

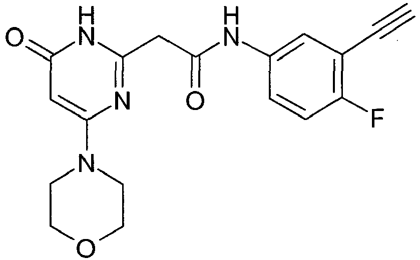

### Etape 1 :

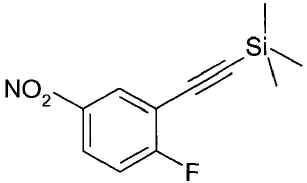

A une solution de 1.169 g de 2-bromo-1-fluoro-4-nitrobenzène dans 13 mL de triéthylamine sous agitation et sous argon, sont ajoutés 1.126 mL d' éthynyl(triméthyl)silane, de 55 mg de triphénylphosphine et de 24 mg d'acétate de paladium (2+). Après 4 heures et trente minutes à une température de 100°C, le milieu réactionnel est refroidit et l'insoluble filtré sur verre fritté. Le filtrat est concentré à sec sous pression réduite. Après deux purifications sur colonne de silice éluant: Heptane/AcOEt, 90/10 puis Heptane/AcOEt, 95/5, on obtient 460 mg de [(2-fluoro-5-nitrophényl)éthynyl](triméthyl)silane sous forme d'un solide jaune dont les caractéristiques sont les suivantes :
Spectrométrie de Masse : Les spectres ont été réalisés par introduction directe sur un appareil WATERS GCTof (introduction directe sans LC) :
El : [M]+. m/z 237 ; pic de base : m/z 222

### Etape 2

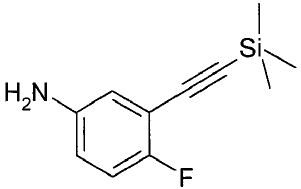

A une solution de 460 mg de [(2-fluoro-5-nitrophényl)éthynyl](triméthyl)silane dans 10 mL de méthanol sont ajoutés 460 mg de fer et le milieu est ajusté à pH 4-5 à l'aide d'acide chlorhydrique concentrée. Après trois heures trente de reflux, le milieu est refroidit puis filtré sur silice, concentré à sec sous pression réduite. Le résidu est repris par de l'oxyde de diéthyle, l'insoluble filtré puis le filtrat concentré à sec sous pression réduite. On obtient 370 mg de 4-fluoro-3-[(triméthylsilyl)éthynyl]aniline sous forme d'une huile orangée dont les caractéristiques sont les suivantes :
Spectrométrie de Masse : méthode A
Temps de rétention Tr (min) = 1,11
[M+H]+ : m/z 208 ; pic de base : m/z 249

### Etape 3

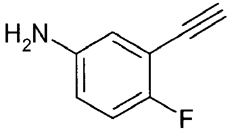

A une solution de 360 mg de 4-fluoro-3-[(triméthylsilyl)éthynyl]aniline dans 5 mL de méthanol, sont ajoutés 16 mg de carbonate de potassium. Après une nuit d'agitation sous argon et à une température voisine de 20°C, le milieu est concentré à sec sous pression réduite puis repris dans 8 mL d'eau, neutralisé avec quelques gouttes d'acide chlorhydrique (1 N) puis extrait trois fois par de l'oxyde de diéthyle. Les phases organiques réunies sont séchées sur sulfate de magnésium anhydre, filtrées et concentrées à sec sous pression réduite. On obtient 225 mg de 3-éthynyl-4-fluoroaniline sous forme d'une huile brune dont les caractéristiques sont les suivantes :
Spectrométrie de Masse : méthode A
Temps de rétention Tr (min) = 0,49
[M+H]+ : m/z 136 ;

### Etape 4

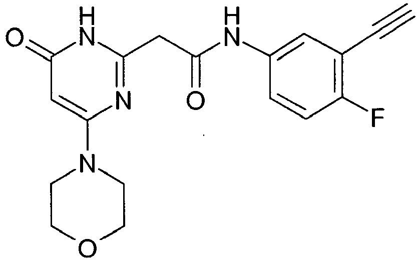

Le produit est préparé en suivant le mode opératoire décrit à l'exemple 5 à partir de 204 mg de [4-(morpholin-4-yl)-6-oxo-1,6-dihydropyrimidin-2-yl]acétate de sodium, de 211 mg de 3-éthynyl-4-fluoroaniline, de 195 mg de N-[3-(diméthylamino)propyl]-N'-éthylcarbodiimide chlorhydrate dans un mélange de 0.126 mL de pyridine et de 3 mL de N,N-diméthylformamide. On obtient 200 mg de N-(3-éthynyl-4-fluorophényl)-2-[4-(morpholin-4-yl)-6-oxo-1,6-dihydropyrimidin-2-yl]acétamide sous forme d'un solide blanc dont les caractéristiques sont les suivantes :
Spectre RMN 1 H (400MHz):
   3,41 (m, 4 H) ; 3,60 (m, 6 H) ; 4,48 (s, 1 H) ; 5,20 (s, 1 H) ; 7,27 (t, J=9,0 Hz, 1 H) ; 7,56 (m, 1 H) ; 7,78 (dd, J=1,5 et 5,9 Hz, 1 H) ; 10,31 (m étalé, 1 H) ; 11,66 (m étalé, 1 H)
Spectrométrie de Masse : méthode A
Temps de rétention Tr (min) = 0,75
[M+H]+ : m/z 357 ; [M-H]- : m/z 355
Point de fusion (Kofler) : supérieur à 260°C

### Exemple 91 : Synthèse de 2-[2-(4-hydroxy-2,3-dihydro-1H-indol-1-yl)-2-oxoéthyl]-6-(morpholin-4-yl)pyrimidin-4(3H)-one

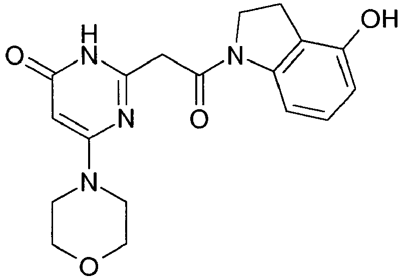

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 270 mg de 2,3-dihydro-1H-indol-4-ol, de 254 mg de N-[3-(diméthylamino)propyl]-N'-éthylcarbodiimide chlorhydrate dans un mélange de 0.16 mL de pyridine et de 4 mL de N,N-diméthylformamide . On obtient 205 mg de 2-[2-(4-hydroxy-2,3-dihydro-1H-indol-1-yl)-2-oxoéthyl]-6-(morpholin-4-yl)pyrimidin-4(3H)-one sous forme d'un solide rose dont les caractéristiques sont les suivantes :
Spectre RMN 1 H (400MHz):
   3,01 (t, J=8,1 Hz, 2 H) ; 3,41 (m, 4 H) ; 3,61 (m, 4 H) ; 3,71 (s, 2 H) ; 4,12 (t, J=8,1 Hz, 2 H) ; 5,20 (s, 1 H) ; 6,49 (d, J=8,1 Hz, 1 H) ; 6,97 (t, J=8,1 Hz, 1 H) ; 7,50 (d, J=8,1 Hz, 1 H) ; 9,44 (s large, 1 H) ; 11,60 (s large, 1 H)
Spectrométrie de Masse : méthode A
Temps de rétention Tr (min) = 0,57
[M+H]+ : m/z 357 ; [M-H]- : m/z 355
Point de fusion (Kofler) : supérieur à 260°C

### Exemple 92 : Synthèse de 2-[2-(4,6-difluoro-2,3-dihydro-1H-indol-1-yl)-2-oxoéthyl]-6-(morpholin-4-yl)pyrimidin-4(3H)-one

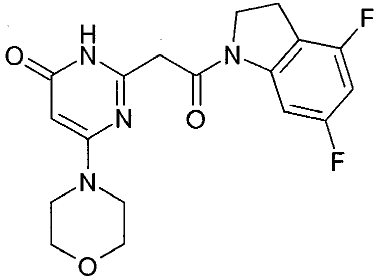

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 310 mg de 4,6-difluoro-2,3-dihydro-1H-indole, de 254 mg de N-[3-(diméthylamino)propyl]-N'-éthylcarbodiimide chlorhydrate dans un mélange de 0.16 mL de pyridine et de 4 mL de N,N-diméthylformamide . On obtient 225 mg de 2-[2-(4,6-difluoro-2,3-dihydro-1H-indol-1-yl)-2-oxoéthyl]-6-(morpholin-4-yl)pyrimidin-4(3H)-one sous forme d'un solide blanc dont les caractéristiques sont les suivantes :
Spectre RMN 1 H (400M Hz):
   3,17 (t, J=8,4 Hz, 2 H) ; 3,41 (m, 4 H) ; 3,61 (m, 4 H) ; 3,78 (s, 2 H) ; 4,24 (t, J=8,4 Hz, 2 H) ; 5,21 (s, 1 H) ; 6,89 (t large, J=8,9 Hz, 1 H) ; 7,64 (d large, J=8,9 Hz, 1 H) ; 11,62 (m étalé, 1 H)
Spectrométrie de Masse : méthode A
Temps de rétention Tr (min) = 0,73
[M+H]+ : m/z 377 ; [M-H]- : m/z 375
Point de fusion (Kofler) : supérieur à 260°C

### Exemple 93 : Synthèse du N-(3-iodo-4-fluorophényl)-2-[1-méthyl-4-(morpholin-4-yl)-6-oxo-1,6-dihydropyrimidin-2-yl]acétamide

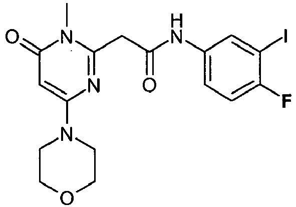

Le produit est préparé en suivant le mode opératoire décrit à l'exemple 68 à partir de 200 mg de [1-méthyl-4-(morpholin-4-yl)-6-oxo-1,6-dihydropyrimidin-2-yl]acétate de sodium et de 210 mg de 3-iodo-4-fluoro-aniline. On obtient 186 mg de N-(3-iodo-4-fluorophényl)-2-[1-méthyl-4-(morpholin-4-yl)-6-oxo-1,6-dihydropyrimidin-2-yl]acétamide sous forme de solide blanc dont les caractéristiques sont les suivantes:
Spectre RMN 1 H (400MHz): 3,33 (s, 3 H) ; 3,40 (m, 4 H) ; 3,58 (m, 4 H) ; 3,90 (s, 2 H) ; 5,36 (s, 1 H) ; 7,22 (t, J=8,4 Hz, 1 H) ; 7,49 (m, 1 H) ; 8,11 (dd, J=2,4 et 5,6 Hz, 1 H) ; 10,33 (m étalé, 1 H)
Spectrométrie de Masse : méthode B
Temps de rétention Tr (min) = 3,50 ;
[M+H]+ : m/z 473 ; [M-H]- : m/z 471

### Exemple 94 : Synthèse de 2-[2-(4,5-difluoro-2,3-dihydro-1H-indol-1-yl)-2-oxoéthyl]-6-(morpholin-4-yl)pyrimidin-4(3H)-one

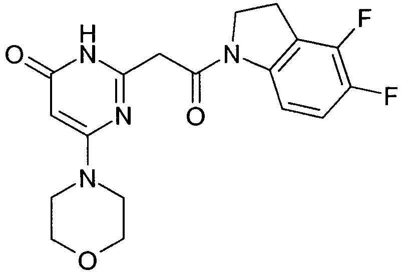

Le produit est préparé en suivant le mode opératoire décrit à l'exemple 5 mais à partir de 261 mg de [4-(morpholin-4-yl)-6-oxo-1,6-dihydropyrimidin-2-yl]acétate de sodium préparé à l'étape 2 de l'exemple 1, de 383 mg de 4,5-difluoro-2,3-dihydro-1H-indole chlorhydrate, de 254 mg de N-[3-(diméthylamino)propyl]-N'-éthylcarbodiimide chlorhydrate dans un mélange de 0.32 mL de pyridine et de 4 mL de N,N-diméthylformamide . On obtient 210 mg de 2-[2-(4,5-difluoro-2,3-dihydro-1H-indol-1-yl)-2-oxoéthyl]-6-(morpholin-4-yl)pyrimidin-4(3H)-one sous forme d'un solide poudreux rose pâle dont les caractéristiques sont les suivantes :
Spectre RMN 1 H (400MHz):
   3,25 (t, J=8,3 Hz, 2 H) ; 3,41 (m, 4 H) ; 3,61 (m, 4 H) ; 3,76 (s, 2 H) ; 4,23 (t, J=8,3 Hz, 2 H) ; 5,21 (s, 1 H) ; 7,05 à 7,34 (m, 1 H) ; 7,79 (dd, J=3,4 et 8,8 Hz, 1 H) ; 11,60 (m étalé, 1 H)
Spectrométrie de Masse : méthode B
Temps de rétention Tr (min) = 3,30 ;
[M+H]+ : m/z 377 ; [M-H]- : m/z 375
Point de fusion (Kofler) : supérieur à 260°C

### Exemple 95 : Synthèse de 2-[2-(6-fluoro-2,3-dihydro-1H-indol-1-yl)-2-oxoéthyl]-6-(morpholin-4-yl)pyrimidin-4(3H)-one

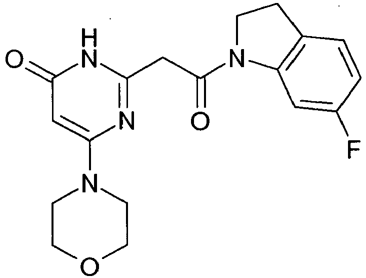

Le produit est préparé en suivant le mode opératoire décrit à l'exemple 5 mais à partir de 200 mg de (4-(morpholin-4-yl)-6-oxo-1,6-dihydropyrimidin-2-yl]acétate de sodium préparé à l'étape 2 de l'exemple 1, de 210 mg de 6-fluoro-2,3-dihydro-1H-indole, de 194 mg de N-[3-(diméthylamino)propyl]-N'-éthylcarbodiimide chlorhydrate dans un mélange de 0.12 mL de pyridine et de 3 mL de N,N-diméthylformamide . On obtient 184 mg de 2-[2-(6-fluoro-2,3-dihydro-1H-indol-1-yl)-2-oxoéthyl]-6-(morpholin-4-yl)pyrimidin-4(3H)-one sous forme d'une poudre blanche dont les caractéristiques sont les suivantes :
Spectre RMN 1 H (400MHz):
   3,14 (t, J=8,3 Hz, 2 H) ; 3,37 à 3,45 (m, 4 H) ; 3,54 à 3,64 (m, 4 H) ; 3,76 (s, 2 H) ; 4,19 (t, J=8,3 Hz, 2 H) ; 5,21 (s, 1 H) ; 6,83 (dt, J=2,0 et 8,6 Hz, 1 H) ; 7,19 à 7,31 (m, 1 H) ; 7,77 (dd, J=2,6 et 10,8 Hz, 1 H) ; 11,61 (m étalé, 1 H)
Spectrométrie de Masse : méthode A
Temps de rétention Tr (min) = 0,67 ;
[M+H]+ : m/z 359 ; [M-H]- : m/z 357
Point de fusion (Kofler) : supérieur à 260°C

### Exemple 96: Synthèse de 2-[2-(2-méthyl-2,3-dihydro-1H-indol-1-yl)-2-oxoéthyl]-6-(morpholin-4-yl)pyrimidin-4(3H)-one

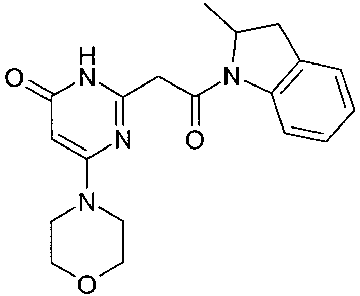

Le produit est préparé en suivant le mode opératoire décrit à l'exemple 5 à partir de 500 mg de [4-(morpholin-4-yl)-6-oxo-1,6-dihydropyrimidin-2-yl]acétate de sodium, de 510 mg de 2-méthyl-2,3-dihydro-1H-indole, de 487 mg de N-[3-(diméthylamino)propyl]-N'-éthylcarbodiimide chlorhydrate dans un mélange de 0.308 mL de pyridine et de 8 mL de N,N-diméthylformamide . On obtient 400 mg de 2-[2-(2-méthyl-2,3-dihydro-1H-indol-1-yl)-2-oxoéthyl]-6-(morpholin-4-yl)pyrimidin-4(3H)-one sous forme d'une poudre blanche dont les caractéristiques sont les suivantes :
Spectre RMN 1 H (400MHz):
   1,26 (d, J=6,1 Hz, 3 H) ; 2,65 à 2,72 (m, 1 H) ; 3,18 à 3,44 (m partiellement masqué, 5 H) ; 3,54 à 3,63 (m, 4 H) ; 3,72 (d, J=15,7 Hz, 1 H) ; 3,92 (d, J=15,7 Hz, 1 H) ; 4,71 (m, 1 H) ; 5,20 (s, 1 H) ; 7,04 (t, J=7,8 Hz, 1 H) ; 7,18 (t, J=7,8 Hz, 1 H) ; 7,29 (d, J=7,8 Hz, 1 H) ; 7,96 (d, J=7,8 Hz, 1 H) ; 11,69 (m étalé, 1 H)
Spectrométrie de Masse : méthode A
Temps de rétention Tr (min) = 0,70 ;
[M+H]+ : m/z 355 ; [M-H]- : m/z 353
Point de fusion (Kofler) : 172°C

### Exemple 97 : Synthèse de N-[3-(difluorométhyl)-4-fluorophényl]-2-[1-méthyl-4-(morpholin-4-yl)-6-oxo-1,6-dihydropyrimidin-2-yl]acétamide

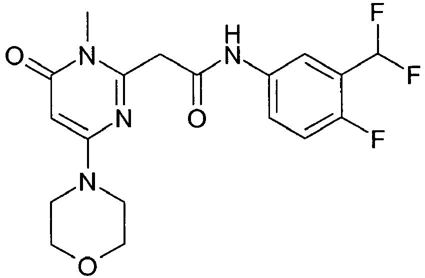

Le produit est préparé en suivant le mode opératoire décrit à l'exemple 5 mais à partir de 275 mg de [1-méthyl-4-(morpholin-4-yl)-6-oxo-1,6-dihydropyrimidin-2-yl]acétate de sodium préparé à l'étape 1 de l'exemple 68, de 322 mg de 3-(difluorométhyl)-4-fluoroaniline (exemple 76 étape 2), et de 250 mg de N-[3-(diméthylamino)propyl]-N'-éthylcarbodiimide chlorhydrate dans un mélange de 0.16 mL de pyridine et de 4 mL de N,N-diméthylformamide . On obtient 98 mg de N-[3-(difluorométhyl)-4-fluorophényl]-2-[1-méthyl-4-(morpholin-4-yl)-6-oxo-1,6-dihydropyrimidin-2-yl]acétamide sous forme d'un solide blanc dont les caractéristiques sont les suivantes:
Spectre RMN 1 H (400MHz):
   3,34 (s, 3 H) ; 3,39 (m, 4 H) ; 3,58 (m, 4 H) ; 3,92 (s, 2 H) ; 5,36 (s, 1 H) ; 7,20 (t, J=54,3 Hz, 1 H) ; 7,34 (t, J=9,5 Hz, 1 H) ; 7,68 (m, 1 H) ; 7,89 (m, 1 H) ; 10,45 (s, 1 H)
Spectrométrie de Masse : méthode B
Temps de rétention Tr (min) = 3,29 ;
[M+H]+ : m/z 397 ; [M-H]- : m/z 395

### Exemple 98: Synthèse du 2-(1-méthyl-4-morpholin-4-yl-6-oxo-1,6-dihydro-pyrimidin-2-yl)-N-(3,4,5-trifluorophényl)-acétamide

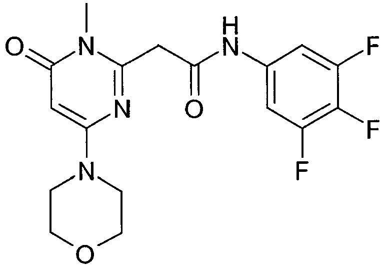

Le produit est préparé en suivant le mode opératoire décrit à l'exemple 68 à partir de 200 mg de [1-méthyl-4-(morpholin-4-yl)-6-oxo-1,6-dihydropyrimidin-2-yl]acétate de sodium, de 214 mg de 3,4,5-trifluoro-aniline, de 180 mg de N-[3-(diméthylamino)propyl]-N'-éthylcarbodiimide chlorhydrate dans un mélange de 0,12 mL de pyridine et de 2,5 mL de N,N-diméthylformamide. On obtient 72 mg de 2-(1-méthyl-4-morpholin-4-yl-6-oxo-1,6-dihydro-pyrimidin-2-yl)-N-(3,4,5-trifluorophényl)-acétamide sous forme d'un solide crème dont les caractéristiques sont les suivantes :
Spectre RMN 1 H (400 MHz) : 3,33 (s, 3 H) ; 3,39 (m, 4 H) ; 3,58 (m, 4 H) ; 3,93 (s, 2 H) ; 5,36 (s, 1 H) ; 7,46 (dd, J=6,4 et 10,3 Hz, 2 H) ; 10,58 (s large, 1 H)
Spectrométrie de Masse : méthode B
Temps de rétention Tr (min) = 3,47 ;
[M+H]+ : m/z 383 ; [M-H]- : m/z 381
Point de fusion (Kofler) : supérieur à 266°C

### Exemple 99 : Synthèse du N-(1-méthyl-1H-indol-4-yl)-2-[4-(morpholin-4-yl)-6-oxo-1,6-dihydropyrimidin-2-yl]acétamide

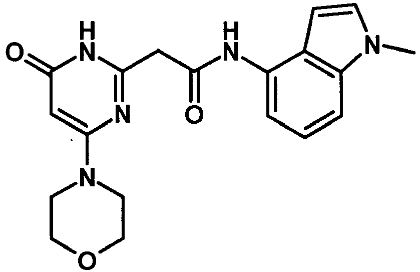

A une solution de 250 mg de [4-(morpholin-4-yl)-6-oxo-1,6-dihydropyrimidin-2-yl]acétate de sodium préparé à l'étape 2 de l'exemple 1 dans 2 mL de N,N-diméthylformamide sont ajoutés 2 mL de pyridine, 300 mg de N-[3-(diméthylamino)propyl]-N'-éthylcarbodiimide chlorhydrate et 272 mg de 1-méthyl-1H-indol-4-ylamine. Le mélange réactionnel est agité à température ambiante pendant 15 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 280 mg du N-(1-méthyl-1H-indol-4-yl)-2-[4-(morpholin-4-yl)-6-oxo-1,6-dihydropyrimidin-2-yl]acétamide sous forme de solide rosé dont les caractéristiques sont les suivantes:
Spectre RMN 1 H (400MHz) : 3,42 (m, 4 H) ; 3,60 (m, 4 H) ; 3,73 (s, 2 H) ; 3,78 (s, 3 H) ; 5,21 (s, 1 H) ; 6,67 (d, J=2,9 Hz, 1 H) ; 7,08 (t, J=8,1 Hz, 1 H) ; 7,19 (d, J=8,1 Hz, 1 H) ; 7,29 (d, J=2,9 Hz, 1 H) ; 7,60 (d, J=8,1 Hz, 1 H) ; 9,85 (s large, 1 H) ; 11,68 (m étalé, 1 H)
Spectrométrie de Masse : méthode
[M+H]+ : m/z 366 ; [M-H]- : m/z 368

### Exemple 100 : Synthèse de la 2-[2-(4-méthyl-2,3-dihydro-1H-indol-1-yl)-2-oxoéthyl]-6-(morpholin-4-yl)pyrimidin-4(3H)-one

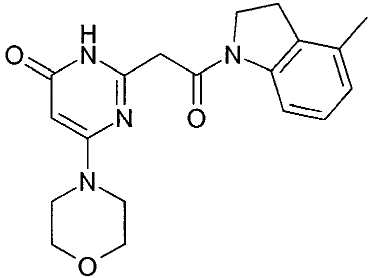

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 339 mg de chlorhydrate de 4-méthyl-indoline, de 254 mg de N-[3-(diméthylamino)propyl]-N'-éthylcarbodiimide chlorhydrate dans un mélange de 241 µL de pyridine et de 4,0 mL de N,N-diméthylformamide. On obtient 22 mg de 2-[2-(4-méthyl-2,3-dihydro-1H-indol-1-yl)-2-oxoéthyl]-6-(morpholin-4-yl)pyrimidin-4(3H)-one sous forme d'une poudre cristalline fuchsia dont les caractéristiques sont les suivantes :
Spectre RMN 1 H (400 MHz): 2,21 (s, 3 H) ; 3,08 (t, J=8,3 Hz, 2 H) ; 3,42 (m, 4 H); 3,61 (m, 4 H) ; 3,75 (s, 2 H) ; 4,15 (t, J=8,3 Hz, 2 H) ; 5,20 (s, 1 H) ; 6,85 (d, J=8,0 Hz, 1 H) ; 7,07 (t, J=8,0 Hz, 1 H) ; 7,85 (d, J=8,0 Hz, 1 H) ; 11,62 (m étalé, 1 H)
Spectrométrie de Masse : méthode B
Temps de rétention Tr (min) = 3,31 ;
[M+H]+ : m/z 355 ; [M-H]- : m/z 353
Point de fusion (Kofler) : supérieur à 260°C

### Exemple 101 : Synthèse de la 2-[2-(3-méthyl-2,3-dihydro-1H-indol-1-yl)-2-oxoéthyl]-6-(morpholin-4-yl)pyrimidin-4(3H)-one

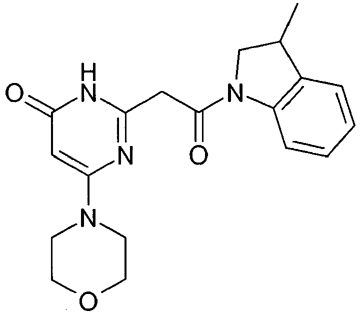

Le produit est préparé en suivant le mode opératoire décrit à l'exemple 5 à partir de 500 mg de [4-(morpholin-4-yl)-6-oxo-1,6-dihydropyrimidin-2-yl]acétate de sodium, de 170 mg de 3-méthyl-indoline, de 487 mg de N-[3-(diméthylamino)propyl]-N'-éthylcarbodiimide chlorhydrate dans un mélange de 310 µL de pyridine et de 6,0 mL de N,N-diméthylformamide. On obtient 367 mg de 2-[2-(3-méthyl-2,3-dihydro-1H-indol-1-yl)-2-oxoéthyl]-6-(morpholin-4-yl)pyrimidin-4(3H)-one sous forme d'une poudre cristalline blanche dont les caractéristiques sont les suivantes :
Spectre RMN 1 H (400 MHz): 1,29 (d, *J*=6,8 Hz, 3 H) ; 3,42 (m, 4 H) ; 3,49 (m, 1 H) ; 3,60 (m, 4 H) ; 3,68 (dd, *J*=6,8 et 9,8 Hz, 1 H) ; 3,75 (s, 2 H) ; 4,33 (t, *J*=9,8 Hz, 1 H) ; 5,21 (s, 1 H) ; 7,04 (t, *J*=7,8 Hz, 1 H) ; 7,18 (t, *J*=7,8 Hz, 1 H) ; 7,27 (d, *J*=7,8 Hz, 1 H) ; 8,01 (d, *J*=7,8 Hz, 1 H) ; 11,62 (m étalé, 1 H)
Spectrométrie de Masse : méthode A
Temps de rétention Tr (min) = 0,73 ;
[M+H]+ : m/z 355 ; [M-H]- : m/z 353
Point de fusion (Kofler) : supérieur à 260°C

### Exemple 102 : Synthèse de la 2-[2-(4-fluoro-2,3-dihydro-1H-indol-1-yl)-2-oxoéthyl]-3-méthyl-6-(morpholin-4-yl)pyrimidin-4(3H)-one

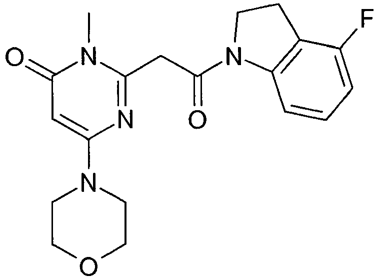

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 274 mg de 4-fluoro-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 102 mg de 2-[2-(4-fluoro-2,3-dihydro-1H-indol-1-yl)-2-oxoéthyl]-3-méthyl-6-(morpholin-4-yl)pyrimidin-4(3H)-one sous forme d'une poudre rose pâle dont les caractéristiques sont les suivantes :
Spectre RMN 1 H (400 MHz): 3,19 (t, *J*=8,4 Hz, 2 H) ; 3,31 (s, 3 H) ; 3,39 (m, 4 H); 3,57 (m, 4 H) ; 4,12 (s, 2 H) ; 4,25 (t, *J*=8,4 Hz, 2 H) ; 5,36 (s, 1 H) ; 6,87 (t, *J*=8,7 Hz, 1 H) ; 7,22 (m, 1 H) ; 7,84 (d, *J*=8,1 Hz, 1 H)
Spectrométrie de Masse : méthode A
Temps de rétention Tr (min) = 0,73 ;
[M+H]+ : m/z 373 ; [M-H]- : m/z 371
Point de fusion (Kofler) : 244°C

### Exemple 103 : 2-[2-(5-Fluoro-2,3-dihydro-indol-1-yl)-2-oxo-éthyl]-6-morpholin-4-yl-3H-pyrimidin-4-one

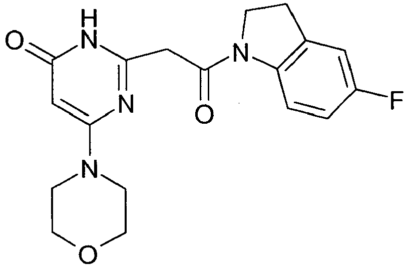

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 274 mg de 5-fluoro-indoline, de 254 mg de N-[3-(diméthylamino)propyl]-N'-éthylcarbodiimide chlorhydrate dans un mélange de 0,16 mL de pyridine et de 4 mL de diméthylformamide. On obtient 197 mg de 2-[2-(5-fluoro-2,3-dihydro-indol-1-yl)-2-oxo-éthyl]-6-morpholin-4-yl-3H-pyrimidin-4-one sous forme d'une poudre rose très pâle dont les caractéristiques sont les suivantes :
Spectre RMN 1 H (400 MHz, δ en ppm, DMSO-d₆): 3,18 (t, *J*=8,2 Hz, 2 H) ; 3,42 (m, 4 H) ; 3,60 (m, 4H) ; 3,74 (s, 2 H) ; 4,16 (t, *J*=8,2 Hz, 2 H) ; 5,20 (s, 1 H) ; 6,98 (t large, *J*=8,9 Hz, 1 H) ; 7,12 (d large, *J*=8,9 Hz, 1 H) ; 8,00 (dd, *J*=5,3 et 8,9 Hz, 1 H) ; 11,61 (m étalé, 1 H).
Spectrométrie de Masse : méthode A
Temps de rétention Tr (min) = 0,70 ;
[M+H]+ : m/z 359 ; [M-H]- : m/z 357
Point de fusion (Kofler) : 264°C

### Exemple 104 : Synthèse de la 2-[2-(4-chloro-2,3-dihydro-1H-indol-1-yl)-2-oxoéthyl]-3-méthyl-6-(morpholin-4-yl)pyrimidin-4(3H)-one

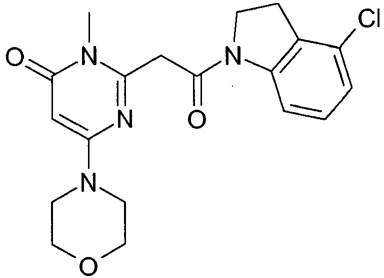

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 307 mg de 4-chloro-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 84 mg de 2-[2-(4-chloro-2,3-dihydro-1H-indol-1-yl)-2-oxoéthyl]-3-méthyl-6-(morpholin-4-yl)pyrimidin-4(3H)-one sous forme d'une poudre légèrement rosée dont les caractéristiques sont les suivantes :
Spectre RMN 1 H (400 MHz): 3,18 (t, *J*=8,4 Hz, 2 H) ; 3,31 (s large, 3 H) ; 3,39 (m, 4 H) ; 3,58 (m, 4 H) ; 4,11 (s, 2 H) ; 4,23 (t, *J*=8,4 Hz, 2 H) ; 5,36 (s, 1 H) ; 7,09 (d, *J*=8,1 Hz, 1 H) ; 7,22 (t, *J*=8,1 Hz, 1 H) ; 7,96 (d, *J*=8,1 Hz, 1 H)
Spectrométrie de Masse : méthode A
Temps de rétention Tr (min) = 0,81 ;
[M+H]+ : m/z 389 ; [M-H]- : m/z 387
Point de fusion (Kofler) : 235°C

### Exemple 105 : Synthèse du N-(1-benzothiophèn-4-yl)-2-[4-(morpholin-4-yl)-6-oxo-1,6-dihydropyrimidin-2-yl]acétamide

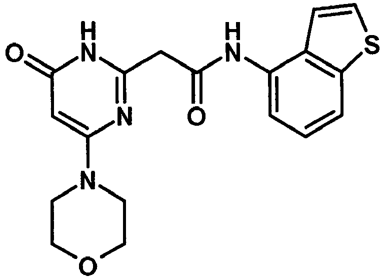

A une solution de 250 mg de [4-(morpholin-4-yl)-6-oxo-1,6-dihydropyrimidin-2-yl]acétate de sodium préparé à l'étape 2 de l'exemple 1 dans 2 mL de N,N-diméthylformamide sont ajoutés 2 mL de pyridine, 257 mg de N-[3-(diméthylamino)propyl]-N'-éthylcarbodiimide chlorhydrate et 223 mg de benzo[B]thiophèn-4-ylamine. Le mélange réactionnel est agité à température ambiante pendant 15 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 262 mg du N-(1-benzothiophèn-4-yl)-2-[4-(morpholin-4-yl)-6-oxo-1,6-dihydropyrimidin-2-yl]acétamide sous forme de solide blanc cassé dont les caractéristiques sont les suivantes:
Spectre RMN 1 H (400MHz) : 3,43 (m, 4 H) ; 3,60 (m, 4 H) ; 3,74 (s, 2 H) ; 5,21 (s, 1 H) ; 7,34 (t, J=7,9 Hz, 1 H) ; 7,65 (d, J=5,6 Hz, 1 H) ; 7,71 à 7,82 (m, 3 H) ; 10,17 (m étalé, 1 H) ; 11,70(m étalé, 1 H)
Spectrométrie de Masse : méthode A
Temps de rétention Tr (min) = 0,66;
[M+H]+ : m/z 369 ; [M-H]- : m/z 371

### Exemple 106 : Synthèse de la 2-{2-[2-(hydroxyméthyl)-2,3-dihydro-1H-indol-1-yl]-2-oxoéthyl)-6-(morpholin-4-yl)pyrimidin-4(3H)-one

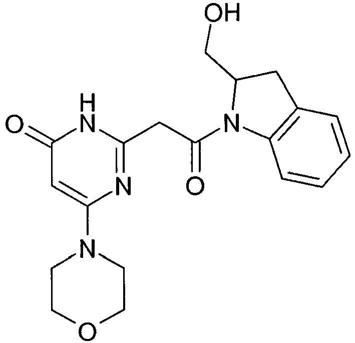

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 298 mg de 2,3-dihydro-1H-indol-2-ylméthanol, de 249 mg de N-[3-(diméthylamino)propyl]-N'-éthylcarbodiimide chlorhydrate dans un mélange de 322 µL de pyridine et de 4,0 mL de N,N-diméthylformamide. On obtient 212 mg de 2-{2-[2-(hydroxyméthyl)-2,3-dihydro-1H-indol-1-yl]-2-oxoéthyl}-6-(morpholin-4-yl)pyrimidin-4(3H)-one sous forme d'une poudre cristalline rose pâle dont les caractéristiques sont les suivantes :
Spectre RMN 1 H (400 MHz): 2,86 (d, J=16,1 Hz, 1 H) ; 3,25 (dd, J=8,6 et 16,1 Hz, 1 H) ; 3,34 à 3,43 (m, 5 H) ; 3,51 (m, 1 H) ; 3,60 (m, 4 H) ; 3,81 (d, J=15,9 Hz, 1 H) ; 4,01 (d, J=15,9 Hz, 1 H) ; 4,64 (m, 1 H) ; 5,13 (m large, 1 H) ; 5,20 (s, 1 H) ; 7,03 (t, J=7,8 Hz, 1 H) ; 7,16 (t, J=7,8 Hz, 1 H) ; 7,26 (d, J=7,8 Hz, 1 H) ; 7,94 (d, J=7,8 Hz, 1 H) ; 11,63 (m étalé, 1 H)
Spectrométrie de Masse : méthode B
Temps de rétention Tr (min) = 2,80 ;
[M+H]+ : m/z 371 ; [M-H]- : m/z 369
Point de fusion (Kofler) : 200°C

**Exemple 107 : Synthèse de 2-[4-(morpholin-4-yl)-6-oxo-1,6-dihydropyrimidin-2-yl]-N-{2-[2-(piperidin-1-yl)ethoxy]phenyl}acetamide**

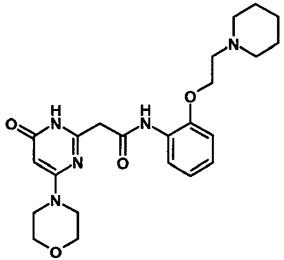

Le produit est préparé en suivant le mode opératoire décrit à l'exemple 5 à partir de 250 mg de [4-(morpholin-4-yl)-6-oxo-1,6-dihydropyrimidin-2-yl]acétate de sodium, de 303 mg de 2-(2-pipéridin-1-yl-éthoxy)-phénylamine, de 300 mg de N-[3-(diméthylamino)propyl]-N'-éthylcarbodiimide chlorhydrate dans un mélange de 2 mL de pyridine et de 2 mL de diméthylformamide. On obtient 255 mg de 2-[4-(morpholin-4-yl)-6-oxo-1,6-dihydropyrimidin-2-yl]-N-{2-[2-(piperidin-1-yl)ethoxy]phenyl}acetamide sous forme d'un solide beige dont les caractéristiques sont les suivantes:
Spectre RMN 1 H (400 MHz): 1,37 (m, 2 H) ; 1,43 à 1,53 (m, 4 H) ; 2,40 (m, 4 H) ; 2,65 (t, J=6,0 Hz, 2 H) ; 3,43 (m, 4 H) ; 3,60 (m, 4 H) ; 3,68 (s, 2 H) ; 4,13 (t, J=6,0 Hz, 2 H) ; 5,22 (s, 1 H) ; 6,89 (m, 1 H) ; 7,00 à 7,13 (m, 2 H) ; 7,93 (d, J=8,3 Hz, 1 H) ; 9,30 (m étalé, 1 H)
Spectrométrie de Masse : méthode B
Temps de rétention Tr (min) = 2,20 ;
[M+H]+ : m/z 442 ; [M-H]- : m/z 440

### Exemple 108: Synthèse du N-[2-(2-méthoxyéthoxy)phényl]-2-[4-(morpholin-4-yl)-6-oxo-1,6-dihydropyrimidin-2-yl]acétamide

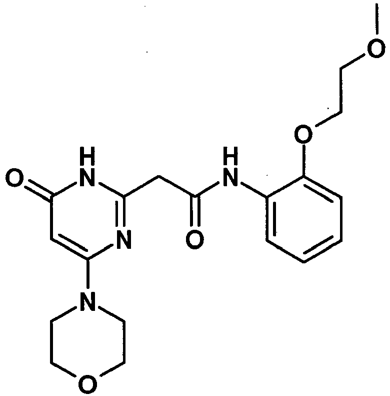

A une solution de 250 mg de [4-(morpholin-4-yl)-6-oxo-1,6-dihydropyrimidin-2-yl]acétate de sodium préparé à l'étape 2 de l'exemple 1 dans 2 mL de N,N-diméthylformamide sont ajoutés 2 mL de pyridine, 300 mg de N-[3-(diméthylamino)propyl]-N'-éthylcarbodiimide chlorhydrate et 303 mg de 2-(2-méthoxy-éthoxy)-phénylamine. Le mélange réactionnel est agité à température ambiante pendant 15 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 223 mg du N-[2-(2-méthoxyéthoxy)phényl]-2-[4-(morpholin-4-yl)-6-oxo-1,6-dihydropyrimidin-2-yl]acétamide sous forme de solide rosé dont les caractéristiques sont les suivantes:
Spectre RMN 1 H (400MHz) : 3,19 à 3,33 (s partiellement masqué, 3 H) ; 3,43 (m, 4H) ; 3,61 (m, 4 H) ; 3,67 à 3,72 (m, 4 H) ; 4,16 (t, J=4,5 Hz, 2 H) ; 5,21 (s, 1 H) ; 6,94 (m, 1 H) ; 7,02 à 7,13 (m, 2 H) ; 7,95 (d, J=8,1 Hz, 1 H) ; 9,25 (s large, 1 H) ; 11,67 (m étalé, 1 H)
Spectrométrie de Masse : méthode B
Temps de rétention Tr (min) = 3,00 ;
[M+H]+ : m/z 387 ; [M-H]- : m/z 389

### Exemple 109 : Synthèse de la 2-[2-(4-hydroxy-2,3-dihydro-1H-indol-1-yl)-2-oxoéthyl]-3-méthyl-6-(morpholin-4-yl)pyrimidin-4(3H)-one

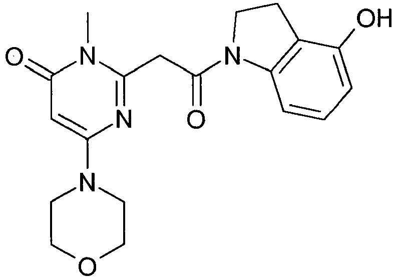

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 270 mg d'indoline-4-ol, de 254 mg de N-[3-{diméthylamino)propyl]-N'-éthylcarbodiimide chlorhydrate dans un mélange de 161 µL de pyridine et de 4,0 mL de N,N-diméthylformamide. On obtient 85 mg de 2-[2-(4-hydroxy-2,3-dihydro-1H-indol-1-yl)-2-oxoéthyl]-3-méthyl-6-(morpholin-4-yl)pyrimidin-4(3H)-one sous forme d'une poudre rose dont les caractéristiques sont les suivantes :
Spectre RMN 1 H (400 MHz): 3,01 (t, J=8,6 Hz, 2 H) ; 3,31 (s masqué, 3 H) ; 3,40 (m, 4 H) ; 3,58 (m, 4 H) ; 4,07 (s, 2 H) ; 4,16 (t, J=8,6 Hz, 2 H) ; 5,36 (s, 1 H) ; 6,50 (d, J=8,2 Hz, 1 H) ; 6,97 (t, J=8,2 Hz, 1 H) ; 7,49 (d, J=8,2 Hz, 1 H) ; 9,47 (s large, 1 H)
Spectrométrie de Masse : méthode A
Temps de rétention Tr (min) = 0,49 ;
[M+H]+ : m/z 371 ; [M-H]- : m/z 369
Point de fusion (Kofler) : supérieur à 260°C

### Exemple 110 : Synthèse de la 2-[2-(4-méthoxy-2,3-dihydro-1H-indol-1-yl)-2-oxoéthyl]-3-méthyl-6-(morpholin-4-yl)pyrimidin-4(3H)-one

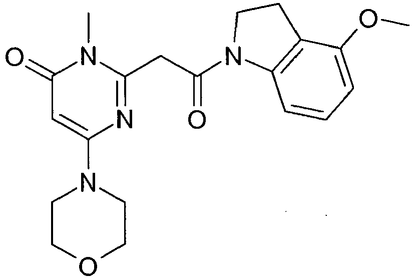

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 298 mg de 4-méthoxy-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 107 mg de 2-[2-(4-méthoxy-2,3-dihydro-1H-indol-1-yl)-2-oxoéthyl]-3-méthyl-6-(morpholin-4-yl)pyrimidin-4(3H)-one sous forme d'une poudre rose saumon dont les caractéristiques sont les suivantes :
Spectre RMN 1 H (400 MHz): 3,04 (t, J=8,5 Hz, 2 H) ; 3,31 (s masqué, 3 H) ; 3,40 (m, 4 H) ; 3,59 (m, 4 H) ; 3,80 (s, 3 H) ; 4,09 (s, 2 H) ; 4,18 (t, J=8,5 Hz, 2 H) ; 5,36 (s, 1 H) ; 6,71 (d, J=8,2 Hz, 1 H) ; 7,15 (t, J=8,2 Hz, 1 H) ; 7,64 (d, J=8,2 Hz, 1 H)
Spectrométrie de Masse : méthode A
Temps de rétention Tr (min) = 0,69 ;
[M+H]+ : m/z 385
Point de fusion (Kofler) : 229°C

### Exemple 111 : Synthèse de la 2-[2-(3,4-dihydroisoquinolin-2(1H)-yl)-2-oxoéthyl]-6-(morpholin-4-yl)pyrimidin-4(3H)-one

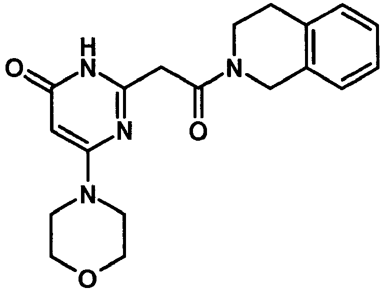

Le produit est préparé en suivant le mode opératoire décrit à l'exemple 70 à partir de 304 µL de 1,2,3,4-tetrahydroisoquinoléine, de 300 mg de [4-(morpholin-4-yl)-6-oxo-1,6-dihydropyrimidin-2-yl]acétate d'éthyle préparé à l'étape 1 de l'exemple 1 et de 1,3 mL d'une solution de triméthylaluminium 2M, dans un mélange de 21 mL de toluène et de 10 mL de tetrahydrofuranne. On obtient ainsi 130 mg de 2-[2-(3,4-dihydroisoquinolin-2(1 H)-yl)-2-oxoéthyl]-6-(morpholin-4-yl)pyrimidin-4(3H)-one sous forme d'une meringue blanchâtre dont les caractéristiques sont les suivantes :
Spectre RMN 1 H (400 MHz)pour ce lot, tous les signaux sont larges avec : 2,86 (m, 2 H) ; 3,33 (m, 4 H) ; 3,56 (m, 4 H) ; 3,72 (m, 4 H) ; 4,67 (s, 2 H) ; 5,13 (s, 1 H) ; 7,18 (s, 4 H) ; 11,23 (m étalé, 1 H)
Spectrométrie de Masse : méthode A
Temps de rétention Tr (min) = 0,62 ;
[M+H]+ : m/z 355 ; [M-H]- : m/z 353
Point de fusion (Kofler) : 107,5°C

### Exemple 112 : Synthèse du 2-[4-(morpholin-4-yl)-6-oxo-1,6-dihydropyrimidin-2-yl]-N-{2-[2-(pyrrolidin-1-yl)éthoxy]phényl}acétamide.

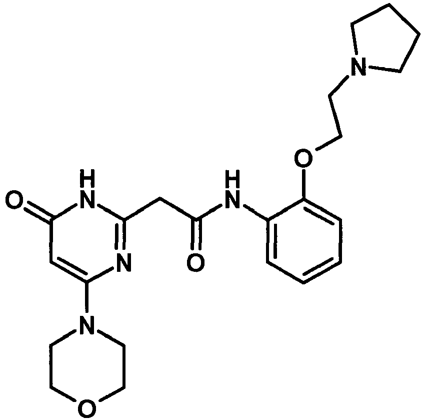

A une solution de 250 mg de [4-(morpholin-4-yl)-6-oxo-1,6-dihydropyrimidin-2-yl]acétate de sodium préparé à l'étape 2 de l'exemple 1 dans 2 mL de N,N-diméthylformamide sont ajoutés 2 mL de pyridine, 300 mg de N-[3-(diméthylamino)propyl]-N'-éthylcarbodiimide chlorhydrate et 273 mg de 2-(2-pyrrolidin-1-yl-éthoxy)-phénylamine. Le mélange réactionnel est agité à température ambiante pendant 2 jours, puis concentré sous pression réduite.

Après purification sur colonne de silice, en éluant par un mélange de dichlorométhane et de méthanol (95/05 en volumes), on obtient 45 mg de 2-[4-(morpholin-4-yl)-6-oxo-1,6-dihydropyrimidin-2-yl]-N-{2-[2-(pyrrolidin-1-yl)éthoxy]phényl}acétamide sous forme de solide blanc dont les caractéristiques sont les suivantes:
Spectre RMN 1H (400MHz) : 1,71 (m, 4 H) ; 2,60 (m large, 4 H) ; 2,84 (m large, 2H) ; 3,43 (m, 4 H) ; 3,61 (m, 4 H) ; 3,67 (s, 2 H) ; 4,16 (t, J=5,9 Hz, 2 H) ; 5,22 (s, 1 H) ; 6,93 (m, 1 H) ; 7,03 à 7,13 (m, 2 H) ; 7,94 (d, J=8,1 Hz, 1 H) ; 9,46 (s large, 1 H) ; 13,33 (m très étalé, 1 H)
Spectrométrie de Masse : méthode A
Temps de rétention Tr (min) = 0,39 ;
[M+H]+ : m/z 426 ; [M-H]- : m/z 428

### Exemple 113 : Synthèse du 2-[4-(morpholin-4-yl)-6-oxo-1,6-dihydropyrimidin-2-yl]-N-[2-(pyridin-3-ylméthoxy)phényl]acétamide

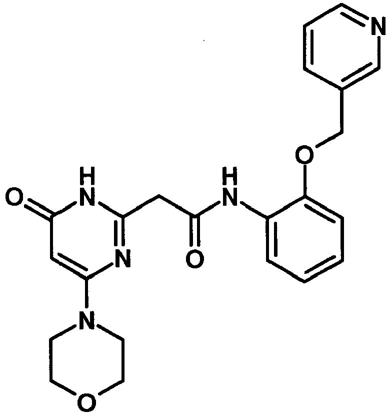

A une solution de 250 mg de [4-(morpholin-4-yl)-6-oxo-1,6-dihydropyrimidin-2-yl]acétate de sodium préparé à l'étape 2 de l'exemple 1 dans 2 mL de N,N-diméthylformamide sont ajoutés 2 mL de pyridine, 300 mg de N-[3-(diméthylamino)propyl]-N'-éthylcarbodiimide chlorhydrate et 366 mg de 2-(pyridin-3-ylméthoxy)-phénylamine. Le mélange réactionnel est agité à température ambiante pendant 20 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 150 mg du 2-[4-(morpholin-4-yl)-6-oxo-1,6-dihydropyrimidin-2-yl]-N-[2-(pyridin-3-ylméthoxy)phényl]acétamide sous forme de solide marron dont les caractéristiques sont les suivantes:
Spectre RMN 1 H (400MHz) : tous les signaux sont larges avec : 3,37 (m, 4 H) ; 3,55 (m, 4 H) ; 3,70 (s, 2 H) ; 5,19 (s, 1 H) ; 5,26 (s, 2 H) ; 6,93 (t, J=7,6 Hz, 1 H) ; 7,07 (t, J=7,6 Hz, 1 H) ; 7,14 (d, J=7,6 Hz, 1 H) ; 7,40 (m, 1 H) ; 7,88 (m, 2 H) ; 8,53 (d, J=5,2 Hz, 1 H) ; 8,70 (s, 1 H) ; 9,47 (s, 1 H) ; 11,66 (m étalé, 1 H)
Spectrométrie de Masse : méthode A
Temps de rétention Tr (min) = 0,44 ;
[M+H]+ : m/z 420 ; [M-H]- : m/z 422

### Exemple 114 : Synthèse de la 3-méthyl-2-[2-(4-méthyl-2,3-dihydro-1H-indol-1-yl)-2-oxoéthyl]-6-(morpholin-4-yl)pyrimidin-4(3H)-one

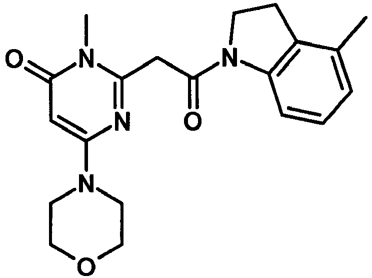

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 339 mg de chlorhydrate de 4-méthyl-indoline, de 254 mg de N-[3-(diméthylamino)propyl]-N'-éthylcarbodiimide chlorhydrate dans un mélange de 240 µL de pyridine et de 4,0 mL de N,N-diméthylformamide. On obtient 93 mg de 3-méthyl-2-[2-(4-méthyl-2,3-dihydro-1H-indol-1-yl)-2-oxoéthyl]-6-(morpholin-4-yl)pyrimidin-4(3H)-one sous forme d'une poudre rose dont les caractéristiques sont les suivantes :
Spectre RMN 1 H (400 MHz): 2,21 (s, 3 H) ; 3,08 (t, J=8,3 Hz, 2 H) ; 3,29 (s, 3 H); 3,41 (m, 4 H) ; 3,58 (m, 4 H) ; 4,10 (s, 2 H) ; 4,19 (t, J=8,3 Hz, 2 H) ; 5,36 (s, 1 H) ; 6,86 (d, J=8,1 Hz, 1 H) 7,07 (t, J=8,1 Hz, 1 H) ; 7,84 (d, J=8,1 Hz, 1 H)
Spectrométrie de Masse : méthode A
Temps de rétention Tr (min) = 0,75 ;
[M+H]+ : m/z 369 ; [M-H]- : m/z 367
Point de fusion (Kofler) : 239°C

### Exemple 115 : Synthèse de la 2-[2-(3-diméthylaminométhyl-2,3-dihydro-indol-1-yl)-2-oxo-éthyl]-6-morpholin-4-yl-3H-pyrimidin-4-one

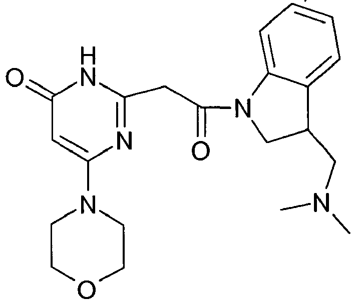

### Etape 1

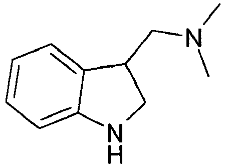

A une solution de 1 g de gramine dans 20 mL d'acide trifluoroacétique sous argon refroidie dans un bain de glace, est ajouté progressivement 1,1 g de cyanoborohydrure de sodium, que l'on agite pendant une demi-heure à 0°C puis 2 h à température ambiante. Le milieu réactionnel est jeté dans 50 g de glace et alcalinisé par de la soude à 30%, puis il est extrait par quatre fois 30 mL d'acétate d'éthyle. La phase organique est lavée avec 20 mL d'eau, séchée sur sulfate de magnésium anhydre, filtrée puis concentrée à sec sous pression réduite. Après une purification sur colonne de silice éluant: Cl2CH2/MeOH, 85/15 v/v, on obtient 660 mg de (2,3-dihydro-1H-indol-3-ylméthyl)-diméthyl-amine sous forme d'un solide jaune dont les caractéristiques sont les suivantes :
Spectrométrie de Masse : méthode A
Temps de rétention Tr (min) = 0,16 ; mélange avec attendu
[M+H]+ : m/z 177

### Etape 2

Le produit est préparé en suivant le mode opératoire décrit à l'exemple 5 à partir de 220 mg de [4-(morpholin-4-yl)-6-oxo-1,6-dihydropyrimidin-2-yl]acétate de sodium, de 300 mg de (2,3-dihydro-1H-indol-3-ylméthyl)-diméthyl-amine, de 210 mg de N-[3-(diméthylamino)propyl]-N'-éthylcarbodiimide chlorhydrate dans un mélange de 0,14 mL de pyridine et de 2,5 mL de N,N-diméthylformamide. On obtient 206 mg de 2-[2-(3-diméthylaminométhyl-2,3-dihydro-indol-1-yl)-2-oxo-éthyl]-6-morpholin-4-yl-3H-pyrimidin-4-one sous forme d'un solide rose dont les caractéristiques sont les suivantes :
Spectre RMN 1 H (400 MHz): 2,22 (s, 6 H) ; 2,35 (m, 1 H) ; 2,45 (m, 1 H) ; 3,28 (m masqué, 1 H) ; 3,41 (m, 4 H) ; 3,60 (m, 4 H) ; 3,76 (m, 2 H) ; 3,91 (dd, J=5,7 et 9,9 Hz, 1 H) ; 4,23 (t, J=9,9 Hz, 1 H) ; 5,21 (s, 1 H) ; 7,02 (t, J=8,1 Hz, 1 H) ; 7,19 (t, J=8,1 Hz, 1 H) ; 7,29 (d, J=8,1 Hz, 1 H) ; 8,02 (d, J=8,1 Hz, 1 H) ; 11,61 (m étalé, 1 H)
Spectrométrie de Masse : méthode A
Temps de rétention Tr (min) = 0,37 ;
[M+H]+ : m/z 398 ; [M+2H]2+ : m/z 199,5 (pic de base)
[M-H]- : m/z 396 ;
Point de fusion (Kofler) : 243°C

### Exemple 116 : Synthèse de la 2-[2-(4-bromo-2,3-dihydro-1H-indol-1-yl)-2-oxoéthyl]-3-méthyl-6-(morpholin-4-yl)pyrimidin-4(3H)-one

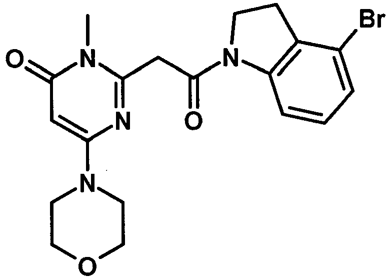

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 352 mg de chlorhydrate de 4-bromo-indoline, de 254 mg de N-[3-(diméthylamino)propyl]-N'-éthylcarbodiimide chlorhydrate dans un mélange de 201 µL de pyridine et de 4,0 mL de N,N-diméthylformamide. On obtient 161 mg de 2-[2-(4-bromo-2,3-dihydro-1H-indol-1-yl)-2-oxoéthyl]-3-méthyl-6-(morpholin-4-yl)pyrimidin-4(3H)-one sous forme d'une poudre rose dont les caractéristiques sont les suivantes:
Spectre RMN 1 H (400 MHz): 3,14 (t, J=8,3 Hz, 2 H) ; 3,30 (s masqué, 3 H) ; 3,39 (m, 4 H) ; 3,58 (m, 4 H) ; 4,11 (s, 2 H) ; 4,22 (t, J=8,3 Hz, 2 H) ; 5,36 (s, 1 H) ; 7,14 (t, J=7,8 Hz, 1 H) ; 7,24 (d, J=7,8 Hz, 1 H) ; 8,01 (d, J=7,8 Hz, 1 H)
Spectrométrie de Masse : méthode A
Temps de rétention Tr (min) = 0,83 ;
[M+H]+ : m/z 431 ; [M-H]- : m/z 433
Point de fusion (Kofler) : 226°C

### Exemple 117 et Exemple 118 : Séparation de la 2-{2-[(2S)-2-méthyl-2,3-dihydro-1H-indol-1-yl]-2-oxoéthyl}-6-(morpholin-4-yl)pyrimidin-4(3H)-one et de la 2-{2-[(2R)-2-méthyl-2,3-dihydro-1H-indol-1-yl]-2-oxoéthyl}-6-(morpholin-4-yl)pyrimidin-4(3H)-one

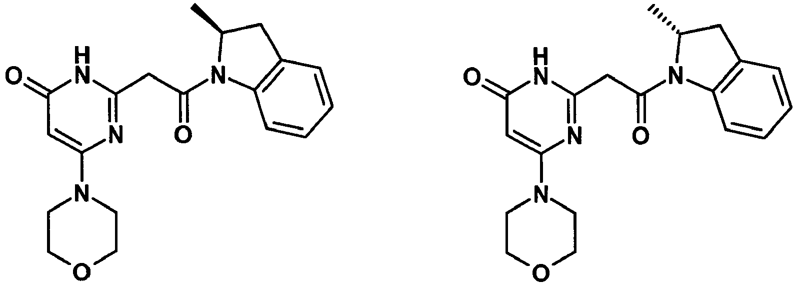

Les produits ont été obtenus par séparation chromatographique chirale de 311 mg de 2-{2-[(2-méthyl-2,3-dihydro-1H-indol-1-yl]-2-oxoéthyl}-6-(morpholin-4-yl)pyrimidin-4(3H)-one (exemple 96) sur colonne chirale Chiralpak T304 20 µm (1080 g, 20 µm, 8/35 cm), éluant : Acétonitrile/Isopropanol : 90/10; débit :185 mL/min. Après purification, on obtient comme premier énantiomère, 160 mg de (+)-2-{2-[(2-méthyl-2,3-dihydro-1H-indol-1-yl]-2-oxoéthyl}-6-(morpholin-4-yl)pyrimidin-4(3H)-one, sous forme d'un solide amorphe rose dont les caractéristiques sont les suivantes:
Spectre RMN 1 H (400MHz): pour ce lot, les signaux sont larges avec : 1,26 (d, J=6,8 Hz, 3 H) ; 2,44 (m partiellement masqué, 1 H) ; 2,69 (d, J=15,2 Hz, 1 H) ; 3,42 (m, 4 H) ; 3,60 (m, 4H) ; 3,72 (d, J=15,7 Hz, 1 H) ; 3,92 (d, J=15,7 Hz, 1 H) ; 4,72 (m, 1 H) ; 5,20 (s, 1 H) ; 7,04 (t, J=7,8 Hz, 1 H) ; 7,18 (t, J=7,8 Hz, 1 H) ; 7,28 (d, J=7,8 Hz, 1 H) ; 7,96 (d, J=7,8 Hz, 1 H) ; 11,67 (m étalé, 1 H)
Spectrométrie de Masse : méthode A
Temps de rétention Tr (min) = 0,70 ;
[M+H]+ : m/z 355 ; [M-H]- : m/z 353;
Pouvoir rotatoire : α_{D} = +65,0° +/-1,3 (c=1,736 mg dans 0,5 mL de méthanol)

Puis le deuxième énantiomère, soit: 143 mg de (-)-2-{2-[(2-méthyl-2,3-dihydro-1H-indol-1-yl]-2-oxoéthyl}-6-(morpholin-4-yl)pyrimidin-4(3H)-one sous forme d'un solide amorphe blanc dont les caractéristiques sont les suivantes :
Spectre RMN 1 H (400MHz): pour ce lot, les signaux sont larges avec : 1,26 (d, J=6,8 Hz, 3 H) ; 2,45 (m partiellement masqué, 1 H) ; 2,69 (m, 1 H) ; 3,41 (m, 4 H) ; 3,61 (m, 4 H) ; 3,72 (d, J=15,7 Hz, 1 H) ; 3,92 (d, J=15,7 Hz, 1 H) ; 4,70 (m, 1 H) ; 5,20 (s, 1 H) ; 7,04 (t, J=7,8 Hz, 1 H) ; 7,18 (t, J=7,8 Hz, 1 H) ; 7,28 (d, J=7,8 Hz, 1 H) ; 7,96 (d, J=7,8 Hz, 1 H) ; 11,64 (m étalé, 1 H)
Spectrométrie de Masse : méthode A
Temps de rétention Tr (min) = 0,70 ;
[M+H]+ : m/z 355 ; [M-H]- : m/z 353;
Pouvoir rotatoire : α_{D} = -72,8° +/-1,2 (c=2,338 mg dans 0,5 mL de méthanol)

### Exemple 119 : Synthèse de la 3-méthyl-2-[2-(3-méthyl-2,3-dihydro-1H-indol-1-yl)-2-oxoéthyl]-6-(morpholin-4-yl)pyrimidin-4(3H)-one

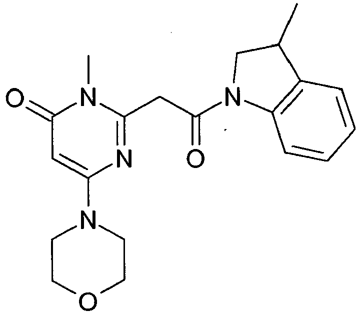

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 254 mg de chlorhydrate de 3-méthyl-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 96 mg de 3-méthyl-2-[2-(3-méthyl-2,3-dihydro-1H-indol-1-yl)-2-oxoéthyl]-6-(morpholin-4-yl)pyrimidin-4(3H)-one sous forme d'une poudre rose dont les caractéristiques sont les suivantes :
Spectre RMN 1 H (400 MHz): 1,30 (d, J=6,8 Hz, 3 H) ; 3,32 (s, 3 H) ; 3,39 (m, 4 H) ; 3,49 (m, 1 H) ; 3,57 (m, 4 H) ; 3,71 (dd, J=6,8 et 10,1 Hz, 1 H) ; 4,10 (s, 2 H) ; 4,36 (t, J=10,1 Hz, 1 H); 5,36 (s, 1 H) ; 7,05 (t, J=8,0 Hz, 1 H) ; 7,17 (t, J=8,0 Hz, 1 H) ; 7,28 (d, J=8,0 Hz, 1 H) ; 8,01 (d, J=8,0 Hz, 1 H)
Spectrométrie de Masse : méthode A
Temps de rétention Tr (min) = 0,77 ;
[M+H]+ : m/z 369 ; [M-H]- : m/z 367
Point de fusion (Kofler) : 225°C

### Exemple 120 : Synthèse de la 2-{2-[2-(méthoxyméthyl)-2,3-dihydro-1H-indol-1-yl]-2-oxoéthyl}-6-(morpholin-4-yl)pyrimidin-4(3H)-one

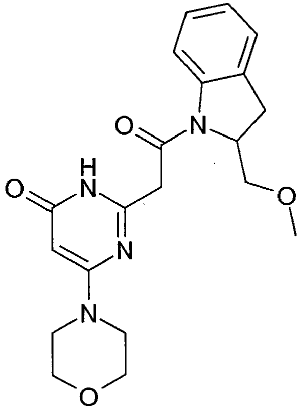

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 326 mg de 2-(méthoxyméthyl)indoline, de 249 mg de N-[3-(diméthylamino)propyl]-N'-éthylcarbodiimide chlorhydrate dans un mélange de 162 µL de pyridine et de 4,0 mL de N,N-diméthylformamide. On obtient 143 mg de 2-{2-[2-(méthoxyméthyl)-2,3-dihydro-1H-indol-1-yl]-2-oxoéthyl}-6-(morpholin-4-yl)pyrimidin-4(3H)-one sous forme d'une poudre beige dont les caractéristiques sont les suivantes :
Spectre RMN 1 H (400 MHz): 2,84 (d, J=16,4 Hz, 1 H) ; 3,26 (s, 3 H) ; 3,34 à 3,46 (m, 7 H) ; 3,60 (m, 4 H) ; 3,79 (d, J=15,6 Hz, 1 H) ; 4,00 (d, J=15,6 Hz, 1 H) ; 4,80 (m, 1 H) ; 5,20 (s, 1 H); 7,03 (t, J=7,8 Hz, 1 H) ; 7,17 (t, J=7,8 Hz, 1 H) ; 7,27 (d, J=7,8 Hz, 1 H) ; 7,93 (d, J=7,8 Hz, 1 H) ; 11,66 (s large, 1 H)
Spectrométrie de Masse : méthode B
Temps de rétention Tr (min) = 3,26 ;
[M+H]+ : m/z 385 ; [M-H]- : m/z 383
Point de fusion (Kofler) : 112-115°C

### Exemple 121 : Synthèse de la 2-[2-(4-éthoxy-2,3-dihydro-1H-indol-1-yl)-2-oxoéthyl]-6-(morpholin-4-yl)pyrimidin-4(3H)-one

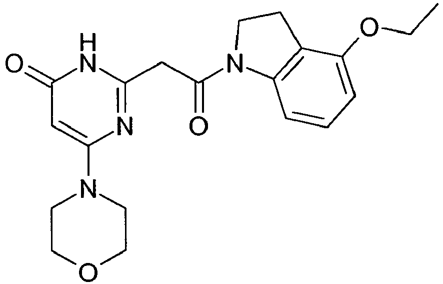

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 163 mg de 4-(éthoxy)-indoline, de 249 mg de N-[3-(diméthylamino)propyl]-N'-éthylcarbodiimide chlorhydrate dans un mélange de 162 µL de pyridine et de 4,0 mL de N,N-diméthylformamide. On obtient 260 mg de 2-[2-(4-éthoxy-2,3-dihydro-1H-indol-1-yl)-2-oxoéthyl]-6-(morpholin-4-yl)pyrimidin-4(3H)-one sous forme d'une poudre blanche dont les caractéristiques sont les suivantes :
Spectre RMN 1H (400 MHz): 1,33 (t, J=6,8 Hz, 3 H) ; 3,03 (t, J=8,6 Hz, 2 H) ; 3,42 (m, 4 H) ; 3,61 (m, 4 H) ; 3,73 (s, 2 H) ; 4,06 (q, J=6,8 Hz, 2 H) ; 4,14 (t, J=8,6 Hz, 2 H) ; 5,20 (s, 1 H); 6,68 (d, J=8,1 Hz, 1 H) ; 7,12 (t, J=8,1 Hz, 1 H) ; 7,63 (d, J=8,1 Hz, 1 H) ; 11,61 (s large, 1 H)
Spectrométrie de Masse : méthode B
Temps de rétention Tr (min) = 3,48 ;
[M+H]+ : m/z 385 ; [M-H]- : m/z 383
Point de fusion (Kofler) : 257°C

### Exemple 122 _{:} Synthèse de la 1-([4-(morpholin-4-yl)-6-oxo-1,6-dihydropyrimidin-2-yl]acétyl}-2,3-dihydro-1H-indole-2-carboxamide

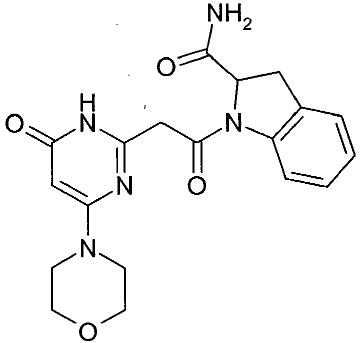

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 324 mg de 2-carboxamido-indoline, de 249 mg de N-[3-(diméthylamino)propyl]-N'-éthylcarbodiimide chlorhydrate dans un mélange de 162 µL de pyridine et de 4,0 mL de N,N-diméthylformamide. On obtient 93 mg de 1-{[4-(morpholin-4-yl)-6-oxo-1,6-dihydropyrimidin-2-yl]acétyl}-2,3-dihydro-1 H-indole-2-carboxamide sous forme d'une poudre blanche dont les caractéristiques sont les suivantes :
Spectre RMN 1 H (400 MHz): 3,11 (d, J=16,9 Hz, 1 H) ; 3,36 à 3,47 (m, 5 H) ; 3,54 à 3,63 (m, 5 H) ; 3,77 (d, J=15,7 Hz, 1 H) ; 5,07 (dd, J=3,8 et 9,1 Hz, 1 H) ; 5,20 (s, 1 H) ; 7,02 (t, J=7,8 Hz, 1 H) ; 7,18 (t, J=7,8 Hz, 1 H) ; 7,23 (d, J=7,8 Hz, 1 H) ; 7,36 (s large, 1 H) ; 7,76 (s large, 1 H) ; 8,01 (d, J=7,8 Hz, 1 H) ; 11,59 (m étalé, 1 H)
Spectrométrie de Masse : méthode A
Temps de rétention Tr (min) = 0,50 ;
[M+H]+ : m/z 384 ; [M-H]- : m/z 382
Point de fusion (Kofler) : supérieur à 260°C

### Exemple 123 : Synthèse de la 3-méthyl-2-[2-(2-méthyl-2,3-dihydro-1H-indol-1-yl)-2-oxoéthyl]-6-(morpholin-4-yl)pyrimidin-4(3H)-one

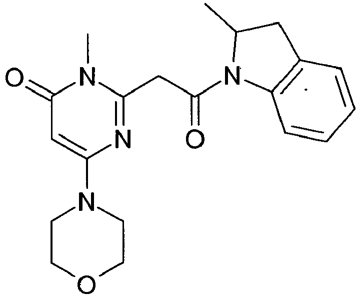

Le produit est préparé en suivant le mode opératoire décrit à l'exemple 68 à partir de 550 mg de [1-méthyl-4-(morpholin-4-yl)-6-oxo-1,6-dihydropyrimidin-2-yl]acétate de sodium, de 400 mg de 2-méthyl-indoline, de 500 mg de N-[3-(diméthylamino)propyl]-N'-éthylcarbodiimide chlorhydrate dans un mélange de 320 µL de pyridine et de 8,0 mL de N,N-diméthylformamide. On obtient 37 mg de 3-méthyl-2-[2-(2-méthyl-2,3-dihydro-1H-indol-1-yl)-2-oxoéthyl]-6-(morpholin-4-yl)pyrimidin-4(3H)-one sous forme d'une poudre rose dont les caractéristiques sont les suivantes :
Spectre RMN 1 H (400 MHz, 1,28 (d, J=6,8 Hz, 3 H) ; 2,70 (d, J=15,9 Hz, 1 H) ; 3,23 à 3,30 (s masqué, 3 H) ; 3,40 (m, 5 H) ; 3,60 (m, 4 H) ; 4,02 (d, J=16,9 Hz, 1 H) ; 4,29 (d, J=16,9 Hz, 1 H) ; 4,70 (m, 1 H) ; 5,36 (s, 1 H) ; 7,05 (t, J=7,8 Hz, 1 H) ; 7,18 (t, J=7,8 Hz, 1 H) ; 7,29 (d, J=7,8 Hz, 1 H); 7,95 (d, J=7,8 Hz, 1 H)
Spectrométrie de Masse : méthode A
Temps de rétention Tr (min) = 0,75 ;
[M+H]+ : m/z 369 ; [M-H]- : m/z 367
Point de fusion (Kofler) : 148°C

### Exemple 124 : Synthèse de la 2-[2-(6-fluoro-2,3-dihydro-1H-indol-1-yl)-2-oxoethyl]-3-methyl-6-(morpholin-4-yl)pyrimidin-4(3H)-one

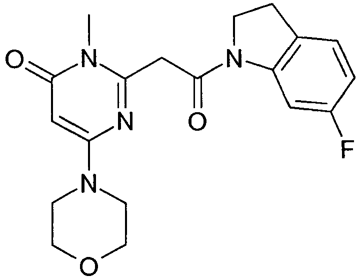

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 205 mg de 6-fluoro-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 140 mg de 2-[2-(6-fluoro-2,3-dihydro-1H-indol-1-yl)-2-oxoethyl]-3-methyl-6-(morpholin-4-yl)pyrimidin-4(3H)-one sous forme d'une poudre rose très pâle dont les caractéristiques sont les suivantes :
Spectre RMN 1 H (400 MHz): 3,14 (t, J=8,2 Hz, 2 H) ; 3,25 à 3,45 (m partiellement masqué, 7 H) ; 3,58 (m, 4 H) ; 4,11 (s, 2 H) ; 4,22 (t, J=8,2 Hz, 2 H) ; 5,36 (s, 1 H) ; 6,84 (t, J=8,7 Hz, 1 H); 7,26 (m, 1 H) ; 7,77 (d large, J=10,3 Hz, 1 H)
Spectrométrie de Masse : méthode A
Temps de rétention Tr (min) = 0,73 ;
[M+H]+ : m/z 373 ; [M-H]- : m/z 371
Point de fusion (Kofler) : 223°C

### Exemple 125 et Exemple 126 : Séparation de la 2-{2-[(3S)-3-méthyl-2,3-dihydro-1H-indol-1-yl]-2-oxoéthyl}-6-(morpholin-4-yl)pyrimidin-4(3H)-one et de la 2-{2-[(3R)-3-méthyl-2,3-dihydro-1H-indol-1-yl]-2-oxoéthyl}-6-(morpholin-4-yl)pyrimidin-4(3H)-one

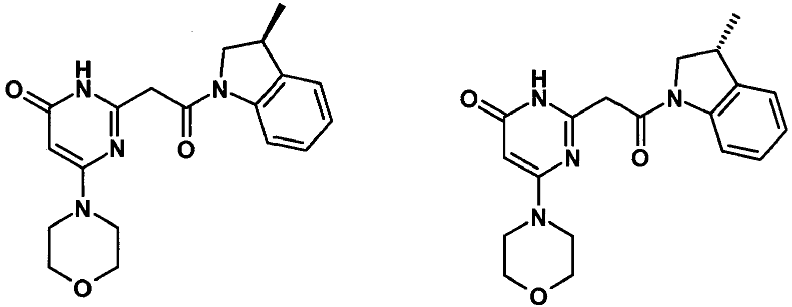

Les produits ont été obtenus par séparation chromatographique chirale de 369 mg de 2-{2-[3-méthyt-2,3-dihydro-1H-indo)-1-yl]-2-oxoéthyl}-6-(morpholin-4-yl)pyrimidin-4(3H)-one (exemple 101) sur colonne chirale Chiralpak T304 20 µm (1100 g, 20 µm, 8/35 cm), éluant : heptane/éthanol/méthanol : 25/40/35 ; débit :200 mL/min. Après purification, on obtient comme premier énantiomère, 168 mg de (+)-2-{2-[3-méthyl-2,3-dihydro-1H-indol-1-yl]-2-oxoéthyl}-6-(morpholin-4-yl)pyrimidin-4(3H)-one, sous forme d'une poudre blanche dont les caractéristiques sont les suivantes:
Spectre RMN 1 H (400MHz) : 1,29 (d, J=6,8 Hz, 3 H) ; 3,42 (m, 4 H) ; 3,49 (m, 1 H) ; 3,60 (m, 4 H) ; 3,68 (dd, J=6,6 et 10,1 Hz, 1 H) ; 3,75 (s, 2 H) ; 4,33 (t, J=10,1 Hz, 1 H) ; 5,21 (s, 1 H); 7,04 (t, J=8,0 Hz, 1 H) ; 7,18 (t, J=8,0 Hz, 1 H) ; 7,27 (d, J=8,0 Hz, 1 H) ; 8,01 (d, J=8,0 Hz, 1 H) ; 11,61 (m étalé, 1 H)
Spectrométrie de Masse : méthode A
Temps de rétention Tr (min) = 0,72 ;
[M+H]+ : m/z 355 ; [M-H]- : m/z 353;
Pouvoir rotatoire : α_{D} = +17,1 +/-0,8 (c=1,656 mg dans 0,5 mL de DMSO)

Puis le deuxième énantiomère, soit: 164 mg de (-)-2-{2-[3-méthyl-2,3-dihydro-1H-indol-1-yl]-2-oxoéthyl}-6-(morpholin-4-yl)pyrimidin-4(3H)-one sous forme d'une poudre blanche dont les caractéristiques sont les suivantes :
Spectre RMN 1 H (400MHz) : 1,29 (d, J=6,8 Hz, 3 H) ; 3,41 (m, 4 H) ; 3,49 (m, 1 H) ; 3,60 (m, 4 H) ; 3,68 (dd, J=6,6 et 10,3 Hz, 1 H) ; 3,75 (s, 2 H) ; 4,33 (t, J=10,3 Hz, 1 H) ; 5,21 (s, 1 H); 7,05 (t, J=8,0 Hz, 1 H) ; 7,18 (t, J=8,0 Hz, 1 H) ; 7,27 (d, J=8,0 Hz, 1 H) ; 8,01 (d, J=8,0 Hz, 1 H) ; 11,60 (m étalé, 1 H)
Spectrométrie de Masse : méthode A
Temps de rétention Tr (min) = 0,72 ;
[M+H]+ : m/z 355 ; [M-H]- : m/z 353;
Pouvoir rotatoire: α_{D} = -13,0° (c=1,386 mg dans 0,5 mL de DMSO)

### Exemple 127 : Synthèse de la 2-[2-(5,6-difluoro-2,3-dihydro-1H-indol-1-yl)-2-oxoéthyl]-3-méthyl-6-(morpholin-4-yl)pyrimidin-4(3H)-one

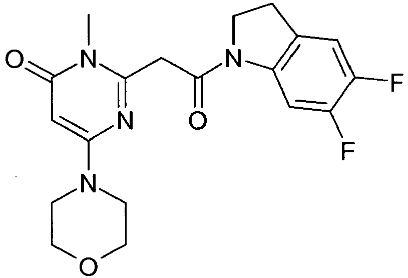

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 232 mg de 5,6-difluoro-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 167 mg de 2-[2-(5,6-difluoro-2,3-dihydro-1H-indol-1-yl)-2-oxoéthyl]-3-méthyl-6-(morpholin-4-yl)pyrimidin-4(3H)-one sous forme d'une poudre blanc cassé dont les caractéristiques sont les suivantes :
Spectre RMN 1 H (400 MHz): 3,16 (t, J=8,6 Hz, 2 H) ; 3,31 (s, 3 H) ; 3,39 (m, 4 H); 3,58 (m, 4 H) ; 4,11 (s, 2 H) ; 4,22 (t, J=8,6 Hz, 2 H) ; 5,36 (s, 1 H) ; 7,37 (dd, J=8,9 et 10,3 Hz, 1 H) ; 7,95 (dd, J=7,5 et 12,1 Hz, 1 H)
Spectrométrie de Masse : méthode A
Temps de rétention Tr (min) = 0,76 ;
[M+H]+ : m/z 391 ; [M-H]- : m/z 389
Point de fusion (Kofler) : 250°C

### Exemple 128 : Synthèse de la 2-[2-(4,5-difluoro-2,3-dihydro-1H-indol-1-yl)-2-oxoéthyl]-3-méthyl-6-(morpholin-4-yl)pyrimidin-4(3H)-one

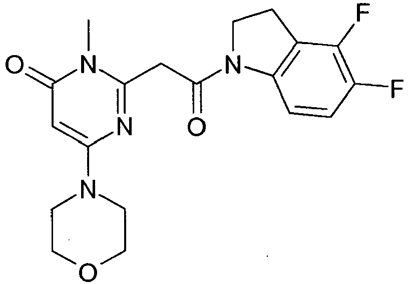

Le produit est préparé en suivant le mode opératoire décrit à l'exemple 68 à partir de 275 mg de [1-méthyl-4-(morpholin-4-yl)-6-oxo-1,6-dihydropyrimidin-2-yl]acétate de sodium, de 287 mg de chlorhydrate de 4,5-difluoro-indoline, de 254 mg de N-[3-(diméthylamino)propyl]-N'-éthylcarbodiimide chlorhydrate dans un mélange de 241 µL de pyridine et de 4,0 mL de N,N-diméthylformamide. On obtient 155 mg de 2-[2-(4,5-difluoro-2,3-dihydro-1H-indol-1-yl)-2-oxoéthyl]-3-méthyl-6-(morpholin-4-yl)pyrimidin-4(3H)-one sous forme d'une poudre de couleur brique dont les caractéristiques sont les suivantes :
Spectre RMN 1 H (400 MHz): 3,25 (t, J=8,4 Hz, 2 H) ; 3,31 (s, 3 H) ; 3,39 (m, 4 H); 3,58 (m, 4 H) ; 4,11 (s, 2 H) ; 4,27 (t, J=8,4 Hz, 2 H) ; 5,36 (s, 1 H) ; 7,23 (m, 1 H) ; 7,79 (dd, J=4,3 et 8,9 Hz, 1 H)
Spectrométrie de Masse : méthode A
Temps de rétention Tr (min) = 0,77 ;
[M+H]+ : m/z 391 ; [M-H]- : m/z 389
Point de fusion (Kofler) : supérieur à 260°C

### Exemple 129 : Synthèse de la 2-[2-(1,3-dihydro-2H-isoindol-2-yl)-2-oxoéthyl]-6-(morpholin-4-yl)pyrimidin-4(3H)-one

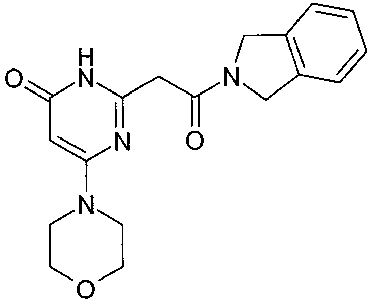

Le produit est préparé en suivant le mode opératoire décrit à l'exemple 70 à partir de 250 mg d'isoindoline, de 267 mg de [4-(morpholin-4-yl)-6-oxo-1,6-dihydropyrimidin-2-yl]acétate d'éthyle préparé à l'étape 1 de l'exemple 1 et de 1,15 mL d'une solution de triméthylaluminium 2M, dans un mélange de 20 mL de toluène et de 10 mL de tetrahydrofuranne. On obtient ainsi 80 mg de 2-[2-(1,3-dihydro-2H-isoindol-2-yl)-2-oxoéthyl]-6-(morpholin-4-yl)pyrimidin-4(3H)-one sous forme d'une poudre blanche dont les caractéristiques sont les suivantes :
Spectre RMN 1 H (400 MHz): 3,39 (m, 4 H) ; 3,59 (m, 4 H) ; 3,67 (s, 2 H) ; 4,67 (s, 2 H) ; 4,90 (s, 2 H) ; 5,19 (s, 1 H) ; 7,26 à 7,42 (m, 4 H) ; 11,60 (m étalé, 1 H)
Spectrométrie de Masse : méthode A
Temps de rétention Tr (min) = 0,60 ;
[M+H]+ : m/z 341 ; [M-H]- : m/z 339
Point de fusion (Kofler) : supérieur à 260°C

### Exemple 130 : Synthèse du N-(1-benzothiophen-4-yl)-2-[1-méthyl-4-(morpholin-4-yl)-6-oxo-1,6-dihydropyrimidin-2-yl]acétamide

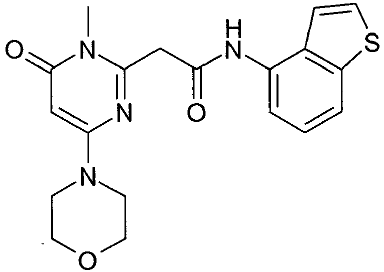

Le produit est préparé en suivant le mode opératoire décrit à l'exemple 68 à partir de 551 mg de [1-méthyl-4-(morpholin-4-yl)-6-oxo-1,6-dihydropyrimidin-2-yllacétate de sodium, de 328 mg de benzo[b]thiophen-4-ylamine, de 498 mg de N-[3-(diméthylamino)propyl]-N'-éthylcarbodiimide chlorhydrate dans un mélange de 324 µL de pyridine et de 8,0 mL de N,N-diméthylformamide. On obtient 110 mg de N-(1-benzothiophen-4-yl)-2-[1-méthyl-4-(morpholin-4-yl)-6-oxo-1,6-dihydropyrimidin-2-yl]acétamide sous forme d'une poudre blanche dont les caractéristiques sont les suivantes :
Spectre RMN 1 H (400 MHz): 3,38 (s, 3 H) ; 3,41 (m, 4 H) ; 3,57 (m, 4 H) ; 4,05 (s, 2 H) ; 5,36 (s, 1 H) ; 7,34 (t, J=7,9 Hz, 1 H) ; 7,63 (d, J=5,6 Hz, 1 H) ; 7,72 à 7,81 (m, 3 H) ; 10,14 (m étalé, 1 H)
Spectrométrie de Masse : méthode A
Temps de rétention Tr (min) = 0,71 ;
[M+H]+ : m/z 385 ; [M-H]- : m/z 383
Point de fusion (Kofler) : 254°C

### Exemple 131 : Synthèse de la 2-[2-(5-chloro-3,4-dihydroquinolin-1(2H)-yl)-2-oxoéthyl]-6-(morpholin-4-yl)pyrimidin-4(3H)-one

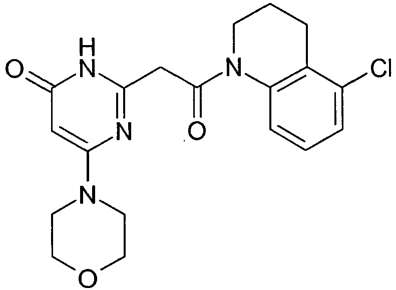

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 184 mg de 5-chloro-1,2,3,4-tetrahydro-quinoléine (peut être préparée selon WO2004/0116388), de 249 mg de N-[3-(diméthylamino)propyl]-N'-éthylcarbodiimide chlorhydrate dans un mélange de 162 µL de pyridine et de 4,0 mL de N,N-diméthylformamide. On obtient 141 mg de 2-[2-(5-chloro-3,4-dihydroquinolin-1 (2H)-yl)-2-oxoéthyl]-6-(morpholin-4-yl)pyrimidin-4(3H)-one sous forme d'une poudre blanche dont les caractéristiques sont les suivantes :
Spectre RMN 1 H (400 MHz): 1,96 (m, 2 H) ; 2,77 (t, J=6,8 Hz, 2 H) ; 3,41 (m, 4H) ; 3,61 (m, 4 H) ; 3,70 (t, J=6,8 Hz, 2 H) ; 3,79 (s, 2 H) ; 5,16 (s, 1 H) ; 7,21 (t, J=8,0 Hz, 1 H) ; 7,27 (d, J=8,0 Hz, 1 H) ; 7,55 (d large, J=8,0 Hz, 1 H); 11,57 (m étalé, 1 H)
Spectrométrie de Masse : méthode A
Temps de rétention Tr (min) = 0,78 ;
[M+H]+ : m/z 389 ; [M-H]- : m/z 387
Point de fusion (Kofler) : 238°C

### Exemple 132 : Synthèse de la 2-{2-[4-(hydroxyméthyl)-2,3-dihydro-1H-indol-1-yl]-2-oxoéthyl}-6-(morpholin-4-yl)pyrimidin-4(3H)-one

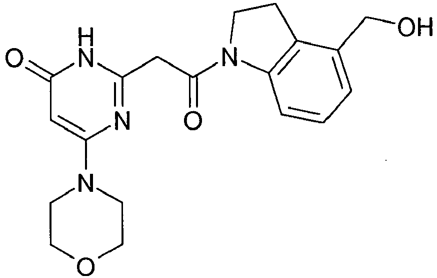

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 173 mg de 4-(hydroxyméthyl)-indoline, de 254 mg de N-[3-(diméthylamino)propyl]-N'-éthylcarbodiimide chlorhydrate dans un mélange de 161 µL de pyridine et de 4,0 mL de N,N-diméthylformamide. On obtient 288 mg de 2-{2-[4-(hydroxyméthyl)-2,3-dihydro-1H-indol-1-yl]-2-oxoéthyl}-6-(morpholin-4-yl)pyrimidin-4(3H)-one sous forme d'une poudre rose dont les caractéristiques sont les suivantes :
Spectre RMN 1 H (400 MHz): 3,12 (t, J=8,3 Hz, 2 H) ; 3,42 (m, 4 H) ; 3,59 (m, 4H) ; 3,75 (s, 2 H) ; 4,15 (t, J=8,3 Hz, 2 H) ; 4,46 (d, J=5,4 Hz, 2 H) ; 5,10 (t, J=5,4 Hz, 1 H) ; 5,21 (s, 1 H) ; 7,04 (d, J=7,8 Hz, 1 H) ; 7,15 (t, J=7,8 Hz, 1 H) ; 7,92 (d, J=7,8 Hz, 1 H) ; 11,62 (s large, 1 H)
Spectrométrie de Masse : méthode B
Temps de rétention Tr (min) = 2,48 ;
[M+H]+ : m/z 371 ; [M-H]- : m/z 369
Point de fusion (Kofler) : 234°C

### Exemple 133 : Synthèse du N-[4-fluoro-2-(pipéridin-4-ylméthoxy)phényl]-2-[4-(morpholin-4-yl)-6-oxo-1,6-dihydropyrimidin-2-yl]acétamide

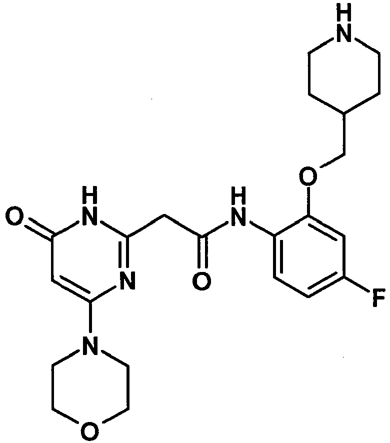

### Etape 1 :

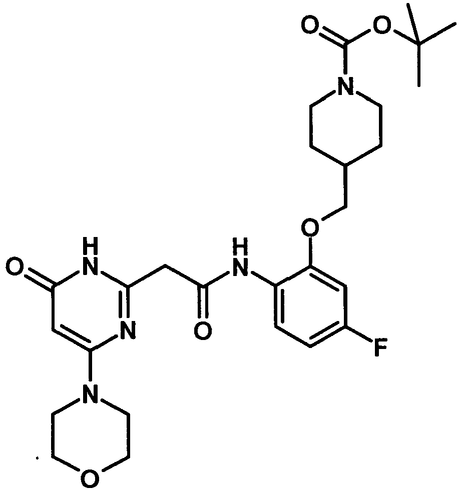

A une solution de 500 mg de [4-(morpholin-4-yl)-6-oxo-1,6-dihydropyrimidin-2-yl]acétate de sodium préparé à l'étape 2 de l'exemple 1 dans 3 mL de N,N-diméthylformamide sont ajoutés 3 mL de pyridine, 500 mg de N-[3-(diméthylamino)propyl]-N'-éthylcarbodiimide chlorhydrate et 930 mg de 4-(2-amino-5-fluoro-phénoxyméthyl)-pipéridine-1-carboxylic acid tert-butyl ester. Le mélange réactionnel est agité à température ambiante pendant 2 jours, puis concentré sous pression réduite. Après purification sur colonne de silice, en éluant par un mélange de dichlorométhane et de méthanol (95/05 en volumes), on obtient 225 mg de 4-{5-Fluoro-2-[2-(4-morpholin-4-yl-6-oxo-1,6-dihydro-pyrimidin-2-yl)-acétylamino]-phenoxymethyl}-piperidine-1-carboxylic acid tert-butyl ester sous forme d'un solide violacé dont les caractéristiques sont les suivantes:
Spectrométrie de Masse : méthode B
Temps de rétention Tr (min) = 4,02 ;
[M+H]+ : m/z 544 ; [M-H]- : m/z 546

### Etape 2:

A une solution de 223 mg de 4-{5-Fluoro-2-[2-(4-morpholin-4-yl-6-oxo-1,6-dihydro-pyrimidin-2-yl)-acétylamino]-phenoxymethyl}-piperidine-1-carboxylic acid tert-butyl ester dans 6 mL d'éthanol sont ajoutés 1.5 mL d'acide chlorhydrique 4M dans le dioxanne. Après 20h d'agitation à température ambiante, le milieu réactionnel est concentré sous pression réduite, repris avec une solution l'ammoniaque dans le méthanol puis de nouveau concentré à sec. Après purification par chromatographie sur colonne de silice, éluant: CH2Cl2/MeOH/, 90/10, on obtient 65 mg de N-[4-fluoro-2-(pipéridin-4-ylméthoxy)phényl]-2-[4-(morpholin-4-yl)-6-oxo-1,6-dihydropyrimidin-2-yl]acétamide dont les caractéristiques sont les suivantes :
Spectre RMN 1 H (400MHz) : 1,52 (m, 2 H) ; 1,89 (m, 2 H) ; 2,06 (m, 1 H) ; 2,86 (m, 2 H) ; 3,25 (m partiellement masqué, 2 H) ; 3,43 (m, 4 H) ; 3,62 (m, 4 H) ; 3,71 (s, 2 H) ; 3,92 (d, J=6,4 Hz, 2 H) ; 5,22 (s, 1 H) ; 6,74 (dt, J=2,7 et 8,8 Hz, 1 H) ; 7,00 (dd, J=2,7 et 11,0 Hz, 1 H) ; 7,75 (dd, J=6,6 et 8,8 Hz, 1 H); 7,87 (m très étalé, 2 H) ; 9,22 (s large, 1 H)
Spectrométrie de Masse : méthode A
Temps de rétention Tr (min) = 0,48 ;
[M+H]+ : m/z 444 ; [M-H]- : m/z 446

### Exemple 134: 2-[2-(5-Chloro-2,3-dihydro-indol-1-yl)-2-oxo-éthyl]-6-morpholin-4-yl-3H-pyrimidin-4-one

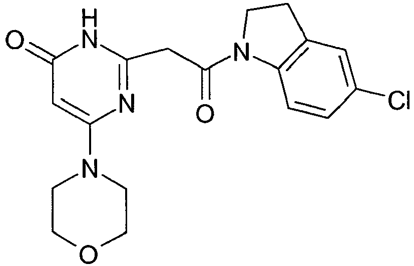

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 307 mg de 5-chloro-indoline, de 254 mg de N-[3-(diméthylamino)propyl]-N'-éthylcarbodiimide chlorhydrate dans un mélange de 0,16 mL de pyridine et de 4 mL de diméthylformamide. On obtient 255 mg de 2-[2-(5-chloro-2,3-dihydro-indol-1-yl)-2-oxo-éthyl]-6-morpholin-4-yl-3H-pyrimidin-4-one sous forme d'une poudre blanche dont les caractéristiques sont les suivantes :
Spectre RMN 1 H (400 MHz, δ en ppm, DMSO-d₆): 3,18 (t, *J*=8,3 Hz, 2 H) ; 3,41 (m, 4 H) ; 3,60 (m, 4H) ; 3,75 (s, 2 H) ; 4,16 (t, *J*=8,3 Hz, 2 H) ; 5,20 (s, 1 H) ; 7,21 (d large, *J*=8,6 Hz, 1 H) ; 7,32 (s large, 1 H); 7,99 (d, *J*=8,6 Hz, 1 H) ; 11,61 (m étalé, 1H).
Spectrométrie de Masse : méthode B
Temps de rétention Tr (min) = 3,42 ;
[M-H]- : m/z 373
Point de fusion (Kofler) : supérieur à 260°C

### Exemple 135 : 2-[2-(4-Bromo-2,3-dihydro-indol-1-yl)-2-oxo-éthyl]-6-morpholin-4-yl-3H-pyrimidin-4-one

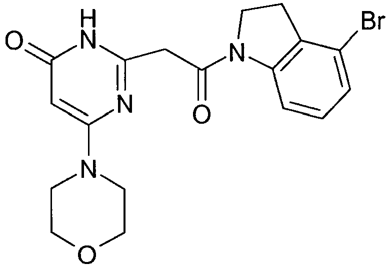

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 402 mg de 4-bromo-indoline, de 254 mg de N-[3-(diméthylamino)propyl]-N'-éthylcarbodiimide chlorhydrate dans un mélange de 0,16 mL de pyridine et de 4 mL de diméthylformamide. On obtient 303 mg de 2-[2-(4-bromo-2,3-dihydro-indo)-1-yl)-2-oxo-éthyl]-6-morpholin-4-yl-3H-pyrimidin-4-one sous forme d'une poudre blanche dont les caractéristiques sont les suivantes :
Spectre RMN 1 H (400 MHz, δ en ppm, DMSO-d₆): 3,14 (t, *J*=8,4 Hz, 2 H) ; 3,41 (m, 4 H) ; 3,61 (m, 4H) ; 3,76 (s, 2 H) ; 4,19 (t, *J*=8,4 Hz, 2 H) ; 5,21 (s, 1 H) ; 7,14 (t, *J*=8,1 Hz, 1 H) ; 7,23 (d large, *J*=8,1 Hz, 1 H) ; 8,01 (d large, *J*=8,1 Hz, 1 H) ; 11,62 (m étalé, 1 H).
Spectrométrie de Masse : méthode A
Temps de rétention Tr (min) = 0,88 ;
[M+H]+ : m/z 419 ; [M-H]- : m/z 417
Point de fusion (Kofler) : 219°C

### Exemple 136 et Exemple 137: Séparation de la 2-(2-{(3S)-3-[(dimethylamino)methyl]-2,3-dihydro-1H-indol-1-yl}-2-oxoethyl)-6-(morpholin-4-yl)pyrimidin-4(3H)-one et de la 2-(2-{(3R)-3-[(dimethylamino)methyl]-2,3-dihydro-1H-indol-1-yl}-2-oxoethyl)-6-(morpholin-4-yl)pyrimidin-4(3H)-one

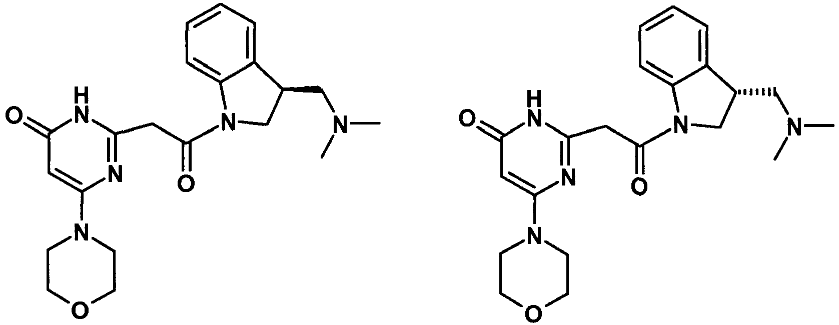

Les produits ont été obtenus par séparation chromatographique chirale de 500 mg de 2-[2-(3-diméthylaminométhyl-2,3-dihydro-indol-1-yl)-2-oxo-éthyl]-6-morpholin-4-yl-3H-pyrimidin-4-one (exemple 115) sur colonne chirale chiralpak AD 20µm (lot CFB003) (1200g, 20µM, 80/35mm), éluant : Heptane/Méthanol/Ethanol : 50/20/30 ; débit :160ml/min. Après purification, on obtient comme premier énantiomère, 226mg de (+)-2-[2-(3-diméthylaminométhyl-2,3-dihydro-indol-1-yl)-2-oxo-éthyl]-6-morpholin-4-yl-3H-pyrimidin-4-one, sous forme d'un solide jaune dont les caractéristiques sont les suivantes:
Spectre RMN 1 H (400 MHz): 2,22 (s, 6 H) ; 2,36 (dd, J=10,0 et 11,7 Hz, 1 H) ; 2,46 (dd, J=5,4 et 11,7 Hz, 1 H) ; 3,41 (m, 4 H) ; 3,60 (m, 5 H) ; 3,76 (m, 2 H) ; 3,91 (dd, J=5,7 et 10,0 Hz, 1 H) ; 4,23 (t, J=10,0 Hz, 1 H) ; 5,21 (s, 1 H) ; 7,02 (t, J=7,8 Hz, 1 H) ; 7,19 (t, J=7,8 Hz, 1 H) ; 7,29 (d, J=7,8 Hz, 1 H) ; 8,03 (d, J=7,8 Hz, 1 H) ; 11,61 (m étalé, 1 H)
Spectrométrie de Masse : méthode A
Temps de rétention Tr (min) = 0,40 ;
[M+H]+ : m/z 398 ; [M-H]- : m/z 396
Point de fusion (Kofler) : 246°C
Pouvoir rotatoire : 1,830 mg dans 1 ml de DMSO signe positif

Puis le deuxième énantiomère, soit : 239 mg de (-)-2-[2-(3-diméthylaminométhyl-2,3-dihydro-indol-1-yl)-2-oxo-éthyl]-6-morpholin-4-yl-3H-pyrimidin-4-one sous forme d'un solide jaune dont les caractéristiques sont les suivantes :
Spectre RMN 1 H (400 MHz): 2,22 (s, 6 H) ; 2,36 (dd, J=10,0 et 11,7 Hz, 1 H) ; 2,46 (dd, J=5,4 et 11,7 Hz, 1 H) ; 3,41 (m, 4 H) ; 3,60 (m, 5 H) ; 3,76 (m, 2 H) ; 3,91 (dd, J=5,7 et 10,0 Hz, 1 H) ; 4,23 (t, J=10,0 Hz, 1 H) ; 5,21 (s, 1 H) ; 7,02 (t, J=7,8 Hz, 1 H) ; 7,19 (t, J=7,8 Hz, 1 H) ; 7,29 (d, J=7,8 Hz, 1 H) ; 8,03 (d, J=7,8 Hz, 1 H) ; 11,62 (m étalé, 1 H)
Spectrométrie de Masse : méthode A
Temps de rétention Tr (min) = 0,40 ;
[M+H]+ : m/z 398 ; [M-H]- : m/z 396
Point de fusion (Kofler) : 256°C
Pouvoir rotatoire : 1,721 mg dans 1 ml de DMSO signe négatif

### Exemple 138 : Synthèse du N-[4-fluoro-2-(2-méthoxyéthoxy)phényl]-2-[4-(morpholin-4-yl)-6-oxo-1,6-dihydropyrimidin-2-yl]acétamide

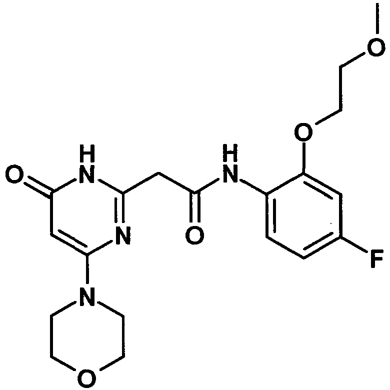

A une solution de 500 mg de [4-(morpholin-4-yl)-6-oxo-1,6-dihydropyrimidin-2-yl]acétate de sodium préparé à l'étape 2 de l'exemple 1 dans 3 mL de N,N-diméthylformamide sont ajoutés 3 mL de pyridine, 500 mg de N-[3-(diméthylamino)propyl]-N'-éthylcarbodiimide chlorhydrate et 590 mg de 4-fluoro-2-(2-méthoxy-éthoxy)-phénylamine. Le mélange réactionnel est agité à température ambiante pendant 15 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 155 mg du N-[4-fluoro-2-(2-méthoxyéthoxy)phényl]-2-[4-(morpholin-4-yl)-6-oxo-1,6-dihydropyrimidin-2-yl]acétamide sous forme de solide rosé dont les caractéristiques sont les suivantes:
Spectre RMN 1H (400MHz) : 3,17 à 3,33 (m partiellement masqué, 3 H) ; 3,43 (m, 4 H) ; 3,62 (m, 4 H) ; 3,68 (m, 4 H) ; 4,21 (m, 2 H) ; 5,21 (s, 1 H) ; 6,75 (dt, J=2,7 et 8,8 Hz, 1 H) ; 7,02 (dd, J=2,7 et 10,8 Hz, 1 H) ; 7,85 (dd, J=6,8 et 8,8 Hz, 1 H) ; 9,28 (s large, 1 H) ; 11,65 (m étalé, 1 H)
Spectrométrie de Masse : méthode B
Temps de rétention Tr (min) = 3,60 ;
[M+H]+ : m/z 405 ; [M-H]- : m/z 407

### Exemple 139 : Synthèse du N-(1H-benzimidazol-4-yl)-2-[4-(morpholin-4-yl)-6-oxo-1,6-dihydropyrimidin-2-yl]acétamide

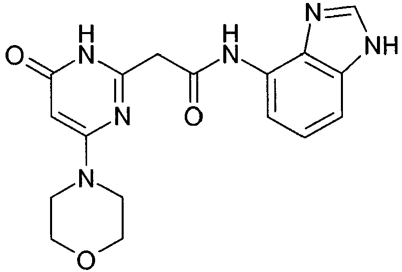

Le produit est préparé en suivant le mode opératoire décrit à l'exemple 5 à partir de 130 mg de [4-(morpholin-4-yl)-6-oxo-1,6-dihydropyrimidin-2-yl]acétate de sodium, de 113 mg de dichlorhydrate de 4-amino-benzimidazole, de 127 mg de N-[3-(diméthylamino)propyl]-N'-éthylcarbodiimide chlorhydrate dans un mélange de 80 µL de pyridine et de 2,0 mL de N,N-diméthylformamide. On obtient 112 mg de N-(1H-benzimidazol-4-yl)-2-[4-(morpholin-4-yl)-6-oxo-1,6-dihydropyrimidin-2-yl]acétamide sous forme d'une poudre blanc cassé dont les caractéristiques sont les suivantes :
Spectre RMN 1 H (400 MHz): 3,48 (m, 4 H) ; 3,60 (m, 4 H) ; 3,79 (s large, 2 H) ; 5,21 (s, 1 H) ; 7,14 (t, J=8,1 Hz, 1 H) ; 7,26 (s large, 1 H) ; 7,94 (s large, 1 H) ; 8,21 (s, 1 H) ; 10,20 (s large, 1 H); 11,70 (m étalé, 1 H) ; 12,54 (m étalé, 1 H)
Spectrométrie de Masse : méthode A
Temps de rétention Tr (min) = 0,32 ;
[M+H]+ : m/z 355 ; [M-H]- : m/z 353
Point de fusion (Kofler) : supérieur à 260°C

### Exemple 140 : Synthèse du méthyl 2-hydroxy-3-({[4-(morpholin-4-yl)-6-oxo-1,6-dihydropyrimidin-2-yl]acétyl}amino)benzoate

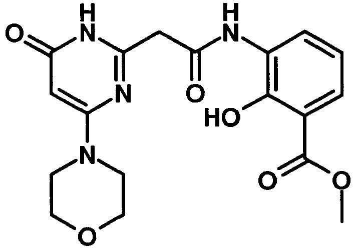

A une solution de 1g de [4-(morpholin-4-yl)-6-oxo-1,6-dihydropyrimidin-2-yl]acétate de sodium préparé à l'étape 2 de l'exemple 1 dans 6 mL de N,N-diméthylformamide sont ajoutés 6 mL de pyridine, 1g de N-[3-(diméthylamino)propyl]-N'-éthylcarbodiimide chlorhydrate et 1g de méthyl 3-amino-2-hydroxy-benzoate. Le mélange réactionnel est agité à température ambiante pendant 15 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 1,27g du méthyl 2-hydroxy-3-({[4-(morpholin-4-yl)-6-oxo-1,6-dihydropyrimidin-2-yl]acétyl}amino)benzoate sous forme de solide gris dont les caractéristiques sont les suivantes:
Spectre RMN 1 H (400MHz) : 3,45 (m, 4 H) ; 3,58 (m, 4 H) ; 3,73 (s, 2 H) ; 3,94 (s, 3 H) ; 5,21 (s, 1 H) ; 6,95 (t, J=7,9 Hz, 1 H) ; 7,56 (dd, J=1,3 et 7,9 Hz, 1 H) ; 8,21 (d large, J=7,9 Hz, 1 H) ; 9,71 (s large, 1 H) ; 11,03 (s large, 1 H) ; 11,70 (m étalé, 1 H)
Spectrométrie de Masse : méthode A
Temps de rétention Tr (min) =0,66 ;
[M+H]+ : m/z 387 ; [M-H]- : m/z 389

### Exemple 141 2-[2-(4-Méthoxy-2,3-dihydro-indol-1-yl)-2-oxo-éthyl]-6-morpholin-4-yl-3H-pyrimidin-4-one

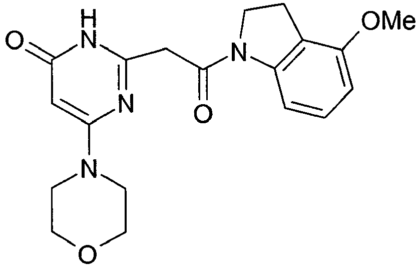

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 308 mg de 4-méthoxy-indoline, de 254 mg de N-[3-(diméthylamino)propyl]-N'-éthylcarbodiimide chlorhydrate dans un mélange de 0,16 mL de pyridine et de 4 mL de diméthylformamide. On obtient 269 mg de 2-[2-(4-méthoxy-2,3-dihydro-indol-1-yl)-2-oxo-éthyl]-6-morpholin-4-yl-3H-pyrimidin-4-one sous forme d'une poudre rosée dont les caractéristiques sont les suivantes :
Spectre RMN 1 H (400 MHz, δ en ppm, DMSO-d₆): 3,04 (t, *J*=8,2 Hz, 2 H) ; 3,41 (m, 4 H) ; 3,61 (m, 4H) ; 3,74 (s, 2 H) ; 3,79 (s, 3 H) ; 4,14 (t, *J*=8,2 Hz, 2 H) ; 5,20 (s, 1 H) ; 6,70 (d large, *J*=7,9 Hz, 1 H) ; 7,15 (t, *J*=7,9 Hz, 1 H) ; 7,64 (d large, *J*=7,9 Hz, 1H) ; 11,61 (m étalé, 1 H).
Spectrométrie de Masse : méthode A
Temps de rétention Tr (min) = 0,75 ;
[M+H]₊ : m/z 371 ; [M-H]- : m/z 369
Point de fusion (Kofler) : supérieur à 260°C

### Exemple 142: N-(3-bromo-2-hydroxyphényl)-2-[4-(morpholin-4-yl)-6-oxo-1,6-dihydropyrimidin-2-yl]acétamide

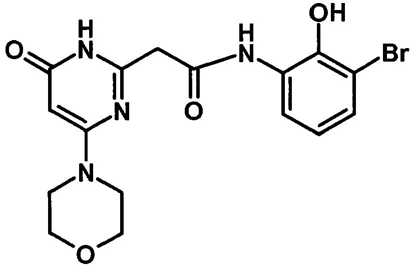

A une solution de 500 mg de [4-(morpholin-4-yl)-6-oxo-1,6-dihydropyrimidin-2-yl]acétate de sodium préparé à l'étape 2 de l'exemple 1 dans 4 mL de pyridine, 600 mg de N-[3-(diméthylamino)propyl]-N'-éthylcarbodiimide chlorhydrate et 550 mg de 2-amino-6-bromophénol. Le mélange réactionnel est agité à température ambiante pendant 20 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 543 mg du N-(3-bromo-2-hydroxyphényl)-2-[4-(morpholin-4-yl)-6-oxo-1 ,6-dihydropyrimidin-2-yl]acétamide sous forme de solide beige dont les caractéristiques sont les suivantes:
Spectre RMN 1 H (400MHz) : 3,44 (m, 4 H) ; 3,62 (m, 4 H) ; 3,70 (s, 2 H) ; 5,21 (s, 1 H) ; 6,78 (t, J=8,1 Hz, 1 H) ; 7,32 (d, J=8,1 Hz, 1 H) ; 7,56 (d, J=8,1 Hz, 1 H) ; 9,82 (m étalé, 2 H); 11,62 (m étalé, 1 H)
Spectrométrie de Masse : méthode A
Temps de rétention Tr (min) =0,64 ;
[M+H]+ : m/z 408 ; [M-H]- : m/z 410

### Exemple 143 : Synthèse du N-(3,4-dihydro-2H-1,4-benzoxazin-8-yl)-2-[4-(morpholin-4-yl)-6-oxo-1,6-dihydropyrimidin-2-yl]acétamide

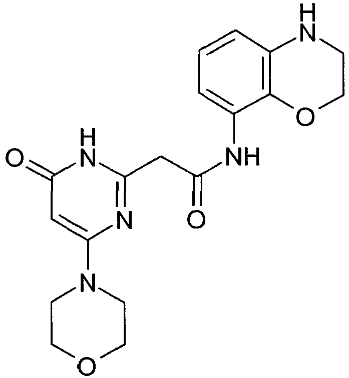

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 166 mg de 3,4-dihydro-2H-1,4-benzoxazin-8-ylamine, de 249 mg de N-[3-(diméthylamino)propyl]-N'-éthylcarbodiimide chlorhydrate dans un mélange de 162 µL de pyridine et de 4,0 mL de N,N-diméthylformamide. On obtient 100 mg de N-(3,4-dihydro-2H-1,4-benzoxazin-8-yl)-2-[4-(morpholin-4-yl)-6-oxo-1,6-dihydropyrimidin-2-yl]acétamide sous forme d'une poudre blanche dont les caractéristiques sont les suivantes :
Spectre RMN 1 H (400 MHz): 3,32 (m partiellement masqué, 2 H) ; 3,44 (m, 4 H) ; 3,62 (m, 6 H) ; 4,08 (m, 2 H) ; 5,08 (m, 1 H) ; 5,19 (s, 1 H) ; 6,49 (t, J=7,8 Hz, 1 H) ; 6,56 (dd, J=1,5 et 7,8 Hz, 1 H) ; 6,80 (dd, J=1,5 et 7,8 Hz, 1 H) ; 9,43 (m étalé, 1 H) ; 11,63 (m étalé, 1 H)
Spectrométrie de Masse : méthode A
Temps de rétention Tr (min) = 2,64 ;
[M+H]+ : m/z 372 ; [M-H]- : m/z 370
Point de fusion (Kofler) : 243°C

### Exemple 144: 5-fluoro-2-({[4-(morpholin-4-yl)-6-oxo-1,6-dihydropyrimidin-2-yl]acétyl}amino)benzoate de méthyle

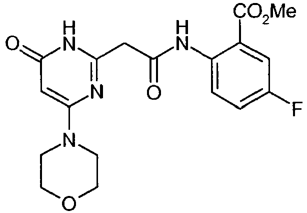

Le produit est préparé en suivant le mode opératoire décrit à l'exemple 5 à partir de 300 mg de [4-(morpholin-4-yl)-6-oxo-1,6-dihydropyrimidin-2-yl]acétate de sodium, de 303 mg de 2-amino-5-fluorobenzoate de méthyle, de 308 mg de N-[3-(diméthylamino)propyl]-N'-éthylcarbodiimide chlorhydrate dans un mélange de 2 mL de pyridine et de 2 mL de diméthylformamide. On obtient 310 mg de 5-fluoro-2-({[4-(morpholin-4-yl)-6-oxo-1,6-dihydropyrimidin-2-yl]acétyl}amino)benzoate de méthyle sous forme d'un solide blanc dont les caractéristiques sont les suivantes:
Spectre RMN 1 H (400 MHz) : 3,44 (m, 4 H) ; 3,61 (m, 4 H) ; 3,67 (s, 2 H) ; 3,84 (s, 3 H) ; 5,21 (s, 1 H) ; 7,50 (m, 1 H) ; 7,64 (dd, J=3,0 et 9,2 Hz, 1 H) ; 8,13 (dd, J=5,3 et 8,9 Hz, 1 H) ; 10,57 (s large, 1 H) ; 11,70 (m étalé, 1 H)
Spectrométrie de Masse : méthode A
Temps de rétention Tr (min) = 0,71 ;
[M+H]+ : m/z 391 ; [M-H]- : m/z 389

### Exemple 145 : Synthèse de la 2-(2-{3-[(diéthylamino)méthyl]-2,3-dihydro-1H-indol-1-yl}-2-oxoéthyl)-6-(morpholin-4-yl)pyrimidin-4(3H)-one

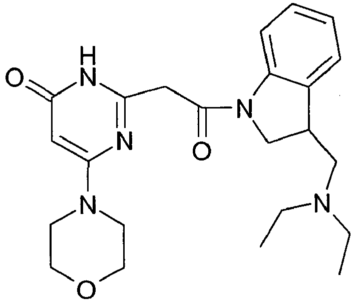

### Etape 1

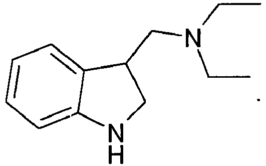

A une solution de 0,5 g de diéthyl-(1H-indol-3-ylméthyl)-amine (préparé selon Synth. Commun. 2006, 1829) dans 10 mL d'acide trifluoroacétique sous argon refroidie dans un bain de glace, est ajouté progressivement 0,48 g de cyanoborohydrure de sodium, puis l'on agite pendant une demi-heure à 0°C et 3h à température ambiante. Le milieu réactionnel est ensuite jeté dans 100 mL d'eau et alcalinisé par de la soude à 30%. Après ajout de 100 mL d'acétate d'éthyle, on agite pendant 10 min à température ambiante, puis le mélange est décanté et la phase aqueuse est extraite par trois fois 50 mL d'acétate d'éthyle. Les phases organiques sont réunies, puis lavées avec 40 mL d'eau. La phase organique est séchée sur sulfate de magnésium anhydre, filtrée et concentrée à sec sous pression réduite. Le résidu est repris par 20 mL de soude 2N, plus 40 mL d'eau et 60 mL d'acétate d'éthyle, agité pendant 5 min, puis décanté. La phase aqueuse est extraite par deux fois 40 mL d'acétate d'éthyle et les phases organiques sont réunies, séchées sur sulfate de magnésium anhydre, filtrées et concentrées à sec sous pression réduite. Après une purification sur colonne de silice, éluant: CH2Cl2/MeOH/NH4OH, 89/10/1, on obtient 246 mg de (2,3-dihydro-1H-indol-3-ylméthyl)-diéthyl-amine sous forme d'une huile jaune dont les caractéristiques sont les suivantes :
Spectrométrie de Masse : méthode A
Temps de rétention Tr (min) = 0,20 ;
[M+H]+ : m/z 205 ;

### Etape 2

Le produit est préparé en suivant le mode opératoire décrit à l'exemple 5 à partir de 150 mg de [4-(morpholin-4-yl)-6-oxo-1,6-dihydropyrimidin-2-yl]acétate de sodium, de 238 mg de (2,3-dihydro-1H-indol-3-ylméthyl)-diéthyl-amine, de 140 mg de N-[3-(diméthylamino)propyl]-N'-éthylcarbodiimide chlorhydrate dans un mélange de 0,1 mL de pyridine et de 3 mL de N,N-diméthylformamide. On obtient 108 mg de 2-(2-{3-[(diéthylamino)méthyl]-2,3-dihydro-1H-indol-1-yl}-2-oxoéthyl)-6-(morpholin-4-yl)pyrimidin-4(3H)-one sous forme d'un solide crème dont les caractéristiques sont les suivantes :
Spectre RMN 1 H (400 MHz): 0,96 (t, J=7,0 Hz, 6 H) ; 2,36 à 2,71 (m partiellement masqué, 6 H) ; 3,41 (m, 4 H) ; 3,56 (m, 1 H) ; 3,61 (m, 4 H) ; 3,75 (s, 2 H) ; 3,88 (dd, J=5,4 et 10,8 Hz, 1 H); 4,21 (t, J=10,8 Hz, 1 H) ; 5,21 (s, 1 H) ; 7,02 (t, J=7,7 Hz, 1 H) ; 7,18 (t, J=7,7 Hz, 1 H) ; 7,32 (d, J=7,7 Hz, 1 H) ; 8,03 (d, J=7,7 Hz, 1 H) ; 11,62 (m étalé, 1 H)
Spectrométrie de Masse : méthode A
Temps de rétention Tr (min) = 0,43 ;
[M+H]+ : m/z 426 ; [M-H]- : m/z 424
Point de fusion (Kofler) : 202°C

### Exemple 146 : Synthèse de la 2-[2-(2,3-dihydro-1H-pyrrolo[3,2-b]pyridin-1-yl)-2-oxoéthyl]-6-(morpholin-4-yl)pyrimidin-4(3H)-one

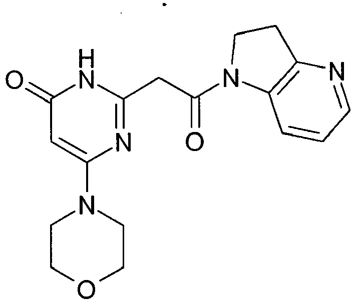

### Etape 1

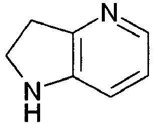

A une solution de 1,5 g de 4-aza-indole dans 45 mL d'éthanol absolu, dans un autoclave sous argon sont ajoutés 1,49 g de nickel de Raney à 50% dans l'eau. Le mélange est hydrogéné sous 100 bar d'hydrogène à une température de 95°C pendant 41 h. Après retour à température ambiante, le catalyseur est filtré sur Clarcel et le filtrat est concentré à sec sous pression réduite. Le résidu est purifié par chromatographie sur une cartouche de 70g silice 15-40 µm, en éluant avec du dichlorométhane pur puis avec un mélange dichlorométhane/ méthanol 99/1 v/v avec un débit de 80 mL/ min, puis sur une cartouche de 30 g silice 15-40 µm, en éluant avec du dichlorométhane pur avec un débit de 30 mL/ min. On obtient ainsi 0,18 g de 4-aza-indoline sous forme d'un solide jaune pâle fondant à 63°C (Kofler).

### Etape 2

Le produit est préparé en suivant le mode opératoire décrit à l'exemple 5 à partir de 350 mg de [4-(morpholin-4-yl)-6-oxo-1,6-dihydropyrimidin-2-yl]acétate de sodium, de 177 mg de 4-aza-indoline, de 340 mg de N-[3-(diméthylamino)propyl]-N'-éthylcarbodiimide chlorhydrate dans un mélange de 216 µL de pyridine et de 6,5 mL de N,N-diméthylformamide. On obtient 252 mg de 2-[2-(2,3-dihydro-1H-pyrrolo[3,2-b]pyrid in-1-yl)-2-oxoéthyl]-6-(morpholin-4-yl)pyrimidin-4(3H)-one sous forme d'une poudre blanche dont les caractéristiques sont les suivantes :
Spectre RMN 1 H (400 MHz): 3,24 à 3,37 (m partiellement masqué, 2H) ; 3,41 (m, 4 H) ; 3,60 (m, 4 H) ; 3,79 (s, 2 H) ; 4,19 (t, J=8,3 Hz, 2 H) ; 5,21 (s, 1 H) ; 7,17 (dd, J=4,4 et 8,3 Hz, 1 H) ; 8,13 (d, J=4,4 Hz, 1 H) ; 8,19 (d, J=8,3 Hz, 1 H) ; 11,63 (m étalé, 1 H)
Spectrométrie de Masse : méthode A
Temps de rétention Tr (min) = 0,37 ;
[M+H]+ : m/z 342 ; [M-H]- : m/z 340
Point de fusion (Kofler) : supérieur à 260°C

### Exemple 147 2-[2-(5,6-Difluoro-2,3-dihydro-indol-1-yl)-2-oxo-éthyl]-6-morpholin-4-yl-3H-pyrimidin-4-one

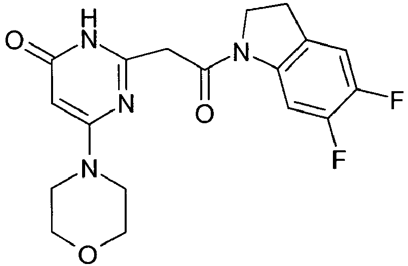

Le produit est préparé en suivant le mode opératoire décrit à l'exemple 5 à partir de 261 mg de [4-(morpholin-4-yl)-6-oxo-1,6-dihydropyrimidin-2-yl]acétate de sodium, de 310 mg de 4-méthoxy-indoline, de 254 mg de N-[3-(diméthylamino)propyl]-N'-éthylcarbodiimide chlorhydrate dans un mélange de 0,16 mL de pyridine et de 4 mL de diméthylformamide. On obtient 245 mg de 2-[2-(5,6-difluoro-2,3-dihydro-indol-1-yl)-2-oxo-éthyl]-6-morpholin-4-yl-3H-pyrimidin-4-one sous forme d'une poudre blanche dont les caractéristiques sont les suivantes :
Spectre RMN 1 H (400 MHz, δ en ppm, DMSO-d₆): 3,16 (t, *J*=8,4 Hz, 2 H) ; 3,41 (m, 4 H) ; 3,60 (m, 4H) ; 3,76 (s, 2 H) ; 4,19 (t, *J*=8,4 Hz, 2 H) ; 5,21 (s, 1 H) ; 7,37 (t, *J*=9,2 Hz, 1 H) ; 7,95 (dd, *J*=7,6 et 12,0 Hz, 1 H) ; 11,61 (m étalé, 1 H).
Spectrométrie de Masse : méthode A
Temps de rétention Tr (min) = 0,70 ;
[M+H]+ : m/z 377 ; [M-H]- : m/z 375
Point de fusion (Kofler) : supérieur à 260°C

### Exemple 148 : Synthèse du 2-[4-(morpholin-4-yl)-6-oxo-1,6-dihydropyrimidin-2-yl]-N-(1,2,3,4-tétrahydroquinolin-8-yl)acétamide

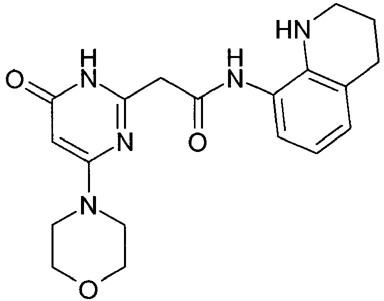

Le produit est préparé en suivant le mode opératoire décrit à l'exemple 5 à partir de 340 mg de [4-(morpholin-4-yl)-6-oxo-1,6-dihydropyrimidin-2-yl]acétate de sodium, de 212 mg de 8-amino-1,2,3,4-tetrahydroquinoléine, de 330 mg de N-[3-(diméthylamino)propyl]-N'-éthylcarbodiimide chlorhydrate dans un mélange de 210 µL de pyridine et de 5,0 mL de N,N-diméthylformamide. On obtient 243 mg de 2-[4-(morpholin-4-yl)-6-oxo-1,6-dihydropyrimidin-2-yl]-N-(1,2,3,4-tétrahydroquinolin-8-yl)acétamide sous forme d'une poudre beige dont les caractéristiques sont les suivantes :
Spectre RMN 1 H (400 MHz): 1,79 (m, 2 H) ; 2,70 (t, J=6,5 Hz, 2 H) ; 3,23 (t large, J=6,5 Hz, 2 H) ; 3,44 (m, 4 H) ; 3,59 (s, 2 H) ; 3,63 (m, 4 H) ; 5,03 (s large, 1 H) ; 5,20 (s, 1 H) ; 6,43 (t, J=7,7 Hz, 1 H) ; 6,75 (d, J=7,7 Hz, 1 H) ; 6,93 (d, J=7,7 Hz, 1 H) ; 9,22 (s large, 1 H) ; 11,62 (m étalé, 1 H)
Spectrométrie de Masse : méthode A
Temps de rétention Tr (min) = 0,57 ;
[M+H]+ : m/z 370 ; [M-H]- : m/z 368
Point de fusion (Kofler) : 232°C

### Exemple 149: Synthèse de la 2-[2-(8-chloro-2,3-dihydro-4H-1,4-benzoxazin-4-yl)-2-oxoéthyl]-6-(morpholin-4-yl)pyrimidin-4(3H)-one

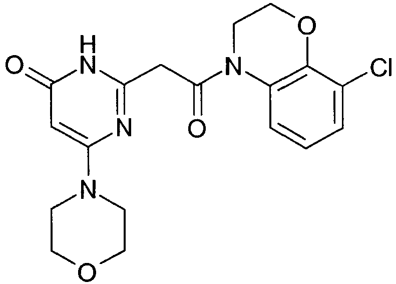

Le produit est préparé en suivant le mode opératoire décrit à l'exemple 5 à partir de 220 mg de [4-(morpholin-4-yl)-6-oxo-1,6-dihydropyrimidin-2-yl]acétate de sodium, de 157 mg de 8-chloro-2H-benzo[b][1,4]oxazine (qui peut être préparé selon WO 2008/100463), de 214 mg de N-[3-(diméthylamino)propyl]-N'-éthylcarbodiimide chlorhydrate dans un mélange de 135 µL de pyridine et de 3,5 mL de N,N-diméthylformamide. On obtient 140 mg de 2-[2-(8-chloro-2,3-dihydro-4H-1,4-benzoxazin-4-yl)-2-oxoéthyl]-6-(morpholin-4-yl)pyrimidin-4(3H)-one sous forme d'une poudre blanche dont les caractéristiques sont les suivantes :
Spectre RMN 1 H (400MHz): pour ce lot, tous les signaux sont larges avec : 3,41 (m, 4 H) ; 3,57 (m, 4 H) ; 3,88 (m, 4 H) ; 4,42 (m, 2 H) ; 5,17 (s, 1 H) ; 6,88 (m, 1 H) ; 7,24 (m, 1 H) ; 7,71 (m très étalé, 1 H) ; 11,57 (m étalé, 1 H)
Spectrométrie de Masse : méthode A
Temps de rétention Tr (min) = 0,71 ;
[M+H]+ : m/z 391 ; [M-H]- : m/z 389
Point de fusion (Kofler) : supérieur à 260°C

### Exemple 150: Synthèse de N-(2-hydroxy-3-méthylphényl)-2-[4-(morpholin-4-yl)-6-oxo-1,6-dihydropyrimidin-2-yl]acétamide

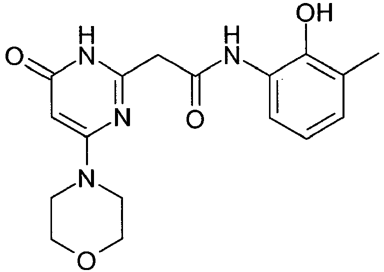

Le produit est préparé en suivant le mode opératoire décrit à l'exemple 5 à partir de 1 g de [4-(morpholin-4-yl)-6-oxo-1,6-dihydropyrimidin-2-yl]acétate de sodium, de 680 mg de 6-amino-2-méthylphénol, de 1,2 g de N-[3-(diméthylamino)propyl]-N'-éthylcarbodiimide chlorhydrate dans un mélange de 6 mL de pyridine et de 8 mL de N,N-diméthylformamide. On obtient 1,1 g de N-(2-hydroxy-3-méthylphényl)-2-[4-(morpholin-4-yl)-6-oxo-1,6-dihydropyrimidin-2-yl]acétamide sous forme d'un solide beige dont les caractéristiques sont les suivantes:
Spectre RMN 1 H (400MHz) : 2,18 (s, 3 H) ; 3,45 (m, 4 H) ; 3,62 (m, 4 H) ; 3,70 (s,2 H) ; 5,21 (s, 1 H) ; 6,73 (t, J=7,8 Hz, 1 H) ; 6,91 (d, J=7,8 Hz, 1 H) ; 7,37 (d, J=7,8 Hz, 1 H) ; 8,80 (s, large, 1 H) ; 9,77 (s, 1 H) ; 11,68 (m étalé, 1 H)
Spectrométrie de Masse : méthode A
Temps de rétention Tr (min) = 0,61 ;
[M+H]+ : m/z 345 ; [M-H]- : m/z 343

### Exemple 151: N-(2-hydroxy-3-nitrophényl)-2-[4-(morpholin-4-yl)-6-oxo-1,6-dihydropyrimidin-2-yl]acétamide

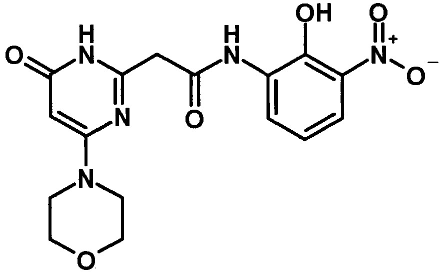

Le produit est préparé en suivant le mode opératoire décrit à l'exemple 5 à partir de 1 g de [4-(morpholin-4-yl)-6-oxo-1,6-dihydropyrimidin-2-yl]acétate de sodium, de 590 mg de 2-amino-6-nitrophénol, de 734 mg de N-[3-(diméthylamino)propyl]-N'-éthylcarbodiimide chlorhydrate dans un mélange de 5 mL de pyridine et de 6 mL de N,N-diméthylformamide . On obtient 976 mg de N-(2-hydroxy-3-nitrophényl)-2-[4-(morpholin-4-yl)-6-oxo-1,6-dihydropyrimidin-2-yl]acétamide sous forme d'un solide jaune dont les caractéristiques sont les suivantes:
Spectre RMN 1 H (400MHz) : 3,48 (m, 4 H) ; 3,63 (m, 4 H) ; 3,65 (s, 2 H) ; 5,19 (s,1 H) ; 5,85 (dd, J=7,2 et 8,7 Hz, 1 H) ; 7,37 (dd, J=1,7 et 8,7 Hz, 1 H) ; 7,91 (dd, J=1,7 et 7,2 Hz, 1 H) ; 9,74 (m étalé, 1 H) ; 11,75 (m étalé, 1 H)
Spectrométrie de Masse : méthode A
Temps de rétention Tr (min) = 0,61 ;
[M+H]+ : m/z 376 ; [M-H]- : m/z 374

### Exemple 152 : N-(3-cyano-2-hydroxyphényl)-2-[4-(morpholin-4-yl)-6-oxo-1,6-dihydropyrimidin-2-yl]acétamide

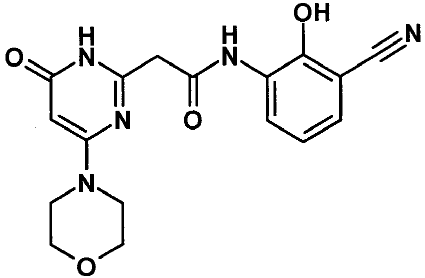

Le produit est préparé en suivant le mode opératoire décrit à l'exemple 5 à partir de 1 g de [4-(morpholin-4-yl)-6-oxo-1,6-dihydropyrimidin-2-yl]acétate de sodium, de 513 mg de 3-amino-2-hydroxybenzonitrile, de 734 mg de N-[3-(diméthylamino)propyl]-N'-éthylcarbodiimide chlorhydrate dans un mélange de 5 mL de pyridine et de 5 mL de N,N-diméthylformamide. On obtient 257 mg de N-(3-cyano-2-hydroxyphényl)-2-[4-(morpholin-4-yl)-6-oxo-1,6-dihydropyrimidin-2-yl]acétamide sous forme d'un solide blanc dont les caractéristiques sont les suivantes:
Spectre RMN 1 H (400MHz) : 3,44 (m, 4 H) ; 3,62 (m, 4 H) ; 3,70 (s, 2 H) ; 5,21 (s, 1 H) ; 6,91 (t, J=7,8 Hz, 1 H) ; 7,40 (d, J=7,8 Hz, 1 H) ; 7,78 (d, J=7,8 Hz, 1 H) ; 9,95 (m étalé, 1 H) ; 10,71 (m très étalé, 1 H) ; 11,64 (m étalé, 1 H)
Spectrométrie de Masse : méthode A
Temps de rétention Tr (min) = 0,57 ;
[M+H]+ : m/z 356 ; [2M+H]+ : m/z 711 (pic de base)
[M-H]- : m/z 354 (pic de base) ; [2M-H]- : m/z 709

### Exemple 153 : N-[2-hydroxy-3-(trifluorométhyl)phényl]-2-[4-(morpholin-4-yl)-6-oxo-1,6-dihydropyrimidin-2-yl]acétamide

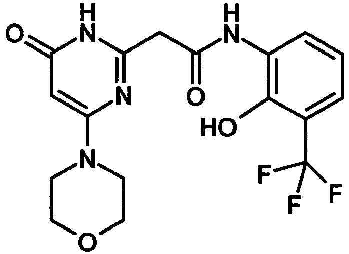

Le produit est préparé en suivant le mode opératoire décrit à l'exemple 5 à partir de 1 g de [4-(morpholin-4-yl)-6-oxo-1,6-dihydropyrimidin-2-yl]acétate de sodium, de 978 mg de 2-hydroxy-3-(trifluorométhyl)aniline, de 1,2 g de N-[3-(diméthylamino)propyl]-N'-éthylcarbodiimide chlorhydrate dans un mélange de 6 mL de pyridine et de 8 mL de N,N-diméthylformamide . On obtient 600 mg de N-[2-hydroxy-3-(trifluorométhyl)phényl]-2-[4-(morpholin-4-yl)-6-oxo-1,6-dihydropyrimidin-2-yl]acétamide sous forme d'un solide beige dont les caractéristiques sont les suivantes:
Spectre RMN 1 H (400MHz) : 3,44 (m, 4 H) ; 3,61 (m, 4 H) ; 3,69 (s, 2 H) ; 5,21 (s, 1 H) ; 6,95 (t, J=7,8 Hz, 1 H) ; 7,38 (d, J=7,8 Hz, 1 H) ; 7,57 (d, J=7,8 Hz, 1 H) ; 10,44 (m très étalé, 3 H)
Spectrométrie de Masse : méthode A
Temps de rétention Tr (min) = 0,71 ;
[M+H]+ : m/z 399 ; [M-H]- : m/z 397

### Exemple 154 : Synthèse de la 2-[2-(3,3-diméthyl-2,3-dihydro-1H-indo)-1-yl)-2-oxoéthyl]-6-(morpholin-4-yl)pyrimidin-4(3H)-one

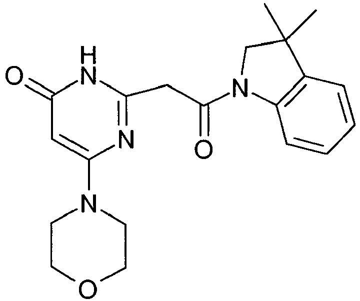

Le produit est préparé en suivant le mode opératoire décrit à l'exemple 5 à partir de 177 mg de [4-(morpholin-4-yl)-6-oxo-1,6-dihydropyrimidin-2-yl]acétate de sodium, de 236 mg de 3,3-diméthylindoline (qui peut être préparée selon T.W. Ramsay et coll. Synth. Commun. 1995, 25, 4029), de 169 mg de N-[3-(diméthylamino)propyl]-N'-éthylcarbodiimide chlorhydrate dans un mélange de 0,11 mL de pyridine et de 5 mL de N,N-diméthylformamide. Le mélange réactionnel est agité pendant 15 h à température ambiante, puis concentré à sec sous pression réduite. On ajoute au résidu 40 mL d'eau que l'on triture à la spatule à température ambiante. Le précipité formé est filtré, puis solubilisé par un mélange dichlorométhane / méthanol et séché. Après concentration à sec sous pression réduite, le produit brut est purifié par chromatographie sur une colonne de 30 g de silice (0,02-0,045 mm), éluant: dichlorométhane/méthanol 90/10 v/v. Après évaporation des différentes fractions, le résidu solide rose est trituré dans 10 mL d'éther diisopropylique, filtré, puis séché sous cloche (1h/40°C/20 mbar). On obtient ainsi 134 mg de 2-[2-(3,3-diméthyl-2,3-dihydro-indol-1-yl)-2-oxo-éthyl]-6-morpholin-4-yl-3H-pyrimidin-4-one sous forme d'un solide rose dont les caractéristiques sont les suivantes:
Spectre RMN 1H (400 MHz): 1,31 (s, 6 H) ; 3,42 (m, 4 H) ; 3,59 (m, 4 H) ; 3,75 (s, 2 H) ; 3,91 (s, 2 H) ; 5,20 (s, 1 H) ; 7,05 (t, J=7,6 Hz, 1 H) ; 7,18 (t, J=7,6 Hz, 1 H) ; 7,27 (d, J=7,6 Hz, 1H) ; 8,00 (d, J=7,6 Hz, 1 H) ; 11,61 (m étalé, 1 H)
Spectrométrie de Masse : méthode A
Temps de rétention Tr (min) = 0,77 ;
[M+H]+ : m/z 369 ; [M-H]- : m/z 367
Point de fusion (Kofler) : 248°C

### Exemple 93 : Composition pharmaceutique

On a préparé des comprimés répondant à la formule suivante :
Produit de l'exemple 1 0,2 g
Excipient pour un comprimé terminé à 1 g
(détail de l'excipient : lactose, talc, amidon, stéarate de magnésium).

L'exemple 1 est pris à titre d'exemple de préparation pharmaceutique, cette préparation pouvant être réalisée si désiré avec d'autres produits en exemples dans la présente demande.

### Partie pharmacologique :

### Protocoles expérimentaux

### Procédures expérimentales in vitro

L'activité inhibitrice des molécules sur la phosphorylation d'AKT est mesurée soit par western blotting par la technique décrite ci-dessous, soit par la technique MSD Multi-spot Biomarker détection de Meso Scale Discovery également décrite ci-dessous. Il a été démontré sur un set de molécules que les 2 techniques donnent des résultats compatibles.

### Etude de l'expression de pAKT dans les cellules humaines PC3 de carcinome de prostate mesurée par western blotting (Test A):

Cet essai est basé sur la mesure de l'expression de la protéine AKT phosphorylée sur la serine 473. La phosphorylation d'AKT (pAKT) est mesurée par western blotting dans la lignée de carcinome de prostate humaine PC3 (ATCC CRL-1435), en utilisant un anticorps reconnaissant spécifiquement pAKT-S473.

Le jour 1, les cellules PC3 sont ensemencées en plaques 6 puits (TPP, # 92006) à la concentration de 0.8x106 cellules/puits dans 1800 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2 heures à 37°C en présence de 5% CO2. Les molécules diluées dans du diméthylsulfoxyde (DMSO Sigma #D2650), sont ajoutées à partir d'une solution mère concentrée 10 fois, le pourcentage final de DMSO étant de 0.1%. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Après aspiration du milieu de culture, les cellules sont rincées par 1ml de PBS (DPBS Gibco, #14190-094), récupérées par grattage dans 200µl de tampon HNTG complet et transfert en plaque 96 puits (Greiner #651201), et lysées pendant 1 H sur glace. Le tampon HNTG est composé du mélange suivant :Hepes 50 mM, NaCl 150 mM, Triton 1%, Glycerol 10%, avec ajoût extemporané d'une pastille de Protease Inhibitor Cocktail Mini (Roche 1836153) et d'une pastille de Phosphatase Inhibitor Cocktail (Roche104906837001) pour 10ml de tampon.

Le lysat est centrifugé 10min à 6000RPM. 155µl de surnageant sont récupérés. 150 µl sont incubés pour dénaturation pendant 5min à 95°C en présence de tampon NuPAGE LDS Sample Buffer 4X dilué 4 fois (Ref InVitrogen NP0007) et de NuPAGE Sample Reducing Agent 10X dilué 10 fois (Ref InVitrogen NP0009). Ces échantillons sont ensuite congelés à -20°C. 5 µl sont dosées par la technique microBCA selon la fiche technique du MicroBCA Proteine Assay Kit (Pierce #23235).

Pour la séparation des protéines, 20µg de protéines sont déposées sur gel NU-PAGE 4-12% Bis Tris Gel 12 puits (Ref InVitrogen NP0322BOX) et la migration est effectuée pendant 1 h30 en tampon de migration NU-PAGE MOPS SDS Running Buffer 20X dilué 20 fois (Ref InVitrogen NP0001), à 150 Volts.

Le gel est ensuite transféré sur une membrane Invitrolon PVDF (Invitrogen #LC2007) préalablement perméabilisée quelques secondes dans de l'éthanol (Ethanol Fischer Scientific #E/0600DF/15).

Le transfert s'effectue dans une cuve Biorad à 30 Volts pendant la nuit ou à 60 volts pendant 3 heures, en présence de tampon de transfert NUPAGE Transfer Buffer 20X dilué 20 fois (Ref InVitrogen NP0006).

La membrane est ensuite saturée en solution de saturation composé de TBS (Tris Buffer Saline 10x, Sigma #T5912 Sigma, dilué 10 fois), Tween 20 0.1% (#P5927 Sigma) et BSA 3% (Bovine Albumin Serum Fraction V, Sigma #A4503) pendant 6h après un transfert d'une durée de une nuit ou bien pendant 1 h après un transfert d'une durée de 3h.

Les anticorps primaires sont dilués au 1/1000e pour l'anticorps anti-phospho AKT-Ser473 (193H2, monoclonal de lapin, cat#4058 de chez Cell Signaling Technology) Abcam), en solution de saturation composée de PBS, Tween 20 0.1%, BSA 3%, puis mis sous agitation pendant la nuit à 4°C. Deux rinçages de 5 min en solution de lavage composée de TBS, Tween 20 0.1 % sont effectués avant l'hybridation des anticorps secondaires.

Les anticorps secondaires sont dilués au 1/10000e pour l'anticorps Rabbit anti-Mouse IgG HRP (W402 Promega) et au 1/10000e pour l'anticorps Goat anti-Rabbit IgG HRP (W401 Promega) en solution de saturation puis mis sous agitation pendant 1 h à température ambiante.

Deux rinçages de 30 min en solution de lavage sont effectués puis un rinçage de 5 min à l'H2O est effectué pour éliminer le Tween 20 restant.

La solution de révélation est préparée volume à volume selon la fiche technique du Western Lightning Chemiluminescence Reagent Plus (Western Lightning Chemiluminescence Reagent Plus Perkin Elmer #NEL104).

La membrane est placée pendant 1 min dans la solution de révélation, égouttée, insérée entre deux transparents puis placée dans l'appareil de mesure pour la lecture de la luminescence et la quantification du signal. La lecture de la luminescence est effectuée avec l'appareil FujiFilm (Ray Test).

L'appareil FUJI mesure le signal total de luminescence obtenu (AU) pour chaque bande sélectionnée. Puis il soustrait le bruit de fond (BG) proportionnel à la taille de la bande sélectionnée (Area), bruit de fond calculé à partir d'une bande de bruit de fond spécifique, en vue d'obtenir le signal spécifique (AU-BG) pour chaque bande. La bande obtenue en absence de produit et en présence de 0.1% DMSO est considérée comme le 100 % de signal. Le logiciel calcule le % d'activité spécifique (Ratio) obtenu pour chaque bande sélectionnée en fonction de ce 100 % de signal. Le calcul du pourcentage d'inhibition est fait pour chaque concentration selon la formule (100% - Ratio).

2 expériences indépendantes permettent de calculer la moyenne des pourcentages d'inhibition obtenus à une concentration donnée pour les produits testés uniquement à une concentration.

Le cas échéant, l'activité des produits est traduite en Cl50 approchée, obtenue à partir d'une courbe dose-réponse de différentes concentrations testées et représentant la dose donnant 50 % d'inhibition spécifique (CI50 absolue). 2 expériences indépendantes permettent de calculer la moyenne des Cl50s.

### Etude de l'expression de pAKT dans les cellules humaines PC3 de carcinome de prostate mesurée par la technique MSD Multi-spot Biomarker Detection de Meso Scale Discovery (Test B):

Cet essai est basé sur la mesure de l'expression de la protéine AKT phosphorylée sur la sérine 473 (P-AKT-S473), dans la lignée de carcinome de prostate humaine PC3, par la technique basée sur un immuno-essai sandwich utilisant le kit MSD Multi-spot Biomarker Detection de Meso Scale Discovery: kits phospho-Akt (Ser473) whole cell lysate (#K151CAD) ou phospho-Akt (Ser473)/Total Akt whole cell lysate (#K151OOD). L'anticorps primaire spécifique de P-AKT-S473 (Kit #K151CAD) est coaté sur une électrode dans chaque puits des plaques 96 puits du kit MSD : après ajoût d'un lysat de protéines dans chaque puits, la révélation du signal se fait par l'addition d'un anticorps secondaire de détection marqué avec un composé électrochimioluminescent. La procédure suivie est celle décrite dans le kit.

Le jour 1, les cellules PC3 sont ensemencées en plaques 96 puits (TPP, #92096) à la concentration de 35000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2h à 37°C en présence de 5% CO2. Les molécules diluées dans du diméthylsulfoxyde (DMSO Sigma #D2650), sont ajoutées à partir d'une solution mère concentrée 20 fois, le pourcentage final de DMSO étant de 0.1 %. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Pour cela, après aspiration du milieu de culture, 50µl de tampon de lyse Tris Lysis Buffer complet du kit MSD contenant les solutions d'inhibiteurs de protéases et phosphatases sont ajoutés dans les puits et les cellules sont lysées pendant 1 H à 4°C sous agitation. A ce stade les plaques contenant les lysats peuvent être congelées à -20°C ou à -80°C.

Les puits des plaques 96 puits du kit MSD sont saturés pendant 1h à température ambiante avec la solution bloquante du kit MSD. Quatre lavages sont effectués avec 150µl de solution de lavage Tris Wash Buffer du kit MSD. Les lysats préparés précédemment sont transférés dans les plaques Multi-spot 96 puits du kit MSD et incubés pendant 1h à température ambiante, sous agitation. Quatre lavages sont effectués avec 150µl de solution de lavage Tris Wash Buffer du kit MSD. 25µl de la solution MSD sulfo-tag détection antibody sont ajoutés dans les puits et incubés pendant 1h à température ambiante, sous agitation. Quatre lavages sont effectués avec 150µl de solution de lavage Tris Wash Buffer du kit MSD. 150µl de tampon de révélation Read Buffer du kit MSD sont ajoutés dans les puits et les plaques sont lues immédiatement sur l'instrument S12400 de Meso Scale Discovery.

L'appareil mesure un signal pour chaque puits. Des puits sans cellules et contenant le tampon de lyse servent à déterminer le bruit de fond qui sera soustrait à toutes les mesures (min). Les puits contenant des cellules en absence de produit et en présence de 0.1 % DMSO sont considérés comme le 100 % de signal (max). Le calcul du pourcentage d'inhibition est fait pour chaque concentration de produit testé selon la formule suivante : (1- ((essai-min)/(max-min)))x100.

L'activité du produit est traduite en Cl₅₀, obtenue à partir d'une courbe dose-réponse de différentes concentrations testées et représentant la dose donnant 50 % d'inhibition spécifique (Cl₅₀ absolue). 2 expériences indépendantes permettent de calculer la moyenne des Cl₅₀ₛ.

Les résultats obtenus pour les produits en exemples dans la partie expérimentale sont donnés dans le tableau de résultats pharmacologiques ci-après:

**Tableau de résultats pharmacologiques :**

| exemple | Test A | Test B |
|---|---|---|
| Exemple 1 | 102 | |
| Exemple 2 | 321 | |
| Exemple 3 | 100 | |
| Exemple 4 | | 145 |
| Exemple 5 | | 51 |
| Exemple 6 | 23 | |
| Exemple 7 | | 23 |
| Exemple 8 | 318 | |
| Exemple 9 | 727 | |
| Exemple 10 | 3498 | |
| Exemple 11 | 632 | |
| Exemple 12 | 737 | |
| Exemple 13 | | 71 |
| Exemple 14 | 570 | 74 |
| Exemple 15 | 1420 | |
| Exemple 16 | 850 | |
| Exemple 17 | 1481 | |
| Exemple 18 | 23 | |
| Exemple 19 | | 42 |
| Exemple 20 | 1314 | |
| Exemple 21 | 10000 | |
| Exemple 22 | 305 | |
| Exemple 23 | 39 | |
| Exemple 24 | | 247 |
| Exemple 25 | | |
| Exemple 26 | 2287 | |
| Exemple 27 | | 71 |
| Exemple 28 | 171 | |
| Exemple 29 | 116 | |
| Exemple 30 | | 1030 |
| Exemple 31 | | 58 |
| Exemple 32 | 3635 | |
| Exemple 33 | 93 | |
| Exemple 34 | 609 | |
| Exemple 35 | 10000 | |
| Exemple 36 | 200 | |
| Exemple 37 | 10000 | |
| Exemple 38 | 480 | |
| Exemple 39 | 1763 | |
| Exemple 40 | 1494 | |
| Exemple 41 | 290 | 60 |
| Exemple 42 | 400 | 148 |
| Exemple 43 | 549 | |
| Exemple 44 | 308 | |
| Exemple 45 | 261 | |
| Exemple 46 | 2498 | |
| Exemple 47 | 346 | |
| Exemple 48 | 184 | |
| Exemple 49 | | 146 |
| Exemple 50 | 6721 | |
| Exemple 51 | 133 | |
| Exemple 52 | 227 | |
| Exemple 53 | | 66 |
| Exemple 54 | 807 | 67 |
| Exemple 55 | 10000 | |
| Exemple 56 | 137 | |
| Exemple 57 | 5865 | |
| Exemple 58 | 760 | 71 |
| Exemple 59 | 417 | 50 |
| Exemple 60 | | 290 |
| Exemple 61 | | 77 |
| Exemple 62 | 530 | |
| Exemple 63 | 6155 | |
| Exemple 64 | | 10000 |
| Exemple 65 | 6460 | |
| Exemple 66 | | 131 |
| Exemple 67 | 2207 | |
| Exemple 68 | 202 | 79 |
| Exemple 69 | 250 | |
| Exemple 70 | | 195 |
| Exemple 71 | 5375 | 120 |
| Exemple 72 | | 89 |
| Exemple 73 | 650 | |
| Exemple 74 | | 10 |
| Exemple 75 | | 69 |
| Exemple 76 | | 77 |
| Exemple 77 | | 698 |
| Exemple 78 | | 129 |
| Exemple 79 | | 20 |
| Exemple 80 | | 94 |
| Exemple 81 | | 1940 |
| Exemple 82 | | 133 |
| Exemple 83 | | 115 |
| Exemple 84 | | 17 |
| Exemple 85 | | 340 |
| Exemple 86 | | 26 |
| Exemple 87 | | 75 |
| Exemple 88 | | 5 |
| Exemple 89 | | 3 |
| Exemple 90 | | 14 |
| Exemple 91 | | 71 |
| Exemple 92 | | 57 |
| Exemple 93 | | 63 |
| Exemple 94 | | 11 |
| Exemple 95 | | 35 |
| Exemple 96 | | 6 |
| Exemple 97 | | 260 |
| Exemple 98 | | 219 |
| Exemple 99 | | 666 |
| Exemple 100 | | 3 |
| Exemple 101 | | 13 |
| Exemple 102 | | 43 |
| Exemple 103 | | 7 |
| Exemple 104 | | 42 |
| Exemple 105 | | 15 |
| Exemple 106 | | 27 |
| Exemple 107 | | 24 |
| Exemple 108 | | 56 |
| Exemple 109 | | 43 |
| Exemple 110 | | 278 |
| Exemple 111 | | 301 |
| Exemple 112 | | 23 |
| Exemple 113 | | 664 |
| Exemple 114 | | 26 |
| Exemple 115 | | 10 |
| Exemple 116 | | 51 |
| Exemple 117 | | 19 |
| Exemple 118 | | 12 |
| Exemple 119 | | 194 |
| Exemple 120 | | 11 |
| Exemple 121 | | 44 |
| Exemple 122 | | 503 |
| Exemple 123 | | 77 |
| Exemple 124 | | 124 |
| Exemple 125 | | 16 |
| Exemple 126 | | 57 |
| Exemple 127 | | 61 |
| Exemple 128 | | 69 |
| Exemple 129 | | 1000 |
| Exemple 130 | | 70 |
| Exemple 131 | | 41 |
| Exemple 132 | | 533 |
| Exemple 133 | | 174 |
| Exemple 134 | | 124 |
| Exemple 135 | | 1 |
| Exemple 136 | | 26 |
| Exemple 137 | | 68 |
| Exemple 138 | | 35 |
| Exemple 139 | | 3000 |
| Exemple 140 | | 302 |
| Exemple 141 | | 60 |
| Exemple 142 | | 22 |
| Exemple 143 | | 473 |
| Exemple 144 | | 432 |
| Exemple 145 | | 38 |
| Exemple 146 | | 180 |
| Exemple 147 | | 4 |
| Exemple 148 | | 234 |
| Exemple 149 | | 43 |
| Exemple 150 | | 189 |
| Exemple 151 | | 29 |
| Exemple 152 | | 938 |
| Exemple 153 | | 49 |
| Exemple 154 | | 28 |

| | | |
|---|---|---|
| Tests A et B : CI₅₀ (nM) | | |

## Revendications

1. Produits de formule (I): 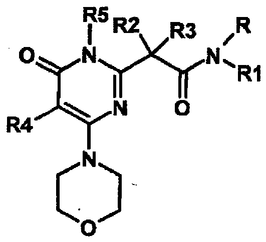 dans laquelle :
R1 représente un radical aryle ou hétéroaryle éventuellement substitués par un ou plusieurs radicaux identiques ou différents choisis parmi les atomes d'halogène et les radicaux hydroxyle, CN, nitro, -COOH, -COOalk, -NRxRy, -CONRxRy, - NRxCORy, -CORy, -NRxCO2Rz, alcoxy, phénoxy, alkylthio, alkyle, alkényle, alkynyle, cycloalkyle, O-cycloalkyle, hétérocycloalkyle, aryle et hétéroaryle;
ces derniers radicaux alcoxy, phénoxy, alkylthio, alkyle, alkényle, alkynyle, hétérocycloalkyle, aryle et hétéroaryle étant eux-mêmes éventuellement substitués par un ou plusieurs radicaux identiques ou différents choisis parmi les atomes d'halogène et les radicaux hydroxyle, alcoxy, NRvRw, hetérocycloalkyle ou hétéroaryle ;
les radicaux aryle et hétéroaryle étant de plus éventuellement substitués par un ou plusieurs radicaux alkyle et alcoxy eux-mêmes éventuellement substitués par un ou plusieurs atomes d'halogène ;
les radicaux hétérocycloalkyle et hétéroaryle pouvant de plus renfermer un radical oxo,
R représente un atome d'hydrogène ou bien forme avec R1 un cycle à 5 ou 6 chaînons saturé ou partiellement ou totalement insaturé fusionné à un reste aryle ou hétéroaryle et renfermant éventuellement un ou plusieurs autres hétéroatomes choisi(s) parmi O, S, N, NH et Nalk, ce radical bicyclique étant éventuellement substitué par un ou plusieurs radicaux identiques ou différents choisis parmi les atomes d'halogène et les radicaux CO-NH2, hydroxyle, alkyle et alcoxy; ce dernier radical alkyle étant lui-même éventuellement substitué par un radical hydroxyle, alcoxy, NH2, NHAlk ou N(alk)2 ;
R2, R3, identiques ou différents représentent indépendamment un atome d'hydrogène, un atome d'halogène ou un radical alkyle éventuellement substitué par un ou plusieurs atomes d'halogène ;
R4 représente un atome d'hydrogène;
R5 représente un atome d'hydrogène ou un radical alkyle éventuellement substitué par un ou plusieurs atomes d'halogène;
NRxRy étant tel que Rx représente un atome d'hydrogéne ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène, un radical cycloalkyle, CO2alk, ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
Rz représente les valeurs de Ry à l'exception de hydrogène ;
Rx, Ry et Rz dans les radicaux -NRxCORy, -CORy et NRxCO₂Rz étant choisis parmi les significations indiquées ci-dessus pour Rx, Ry, et Rz ;
tous les radicaux alkyle ou alk, alcoxy et alkylthio ci-dessus étant linéaires ou ramifiés et renfermant de 1 à 6 atomes de carbone,
lesdits produits de formule (I) étant sous toutes les formes stéréoisomères possibles racémiques, énantiomères et diastéréoisomères, ainsi que les sels d'addition avec les acides minéraux et organiques ou avec les bases minérales et organiques desdits produits de formule (I).

2. Produits de formule (I) tels que définis à la revendication 1 dans laquelle :
R1 représente un radical phényle, pyridine, thiényle, benzoxazolyle, benzofuryle, indazolyle, indolyle, benzothiényle, benzimidazolyle, benzoxazinyle, tetrahydroquinolyle, éventuellement substitués par un ou plusieurs radicaux identiques ou différents choisis parmi les atomes d'halogène et les radicaux CN, nitro, -COOH, -COOalk, -NRxRy, alcoxy, alkyle, alkynyle et cycloalkyle ;
ces derniers radicaux alcoxy, alkyle et alkynyle, étant eux-mêmes éventuellement substitués par un ou plusieurs radicaux identiques ou différents choisis parmi les atomes d'halogène et les radicaux hydroxyle, alcoxy, NRvRw, pipéridyle, pyrrolidinyle ou hétéroaryle;
les radicaux phényle et hétéroaryle étant de plus éventuellement substitués par un ou plusieurs radicaux alkyle et alcoxy ;
R représente un atome d'hydrogène ou bien forme avec R1 un cycle benzoxazinyle, dihydroindolyle, tetrahydroisoquinolyle, tetrahydroquinolyle, dihydropyrrolopyridyle, ces cycles étant éventuellement substitués par un ou plusieurs radicaux identiques ou différents choisis parmi les atomes d'halogène et les radicaux CO-NH2, hydroxyle, alkyle et alcoxy; ce dernier radical alkyle étant lui-même éventuellement substitué par un radical hydroxyle, alcoxy, NH2, NHAlk ou N(alk)2 ;
R2, R3, identiques ou différents représentent indépendamment un atome d'hydrogène, un atome de fluor ou un radical alkyle;
R4 représente un atome d'hydrogène;
R5 représente un atome d'hydrogène ou un radical alkyle;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène ou un radical alkyle;
tous les radicaux alkyle ou alk et alcoxy ci-dessus étant linéaires ou ramifiés et renfermant de 1 à 6 atomes de carbone,
lesdits produits de formule (I) étant sous toutes les formes stéréoisomères possibles racémiques, énantiomères et diastéréoisomères, ainsi que les sels d'addition avec les acides minéraux et organiques ou avec les bases minérales et organiques desdits produits de formule (I).

3. Produits de formule (I) tels que définis à l'une quelconque des revendications 1 ou 2, répondant aux formules suivantes :
- 2-[4-(morpholin-4-yl)-6-oxo-1,6-dihydropyrimidin-2-yl]-N-phénylacétamide
- N-(4-fluorophényl)-2-[4-(morpholin-4-yl)-6-oxo-1,6-dihydropyrimidin-2-yl]acétamide
- N-(3-chlorophényl)-2-[4-(morpholin-4-yl)-6-oxo-1,6-dihydropyrimidin-2-yl]acétamide
- N-[3-(diméthylamino)phényl]-2-[4-(morpholin-4-yl)-6-oxo-1,6-dihydropyrimidin-2-yl]acétamide
- N-(2,4-difluorophényl)-2-[4-(morpholin-4-yl)-6-oxo-1,6-dihydropyrimidin-2-yl]acétamide
- N-(3,4-difluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(thiophén-3-yl)acétamide
- N-(4-fluoro-3-méthoxyphényl)-2-[4-(morpholin-4-yl)-6-oxo-1,6-dihydropyrimidin-2-yl]acétamide
- N-(2-fluorophényl)-2-[4-(morpholin-4-yl)-6-oxo-1,6-dihydropyrimidin-2-yl]acétamide
- N-(2-méthylphényl)-2-[4-(morpholin-4-yl)-6-oxo-1,6-dihydropyrimidin-2-yl]acétamide
- N-(2-méthoxyphényl)-2-[4-(morpholin-4-yl)-6-oxo-1,6-dihydropyrimidin-2-yl]acétamide
- N-(2,3-difluorophényl)-2-[4-(morpholin-4-yl)-6-oxo-1,6-dihydropyrimidin-2-yl]acétamide
- N-(3,5-difluorophényl)-2-[4-(morpholin-4-yl)-6-oxo-1,6-dihydropyrimidin-2-yl]acétamide
- N-(3-fluorophényl)-2-[4-(morpholin-4-yl)-6-oxo-1,6-dihydropyrimidin-2-yl]acétamide
- N-(4-chlorophényl)-2-[4-(morpholin-4-yl)-6-oxo-1,6-dihydropyrimidin-2-yl]acétamide
- N-(3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhyl)phényl]acétamide
- N-(3-bromophényl)-2-[4-(morpholin-4-yl)-6-oxo-1,6-dihydropyrimidin-2-yl]acétamide
- N-[3-(2-méthylpropan-2-yl)phényl]-2-[4-(morpholin-4-yl)-6-oxo-1,6-dihydropyrimidin-2-yl]acétamide
- 3-({[4-(morpholin-4-yl)-6-oxo-1,6-dihydropyrimidin-2-yl]acétyl}amino)benzoate de méthyle
- acide 3-({[4-(morpholin-4-yl)-6-oxo-1,6-dihydropyrimidin-2-yl]acétyl}amino)benzoïque
- 2-[4-(morpholin-4-yl)-6-oxo-1,6-dihydropyrimidin-2-yl]-N-[3-(propan-2-yl)phényl]acétamide
- N-(3-méthylphényl)-2-[4-(morpholin-4-yl)-6-oxo-1,6-dihydropyrimidin-2-yl]cétamide
- N-(3-cyano-4-fluorophényl)-2-[4-(morpholin-4-yl)-6-oxo-1,6-dihydropyrimidin-2-yl]acétamide
- N-(1H-indazol-6-yl)-2-[4-(morpholin-4-yl)-6-oxo-1,6-dihydropyrimidin-2-yl]acétamide
- N-(3-cyanophényl)-2-[4-(morpholin-4-yl)-6-oxo-1,6-dihydropyrimidin-2-yl]acétamide
- N-(5-fluoropyridin-2-yl)-2-[4-(morpholin-4-yl)-6-oxo-1,6-dihydropyrimidin-2-yl]acétamide
- N-(4-fluoro-3-méthylphényl)-2-[4-(morpholin-4-yl)-6-oxo-1,6-dhydropyramidin-2-yl]acétamide
- N-(3-chloro-4-fluorophényl)-2-[4-(morphoùn-4-yl)-6-oxo-1,6-dihydropyàmidin-2-yl]acétamide
- 2-[4-(morpholin-4-yl)-6-oxo-1,6-dihydropyrimidin-2-yl]-N-pyridin-3-yl)acétamide
- N-(4-fluoro-2-méthylphényl)-2-[4-(morpholin-4-yl)-6-oxo-1,6-dihydropyrimidin-2-yl]acétamide
- N-(3-hydroxyphényl)-2-[4-(morpholin-4-yl)-6-oxo-1,6-dihydropyàmidin-2-yl]acétamide
- N-(3-bromo-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(3,4,5-trifluorophényl)acétamide
- N-[4-fluoro3-(hydroxyméthyl)phényl]-2-[4-(morpholin-4-yl)-6-oxo-1,6-dihydropyrimidin-2-yl]acétamide
- N-(3-cyclopropylephényl)-2-[4-(morpholin-4-yl)-6-oxo-1,6-dihydropyrimidin-2-yl]acétamide
- N-(2-hydroxyphényl)-2-[4-(morpholin-4-yl)-6-oxo-1,6-dihydropyrimidin-2-yl]acétamide
- N-[3-(difluorométhoxy)phényl]-2-[4-(morpholin-4-yl)-6-oxo-1,6-dihydropyrimidin-2-yl]acétamide
- N-(4-fluoro-3-méthoxyphényl)-2-[4-(morpholin-4-yl)-6-oxo-1,6-dihydropyrimidin-2-yl]propanamide
- N-(2,3-diméthylphényl)-2-[4-(morpholin-4-yl)-6-oxo-1,6-dihydropyrimidin-2-yl]acétamide
- N-(2-fluoro-3-méthylphényl)-2-[4-(morpholin-4-yl)-6-oxo-1,6-dihydropyrimidin-2-yl]acétamide
- N-(1,3-benzoxazol-4-yl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(trifluorométhoxy)phényl]acétamide
- 2-[4-(morpholin-4-yl)-6-oxo-1,6-dihydropyrimidin-2-yl]-N-[3-(propan-2-yloxy)phényl]acétamide
- N-(4-fluoro-2-méthoxyphényl)-2-[4-(morpholin-4-yl)-6-oxo-1,6-dihydropyrimidin-2-yl]acétamide
- {2-[3-({[4-(morpholin-4-yl)-6-oxo-1,6-dihydropyrimidin-2-yl]acétyl}amino)phényl]éthyl}carbamate de 2-méthylpropan-2-yle
- N-[4-fluoro3-fluorométhyl)phényl]-2-[4-(morpholin-4-yl)-6-oxo-1,6-dihydropyrimidin-2-yl]acétamide
- N-(3-éthynylphényl)-2-[4-(morpholin-4-yl)-6-oxo-1,6-dihydropyrimidin-2-yl]acétamide
- N-[3-(cyclopentyloxy)phényl]-2-[4-(morpholin-4-yl)-6-oxo-1,6-dihydropyrimidin-2-yl]acétamide
- N-(4-fluoro-2-hydroxyphényl)-2-[4-(morpholin-4-yl)-6-oxo-1,6-dihydropyrimidin-2-yl]acétamide
- 2-[2-(2,3-dihydro-1H-indol-1-yl)-2-oxoéthyl]-6-(morpholin-4-yl)pyrimidin-4(3H)-one
- N-(3-cyclopropyl-4-fluorophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,3,4-trifluorophényl)acétamide
- N-[4-fluoro3-(trifluorométhoxy)phényl]-2-[4-(morpholin-4-yl)-6-oxo-1,6-dihydropyrimidin-2-yl]acétamide
- N-[3-(2-hydroxyéthoxy)phényl]-2-[4-(morpholin-4-yl)-b-oxo-1,6-dihydropyrimidin-2-yl]acétamide
- N-(3-iodophényl)-2-[4-(morpholin-4-yl)-6-oxo-1,6-dihydropyrimidin-2-yl]acétamide
- 2-fluoro-5-({[4-(morpholin-4-yl)-6-oxo-1,6-dihydropyrimidin-2-yl]acétyl]amino)benzoate de méthyle
- N-(3-éthoxyphényl)-2-[4-(morpholin-4-yl)-6-oxo-1,6-dihydropyrimidin-2-yl]acétamide
- N-(2,4-difluoro-3-méthoxyphényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-(2,4,5-trifluorophényl)acétamide
- N-(3,5-dichloro-4-fluorophényl)-2-[4-(morpholin-4-yl)-6-oxo-1,6-dihydropyrimidin-2-yl]acétamide
- 2-[2-(2,3-dihydro-4H-1,4-benzoxazin-4-yl)-2-oxoéthyl]-6-(morpholin-4-yl)pyrimidin-4(3H)-one
- N-(4-fluoro-3-nitrophényl)-2-[4-(morpholin-4-yl)-6-oxo-1,6-dihydropyrimidin-2-yl]acétamide
- acide 2-fluoro-5-({[4-(morpholin-4-yl)-6-oxo-1,6-dihydropyrimidin-2-yl]acétyl}amino)benzoïque
- N-(5-fluoro-2-hydroxyphényl)-2-[4-(morpholin-4-yl)-6-oxo-1,6-dihydropyrimidin-2-yl]acétamide
- N-(2-bromo-4-fluorophényl)-2-[4-(morpholin-4-yl)-6-oxo-1,6-dihydropyrimidin-2-yl]acétamide
- N-(4-fluorophényl)-2-[1-méthyl-4-(morpholin-4-yl)-6-oxo-1,6-dihydropyrimidin-2-yl]acétamide
- N-(3-chloro-4-fluorophényl)-2-[1-méthyl-4-(morpholin-4-yl)-6-oxo-1,6-dihydropyrimidin-2-yl]acétamide
- N-(3-bromophényl)-2-[1-méthyl-4-(morpholin-4-yl)-6-oxo-1,6-dihydropyrimidin-2-yl]acétamide
- 2-[1-éthyl-4-(morpholin-4-yl)-6-oxo-1,6-dihydropyrimidin-2-yl]-N-(4-fluorophényl)acétamide
- N-(1H-indol-4-yl)-2-(4-(morpholin-4-yl)-6-oxo-1,6-dihydropyrimidin-2-yl]acétamide
- N-(4-fluorophényl)-3-méthyl-2-[4-(morpholin-4-yl)-6-oxo-1,6-dihydropyrimidîn-2-yl]butanamide
- N-[4-fluoro3-(méthoxyméthyl)phényl]-2-[4-(morpholin-4-yl)-6-oxo-1,6-dihydropyrimidin-2-yl]acétamide
- N-(4-fluoro-3-iodophényl)-2-[4-(morpholin-4-yl)-6-oxo-1,6-dihydropyrimidin-2-yl]acétamide
- 2-[4-(morpholin-4-yl)-6-oxo-1,6-dihydropyrimidin-2-yl]-N-[3-(1,1,2,2-tétrafluoroéthoxy)phényl]acétamide
- N-[3-(difluorométhyl)-4-fluorophényl]-2-[4-(morpholin-4-yl)-6-oxo-1,6-dihydropyrimidin-2-yl]acétamide
- 2,2-difluoro-N-(4-fluorophényl)-2-[4-(morpholin-4-yl)-6-oxo-1,6-dihydropyrimidin-2-yl]acétamide
- N-(3,4-difluorophényl)-2-[1-méthyl-4-(morpholin-4-yl)-6-oxo-1,6-dihydropyrimidin-2-yl]acétamide
- 2-[2-(2,3-dihydro-1H-indol-1-yl)-2-oxoéthyl]-3-méthyl-6-(morpholin-4-yl)pyrimidin-4(3H)-one
- N-(3-bromo-4-fluorophényl)-2-[1-méthyl-4-(morpholin-4-yl)-6-oxo-1,6-dihydropyrimidin-2-yl]acétamide
- N-[4-fluoro3-(hydroxyméthyl)phényl]-2-[1-méthyl-4-(morpholin-4-yl)-6-oxo-1,6-dihydropyrimidin-2-yl]acétamide
- N-(3-cyclopropylphényl)-2-[1-méthyl-4-(morpholin-4-yl)-6-oxo-1,6-dihydropyrimidin-2-yl]acétamide.
- N-(4-fluoro-3-méthoxyphényl)-2-[1-méthyl-4-(morpholin-4-yl)-6-oxo-1,6-dihydropyrimidin-2-yl]acétamide
- N-(1-benzofur-4-yl)-2-[4-(morpholin-4-yl)-6-oxo-1,6-dihydropyrimidin-2-yl]acétamide
- 2-[1-méthyl-4-(morpholin-4-yl)-6-oxo-1,6-dihydropyrimidin-2-yl]-N-phénylacétamide
- N-(3-cyclopropyl-4-fluorophényl)-2-[1-méthyl-4-(morpholin-4-yl)-6-oxo-1,6-dihydropyrimidin-2-yl]acétamide
- N-(3-fluoro-2-hydroxyphényl)-2-[4-(morpholin-4-yl)-6-oxo-1,6-dihydropyrimidin-2-yl]acétamide
- 2-[2-(4-fluoro-2,3-dihydro-1H-indol-1-yl)-2-oxoéthyl]-6-(morpholin-4-yl)pyrimidin-4(3H)-one
- 2-[2-(4-chloro-2,3-dihydro-1H-indol-1-yl)-2-oxoéthyl]-6-(morpholin-4-yl)pyrimidin-4(3H)-one
- N-(3-éthynyl-4-fluorophényl)-2-[4-(morpholin-4-yl)-6-oxo-1,6-dihydropyrimidin-2-yl]acétamide
- 2-[2-(4-hydroxy-2,3-dihydro-1H-indol-1-yl)-2-oxoéthyl]-6-(morpholin-4-yl)pyrimidin-4(3H)-one
- 2-[2-(4,6-difluoro-2,3-dihydro-1H-indol-1-yl)-2-oxoéthyl]-6-(morpholin-4-yl)pyrimidin-4(3H)-one
- N-(4-Fluoro-3-iodo-phenyl)-2-(1-methyl-4-morpholin-4-yl-6-oxo-1,6-dihydro-pyrimidin-2-yl)-acetamide
- 2-[2-(4,5-difluoro-2,3-dihydro-1H-indol-1-yl)-2-oxoéthyl]-6-(morpholin-4-yl)pynmidin-4(3H)-one
- 2-[2-(6-fluoro-2,3-dihydro-1H-indol-1-yl)-2-oxoéthyl]-6-(morpholin-4-yl)pyrimidin-4(3H)-one
- 2-[2-(2-méthyl-2,3-dihydro-1H-indol-1-yl)-2-oxoéthyl]-6-(morpholin-4-yl)pyrimidin-4(3H)-one
- N-[3-(difluorométhyl)-4-fluorophényl]-2-[1-méthyl-4-(morpholin-4-yl)-6-oxo-1,6-dihydropyrimidin-2-yl]acétamide
- 2-[1-methyl-4-(morpholin-4-yl)-6-oxo-1,6-dihydropyrimidin-2-yl]-N-(3,4,5-trifluorophenyl)acetamide
- N-(1-methyl-1H-indol-4-yl)-2-[4-(morpholin-4-yl)-6-oxo-1,6-dihydropyrimidin-2-yl]acetamide
- 2-[2-(4-methyl-2,3-dihydro-1H-indol-1-yl)-2-oxoethyl]-6-(morpholin-4-yl)pyrimidin-4(3H)-one
- 2-[2-(3-methyl-2,3-dihydro-1H-indol-1-yl)-2-oxoethyl]-6-(morpholin-4-yl)pyrimidin-4(3H)-one
- 2-[2-(4-fluoro-2,3-dihydro-1H-indol-1-yl)-2-oxoethyl]-3-methyl-6-(morpholin-4-yl)pyrimidin-4(3H)-one
- 2-[2-(5-Fluoro-2,3-dihydro-indol-1-yl)-2-oxo-ethyl]-6-morpholin-4-yl-3H-pyrimidin-4-one
- 2-[2-(4-chloro-2,3-dihydro-1H-indol-1-yl)-2-oxoethyl]-3-methyl-6-(morpholin-4-yl)pyrimidin-4(3H)-one
- N-(1-benzothiophen-4-yl)-2-[4-(morpholin-4-yl)-6-oxo-1,6-dihydropyrimidin-2-yl]acetamide
- 2-{2-[2-(hydroxymethyl)-2,3-dihydro-1H-indol-1-yl]-2-oxoethyl}-6-(morpholin-4-yl)pyrimidin-4(3H)-one
- 2-[4-(morpholin-4-yl)-6-oxo-1,6-dihydropyrimidin-2-yl]-N-{2-[2-(piperidin-1-yl)ethoxy]phenyl]acetamide
- N-[2-(2-methoxyethoxy)phenyl]-2-[4-(morpholin-4-yl)-6-oxo-1,6-dihydropyrimidin-2-yl]acetamide
- 2-[2-(4-hydroxy-2,3-dihydro-1H-indol-1-yl)-2-oxoethyl]-3-methyl-6-(morpholin-4-yl)pyrimidin-4(3H)-one
- 2-[2-(4-methoxy-2,3-dihydro-1H-indol-1-yl)-2-oxoethyl]-3-methyl-6-(morpholin-4-yl)pyrimidin-4(3H)-one
- 2-[2-(3,4-dihydroisoquinolin-2(1H)-yl)-2-oxoethyl]-6-(morpholin-4-yl)pyrimidin-4(3H)-one
- 2-[4-(morpholin-4-yl)-6-oxo-1,6-dihydropyrimidin-2-yl]-N-{2-[2-(pyrrolidin-1-yl)ethoxy]phenyl}acetamide
- 2-[4-(morpholin-4-yl)-6-oxo-1,6-dihydropyrimidin-2-yl]-N-[2-(pyridin-3-ylmethoxy)phenyl]acetamide
- 3-methyl-2-[2-(4-methyl-2,3-dihydro-1H-indol-1-yl)-2-oxoethyl]-6-(morpholin-4-yl)pyrimidin-4(3H)-one
- 2-(2-{3-[(dimethylamino)methyl]-2,3-dihydro-1H-indol-1-yl}-2-oxoethyl)-6-(morpholin-4-yl)pyrimidin-4(3H)-one
- 2-[2-(4-bromo-2,3-dihydro-1H-indol-1-yl)-2-oxoethyl]-3-methyl-6-(morpholin-4-yl)pyrimidin-4(3H)-one
- 2-{2-[(2S)-2-methyl-2,3-dihydro-1H-indol-1-yl]-2-oxoethyl}-6-(morpholin-4-yl)pyrimidin-4(3H)-one
- 2-{2-[(2R)-2-methyl-2,3-dihydro-1H-indol-1-yl]-2-oxoethyl}-6-(morpholin-4-yl)pyrimidin-4(3H)-one
- 3-methyl-2-[2-(3-methyl-2,3-dihydro-1H-indol-1-yl)-2-oxoethyl]-6-(morpholin-4-yl)pyrimidin-4(3H)-one
- 2-{2-[2-(methoxymethyl)-2,3-dihydro-1H-indol-1-yl]-2-oxoethyl}-6-(morpholin-4-yl)pyrimidin-4(3H)-one
- 2-[2-(4-ethoxy-2,3-dihydro-1H-indol-1-yl)-2-oxoethyl]-6-(morpholin-4-yl)pyrimidin-4(3H)-one
- 1-{[4-(morpholin-4-yl)-6-oxo-1,6-dihydropyrimidin-2-yl]acetyl}-2,3-dihydro-1H-indole-2-carboxamide
- 3-methyl-2-[2-(2-methyl-2,3-dihydro-1H-indol-1-yl)-2-oxoethyl]-6-(morpholin-4-yl)pyrimidin-4(3H)-one
- 2-[2-(6-fluoro-2,3-dihydro-1H-indol-1-yl)-2-oxoethyl]-3-methyl-6-(morpholin-4-yl)pyrimidin-4(3H)-one
- 2-{2-[(3S)-3-methyl-2,3-dihydro-1H-indol-1-yl]-2-oxoethyl}-6-(morpholin-4-yl)pyrimidin-4(3H)-one
- 2-{2-[(3R)-3-methyl-2,3-dihydro-1H-indol-1-yl]-2-oxoethyl}-6-(morpholin-4-yl)pyrimidin-4(3H)-one
- 2-[2-(5,6-difluoro-2,3-dihydro-1H-indol-1-yl)-2-oxoethyl]-3-methyl-6-(morpholin-4-yl)pyrimidin-4(3H)-one
- 2-[2-(4,5-difluoro-2,3-dihydro-1H-indol-1-yl)-2-oxoethyl]-3-methyl-6-(morpholin-4-yl)pyrimidin-4(3H)-one
- 2-[2-(1,3-dihydro-2H-isoindol-2-yl)-2-oxoethyl]-6-(morpholin-4-yl)pyrimidin-4(3H)-one
- N-(1-benzothiophen-4-yl)-2-[1-methyl-4-(morpholin-4-y1)-6-oxo-1,6-dihydropyrimidin-2-yl]acetamide
- 2-[2-(5-chloro-3,4-dihydroquinolin-1(2H)-yl)-2-oxoethyl]-6-(morpholin-4-yl)pyrimidin-4(3H)-one
- 2-{2-[4-(hydroxymethyl)-2,3-dihydro-1H-indol-1-yl]-2-oxoethyl}-6-(morpholin-4-yl)pyrimidin-4(3H)-one
- N-[4-fluoro-2-(piperidin-4-ylmethoxy)phenyl]-2-[4-(morpholin-4-yl)-6-oxo-1,6-dihydropyrimidin-2-yl]acetamide
- 2-[2-(5-Chloro-2,3-dihydro-indol-1-yl)-2-oxo-ethyl]-6-morpholin-4-yl-3H-pyrimidin-4-one
- 2-[2-(4-Bromo-2,3-dihydro-indol-1-yl)-2-oxo-ethyl]-6-morpholin-4-yl-3H-pyrimidin-4-one
- 2-(2-{(3S)-3-[(dimethylamino)methyl]-2,3-dihydro-1H-indol-1-yl}-2-oxoethyl)-6-(morpholin-4-yl)pyrimidin-4(3H)-one
- 2-(2-{(3R)-3-[(dimethylamino)methyl]-2,3-dihydro-1H-indol-1-yl}-2-oxoethyl)-6-(morpholin-4-yl)pyrimidin-4(3H)-one
- N-[4-fluoro-2-(2-methoxyethoxy)phenyl]-2-[4-(morpholin-4-yl)-6-oxo-1,6-dihydropyrimidin-2-yl]acetamide
- N-(1H-benzimidazol-4-yl)-2-[4-(morpholin-4-yl)-6-oxo-1,6-dihydropyrimidin-2-yl]acetamide
- methyl 2-hydroxy-3-({[4-(morpholin-4-yl)-6-oxo-1,6-dihydropyrimidin-2-yl]acetyl}amino)benzoate
- 2-[2-(4-Methoxy-2,3-dihydro-indol-1-yl)-2-oxo-ethyl]-6-morpholin-4-yl-3H-pyrimidin-4-one
- N-(3-bromo-2-hydroxyphenyl)-2-[4-(morpholin-4-yl)-6-oxo-1,6-dihydropyrimidin-2-yl]acetamide
- N-(3,4-dihydro-2H-1,4-benzoxazin-8-yl)-2-[4-(morpholin-4-yl)-6-oxo-1,6-dihydropyrimidin-2-yl]acetamide
- methyl 5-fluoro-2-({[4-(morpholin-4-yl)-6-oxo-1,6-dihydropyrimidin-2-yl]acetyl}amino)benzoate
- 2-(2-{3-[(diethylamino)methyl]-2,3-dihydro-1H-indol-1-yl}-2-oxoethyl)-6-(morpholin-4-yl)pyrimidin-4(3H)-one
- 2-[2-(2,3-dihydro-1H-pyrrolo[3,2-b]pyridin-1-yl)-2-oxoethyl]-6-(morpholin-4-yl)pyrimidin-4(3H)-one
- 2-[2-(5,6-Difluoro-2,3-dihydro-indol-1-yl)-2-oxo-ethyl]-6-morpholin-4-yl-3H-pyrimidin-4-one
- 2-[4-(morpholin-4-yl)-6-oxo-1,6-dihydropyrimidin-2-yl]-N-(1,2,3,4-tetrahydroquinolin-8-yl)acetamide
- 2-[2-(8-chloro-2,3-dihydro-4H-1,4-benzoxazin-4-yl)-2-oxoéthyl]-6-(morphohn-4-yl)pyrimidin-4(3H)-one
- N-(2-hydroxy-3-méthylphényl)-2-[4-(morpholin-4-yl)-6-oxo-1,6-dihydropyrimidin-2-yl]acétamide
- N-(2-hydroxy-3-nitophényl)-2-[4-(morpholin-4-yl)-6-oxo-1,6-dihydropyrimidin-2-yl]acétamide
- N-(3-cyano-2-hydroxyphényl)-2-[4-{morpholin-4-yl)-6-oxo-1,6-dihydropyrimidin-2-yl]acétamide
- N-[2-hydroxy-3-(trifluorométhyl)phényl]-2-[4-(morpholin-4-yl)-6-oxo-1,6-dihydropyrimidin-2-yl]acétamide
- 2-[2-(3,3-diméthyl-2,3-dihydro-1H-indol-1-yl)-2-oxoéthyl]-6-(morpholin-4-yl)pyrimidin-4(3H)-one
ainsi que les sels d'addition avec les acides minéraux et organiques ou avec les bases minérales et organiques desdits produits de formule (I).

4. Procédé de préparation des produits de formule (I) tels que définis à l'une quelconque des revendications 1 à 3 selon le schéma 1A tel que défini ci-après. 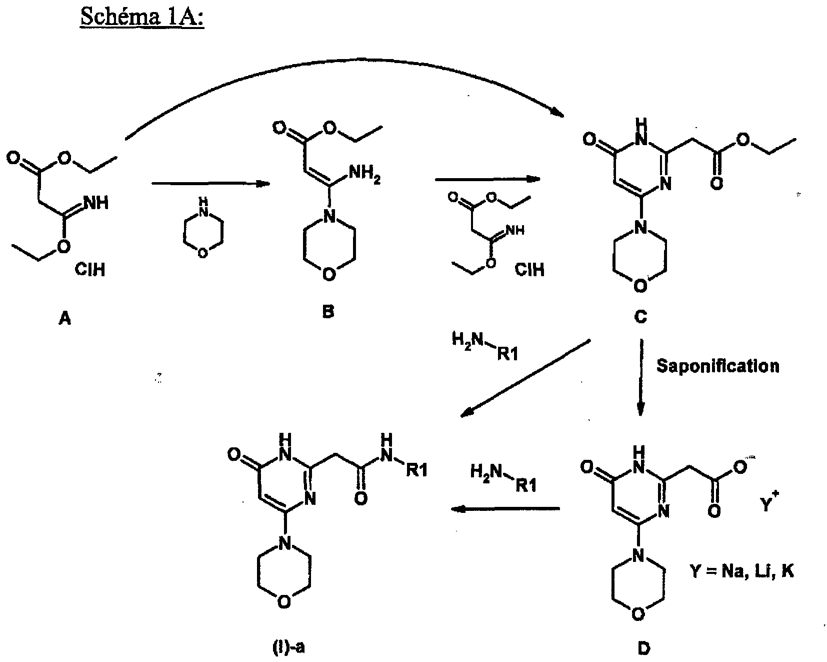 dans lequel le substituant R1 a les significations indiquées à l'une quelconque des revendications 1 ou 2.

5. Procédé de préparation des produits de formule (I) tels que définis à l'une quelconque des revendications 1 à 3 selon le schéma 1B tel que défini ci-après. 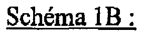 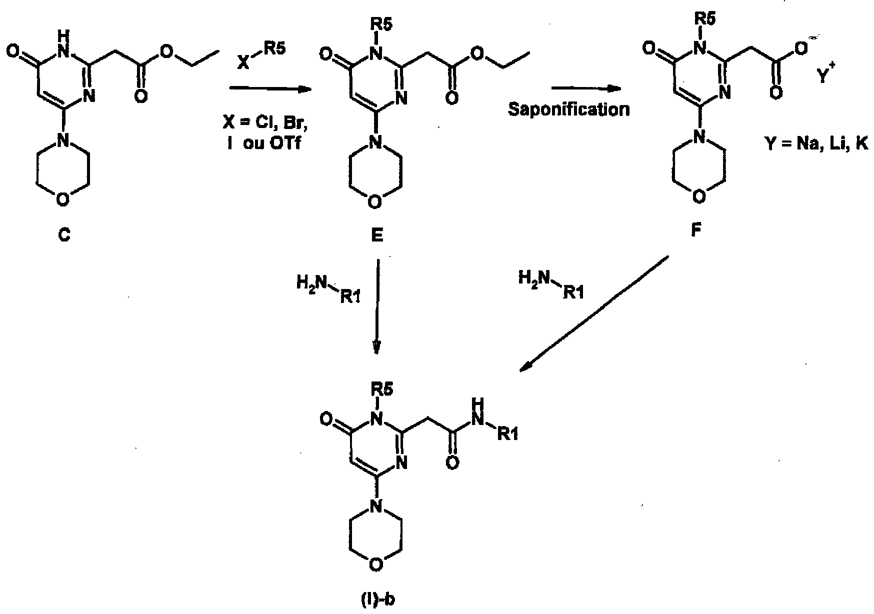 dans lequel les substituants R1 et R5 ont les significations indiquées à l'une quelconque des revendications 1 ou 2.

6. Procédé de préparation des produits de formule (I) tels que définis à l'une quelconque des revendications 1 à 3 selon le schéma 1C tel que défini ci-après. 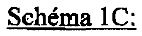 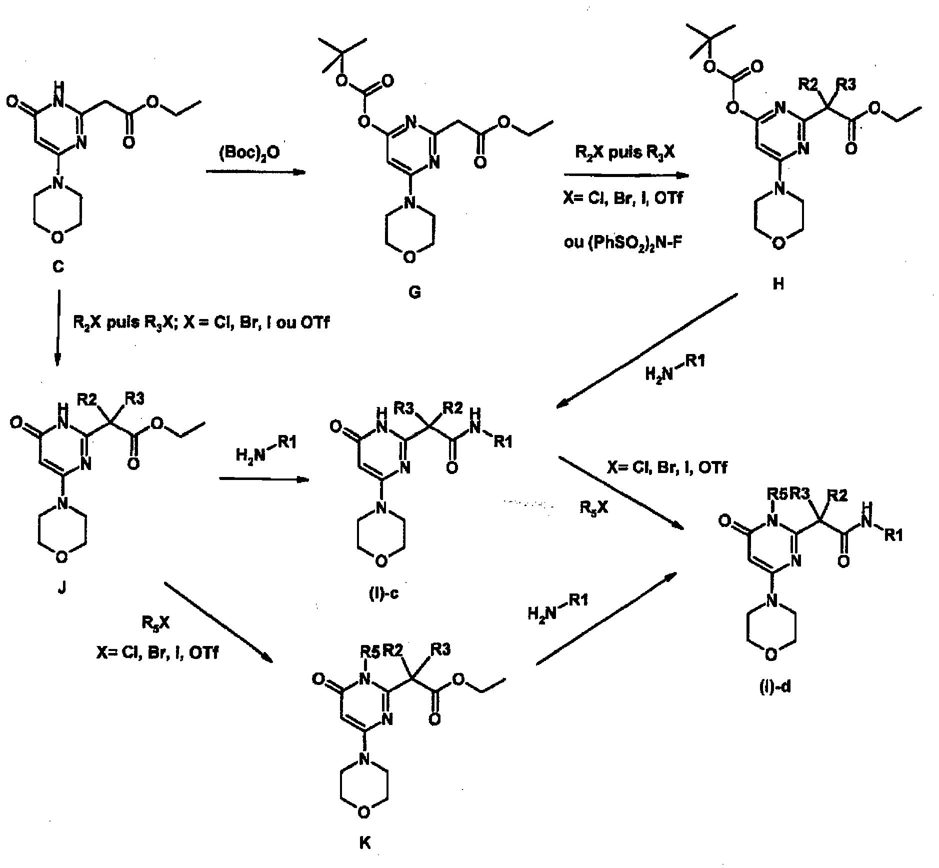 dans lequel les substituants R1, R2, R3 et R5 ont les significations indiquées à l'une quelconque des revendications 1 ou 2.

7. Produits de formule (I) telle que définie à l'une quelconque des revendications 1 à 3, ainsi que les sels d'addition avec les acides minéraux et organiques ou avec les bases minérales et organiques pharmaceutiquement acceptables desdits produits de formule (I), pour leur utilisation en tant que médicaments.

8. Produits de formule (I) telle que définie à la revendication 3, ainsi que les sels d'addition avec les acides minéraux et organiques ou avec les bases minérales et organiques pharmaceutiquement acceptables desdits produits de formule (I), pour leur utilisation en tant que médicaments.

9. Compositions pharmaceutiques contenant à titre de principe actif l'un au moins des produits de formule (I) tel que défini à l'une quelconque des revendications 1 à 3, ou un sel pharmaceutiquement acceptable de ce produit et un support pharmaceutiquement acceptable.

10. Produits de formule (I) tels que définis à l'une quelconque des revendications 1 à 3 pour leur utilisation pour le traitement de cancers.

11. Produits de formule (I) tels que définis à l'une quelconque des revendications 1 à 3 pour leur utilisation pour le traitement de tumeurs solides ou liquides.

12. Produits de formule (I) tels que définis à l'une quelconque des revendications 1 à 3 pour leur utilisation pour le traitement de cancers résistant à des agents cytotoxiques.

13. Produits de formule (I) tels que définis à l'une quelconque des revendications 1 à 3 pour leur utilisation pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

14. Produits de formule (I) tels que définis à l'une quelconque des revendications 1 à 3 pour leur utilisation pour la chimiothérapie de cancers.

15. Produits de formule (I) tels que définis à l'une quelconque des revendications 1 à 3 pour leur utilisation pour la chimiothérapie de cancers, seuls ou en en association:

16. Produits de formule (I) tels que définis à l'une quelconque des revendications 1 à 3 comme inhibiteurs de phosphorylation de AKT(PKB).

17. Intermédiaires de synthèse de formules C, D, E et F tels que définis à la revendication 4 et rappelés ci-après : 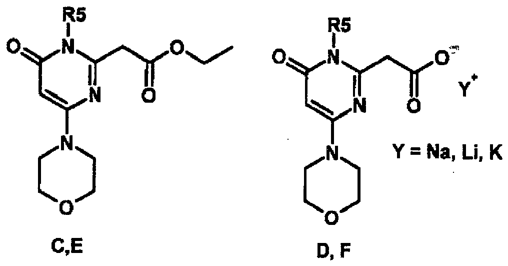 dans lesquels R5 a la définition indiquée à l'une quelconque des revendications 1 à 2.

## Patentansprüche

1. Produkte der Formel (I): 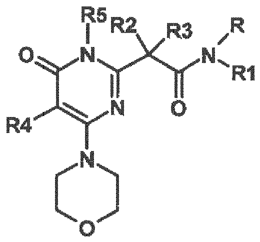 worin:
R1 für einen Aryl- oder Heteroarylrest steht, der gegebenenfalls durch einen oder mehrere gleiche oder verschiedene Reste, die aus Halogenatomen und Hydroxyl-, CN-, Nitro-, -COOH-, -COOalk-, -NRxRy-, -CONRxRy-, -NRxCORy-, -CORy-, -NRxCO₂Rz-, Alkoxy-, Phenoxy-, Alkylthio-, Alkyl-, Alkenyl-, Alkinyl-, Cycloalkyl-, O-Cycloalkyl-, Heterocycloalkyl-, Aryl- un Heteroarylresten ausgewählt sind, substituiert sind;
wobei diese letzteren Alkoxy-, Phenoxy-, Alkylthio-, Alkyl-, Alkenyl-, Alkinyl-, Heterocycloalkyl-, Aryl- und Heteroarylreste selbst gegebenenfalls durch einen oder mehrere gleiche oder verschiedene Reste, die aus Halogenatomen und Hydroxyl-, Alkoxy-, NRvRw-, Heterocycloalkyl- oder Heteroarylresten ausgewählt sind, substituiert sind;
wobei die Aryl- und Heteroarylreste außerdem gegebenenfalls durch einen oder mehrere Alkyl- und Alkoxyreste, die selbst gegebenenfalls durch ein oder mehrere Halogenatome substituiert sind, substituiert sind;
wobei die Heterocycloalkyl- und Heteroarylreste außerdem einen Oxorest enthalten können;
R für ein Wasserstoffatom steht oder mit R1 einen gesättigten oder teilweise oder vollständig ungesättigten 5- oder 6-gliedrigen Ring bildet, der an einen Aryl- oder Heteroarylrest ankondensiert ist und gegebenenfalls ein oder mehrere weitere Heteroatome, die aus O, S, N, NH und Nalk ausgewählt sind, enthält, wobei dieser bicyclische Rest gegebenenfalls durch einen oder mehrere gleiche oder verschiedene Reste, die aus Halogenatomen und CO-NH₂-, Hydroxyl-, Alkyl- und Alkoxyresten ausgewählt sind, substituiert ist; wobei dieser letztere Alkylrest selbst gegebenenfalls durch einen Hydroxyl-, Alkoxy-, NH₂-, NHalk- oder N(alk)₂-Rest substituiert ist;
R2 und R3 gleich oder verschieden sind und unabhängig voneinander für ein Wasserstoffatom, ein Halogenatom oder einen Alkylrest, der gegebenenfalls durch ein oder mehrere Halogenatome substituiert ist, stehen;
R4 für ein Wasserstoffatom steht;
R5 für ein Wasserstoffatom oder einen Alkylrest, der gegebenenfalls durch ein oder mehrere Halogenatome substituiert ist, steht;
wobei NRxRy so beschaffen ist, dass Rx für ein Wasserstoffatom oder einen Alkylrest steht und Ry für ein Wasserstoffatom, einen Cycloalkylrest oder einen Alkylrest, der gegebenenfalls durch einen oder mehrere gleiche oder verschiedene Reste, die aus Hydroxyl-, Alkoxy-, NRvRw- und Heterocycloalkylresten ausgewählt sind, substituiert ist, steht; oder Rx und Ry mit dem Stickstoffatom, an das sie gebunden sind, einen 3- bis 10-gliedrigen cyclischen Rest bilden, der gegebenenfalls ein oder mehrere aus O, S, NH und N-Alkyl ausgewählte Heteroatome enthält und gegebenenfalls substituiert ist;
wobei NRvRw so beschaffen ist, dass Rv für ein Wasserstoffatom oder einen Alkylrest steht und Rw für ein Wasserstoffatom, einen Cycloalkylrest, CO₂alk oder einen Alkylrest, der gegebenenfalls durch einen oder mehrere gleiche oder verschiedene Reste, die aus Hydroxyl-, Alkoxy- und Heterocycloalkylreste ausgewählt sind, substituiert ist, steht; oder Rv und Rw mit dem Stickstoffatom, an das sie gebunden sind, einen 3- bis 10-gliedrigen cyclischen Rest bilden, der gegebenenfalls ein oder mehrere aus O, S, NH und N-Alkyl ausgewählte Heteroatome enthält und gegebenenfalls substituiert ist;
wobei die cyclischen Reste, die Rx und Ry bzw. Rv und Rw mit dem Stickstoffatom, an das sie gebunden sind, bilden können, gegebenenfalls durch einen oder mehrere gleiche oder verschiedene Reste, die aus Halogenatomen und Alkyl-, Hydroxyl-, Oxo-, Alkoxy-, NH₂-, Nhalk- und N(alk)₂-Resten ausgewählt sind, substituiert sind;
Rz für die Werte von Ry mit Ausnahme von Wasserstoff steht;
wobei Rx, Ry und Rz in den Resten -NRxCORy, -CORy und NRxCO₂Rz aus den oben für Rx, Ry und Rz angegebenen Bedeutungen ausgewählt sind;
wobei alle obigen Alkyl- oder alk-, Alkoxy- und Alkylthioreste linear oder verzweigt sind und 1 bis 6 Kohlenstoffatome enthalten,
wobei die Produkte der Formel (I) in allen möglichen racemischen, enantiomeren und diastereoisomeren Stereoisomerformen vorliegen, sowie die Säureadditionssalze der Produkte der Formel (I) mit anorganischen und organischen Säuren oder mit anorganischen und organischen Basen.

2. Produkte der Formel (I) gemäß Anspruch 1, worin:
R₁ für einen Phenyl-, Pyridin-, Thienyl-, Benzoxazolyl-, Benzofuryl-, Indazolyl-, Indolyl-, Benzothienyl-, Benzimidazolyl-, Benzoxazinyl- oder Tetrahydrochinolylrest steht, der gegebenenfalls durch einen oder mehrere gleiche oder verschiedene Reste, die aus Halogenatomen und CN-, Nitro-, -COOH-, -COOalk-, -NRxRy-, Alkoxy-, Alkyl-, Alkinyl- und Cycloalkylresten ausgewählt sind, substituiert ist;
wobei diese letzteren Alkoxy-, Alkyl- und Alkinylreste selbst gegebenenfalls durch einen oder mehrere gleiche oder verschiedene Reste, die aus Halogenatomen und Hydroxyl-, Alkoxy-, NRvRw-, Piperidyl-, Pyrrolidinyl- oder Heteroarylresten ausgewählt sind, substituiert sind;
wobei die Phenol- und Heteroarylreste außerdem gegebenenfalls durch einen oder mehrere Alkyl- und Alkoxyreste substituiert sind;
R für ein Wasserstoffatom steht oder mit R1 einen Benzoxazinyl-, Dihydroindolyl-, Tetrahydroisochinolyl-, Tetrahydrochinolyl- oder Dihydropyrrolopyridylring bildet, wobei diese Ringe gegebenenfalls durch einen oder mehrere gleiche oder verschiedene Reste, die aus Halogenatomen und CO-NH₂-, Hydroxyl-, Alkyl- und Alkoxyresten ausgewählt sind, substituiert sind; wobei dieser letztere Alkylrest selbst gegebenenfalls durch einen Hydroxyl-, Alkoxy-, NH₂-, NHalk- oder N(alk)₂-Rest substituiert ist;
R2 und R3 gleich oder verschieden sind und unabhängig voneinander für ein Wasserstoffatom, ein Fluoratom oder einen Alkylrest stehen;
R4 für ein Wasserstoffatom steht;
R5 für ein Wasserstoffatom oder einen Alkylrest steht;
wobei NRxRy so beschaffen ist, dass Rx für ein Wasserstoffatom oder einen Alkylrest steht und Ry für ein Wasserstoffatom oder einen Alkylrest steht; oder Rx und Ry mit dem Stickstoffatom, an das sie gesunden sind, einen 3- bis 10-gliedrigen cyclischen Rest bilden, der gegebenenfalls ein oder mehrere aus O, S, NH und N-Alkyl ausgewählte Heteroatome enthält und gegebenenfalls substituiert ist;
wobei NRvRw so beschaffen ist, dass Rv für ein Wasserstoffatom oder einen Alkylrest steht und Rw für ein Wasserstoffatom oder einen Alkylrest steht;
wobei alle obigen Alkyl- oder alk- und Alkoxyreste linear oder verzweigt sind und 1 bis 6 Kohlenstoffatome enthalten,
wobei die Produkte der Formel (I) in allen möglichen racemischen, enantiomeren und diastereoisomeren Stereoisomerformen vorliegen, sowie die Säureadditionssalze der Produkte der Formel (I) mit anorganischen und organischen Säuren oder mit anorganischen und organischen Basen.

3. Produkte der Formel (I) gemäß einem der Ansprüche 1 oder 2, die den folgenden Formeln entsprechen:
- 2-[4-(Morpholin-4-yl)-6-oxo-1,6-dihydropyrimidin-2-yl]-N-phenylacetamid
- N-(4-Fluorphenyl)-2-[4-(morpholin-4-yl)-6-oxo-1,6-dihydropyrimidin-2-yl]acetamid
- N-(3-Chlorphenyl)-2-4-(morpholin-4-yl)-6-oxo-1,6-dihydropyrimidin-2-yl]acetamid
- N-[3(Dimethylamino)phenyl]-2-4(morpholin-4-yl)-6-oxo-1,6-dihydropyrimidin-2-yl]acetamid
- N-(2,4-Difluorphenyl)-2-[4-(morpholin-4-yl)-6-oxo-1,6-dihydropyrimidin-2-yl]acetamid
- N-(3,4-Difluorphenyl)-2-[4-(morpholin-4-yl)-6-oxo-1,6-dihydropyrimidin-2-yl]acetamid
- 2-[4-(Morpholin-4-yl)-6-oxo-1,6-dihydropyrimidin-2-yl]-N-(thiophen-3-yl)acetamid
- N-(4-Fluor-3-methoxyphenyl)-2-[4-(morpholin-4-yl)-6-oxo-1,6-dihydropyrimidin-2-yl]acetamid
- N-(2-Fluorphenyl)-2-[4-(morpholin-4-yl)-6-oxo-1,6-dihydropyrimidin-2-yl]acetamid
- N-(2-Methylphenyl-2-[4-(morpholin-4-yl)-6-oxo-1,6-dihydropyrimidin-2-yl]acetamid
- N-(2-Methoxyphenyl)-2-[4-(morpholin-4-yl)-6-oxo-1,6-dihydropyrimidin-2-yl]acetamid
- N-(2,3-Difluorphenyl)-2-4-(morpholin-4-yl)-6-oxo-1,6-dihydropyrimidin-2-yl]acetamid
- N-(3,5-Difluorphenyl)-2-[4-(morpholin-4-yl)-6-oxo-1,6-dihydropyrimidin-2-yl]acetamid
- N-(3-Fluorphenyl)-2-[4-(morpholin-4-yl)-6-oxo-1,6-dihydropyrimidin-2-yl]acetamid
- N-(4-Chlorphenyl)-2-[4-(morpholin-4-yl)-6-oxo-1,6-dihydropyrimidin-2-yl]acetamid
- N-(3-Methoxyphenyl)-2-[4-(morpholin-4-yl)-6-oxo-1,6-dihydropyrimidin-2-yl]acetamid
- 2-[4-(Morpholin-4-yl)-6-oxo-1,6-dihydro-pyrimidin-2-yl]-N-[3-(trifluormethyl)phenyl]-acetamid
- N-(3-Bromphenyl)-2-[4-(morpholin-4-yl)-6-oxo-1,6-dihydropyrimidin-2-yl]acetamid
- N-[3-(2-Methylpropan-2-yl)phenyl]-2-[4-(morpholin-4-yl)-6-oxo-1,6-dihydropyrimidin-2-yl]acetamid
- 3-({[4-(Morpholin-4-yl)-6-oxo-1,6-dihydro-pyrimidin-2-yl]acetyl}amino)benzoesäuremethylester
- 3-({[4-(Morpholin-4-yl)-6-oxo-1,6-dihydro-pyrimidin-2-yl]acetyl}amino)benzoesäure
- 2-[4-(Morpholin-4-yl)-6-oxo-1,6-dihydro-pyrimidin-2-yl]-N-[3-(propan-2-yl)phenyl]acetamid
- N-(3-Methylphenyl)-2-[4-(morpholin-4-yl)-6-oxo-1,6-dihydropyrimidin-2-yl]acetamid
- N-(3-Cyano-4-fluorphenyl)-2-[4-(morpholin-4-yl)-6-oxo-1,6-dihydropyrimidin-2-yl]acetamid
- N-(1H-Indazol-6-yl)-2-[4-(morpholin-4-yl)-6-oxo-1,6-dihydropyrimidin-2-yl]acetamid
- N-(3-Cyanophenyl)-2-[4-(morpholin-4-yl)-6-oxo-1,6-dihydropyrimidin-2-yl]acetamid
- N-(5-Fluorpyridin-2-yl)-2-[4-(morpholin-4-yl)-6-oxo-1,6-dihydropyrimidin-2-yl]acetamid
- N-(4-Fluor-3-methylphenyl)-2-[4-(morpholin-4-yl)-6-oxo-1,6-dihydropyrimidin-2-yl]acetamid
- N-(3-Chlor-4-fluorphenyl)-2-[4-(morpholin-4-yl)-6-oxo-1,6-dihydropyrimidin-2-yl]acetamid
- 2-[4-(Morpholin-4-yl)-6-oxo-1,6-dihydro-pyrimidin-2-yl]-N-(pyridin-3-yl)acetamid
- N-(4-Fluor-2-methylphenyl)-2-[4-(morpholin-4-yl)-9-oxo-1,6-dihydropyrimidin-2-yl]acetamid
- N-(3-Hydroxyphenyl)-2-[4-(morpholin-4-yl)-6-oxo-1,6-dihydropyrimidin-2-yl]acetamid
- N-(3-Brom-4-fluorphenyl)-2-[4-(morpholin-4-yl)-6-oxo-1,6-dihydropyrimidin-2-yl]acetamid
- 2-[4-(Morpholin-4-yl)-6-oxo-1,6-dihydro-pyrimidin-2-yl]-N-(3,4,5-trifluorphenyl)acetamid
- N-[4-Fluor-3-(hydroxymethyl)phenyl]-2-4-(morpholin-4-yl)-6-oxo-1,6-dihydropyrimidin-2-yl]acetamid
- N-(3-Cyclopropylphenyl)-2-[4-(morpholin-4-yl)-6-oxo-1,6-dihydropyrimidin-2-yl]acetamid
- N-(2-Hydroxyphenyl)-2-[4-(morpholin-4-yl)-6-oxo-1,6-dihydropyrimidin-2-yl]acetamid
- N-[3-(Difluormethoxy)phenyl]-2-[4-(morpholin-4-yl)-6-oxo-1,6-dihydropyrimidin-2-yl]acetamid
- N-(4-Fluor-3-methoxyphenyl)-2-[4-(morpholin-4-yl)-6-oxo-1,6-dihydropyrimidin-2-yl]propanamid
- N-(2,3-Dimethylphenyl)-2-[4-(morpholin-4-yl)-6-oxo-1,6-dihydropyrimidin-2-yl]acetamid
- N-(2-Fluor-3-methylphenyl)-2-[4-(morpholin-4-yl)-6-oxo-1,6-dihydropyrimidin-2-yl]acetamid
- N-(1,3-Benzoxazol-4-yl)-2-[4-(morpholin-4-yl)-6-oxo-1,6-dihydropyrimidin-2-yl]acetamid
- 2-[4-(Morpholin-4-yl)-6-oxo-1,6-dihydro-pyrimidin-2-yl]-N-[3-(trifluormethoxy)phenyl]-acetamid
- 2-[4-(Morpholin-4-yl)-6-oxo-1,6-dihydro-pyrimidin-2-yl]-N-3-(propan-2-yloxy)phenyl] - acetamid
- N-(4-Fluor-2-methoxyphenyl)-2-[4-(morpholin-4-yl)-6-oxo-1,6-dihydropyrimidin-2-yl]acetamid
- {2-[3-({[4-(Morpholin-4-yl)-6-oxo-1,6-dihydropyrimidin-2-yl]acetyl}amino)phenyl]ethyl}carbamidsäure-2-methylpropan-2-ylester
- N-[4-Fluor-3-(trifluormethyl)phenyl]-2-[4-(morpholin-4-yl)-6-oxo-1,6-dihydropyrimidin-2-yl]cetamid
- N-(3-Ethinylphenyl)-2-4-(morpholin-4-yl)-6-oxo-1,6-dihydropyrimidin-2-yl]acetamid
- N-[3-(Cyclopentyloxy)phenyl]-2-[4-(morpholin-4-yl)-6-oxo-1,6-dihydropyrimidin-2-yl]acetamid
- N-(4-Fluor-2-hydroxyphenyl)-2-[4-(morpholin-4-yl)-6-oxo-1,6-dihydropyrimidin-2-yl]acetamid
- 2-[2-(2,3-Dihydro-1H-indol-1-yl)-2-oxoethyl]-6-(morpholin-4-yl)pyrimidin-4(3H)-on
- N-(3-Cyclopropyl-4-fluorphenyl)-2- 4-(morpholin-4-yl)-6-oxo-1,6-dihydropyrimidin-2-yl]acetamid
- 2-[4-(Morpholin-4-yl)-6-oxo-1,6-dihydro-pyrimidin-2-yl]-N-(2,3,4-trifluorphenyl)acetamid
- N-[4-Fluor-3-(trifluormethoxy)phenyl]-2-[4-(morpholin-4-yl)-6-oxo-1,6-dihydropyrimidin-2-yl]acetamid
- N-[3-(2-Hydroxyethoxy)phenyl]-2-[4-(morpholin-4-yl)-6-oxo-1,6-dihydropyrimidin-2-yl]acetamid
- N-(3-Iodphenyl)-2-[4-(morpholin-4-yl)-6-oxo-1,6-dihydropyrimidin-2-yl]acetamid
- 2-Fluor-5-({[4-(morpholin-4-yl)-6-oxo-1,6-dihydropyrimidin-2-yl]acetyl}amino)benzoesäuremethylester
- N-(3-Ethoxyphenyl)-2-[4-(morpholin-4-yl)-6-oxo-1,6-dihydropyrimidin-2-yl]acetamid
- N-(2,4-Difluor-3-methoxyphenyl)-2-[4-(morpholin-4-yl)-6-oxo-1,6-dihydropyrimidin-2-yl]acetami
- 2-[4-(Morpholin-4-yl)-6-oxo-1,6-dihydropyrimidin-2-yl]-N-(2,4,5-trifluorphenyl)acetamid
- N-3,5-Dichlor-4-fluorphenyl)-2-[4-(morpholin-4-yl)-6-oxo-1,6-dihydropyrimidin-2-yl]acetamid
- 2-[2-(2,3-Dihydro-4H-1,4-benzoxazin-4-yl)-2-oxoethyl]-6-(morpholin-4-yl)pyrimidin-4(3H)-on
- N-(4-Fluor-3-nitrophenyl)-2-[4-(morpholin-4-yl)-6-oxo-1,6-dihydropyrimidin-2-yl]acetamid
- 2-Fluor-5-({[4-(morpholin-4-yl)-6-oxo-1,6-dihydropyrimidin-2-yl]acetyl}amino)benzoesäure
- N-(5-Fluor-2-hydroxyphenyl)-2-[4-(morpholin-4-yl)-6-oxo-1,6-dihydropyrimidin-2-yl]acetamid
- N-(2-Brom-4-fluorphenyl)-2-[4-morpholin-4-yl) - 6-oxo-1,6-dihydropyrimidin-2-yl]acetamid
- N-(4-Fluorphenyl)-2-[1-methyl-4-(morpholin-4-yl)-6-oxo-1,6-dihydropyrimidin-2-yl]acetamid
- N-(3-Chlor-4-fluorphenyl)-2-[1-methyl-4-(morpholin-4-yl)-6-oxo-1,6-dihydropyrimidin-2-yl]acetamid
- N-(3-Bromphenyl)-2-[1-methyl-4-(morpholin-4-yl)-6-oxo-1,6-dihydropyrimidin-2-yl]acetamid
- 2-[1-Ethyl-4-(morpholin-4-yl)-6-oxo-1,6-dihydropyrimidin-2-yl]-N-(4-fluorphenyl)acetamid
- N-(1H-Indol-4-yl)-2-[4-(morpholin-4-yl)-6-oxo-1,6-dihydropyrimidin-2-yl]acetamid
- N-(4-Fluorphenyl)-3-methyl-2-[4-(morpholin-4-yl)-6-oxo-1,6-dihydropyrimidin-2-yl]butanamid
- N-[4-Fluor-3-(methoxymethyl)phenyl]-2-[4-(morpholin-4-yl)-6-oxo-1,6-dihydropyrimidin-2-yl]acetamid
- N-(4-Fluor-3-iodphenyl)-2-[4-(morpholin-4-yl)-6-oxo-1,6-dihydropyrimidin-2-yl]acetamid
- 2-[4-(Morpholin-4-yl)-6-oxo-1,6-dihydro-pyrimidin-2-yl]-N-[3-(1,1,2,2-tetrafluorethoxy)-phenyl]acetamid
- N-[3-(Difluormethyl)-4-fluorphenyl]-2-[4-(morpholin-4-yl)-6-oxo-1,6-dihydropyrimidin-2-yl]acetamid
- 2,2-Difluor-N-(4-fluorphenyl)-2-4-(morpholin-4-yl)-6-oxo-1,6-dihydropyrimidin-2-yl]acetamid
- N-(3,4-Difluorphenyl)-2-[1-methyl-4-(morpholin-4-yl)-6-oxo-1,6-dihydropyrimidin-2-yl]acetamid
- 2-[2-(2,3-Dihydro-1H-indol-1-yl)-2-oxoethyl]-3-methyl-6-(morpholin-4-yl)pyrimidin-4(3H)-on
- N-(3-Brom-4-fluorphenyl)-2-[1-methyl-4-(morpholin-4-yl)-6-oxo-1,6-dihydropyrimidin-2-yl]acetamid
- N-[4-Fluor-3-(hydroxymethyl)phenyl]-2-[1-methyl-4-(morpholin-4-yl)-6-oxo-1,6-dihydropyrimidin-2-yl]acetamid
- N-(3-Cyclopropylphenyl)-2-[1-methyl-4-(morpholin-4-yl)-6-oxo-1,6-dihydropyrimidin-2-yl]acetamid
- N-(4-Fluor-3-methoxyphenyl)-2-[1-methyl-4-(morpholin-4-yl)-6-oxo-1,6-dihydropyrimidin-2-yl]acetamid
- N-(1-Benzofur-4-yl)-2-[4-(morpholin-4-yl)-6-oxo-1,6-dihydropyrimidin-2-yl]acetamid
- 2-[1-Methyl-4-(morpholin-4-yl)-6-oxo-1,6-dihydropyrimidin-2-yl]-N-phenylacetamid
- N-(3-Cyclopropyl-4-fluorphenyl)-2-[1-methyl-4-(morpholin-4-yl)-6-oxo-1,6-dihydropyrimidin-2-yl]acetamid
- N-(3-Fluor-2-hydroxyphenyl)-2-[4-(morpholin-4-yl)-6-oxo-1,6-dihydropyrimidin-2-yl]acetamid
- 2-[2-(4-Fluor-2,3-dihydro-1H-indol-1-yl)-2-oxoethyl]-6-(morpholin-4-yl)pyrimidin-4(3H)-on
- 2-[2-(4-Chlor-2,3-dihydro-1H-indol-1-yl)-2-oxoethyl]-6-(morpholin-4-yl)pyrimidin-4(3H)-on
- N-(3-Ethinyl-4-fluorphenyl)-2-[4-(morpholin-4-yl)-6-oxo-1,6-dihydropyrimidin-2-yl]acetamid
- 2-[2-(4-Hydroxy-2,3-dihydro-1H-indol-1-yl)-2-oxoethyl]-6-(morpholin-4-yl)pyrimidin-4(3H)-on
- 2-[2-(4,6-Difluor-2,3-dihydro-1H-indol-1-yl)-2-oxoethyl]-6-(morpholin-4-yl)pyrimidin-4(3H)-on
- N-(4-Fluor-3-iodphenyl)-2-(1-methyl-4-morpholin-4-yl-6-oxo-1,6-dihydropyrimidin-2-yl)acetamid
- 2-[2-(4,5-Difluor-2,3-dihydro-1H-indol-1-yl)-2-oxoethyl]-6-(morpholin-4-yl)pyrimidin-4(3H)-on
- 2-[2-(6-Fluor-2,3-dihydro-1H-indol-1-yl)-2-oxoethyl]-6-(morpholin-4-yl)pyrimidin-4(3H)-on
- 2-[2-(2-Methyl-2,3-dihydro-1H-indol-1-yl)-2-oxoethyl]-6-(morpholin-4-yl)pyrimidin-4(3H)-on
- N-[3-(Difluormethyl)-4-fluorphenyl]-2-[1-methyl-4-morpholin-4-yl)-6-oxo-1,6-dihydropyrimidin-2-yl]acetamid
- 2-[1-Methyl-4-(morpholin-4-yl)-6-oxo-1,6-dihydropyrimidin-2-yl]-N-(3,4,5-trifluorphenyl)-acetamid
- N-(1-Methyl-1H-indol-4-yl)-2-[4-(morpholin-4-yl)-6-oxo-1,6-dihydropyrimidin-2-yl]acetamid
- 2-[2-(4-Methyl-2,3-dihydro-1H-indol-1-yl)-2-oxoethyl]-6-(morpholin-4-yl)pyrimidin-4(3H)-on
- 2-[2-(3-Methyl-2,3-dihydro-1H-indol-1-yl)-2-oxoethyl]-6-(morpholin-4-yl)pyrimidin-4(3H)-on
- 2-[2-(4-Fluor-2,3-dihydro-1H-indol-1-yl)-2-oxoethyl]-3-methyl-6-(morpholin-4-yl)pyrimidin-4(3H)-on
- 2-[2-(5-Fluor-2,3-dihydroindol-1-yl)-2-oxoethyl]-6-morpholin-4-yl-3H-pyrimidin-4-on
- 2-[2-(4-Chlor-2,3-dihydro-1H-indol-1-yl)-2-oxoethyl]-3-methyl-6-(morpholin-4-yl)pyrimidin-4(3H)-on
- N-(1-Benzothiophen-4-yl)-2-[4-(morpholin-4-yl)-6-oxo-1,6-dihydropyrimidin-2-yl]acetamid
- 2-{2-[2-(Hydroxymethyl)-2,3-dihydro-1H-indol-1-yl]-2-oxoethyl}-6-(morpholin-4-yl)pyrimidin-4(3H)-on
- 2-[4-(Morpholin-4-yl)-6-oxo-1,6-dihydro-pyrimidin-2-yl]-N-{2-[2-(piperidin-1-yl)ethoxy]-phenyl}acetamid
- N-[2-(2-Methoxyethoxy)phenyl]-2-4-(morpholin-4-yl)-6-oxo-1,6-dihydropyrimidin-2-yl]acetamid
- 2-[2-(4-Hydroxy-2,3-dihydro-1H-indol-1-yl)-2-oxoethyl]-3-methyl-6-(morpholin-4-yl)pyrimidin-4(3H)-on
- 2-[2-(4-Methoxy-2,3-dihydro-1H-indol-1-yl)-2-oxoethyl]-3-methyl-6-(morpholin-4-yl)pyrimidin-4(3H)-on
- 2-[2-(3,4-Dihydroisochinolin-2(1H)-yl)-2-oxoethyl]-6-(morpholin-4-yl)pyrimidin-4(3H)-on
- 2-[4-(Morpholin-4-yl)-6-oxo-1,6-dihydro-pyrimidin-2-yl]-N-{2-[2-(pyrrolidin-1-yl)ethoxy]-phenyl}acetamid
- 2-[4-(Morpholin-4-yl)-6-oxo-1,6-dihydro-pyrimidin-2-yl]-N-[2-(pyridin-3-ylmethoxy)phenyl]-acetamid
- 3-Methyl-2-[2-(4-methyl-2,3-dihydro-1H-indol-1-yl)-2-oxoethyl]-6-(morpholin-4-yl)pyrimidin-4(3H)-on
- 2-(2-{3-[(Dimethylamino)methyl]-2,3-dihydro-1H-indol-1-yl}-2-oxoethyl)-6-(morpholin-4-yl)-pyrimidin-4(3)-on
- 2-[2-(4-Brom-2,3-dihydro-1H-indol-1-yl)-2-oxoethyl]-3-methyl-6-(morpholin-4-yl)pyrimidin-4(3H)-on
- 2-{2-[(2S)-2-Methyl-2,3-dihydro-1H-indol-1-yl]-2-oxoethyl}-6-(morpholin-4-yl)pyrimidin-4(3H)-on
- 2-{2-[(2R)-2-Methyl-2,3-dihydro-1H-indol-1-yl] - 2-oxoethyl}-6-(morpholin-4-yl)pyrimidin-4(3H)-on
- 3-Methyl-2-[2-(3-methyl-2,3-dihydro-1H-indol-1-yl)-2-oxoethyl]-6-(morpholin-4-yl)pyrimidin-4(3H)-on
- 2-{2-[2-(Methoxymethyl)-2,3-dihydro-1H-indol-1-yl]-2-oxoethyl}-6-(morpholin-4-yl)pyrimidin-4(3H)-on
- 2-[2-(4-Ethoxy-2,3-dihydro-1H-indol-1-yl)-2-oxoethyl]-6-(morpholin-4-yl)pyrimidin-4(3H)-on
- 1-{[4-(Morpholin-4-yl)-6-oxo-1,6-dihydro-pyrimidin-2-yl]acetyl}-2,3-dihydro-1H-indol-2-carboxamid
- 3-Methyl-2-[2-(2-methyl-2,3-dihydro-1H-indol-1-yl)-2-oxoethyl]-6-(morpholin-4-yl)pyrimidin-4(3H)-on
- 2-[2-(6-Fluor-2,3-dihydro-1H-indol-1-yl)-2-oxoethyl]-3-methyl-6-(morpholin-4-yl)pyrimidin-4(3H)-on
- 2-{2-[(3S)-3-Methyl-2,3-dihydro-1H-indol-1-yl]-2-oxoethyl}-6-(morpholin-4-yl)pyrimidin-4(3H)-on
- 2-{2-[(3R)-3-Methyl-2,3-dihydro-1H-indol-1-yl]-2-oxoethyl}-6-(morpholin-4-yl)pyrimidin-4(3H)-on
- 2-[2-(5,6-Difluor-2,3-dihydro-1H-indol-1-yl)-2-oxoethyl]-3-methyl-6-(morpholin-4-yl)pyrimidin-4(3H)-on
- 2-[2-(4,5-Difluor-2,3-dihydro-1H-indol-1-yl)-2-oxoethyl]-3-methyl-6-(morpholin-4-yl)pyrimidin-4(3H)-on
- 2-[2-(1,3-Dihydro-2H-isoindol-2-yl)-2-oxoethyl]-6-morpholin-4-yl)pyrimidin-4(3H)-on
- N-(1-Benzothiophen-4-yl)-2-[1-methyl-4-(morpholin-4-yl)-6-oxo-1,6-dihydropyrimidin-2-yl]-acetamid
- 2-[2-(5-Chlor-3,4-dihydroquinolin-1(2H)-yl)-2-oxoethyl]-6-(morpholin-4-yl)pyrimidin-4(3H)-on
- 2-{2-[4-(Hydroxymethyl)-2,3-dihydro-1H-indol-1-yl]-2-oxoethyl)-6-(morpholin-4-yl)pyrimidin-4(3H)-on
- N-[4-Fluor-2-(piperidin-4-ylmethoxy)phenyl]-2-[4-(morpholin-4-yl)-6-oxo-1,6-dihydropyrimidin-2-yl]acetamid
- 2-[2-(5-Chlor-2,3-dihydroindol-1-yl)-2-oxoethyl]-6-morpholin-4-yl-3H-pyrimidin-4-on
- 2-[2-(4-Brom-2,3-dihydroindol-1-yl)-2-oxoethyl]-6-morpholin-4-yl-3H-pyrimidin-4-on
- 2-(2-{(3S)-3-[(Dimethylamino)methyl]-2,3-dihydro-1H-indol-1-yl}-2-oxoethyl)-6-(morpholin-4-yl)pyrimidin-4(3H)-on
- 2-(2-{(3R)-3-[(Dimethylamino)methyl]-2,3-dihydro-1H-indol-1-yl)-2-oxoethyl)-6-(morpholin-4-yl)pyrimidin-4(3H)-on
- N-[4-Fluor-2-(2-methoxyethoxy)phenyl]-2-[4-(morpholin-4-yl)-6-oxo-1,6-dihydropyrimidin-2-yl]acetamid
- N-(1H-Benzimidazol-4-yl)-2-[4-(morpholin-4-yl)-6-oxo-1,6-dihydropyrimidin-2-yl]acetamid
- 2-Hydroxy-3-({[4-(morpholin-4-yl)-6-oxo-1,6-dihydropyrimidin-2-yl]acetyl}amino)benzoesäure-methylester
- 2-[2-(4-Methoxy-2,3-dihydroindol-1-yl)-2-oxoethyl]-6-morpholin-4-yl-3H-pyrimidin-4-on
- N-(3-Brom-2-hydroxyphenyl)-2-[4-(morpholin-4-yl)-6-oxo-1,6-dihydropyrimidin-2-yl]acetamid
- N-(3,4-Dihydro-2H-1,4-benzoxazin-8-yl)-2-[4-(morpholin-4-yl)-6-oxo-1,6-dihydropyrimidin-2-yl]acetamid
- 5-Fluor-2-({[4-(morpholin-4-yl)-6-oxo-1,6-dihydropyrimidin-2-yl]acetyl}amino)benzoesäure-methylester
- 2-(2-{3-[(Diethylamino)methyl]-2,3-dihydro-1H-indol-1-yl}-2-oxoethyl)-6-(morpholin-4-yl)-pyrimidin-4(3)-on
- 2-[2-(2,3-Dihydro-1H-pyrrolo[3,2-b]pyridin-1-yl)-2-oxoethyl]-6-(morpholin-4-yl)pyrimidin-4(3H)-on
- 2-[2-(5,6-Difluor-2,3-dihydroindol-1-yl)-2-oxoethyl]-6-morpholin-4-yl-3H-pyrimidin-4-on
- 2-[4-(Morpholin-4-yl)-6-oxo-1,6-dihydro-pyrimidin-2-yl]-N-(1,2,3,4-tetrahydrochinolin-8-yl)acetamid
- 2-[2-(8-Chlor-2,3-dihydro-4H-1,4-benzoxazin-4-yl)-2-oxoethyl]-6-(morpholin-4-yl)pyrimidin-4(3H)-on
- N-(2-Hydroxy-3-methylphenyl)-2-4-(morpholin-4-yl)-6-oxo-1,6-dihydropyrimidin-2-yl]acetamid
- N-(2-Hydroxy-3-nitrophenyl)-2-[4-(morpholin-4-yl)-6-oxo-1,6-dihydropyrimidin-2-yl]acetamid
- N-(3-Cyano-2-hydroxyphenyl)-2-[4-(morpholin-4-yl)-6-oxo-1,6-dihydropyrimidin-2-yl]acetamid
- N-[2-Hydroxy-3-(trifluormethyl)phenyl]-2-[4-(morpholin-4-yl)-6-oxo-1,6-dihydropyrimidin-2-yl]acetamid
- 2-[2-(3,3-Dimethyl-2,3-dihydro-1H-indol-1-yl)-2-oxoethyl]-6-(morpholin-4-yl)pyrimidin-4(3H)-on
sowie die Säureadditionssalze der Produkte der Formel (I) mit anorganischen und organischen Säuren oder mit anorganischen und organischen Basen.

4. Verfahren zur Herstellung der Produkte der Formel (I) gemäß einem der Ansprüche 1 bis 3 gemäß nachstehendem Schema 1A 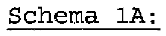 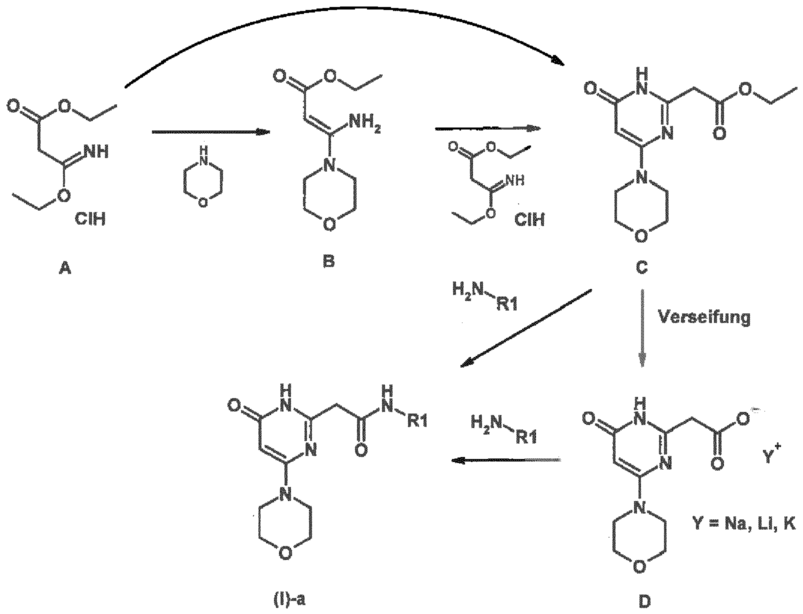 wobei der Substituent R1 die in einem der Ansprüche 1 oder 2 angegebenen Bedeutungen beisitzt.

5. Verfahren zur Herstellung der Produkte der Formel (I) gemäß einem der Ansprüche 1 bis 3 gemäß nachstehendem Schema 1B 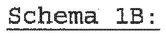 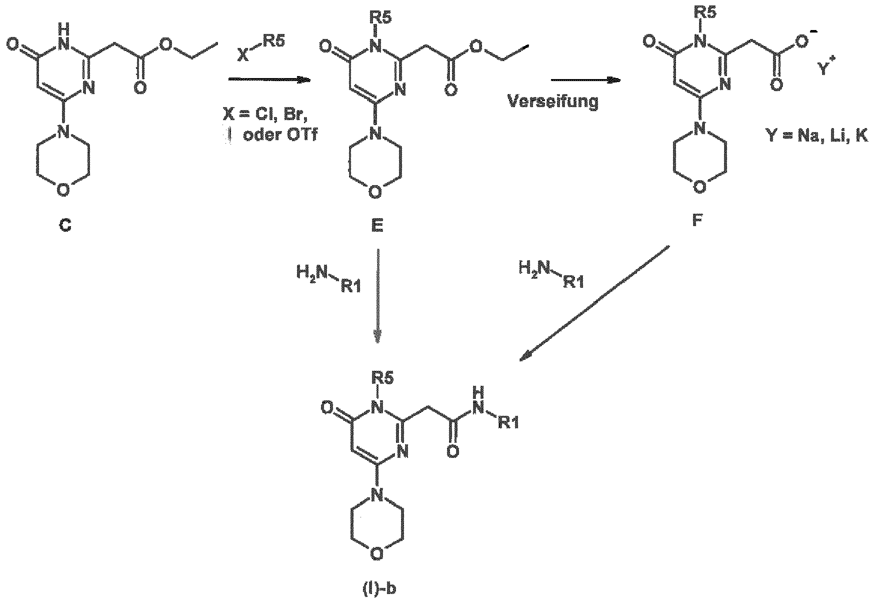 wobei die Substitutenten R1 und R5 die in einem der Ansprüche 1 oder 2 angegebenen Bedeutungen besitzen.

6. Verfahren zur Herstellung der Produkte der Formel (I) gemäß einem der Ansprüche 1 bis 3 gemäß nachstehendem Schema 1C 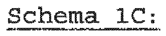 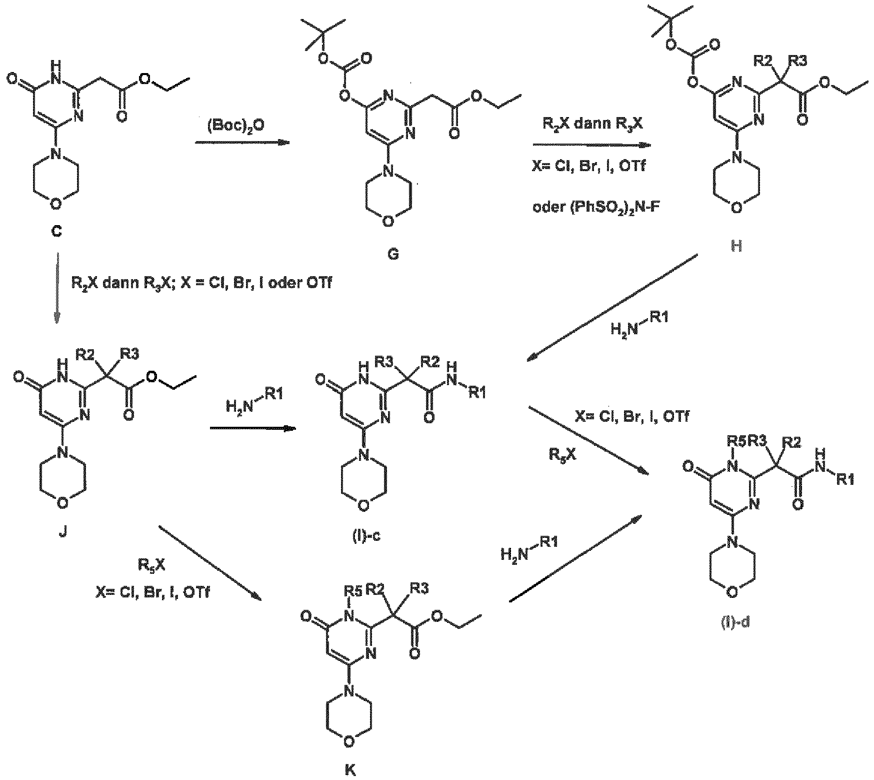 wobei die Substituenten R1, R2, R3 und R5 die in einem der Ansprüche 1 oder 2 angegebenen Bedeutungen besitzen.

7. Produkte der Formel (I) gemäß einem der Ansprüche 1 bis 3 sowie die pharmazeutisch unbedenklichen Säureadditionssalze der Produkte der Formel (I) mit anorganischen und organischen Säuren oder mit anorganischen und organischen Basen zur Verwendung als Medikamente.

8. Produkte der Formel (I) gemäß Anspruch 3 sowie die pharmazeutisch unbedenklichen Säureadditionssalze der Produkte der Formel (I) mit anorganischen und organischen Säuren oder mit anorganischen und organischen Basen zur Verwerdung als Medikamente.

9. Pharmazeutische Zusammensetzungen, die als Wirkstoff mindestens eines der Produkte der Formel (I) gemäß einem der Ansprüche 1 bis 3 oder ein pharmazeutisch unbedenkliches Salz dieses Produkts und einen pharmazeutisch unbedenklichen Träger enthalten.

10. Produkte der Formel (I) gemäß einem der Ansprüche 1 bis 3 zur Verwendung für die Behandlung von Krebserkrankungen.

11. Produkte der Formel (I) gemäß einem der Ansprüche 1 bis 3 zur Verwendung für die Behandlung von soliden oder flüssigen Tumoren.

12. Produkte der Formel (I) gemäß einem der Ansprüche 1 bis 3 zur Verwendung für die Behandlung von Krebserkrankungen, die gegenüber Zytotoxika resistent sind.

13. Produkte der Formel (I) gemäß einem der Ansprüche 1 bis 3 zur Verwendung für die Behandlung von primären Tumoren und/oder Metastasen, insbesondere bei Krebserkrankungen des Magens, der Leber, der Niere, der Eierstöcke, des Kolons, der Prostata, des Endometriums, der Lunge (NSCLC und SCLC), Glioblastomen, Krebserkrankungen der Schilddrüse, der Blase, der Brust, bei Melanom, bei lymphoiden oder myeloiden hämatopoetischen Tumoren, bei Sarkomen, bei Krebserkrankungen des Gehirns, des Kehlkopfs, des Lymphsystems, Krebserkrankungen der Knochen und der Bauchspeicheldrüse, und bei Hamartomen.

14. Produkte der Formel (I) gemäß einem der Ansprüche 1 bis 3 zur Verwendung für die Chemotherapie von Krebserkrankungen.

15. Produkte der Formel (I) gemäß einem der Ansprüche 1 bis 3 zur Verwendung für die Chemotherapie von Krebserkrankungen, allein oder in Kombination.

16. Produkte der Formel (I) gemäß einem der Ansprüche 1 bis 3 als Inhibitoren der Phosphorylierung von AKT (PKB)

17. Synthesezwischenprodukte der Formeln C, D, E und F gemäß Anspruch 4 oder 5, die nachstehend noch einmal aufgeführt sind: 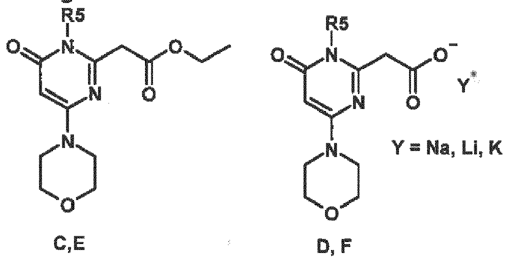 wobei 5 die in einem der Ansprüche 1 oder 2 angegebene Definition aufweist.

## Claims

1. Products of formula (I): 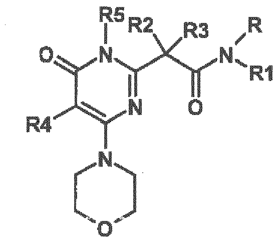 in which:
R1 represents an aryl or heteroaryl radical optionally substitutes with one or more radicals, which may be identical or different, chosen from halogen atoms and hydroxyl, CN, nitro, -COOH, -COOalk, -CONRxRy, -NRxCORy, -CORy, -NRxCO₂Rz, alkoxy, phenoxy, alkylthio, alkyl, alkenyl, alkynyl, cycloalkyl, O-cycloalkyl, heterocycloalkyl, aryl and heteroaryl radicals;
the latter alkoxy, phenoxy, alkylthio, alkyl, alkenyl, alkynyl, heterocycloalkyl, aryl and heteroaryl radicals being themselves optionally substituted with one or more radicals, which may be identical or different, chosen from halogen atoms and hydroxyl, alkoxy, NRvRw, heterocycloalkyl or heteroaryl radicals;
the aryl and heteroaryl radicals being, in addition, optionally substituted with one or more alkyl and alkoxy radicals, themselves optionally substituted with one or more halogen atoms;
it being possible for the heterocycloalkyl and heteroaryl radicals to additionally contain an oxo radical;
R represents a hydrogen atom or else forms, with R1, a saturated or partially or totally unsaturated 5- or 6-membered ring fused to an aryl or heteroaryl residue and optionally containing one or more other heteroatoms chosen from O, S, N, NH and Nalk, this bicyclic radical being optionally substituted with one or more radicals, which may be identical or different, chosen from halogen atoms and CO-NH₂, hydroxyl, alkyl and alkoxy radicals; the latter alkyl radical being itself optionally substituted with a hydroxyl, alkoxy, NH₂, NHalk or N(alk)₂ radical;
R2 and R3, which may be identical or different, independently represent a hydrogen atom, a halogen atom or an alkyl radical optionally substituted with one or more halogen atoms;
R4 represents a hydrogen atom;
R5 represents a hydrogen atom or an alkyl radical optionally substituted with one or more halogen atoms;
NRxRy being such that Rx represents a hydrogen atom or an alkyl radical and Ry represents a hydrogen atom or a cycloalkyl radical or an alkyl radical optionally substituted with one or more radicals, which may be identical or different, chosen from hydroxyl, alkoxy, NRvRw and heterocycloalkyl radicals; or Rx and Ry form, with the nitrogen atom to which they are attached, a cyclic radical containing from 3 to 10 ring members and optionally one or more other heteroatoms chosen from O, S, NH and N-alkyl, this radical being optionally substituted;
NRvRw being such that Rv represents a hydrogen atom or an alkyl radical and Rw represents a hydrogen atom or a cycloalkyl radical, CO₂alk, or an alkyl radical optionally substituted with one or more radicals, which may be identical or different, chosen from hydroxyl, alkoxy and heterocycloalkyl radicals; or Rv and Rw form, with the nitrogen atom to which they are attached, a cyclic radical containing from 3 to 10 ring members and optionally one or more other heteroatoms chosen from O, S, NH and N-alkyl, this cyclic radical being optionally substituted;
the cyclic radicals that Rx and Ry or Rv and Rw, respectively, can form, with the nitrogen atom to which they are attached, being optionally substituted with one or more radicals, which may be identical or different, chosen from halogen atoms and alkyl, hydroxyl, oxo, alkoxy, NH₂; NHalk and N(alk)₂ radicals;
Rz represents the values of Ry except for hydrogen;
Rx, Ry and Rz in the -NRxCORy, -CORy and CO₂Rz radicals being chosen from the meanings indicated above for Rx, Ry and Rz;
all the above alkyl (alk), alkoxy and alkylthio, radicals being linear or blanched and containing from 1 to 6 carbon atoms,
said products of formula (I) being in all the possible racemic, enantiomeric and diastereoisomeric stereoisomer forms, and also the addition salts with inorganic and organic acids or with inorganic and organic bases, of said products of formula (I).

2. Products of formula (I) as defined in Claim 1, in which:
R₁ represents a phenyl, pyridine, thienyl, benzoxazolyl, benzofuryl, indazolyl, indolyl, benzothienyl, benzimidazolyl, benzoxazinyl or tetrahydroquinolyl radical, optionally substituted with one or more radicals, which may be identical or different, chosen from halogen atoms and CN, nitro, - COOH, -COOalk, -NRxRy, alkoxy, alkyl, alkynyl and cycloalkyl radicals;
the latter alkoxy, alkyl and alkynyl radicals being themselves optionally substituted with one or more radicals, which may be identical or different, chosen from halogen atoms and hydroxyl, alkoxy, NRvRw, piperidyl, pyrrolidinyl or heteroaryl radicals;
the phenyl and heteroaryl radicals being, in addition, optionally substituted with one or more alkyl and alkoxy radicals;
R represents a hydrogen atom or else forms, with R₁ a benzoxazinyl, dihydroindolyl, tetrahydroisoquinolyl, tetrahydroquinolyl or dihydropyrrolopyridyl ring, these rings being optionally substituted with one or more radicals, which may be identical or different, chosen from halogen atoms and CO-NH₂, hydroxyl, alkyl and alkoxy radicals;
the latter alkyl radical being itself optionally substituted with a hydroxyl, alkoxy, NH₂, NHalk or N(alk)₂ radical;
R2 and R3, which may be identical or different, independently represent a hydrogen atom, a fluorine atom or an alkyl radical;
R4 represents a hydrogen atom;
R5 represents a hydrogen atom or an alkyl radical;
NRxRy being such that Rx represents a hydrogen atom or an alkyl radical and Ry represents a hydrogen atom or an alkyl radical; or Rx and Ry form, with the nitrogen atom to which they are attached, a cyclic radical containing from 3 to 10 ring members and optionally one or more other heteroatoms chosen from O, S, NH and N-alkyl, this cyclic radical being optionally substituted;
NRvRw being such that Rv represents a hydrogen atom or an alkyl radical and Rw represents a hydrogen atom or an alkyl radical;
all the above alkyl (alk) and alkoxy radicals being linear or blanched and containing from 1 to 6 carbon atoms,
said products of formula (I) being in all the possible racemic, enantiomeric and diastereoisomeric stereoisomer forms, and also the addition salts with inorganic and organic acids or with inorganic and organic bases, of said products of formula (I).

3. Products of formula (I) as defined in either one of Claims 1 and 2, corresponding to the following formulae:
- 2-[4-(morpholin-4-yl)-6-oxo-1,6-dihydropyrimidin-2-yl]-N-phenylacetamide
- N-(4-fluorophenyl)-2-[4-(morpholin-4-yl)-6-oxo-1,6-dihydropyrimidin-2-yl]acetamide
- N-(3-chlorophenyl)-2-[4-(morpholin-4-yl)-6-oxo-1,6-dihydropyrimidin-2-yl]acetamide
- N-[3-(dimethylamino)phenyl]-2-[4-(morpholin-4-yl)-6-oxo-1,6-dihydroyrimidin-2-yl]acetamide
- N-(2,4-difluorophenyl)-2-[4-(morpholin-4-yl)-6-oxo-1,6-dihydropyrimidin-2-yl]acetamide
- N-(3,4-difluorophenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-(thiophen-3-yl)acetamide
- N-(4-fluoro-3-methoxyphenyl)-2-[4-(morpholin-4-yl)-6-oxo-1,6-dihydropyrimidin-2-yl]acetamide
- N-(2-fluorophenyl)-2-[4-(morpholin-4-yl)-6-oxo-1,6-dihydropyrimidin-2-yl]acetamide
- N-(2-methylphenyl)-2-[4-(morpholin-4-yl)-6-oxo-1,6-dihydropyrimidin-2-yl]acetamide
- N-(2-methoxyphenyl)-2-[4-(morpholin-4-yl)-6-oxo-1,6-dihydropyrimidin-2-yl]acetamide
- N-(2,3-difluorophenyl)-2-[4-(morpholin-4-yl)-6-oxo-1,6-dihydropyrimidin-2-yl]acetamide
- N-(3,5-difluorophenyl)-2-[4-(morpholin-4-yl)-6-oxo-1,6-dihydropyrimidin-2-yl]acetamide
- N-(3-fluorophenyl)-2-[4-(morpholin-4-yl)-6-oxo-1,6-dihydropyrimidin-2-yl]acetamide
- N-(4-chlorophenyl)-2-[4-(morpholin-4-yl)-6-oxo-1,6-dihydropyrimidin-2-yl]acetamide
- N-(3-methoxyphenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-[3-(trifluoromethyl)phenyl]acetamide
- N-(3-bromophenyl)-2-[4-(morpholin-4-yl)-6-oxo-1,6-dihydropyrimidin-2-yl]acetamide
- N-[3-(2-methylpropan-2-yl)phenyl]-2-[4-(morpholin-4-yl)-6-oxo-1,6-dihydropyrimidin-2-yl]acetamide
- methyl 3-({[4-(morpholin-4-yl)-6-oxo-1,6-dihydropyrimidin-2-yl]acetyl}amino)benzoate
- 3-({[4-(morpholin-4-yl)-6-oxo-1,6-dihydropyrimidin-2-yl]acetyl}amino)benzoic acrid
- 2-[4-(morpholin-4-yl)-6-oxo-1,6-dihydropyrimidin-2-yl]-N-[3-(propan-2-yl)phenyl]acetamide
- N-(3-methylphenyl-2-[4-(morpholin-4-yl)-6-oxo-1,6-dihydropyrimidin-2-yl]acetamide
- N-(3-cyano-4-fluorophenyl)-2-[4-(morpholin-4-yl)-6-oxo-1,6-dihydropyrimidin-2-yl]acetamide
- N-(1H-indazol-6-yl)-2-[4-(morpholin-4-yl)-6-oxo-1,6-dihydropyrimidin-2-yl]acetamide
- N-(3-cyanophenyl)-2-[4-(morpholin-4-yl)-6-oxo-1,6-dihydropyrimidin-2-yl]acetamide
- N-(5-fluoropyridin-2-yl)-2-[4-(morpholin-4-yl)-6-oxo-1,6-dihydropyrimidin-2-yl]acetamide
- N-(4-fluoro-3-methylphenyl)-2-[4-(morpholin-4-yl)-6-oxo-1,6-dihydropyrimidin-2-yl]acetamide
- N-(3-chloro-4-fluorophenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-(pyridin-3-yl)acetamide
- N-(4-fluoro-2-methylphenyl)-2-[4-(morpholin-4-yl)-6-oxo-1,6-dihydropyrimidin-2-yl]acetamide
- N-(3-hydroxyphenyl)-2-[4-(morpholin-4-yl)-6-oxo-1,6-dihydropyrimidin-2-yl]acetamide
- N-(3-bromo-4-fluorophenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-(3,4,5-trifluorophenyl)acetamide
- N-[4-fluoro-3-(hydroxymethyl)phenyl]-2-[4-(morpholin-4-yl)-6-oxo-1,6-dihydropyrimidin-2-yl]acetamide
- N-(3-cyclopropylephenyl)-2-[4-(morpholin-4-yl)-6-oxo-1,6-dihydropyrimidin-2-yl]acetamide
- N-(2-hydroxyphenyl)-2-[4-(morpholin-4-yl)-6-oxo-1,6-dihydropyrimidin-2-yl]acetamide
- N-[3-(difluoromethoxy)phenyl]-2-[4-(morpholin-4-yl)-6-oxo-1,6-dihydropyrimidin-2-yl]acetamide
- N-(4-fluoro-3-methoxyphenyl)-2-[4-(morpholin-4-yl)-6-oxo-1,6-dihydropyrimidin-2-yl]propanamide
- N-(2,3-dimethylphenyl)-2-[4-(morpholin-4-yl)-6-oxo-1,6-dihydropyrimidin-2-yl]acetamide
- N-(2-fluoro-3-methylphenyl)-2-[4-(morpholin-4-yl)-6-oxo-1,6-dihydropyrimidin-2-yl]acetamide
- N-(1,3-benzoxazol-4-yl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-[3-(trifluoromethoxy)phenyl]acetamide
- 2-[4-(morpholin-4-yl)-6-oxo-1,6-dihydropyrimidin-2-yl]-N-[3-(propan-2-yloxy)phenyl]acetamide
- N-(4-fluoro-2-methoxyphenyl)-2-[4-(morpholin-4-yl)-5-oxo-1,6-dihydropyrimidin-2-yl]acetamide
- 2-methylpropan-2-yl {2-[3-({[4-(morpholin-4-yl)-6-oxo-1,6-dihydropyrimidin-2-yl]acetyl}amino)phenyl]ethyl}carbamate
- N-[4-fluoro-3-(trifluoromethyl)phenyl]-2-[4-(morpholin-4-yl)-6-oxo-1,6-dihydropyrimidin-2-yl]acetamide
- N-(3-ethynylphenyl)-2-[4-(morpholin-4-yl)-6-oxo-1,6-dihydropyrimidin-2-yl]acetamide
- N-[3-(cyclopentyloxy)phenyl]-2-[4-(morpholin-4-yl)-6-oxo-1,6-dihydropyrimidin-2-yl]acetamide
- N-(4-fluoro-2-hydroxyphenyl)-2-[4-(morpholin-4-yl)-6-oxo-1,6-dihydropyrimidin-2-yl]acetamide
- 2-[2-(2,3-dihydro-1H-indol-1-yl)-2-oxoethyl]-6-(morpholin-4-yl)pyrimidin-4(3H)-one
- N-(3-cyclopropyl-4-fluorophenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl-N-2,3,4-trifluorophenyl)acetamide
- N-[4-fluoro-3-(trifluoromethoxy)phenyl]-2-[4-(morpholin-4-yl)-6-oxo-1,6-dihydropyrimidin-2-yl]acetamide
- N-[3-(2-hydroxyethoxy)phenyl]-2-[4-(morpholin-4-yl)-6-oxo-1,6-dihydropyrimidin-2-yl]acetamide
- N-(3-iodophenyl)-2-[4-(morpholin-4-yl)-6-oxo-1,6-dihydropyrimidin-2-yl]acetamide
- methyl 2-fluoro-5-({[4-(morpholin-4-yl)-6-oxo-1,6-dihydropyrimidin-2-yl]acetyl}amino)benzoate
- N-(3-ethoxyphenyl)-2-[4-(morpholin-4-yl)-6-oxo-1,6-dihydropyrimidin-2-yl]acetamide
- N-(2,4-difluoro-3-methoxyphenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-(2,4,5-trifluorophenyl)acetamide
- N-(3,5-dichloro-4-fluorophenyl)-2-[4-(morpholin-4-yl)-6-oxo-1,6-dihydropyrimidin-2-yl]acetamide
- 2-[2-(2,3-dihydro-4H-1,4-benzoxazin-4-yl)-2-oxoethyl]-6-(morpholin-4-yl)pyrimidin-4(3)-one
- N-(4-fluoro-3-nitrophenyl)-2-[4-(morpholin-4-yl)-6-oxo-1,6-dihydropyrimidin-2-yl]acetamide
- 2-fluoro-5-({[4-(morpholin-4-yl)-6-oxo-1,6-dihydropyrimidin-2-yl]acetyl}amino)benzoic acid
- N-(5-fluoro-2-hydroxyphenyl)-2-[4-morpholin-4-yl)-6-oxo-1,6-dihydropyrimidin-2-yl]acetamide
- N-(2-bromo-4-fluorophenyl)-2-[4-(morpholin-4-yl)-6-oxo-1,6-dihydropyrimidin-2-yl]acetamide
- N-(4-fluorophenyl)-2-[1-methyl-4-(morpholin-4-yl)-6-oxo-1,6-dihydropyrimidin-2-yl]acetamide
- N-(3-chloro-4-fluorophenyl)-2-[1-methyl-4-(morpholin-4-yl)-6-oxo-1,6-dihydropyrimidin-2-yl]acetamide
- N-(3-bromophenyl)-2-[1-methyl-4-(morpholin-4-yl)-6-oxo-1,6-dihydropyrimidin-2-yl]acetamide
- 2-[1-ethyl-4-(morpholin-4-yl)-6-oxo-1,6-dihydropyrimidin-2-yl]-N-(4-fluorophenyl)acetamide
- N-(1H-indol-4-yl)-2-[4-(morpholin-4-yl)-6-oxo-1,6-dihydropyrimidin-2-yl]acetamide
- N-(4-fluorophenyl)-3-methyl-2-[4-(morpholin-4-yl)-6-oxo-1,6-dihydropyrimidin-2-yl]butanamide
- N-[4-fluoro-3-(methoxymethyl)phenyl]-2-[4-(morpholin-4-yl)-6-oxo-1,6-dihydropyrimidin-2-yl]acetamide
- N-(4-fluoro-3-iodophenyl)-2-[4-(morpholin-4-yl)-6-oxo-1,6-dihydropyrimidin-2-yl]acetamide
- 2-[4-(morpholin-4-yl)-6-oxo-1,6-dihydropyrimidin-2-yl]-N-[3-(1,1,2,2-tetrafluoroethoxy)phenyl]acetamide
- N-[3-(difluoromethyl)-4-fluorophenyl]-2-[4-(morpholin-4-yl)-6-oxo-1,6-dihydropyrimidin-2-yl]acetamide
- 2,2-difluoro-N-(4-fluorophenyl)-2-[4-(morpholin-4-yl)-6-oxo-1,6-dihydropyrimidin-2-yl]acetamide
- N-(3,4-difluorophenyl)-2-[1-methyl-4-(morpholin-4-yl)-6-oxo-1,6-dihydropyrimidin-2-yl]acetamide
- 2-[2-(2,3-dihydro-1H-indol-1-yl)-2-oxoethyl]-3-methyl-6-(morpholin-4-yl)pyrimidin-4(3H)-one
- N-(3-bromo-4-fluorophenyl)-2-[1-methyl-4-(morpholin-4-yl)-6-oxo-1,6-dihydropyrimidin-2-yl]acetamide
- N-[4-fluoro-3-(hydroxymethyl)phenyl]-2-[1-methyl-4-(morpholin-4-yl)-6-oxo-1,6-dihydropyrimidin-2-yl]acetamide
- N-(3-cyclopropylphenyl)-2-[1-methyl-4-(morpholin-4-yl)-6-oxo-1,6-dihydropyrimidin-2-yl]acetamide
- N-(4-fluoro-3-methoxyphenyl)-2-[1-methyl-4-(morpholin-4-yl)-6-oxo-1,6-dihydropyrimidin-2-yl]acetamide
- N-(1-benzofur-4-yl)-2-[4-(morpholin-4-yl)-6-oxo-1,6-dihydropyrimidin-2-yl]acetamide
- 2-[1-methyl-4-(morpholin-4-yl)-6-oxo-1,6-dihydropyrimidin-2-yl]-N-phenylacetamide
- N-(3-cyclopropyl-4-fluorophenyl)-2-[1-methyl-4-(morpholin-4-yl)-6-oxo-1,6-dihydropyrimidin-2-yl]acetamide
- N-(3-fluoro-2-hydroxyphenyl)-2-[4-(morpholin-4-yl)-6-oxo-1,6-dihydropyrimidin-2-yl]acetamide
- 2-[2-(4-fluoro-2,3-dihydro-1H-indol-1-yl)-2-oxoethyl]-6-(morpholin-4-yl)pyrimidin-4(3H)-one
- 2-[2-(4-chloro-2,3-dihydro-1H-indol-1-yl)-2-oxoethyl]-6-(morpholin-4-yl)pyrimidin-4(3H)-one
- N-(3-ethynyl-4-fluorophenyl)-2-[4-(morpholin-4-yl)-6-oxo-1,6-dihydropyrimidin-2-yl]acetamide
- 2-[2-(4-hydroxy-2,3-dihydro-1H-indol-1-yl)-2-oxoethyl]-6-(morpholin-4-yl)pyrimidin-4(3H)-one
- 2-[2-(4,6-difluoro-2,3-dihydro-1H-indol-1-yl)-2-oxoethyl]-6-(morpholin-4-yl)pyrimidin-4(3H)-one
- N-(4-fluoro-3-iodophenyl)-2-(1-methyl-4-morpholin-4-yl-6-oxo-1,6-dihydropyrimidin-2-yl)acetamide
- 2-[2-(4,5-difluoro-2,3-dihydro-1H-indol-1-yl)-2-oxoethyl]-6-(morpholin-4-yl)pyrimidin-4(3H)-one
- 2-[2-(6-fluoro-2,3-dihydro-1H-indol-1-yl)-2-oxoethyl]-6-(morpholin-4-yl)pyrimidin-4(3H)-one
- 2-[2-(2-methyl-2,3-dihydro-1H-indol-1-yl)-2-oxoethyl]-6-(morpholin-4-yl)pyrimidin-4(3H)-one
- N-3-(difluoromethyl)-4-fluorophenyl]-2-[1-methyl-4-(morpholin-4-yl)-6-oxo-1,6-dihydropyrimidin-2-yl]acetamide
- 2-[1-methyl-4-(morpholin-4-yl)-6-oxo-1,6-dihydropyrimidin-2-yl]-N-(3,4,5-trifluorophenyl)acetamide
- N-(1-methyl-1H-indol-4-yl)-2-[4-(morpholin-4-yl)-6-oxo-1,6-dihydropyrimidin-2-yl]acetamide
- 2-[2-(4-methyl-2,3-dihydro-1H-indol-1-yl)-2-oxoethyl]-6-(morpholin-4-yl)pyrimidin-4(3H)-one
- 2-[2-(3-methyl-2,3-dihydro-1H-indol-1-yl)-2-oxoethyl]-6-(morpholin-4-yl)pyrimidin-4(3H)-one
- 2-[2-(4-fluoro-2,3-dihydro-1H-indol-1-yl)-2-oxoethyl]-3-methyl-6-(morpholin-4-yl)pyrimidin-4(3H)-one
- 2-[2-(5-fluoro-2,3-dihydroindol-1-yl)-2-oxoethyl]-6-morpholin-4-yl-3H-pyrimidin-4-one
- 2-[2-(4-chloro-2,3-dihydro-1H-indol-1-yl)-2-oxoethyl]-3-methyl-6-(morpholin-4-yl)pyrimidin-4(3H)-one
- N-(1-benzothiophen-4-yl)-2-[4-(morpholin-4-yl)-6-oxo-1,6-dihydropyrimidin-2-yl]acetamide
- 2-{2-[2-(hydroxymethyl)-2,3-dihydro-1H-indol-1-yl]-2-oxoethyl}-6-(morpholin-4-yl)pyrimidin-4(3H)-one
- 2-4-(morpholin-4-yl)-6-oxo-1,6-dihydropyrimidin-2-yl]-N-{2-[2-(piperidin-1-yl)ethoxy]phenyl}acetamide
- N-[2-(2-methoxyethoxy)phenyl]-2-[4-(morpholin-4-yl)-6-oxo-1,6-dihydropyrimidin-2-yl]acetamide
- 2-[2-(4-hydroxy-2,3-dihydro-1H-indol-1-yl)-2-oxoethyl]-3-methyl-6-(morpholin-4-yl)pyrimidin-4(3H)-one
- 2-[2-(4-methoxy-2,3-dihydro-1H-indol-1-yl)-2-oxoethyl]-3-methyl-6-(morpholin-4-yl)pyrimidin-4(3H)-one
- 2-[2-(3,4-dihydroisoquinolin-2(1H)-yl)-2-oxoethyl]-6-(morpholin-4-yl)pyrimidin-4(3H)-one
- 2-[4-(morpholin-4-yl)-6-oxo-1,6-dihydropyrimidin-2-yl]-N-{2-[2-(pyrrolidin-1-yl)ethoxy]phenyl}acetamide
- 2-[4-(morpholin-4-yl)-6-oxo-1,6-dihydropyrimidin-2-yl]-N-[2-(pyridin-3-ylmethoxy)phenyl]acetamide
- 3-methyl-2-[2-(4-methyl-2,3-dihydro-1H-indol-1-yl)-2-oxoethyl]-6-(morpholin-4-yl)pyrimidin-4(3H)-one
- 2-(2-{3-[(dimethylamino)methyl]-2,3-dihydro-1H-indol-1-yl}-2-oxoethyl)-6-(morpholin-4-yl)pyrimidin-4(3H)-one
- 2-[2-(4-bromo-2,3-dihydro-1H-indol-1-yl)-2-oxoethyl]-3-methyl-6-(morpholin-4-yl)pyrimidin-4(3H)-one
- 2-{2-[(2S)-2-methyl-2,3-dihydro-1H-indol-1-yl]-2-oxoethyl}-6-(morpholin-4-yl)pyrimidin-4(3H)-one
- 2-{2-[(2R)-2-methyl-2,3-dihydro-1H-indol-1-yl]-2-oxoethyl}-6-(morpholin-4-yl)pyrimidin-4(3H)-one
- 3-methyl-2-[2-(3-methyl-2,3-dihydro-1H-indol-1-yl)-2-oxoethyl]-6-(morpholin-4-yl)pyrimidin-4(3H)-one
- 2-{2-[2-(methoxymethyl)-2,3-dihydro-1H-indol-1-yl]-2-oxoethyl}-6-(morpholin-4-yl)pyrimidin-4(3H)-one
- 2-[2-(4-ethoxy-2,3-dihydro-1H-indol-1-yl)-2-oxoethyl]-6-(morpholin-4-yl)pyrimidin-4(3H)-one
- 1-{[4-(morpholin-4-yl)-6-oxo-1,6-dihydropyrimidin-2-yl]acetyl}-2,3-dihydro-1H-indole-2-carboxamide
- 3-methyl-2-[2-(2-methyl-2,3-dihydro-1H-indol-1-yl)-2-oxoethyl]-6-(morpholin-4-yl)pyrimidin-4(3H)-one
- 2-[2-(6-fluoro-2,3-dihydro-1H-indol-1-yl)-2-oxoethyl]-3-methyl-6-(morpholin-4-yl)pyrimidin-4(3H)-one
- 2-{2-[(3S)-3-methyl-2,3-dihydro-1H-indol-1-yl]-2-oxoethyl}-6-(morpholin-4-yl)pyrimidin-4(3H)-one
- 2-{2-[(3R)-3-methyl-2,3-dihydro-1H-indol-1-yl]-2-oxoethyl}-6-(morpholin-4-yl)pyrimidin-4(3H)-one 2-[2-(5,6-difluoro-2,3-dihydro-1H-indol-1-yl)-2-oxoethyl]-3-methyl-6-(morpholin-4-yl)pyrimidin-4(3H)-one
- 2-[2-(4,5-difluoro-2,3-dihydro-1H-indol-1-yl)-2-oxoethyl]-3-methyl-6-(morpholin-4-yl)pyrimidin-4(3H)-one
- 2-[2-(1,3-dihydro-2H-isoindol-2-yl)-2-oxoethyl]-6-(morpholin-4-yl)pyrimidin-4-(3H)-one
- N-(1-benzothiophen-4-yl)-2-[1-methyl-4-(morpholin-4-yl)-6-oxo-1,6-dihydropyrimidin-2-yl]acetamide
- 2-[2-(5-chloro-3,4-dihydroquinolin-1(2H)-yl)-2-oxoethyl]-6-morpholin-4-yl)pyrimidin-4(3H)-one
- 2-{2-[4-(hydroxymethyl)-2,3-dihydro-1H-indol-1-yl]-2-oxoethyl}-6-(morpholin-4-yl)pyrimidin-4(3H)-one
- N-[4-fluoro-2-(piperidin-4-ylmethoxy)phenyl]-2-[4-(morpholin-4-yl)-6-oxo-1,6-dihydropyrimidin-2-yl]acetamide
- 2-[2-(5-chloro-2,3-dihydroindol-1-yl)-2-oxoethyl]-6-morpholin-4-yl-3H-pyrimidin-4-one
- 2-[2-(4-bromo-2,3-dihydroindol-1-yl)-2-oxoethyl]-6-morpholin-4-yl-3H-pyrimidin-4-one
- 2-(2-{(3S)-3-[(dimethylamino)methyl]-2,3-dihydro-1H-indol-1-yl}-2-oxoethyl)-6-(morpholin-4-yl)pyrimidin-4(3H)-one
- 2-(2-{(3R)-3-[(dimethylamino)methyl]-2,3-dihydro-1H-indol-1-yl}-2-oxoethyl)-6-(morpholin-4-yl)pyrimidin-4(3H)-one
- N-[4-fluoro-2-(2-methoxyethoxy)phenyl]-2-[4-(morpholin-4-yl)-6-oxo-1,6-dihydropyrimidin-2-yl]acetamide
- N-(1H-benzimidazol-4-yl)-2-[4-(morpholin-4-yl)-6-oxo-1,6-diydroyrimidin-2-yl]acetamide
- methyl 2-hydroxy-3-({[4-(morpholin-4-yl)-6-oxo-1,6-dihydropyrimidin-2-yl]acetyl}amino)benzoate
- 2-[2-(4-methoxy-2,3-dihydroindol-1-yl)-2-oxoethyl]-6-morpholin-4-yl-3H-pyrimidin-4-one
- N-(3-bromo-2-hydroxyphenyl)-2-[4-(morpholin-4-yl)-6-oxo-1,6-dihydropyrimidin-2-yl]acetamide
- N-(3,4-dihydro-2H-1,4-benzoxazin-8-yl)-2-[4-(morpholin-4-yl)-6-oxo-1,G-dihydropyrimidin-2-yl]acetamide
- methyl 5-fluoro-2-({[4-(morpholin-4-yl)-6-oxo-1,6-dihydropyrimidin-2-yl]acetyl}amino)benzoate
- 2-(2-{3-[(diethylamino)methyl]-2,3-dihydro-1H-indol-1-yl}-2-oxoethyl)-6-(morpholin-4-yl)pyrimidin-4(3H)-one
- 2-[2-(2,3-dihydro-1H-pyrrolo[3,2-b]pyridin-1-yl)-2-oxoethyl]-6-(morpholin-4-yl)pyrimidin-4(3H)-one
- 2-[2-(5,6-difluoro-2,3-dihydroindol-1-yl)-2-oxoethyl]-6-morpholin-4-yl-3H-pyrimidin-4-one
- 2-[4-(morpholin-4-yl)-6-oxo-1,6-dihydropyrimidin-2-yl]-N-[1,2,3,4-tetrahydroquinolin-8-yl)acetamide
- 2-[2-(8-chloro-2,3-dihydro-4H-1,4-benzoxazin-4-yl)-2-oxoethyl]-6-(morpholin-4-yl)pyrimidin-4(3H)-one
- N-(2-hydroxy-3-methylphenyl)-2-[4-(morpholin-4-yl)-6-oxo-1,6-dihydropyrimidin-2-yl]acetamide
- N-(2-hydroxy-3-nitrophenyl)-2-[4-(morpholin-4-yl)-6-oxo-1,6-dihydropyrimidin-2-yl]acetamide
- N-(3-cyano-2-hydroxyphenyl)-2-[4-(morpholin-4-yl)-6-oxo-1,6-dihydropyrimidin-2-yl]acetamide
- N-[2-hydroxy-3-(trifluoromethyl)phenyl]-2-4-(morpholin-4-yl)-6-oxo-1,6-dihydropyrimidin-2-yl]acetamide
- 2-[2-(3,3-dimethyl-2,3-dihydro-1H-indol-1-yl)-2-oxoethyl]-6-(morpholin-4-yl)pyrimidin-4(3H)-one
and also the addition salts with inorganic and organic acids or with inorganic and organic bases, of said products of formula (I).

4. Process for prepaying the products of formula (I) as define in any one of Claims 1 to 3, according to scheme 1A as defined hereinafter: 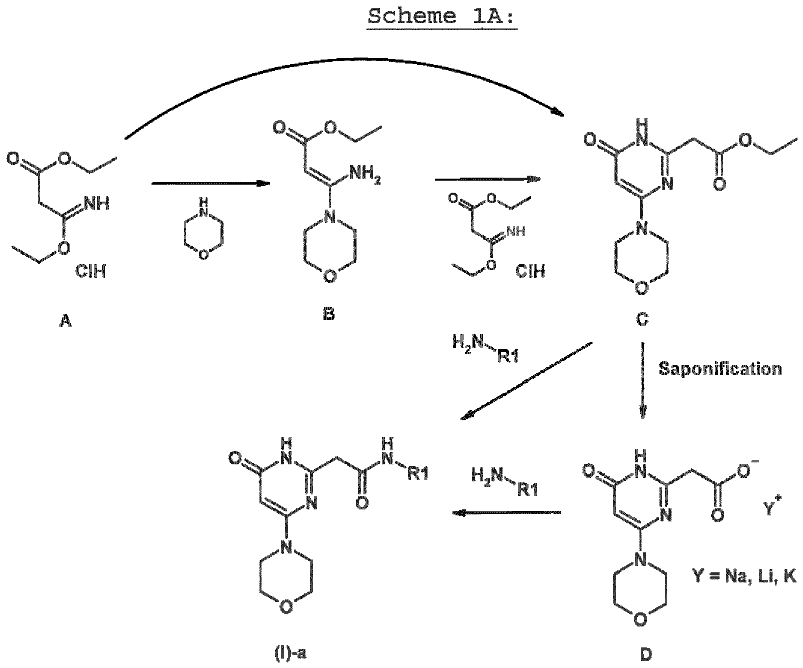 in which the substituent R1 has the meanings indicated in either one of Claims 1 and 2.

5. Process for preparing the products of formula (I) as defined in any one of Claims 1 to 3, according to scheme 1B as defined hereinafter: 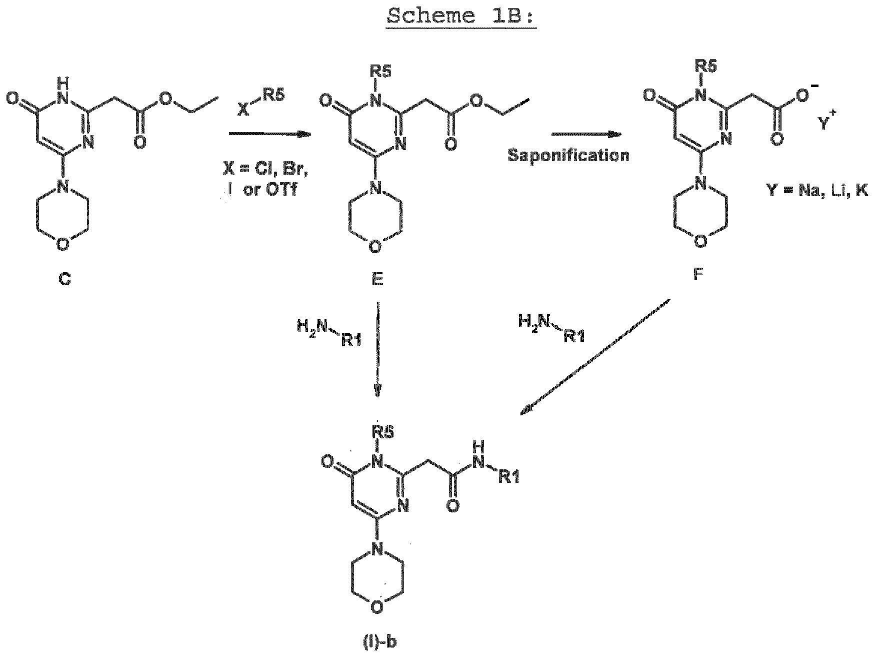 in which the substituents R1 and R5 have the meanings indicated in either one of Claims 1 and 2.

6. Process for preparing the products of formula (I) as define in any one of Claims 1 to 3 according to scheme 1C as defined hereinafter: 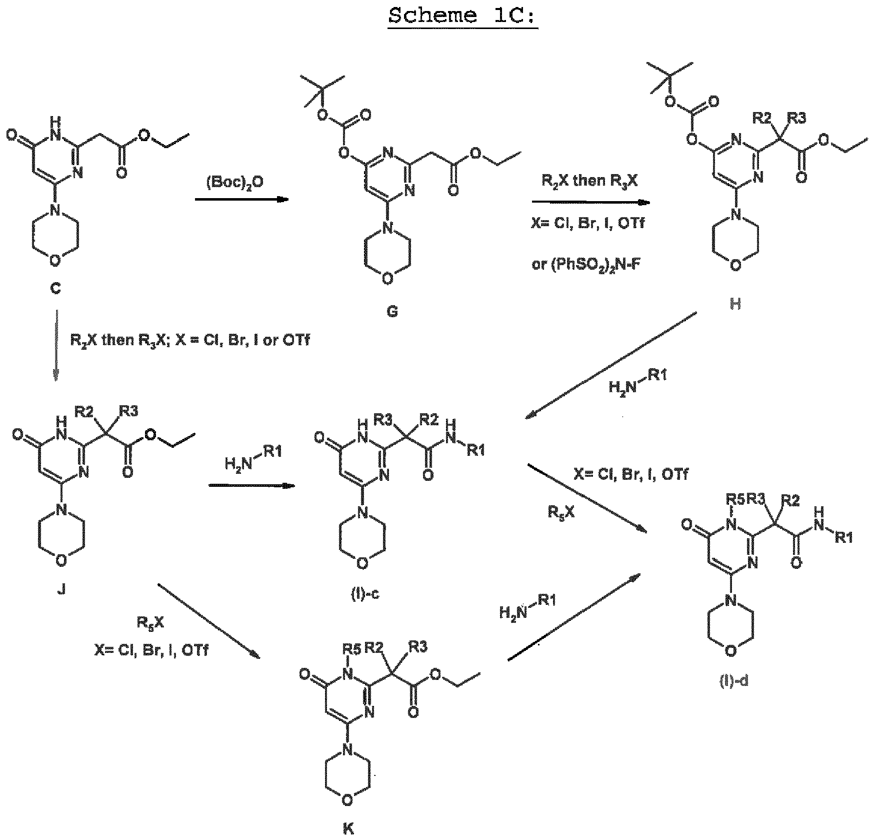 in which the substituents R1, R2, R3 and R5 have the meanings indicated in either one of Claims 1 and 2.

7. Products of formula (I) as defined in any one of Claims 1 to 3, and also the pharmaceutically acceptable addition salts with inorganic an organic acids or with inorganic and organic bases, of said products of formula (I), for their use as medicaments.

8. Products of formula (I) as defined in Claim 3, and also the pharmaceutically acceptable addition salts with inorganic organic acids or it inorganic and organic bases, of said products of formula (I), for their use as medicaments.

9. Pharmaceutical compositions containing, as active ingredient, at least one of the products of formula (I) as defined in any one of Claims 1 to 3, or a pharmaceutically acceptable salt of this product, and a pharmaceutically acceptable carrier.

10. Products of formula (I) as defined in any one of Claims 1 to 3, for their use in the treatment of cancers.

11. Products of formula (1) as defined in any one of Claims 1 to 3, for their use in the treatment of solid or liquid tumours.

12. Products of formula (1) as defined in any one of Claims 1 to 3, for their use in the treatment of cancers résistant to cytotoxic agents.

13. Products of formula (1) as defined in any one of Claims 1 to 3, for their use in the treatment of primary tumours and/or of metastases, in particular in gastric, hepatic, renal, ovarian, colon, prostate, endometrial and lung (NSCLC and SCLC) cancers, glioblastomas, thyroid, bladder and breast cancers, in melanoma, in lymphoid or yeloid hematopoietic tumours, in sarcomas, in brain, larynx and lymphatic system cancers, bone and pancreatic cancers, and in hamartomas.

14. Products of formula (I) as defined in any one of Claims 1 to 3, for their use in cancer chemotherapy.

15. Products of formula (I) as defined in any one of Claims 1 to 3, for their use in cancer chemotherapy, alone or in combination.

16. Products of formula (I) as defined in any one of Claims 1 to 3, as inhibitors of AKT (PKB) phosphorylation.

17. Synthesis intermediates of formulae C, D, E and F as defined in Claim 4 and recalled below: 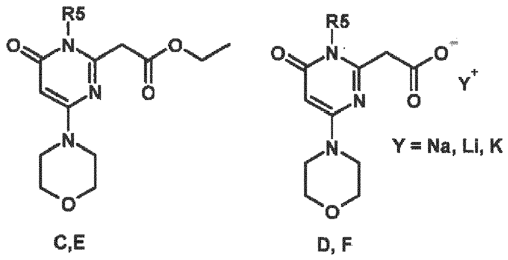 in which R5 has the definition indicated in either one of Claims 1 and 2.
